# EUROPEAN PATENT SPECIFICATION

(11) **EP 1 377 609 B1**
(45) Date of publication and mention of the grant of the patent: **24.06.2015**
(21) Application number: 02706685.1
(22) Date of filing: 13.03.2002
(51) Int. Cl.: C07K 14/705, G01N 33/68, G01N 33/58, G01N 33/543

(54) **MHC MOLECULE CONSTRUCTS AND THEIR USES FOR DIAGNOSIS AND THERAPY**
MHC-MOLEKÜL-KONSTRUKTEN UND IHREN VERWENDUNGEN FÜR DIAGNOSE UND THERAPIE
NOUVELLES CONSTRUCTIONS DE MOLECULES MHC, METHODES D'UTILISATION DE CES CONSTRUCTIONS A DES FINS DE DIAGNOSTIC ET DE THERAPIE ET UTILISATIONS DE MOLECULES MHC

(30) Priority: 14.03.2001 DK 200100435; 14.03.2001 DK 200100436; 14.03.2001 DK 200100441; 14.03.2001 US 275470 P; 14.03.2001 US 275448 P; 14.03.2001 US 275447 P
(43) Date of publication of application: 07.01.2004
(60) Divisional application: 10178826.3
(73) Proprietor: Dako Denmark A/S, 2600 Glostrup (DK); Immudex ApS, 2100 Copenhagen (DK)
(72) Inventor: WINTHER, Lars, DK-2765 Smoerum (DK); PETERSEN, Lars, Oestergaard, DK-2400 Copenhagen NV (DK); BUUS, Soeren, DK-2700 Broenshoej (DK); SCHOELLER, Joergen, DK-2800 Kgs. Lyngby (DK); RUUD, Erik, N-1177 Oslo (NO); AAMELLEM, Oeystein, N-1358 Jar, Norway (NO)
(74) Representative: Høiberg A/S
(86) International application number: PCT/DK2002/000169
(87) International publication number: WO 2002/072631

(56) References cited:
- EP-A- 0 352 761
- EP-A- 0 636 696
- WO-A-00/23053
- WO-A-96/26962
- WO-A-97/44667
- WO-A-98/05965
- WO-A-99/14236
- US-A- 5 312 744

## Description

The present invention relates to the field of poly-valent compounds carrying ligands capable of ligating to counter receptors on relevant target cells. The compounds possess a number of advantageous features, rendering them very suitable for a wide range of applications. In particular, the present invention relates to novel MHC molecule constructs comprising one or more MHC molecules. The affinity and avidity of the MHC molecules of the constructs are surprisingly high. The possibility of presenting to target cells a plurality of MHC molecules makes the MHC molecule constructs of the invention an extremely powerful tool e.g. in the field of diagnosis. Comprised by the present invention is the sample-mounted use of MHC molecules, MHC molecule multimers, and MHC molecule constructs. The invention relates further in general to the field of therapy, including therapeutic methods and therapeutic compositions. The therapeutic compositions may be applied both in vivo and ex vivo.

### BACKGROUND OF THE INVENTION

Specific ligation of ligands to counter receptors on target cells control many important responses in the human organism. The physiological implications of ligand binding to receptors for peptide hormones e.g. insulin receptors (IR) have been known for decades. Peptide hormones may be secreted from one organ and exert their effect locally or be distributed with blood to distantly located cell types or tissues. Our present understanding of the biological functions related to a variety of receptors has emerged from detailed studies of ligand binding, signal transduction and other measurements of physiological responses.

Recently, more complicated ligand-receptor interactions that relate to regulation of specific immune responses have been described. For example, it is now well known that biochemical interactions between peptide epitope specific membrane molecules encoded by the Major Histocompatibility Complex (MHC, in humans HLA) and T-cell receptors (TCR) are required to elicit specific immune responses. This type of ligand-receptor interaction is somewhat more complicated, in comparison to more "conventional" ligand-receptor models like insulin and IR. Activation of T-cells requires simultaneous signalling through other receptors as well, and may acquire additional binding energy from ligation between other membrane molecules, e.g. adhesion proteins, to ensure a close physical contact between the involved cells.

The specific (adaptive) immune system is a complex network of cells and organs that work together to defend the body specifically against attacks by "foreign" or "non-self" invaders. In addition, the immune system also comprise a more unspecific defence line (so-called innate immune system) consisting of cell types and tissues, which by physical and chemical means provide resistance toward foreign invaders.

The cellular network of the specific immune system is one of the body's main defences against disease. It works against disease, including cancer, in a variety of ways.

Thus, a detailed knowledge of the molecular mechanisms underlying activation of the human immune system may not only have a profound effect and importance for control of diseases in humans. A detailed understanding will also provide diagnostic tools for control of infections and other diseases in other vertebrate species of economical importance. A detailed understanding will further provide the possibility of manipulating the immune system (both in vivo and ex vivo) so as to control infections and other diseases.

The genes located in the human MHC locus (HLA locus) encode a set of highly polymorphic membrane proteins that sample peptides in intracellular compartments and present such peptide epitopes to antigen specific receptors (TCR) on T-cells. The extensive genetic polymorphism of this locus is the background for the unique genetic finger print of the immune system in individuals and defines the repertoire of antigenic peptide epitopes which the human population is capable of recognising and respond to. Thus, HLA molecules are key players in determining penetrance and spreading of human diseases. MHC molecules of other higher vertebrate species exert identical biological functions as those described for HLA in man.

To elicit a full and adequate response, the immune system acquire, in addition to the signal transduction resulting from the peptide specific interaction of MHC and TCR molecules (signal 1), stimuli by ligation of other so-called co-stimulatory molecules as well (signal 2). Both groups of immune activating ligands bind to their specific counter receptors with low affinity. For example, it has been measured that monovalent MHC-TCR interaction has an affinity constant of 10µM with a half-life corresponding to few minutes. Interaction between CD28 on T-cells and the co-stimulatory molecules on antigen presenting cells has an affinity of same level. The low intrinsic affinity has been a significant factor that limited quantitative analysis of specific interactions required for specific immune responses. A recently established technology to produce soluble tetramer MHC complexes, first reported by Davis and coworkers in 1996 (ref. 1, Science 274, 94-96 (1996)) has provided a tool for analysis and detection of specific interaction between peptide epitope specific MHC and TCR molecules on T-cells in certain applications. Tetramers result from biotinylating MHC Class I molecules folded in the presence of a specific peptide determinant prior to cross-linking with streptavidin. Until now, several in vitro and in vivo assays (limited dilution assay, proliferation assay, cytokine or cytotoxic activity, ELISPOT and flow cytometry) have been established for monitoring of cytotoxic T-cell responses toward cancers, infectious diseases and auto-antigens. Though the most useful of these approaches to predict clinical efficacy of e.g. cancer vaccines remains to be defined, there is high expectation in the field about the potential of tetramers. In a more recently developed alternative approach, the group of Schneck used immunoglobulin as a molecular scaffold to produce a divalent peptide-MHC-IgG complex (ref. 2). Numerous reports have shown that both types of oligovalent MHC complexes bind to antigen specific T-cells. Such approaches have proven to be valuable for scientific, practical or clinical uses.

However, the previous reported findings still leaves room for improvement. Many MHC molecules are labile compounds not very easy obtainable, they need to be folded correctly to be functional, and they have low intrinsic binding affinity to TCRs making it difficult to obtain sufficient interaction with the TCR. These obstacles with the combined effects thereof have resulted in limited utilisation of MHC multimer technology and uses in many laboratories. Previous reports have only demonstrated binding of multi-valent complexes consisting of 2-4 peptide epitope specific MHC Class I or II molecules. The binding of such multi-valent complexes allows only detection of high affinity T-cell clones i.e. excludes a variety of T-cell clones that respond specifically towards e.g. sub-dominant peptide/MHC epitopes.

Many techniques, like e.g. flow cytometry, ELISA or electrophoresis, use homogenised tissue, cells or cell fragments. In many applications, however, it is a major advantage being able to identify and study potential targets in their native environment and surroundings, i.e. in samples wherein the morphology is preserved. However, this is very demanding on the means used in the determination. The means must be able to bind to the target of interest when the morphology is preserved.

The benefits of early diagnosis are extremely high. The earlier the diagnosis of a serious disease or condition is established, the better the possibilities of curing or at least control. However, this requires sensitive, specific, and, not least, very safe means. The latter is e.g. important in order to avoid both a high number of false positive and a high number of false negative results.

An widely used method for diagnostic procedures, prognostic procedures as well as patient monitoration and stratification is histochemistry, especially immunohisto-chemistry (IHC) (or immunocytochemistry as it is sometimes called). IHC is a potent tool for demonstrating a variety of bio-macromolecules or related biochemical events in situ, combined with the study of tissue morphology and has been and continuously is extensively applied in biomedical and clinical diagnostic studies especially for tumour diagnostics.

Thus, histochemistry is for example used to differentiate between tumours with similar histological patterns, to define the origin of metastasising tumours, in prognostication of tumours (i.e. detecting early neoplatic processes), to identify tumour recurrence and to measure the effectiveness of various therapies.

In research applications, IHC has an obvious role in determining the cytological and histological distribution of biological structures as a complimentary and/or confirmative analysis to results on DNA and RNA level.

The present invention further provides powerful tools in the field of therapy. Diagnosis and therapy are two fields closely connected. Diagnosis is also about determining or selecting the appropriate and optimal therapy. As medicaments become more specific targeting effectively specific variants of diseases (so-called designer drugs), the interrelation between diagnosis and therapy becomes more important and inseparable. Furthermore, the increasing understanding of the immune system raises the possibility of designing medicaments as well as the demands therefore.

A major goal of anti-tumour or anti-virus immunotherapy is to generate antigen-specific, long-lived protective T-cells that enable killing of target cells. Once the antigen-specific T-cells have been isolated they can be cultured in the presence of co-stimulatory molecules. Magnetic beads, such as Dynabeads^{®}, can be coated with antibodies developed against various co-stimulatory molecules, and used as artificial APCs to support the long-term growth ex vivo of various subsets of T-cells. This ex vivo priming and expansion of human effector T-cell populations has a great potential for use in immunotherapy applications against various types of cancer and infectious diseases.

Admittedly, the success of immunotherapies that substantially interfere with the function of cells has been limited, but it is continuously believed that this would be a way of treating a broad range of serious diseases, including cancer and auto-immune diseases.

EP0352761 discloses antigen constructs resulting from linkage of major histocompatibility complex Class I antigens to specific carrier molecules. The specific carrier molecules bind selectively to target cells and are preferably monoclonal antibodies.

WO9626962 discloses that T cells can be specifically labeled according to their antigen receptor by binding of a multimeric binding complex. The complex is prepared with major histocompatibility complex protein subunits having a homogeneous population of peptides bound in the antigen presentation site. The multimeric MHC-antigen complex forms a stable structure with T cells, thereby allowing for the labeling, identification and separation of specific T cells.

WO9914236 discloses that two-domain MHC polypeptides are useful for manipulation of antigen-specific T-cells. These polypeptides include MHC class II-based molecules that comprise covalently linked beta 1 and alpha 1 domains, and MHC class I-based molecules that comprise covalently linked alpha 1 and alpha 2 domains. These polypeptides may also include covalently linked antigenic determinants, toxic moieties, and/or detectable labels. The disclosed polypeptides can be used to target antigen-specific T-cells.

EP0636696 discloses a process for producing a major histocompatibility angiten class II protein; a material having the MHC class II immobilized thereon, prepared by linking the MHC class II, the subunits alpha and/or beta of the MHC class II, or a part thereof to a material such as bead, fiber or hollow yarn through covalent linkage; and a superantigen-removing module prepared from the material.

WO9744667 relates to methods for using MHC/peptide complexes. More specifically, a method for isolating, purifying and quantifying of tumoral specific T lymphocytes, from peritoneal exudate and tumor-infiltrating lymphocytes is disclosed.

WO0023053 is directed to artificial antigen presenting cells and methods of making artificial antigen presenting cells. Such artificial antigen presenting cells may be used in certain methods of isolating and expanding T cell populations as well as modulating T cell responses.

WO9805965 concerns a method for identifying one or more features of eukaryotic cells, in particular of mammals, characterised in that a substantially monocellular layer of a population of eukaryotic cells is covalently immobilised on a functionalised solid support and in that the layer of immobilised cells is subjected to a reaction for determining one cytological and/or cytogenetic feature of said cells and in that the result is evaluated. The invention also concerns a solid support functionalised by a spacer having terminal function capable of reacting with functional groups present in the cell wall.

US5312744 relates to a method for immobilizing and preserving an immunologically reactive antigen substance such as antigenic cells or non-cell bound antigens for use in solid-phase immunoassay. The antigen substance is adsorbed and crosslinked on a support and contacted with a protecting solution and containing sugar and antiseptic substance.

In spite of the previous reported findings, there is still plenty of room for new approaches. The present invention offers such new approach both in the field of diagnosis and the field of therapy.

### SUMMARY OF THE INVENTION

The present invention relates to the following aspects:
- A MHC molecule construct in soluble form in a solubilizing medium or immobilized onto a solid or semi-solid support, said MHC molecule construct comprising a soluble dextran carrier molecule attached to at least 5 MHC molecules, wherein said MHC molecules are attached to the dextran carrier molecule via more than one binding entity, wherein each binding entity is attached to from 2 to 4 MHC molecules.
- A MHC molecule construct in soluble form in a solubilizing medium or immobilized onto a solid or semi-solid support, said MHC molecule construct comprising a soluble dextran carrier molecule attached to at least 5 MHC molecules, wherein said MHC molecules are directly attached to the dextran carrier molecule and said MHC molecule construct comprises one or more labels.

- A composition comprising a MHC molecule construct as defined herein above.
- Use of the composition defined herein above for the manufacture of a medicament for up-regulating, down-regulating or modulating an immune response in an animal, for inducing anergy of a cell or for adoptive cellular immunotherapy.
- A method of obtaining MHC recognising cells comprising bringing into contact a MHC molecule construct as defined herein above and a sample suspected of comprising MHC recognising cells under conditions whereby the MHC recognising cells bind to the MHC molecule construct, and isolating the bound MHC molecule construct and MHC recognising cells.
- A method for producing a composition as defined above comprising providing a MHC molecule construct as defined above, solubilising or dispersing the MHC molecule construct in a medium suitable for therapeutic substances, and optionally adding other excipients.
- A method for detecting or monitoring the presence of MHC recognising cells in a sample comprising the steps of
   (a) contacting a sample suspected of comprising MHC recognising cells with a MHC molecule construct as defined above, and
   (b) determining any binding of the MHC molecule construct, which binding indicates the presence of MHC recognising cells.
- A method for establishing a prognosis of a disease, determining the status of a disease or diagnosing a disease involving MHC recognising cells comprising the steps of
   (a) contacting a sample suspected of comprising MHC recognising cells with a MHC molecule construct as defined above, and
   (b) determining any binding of the MHC molecule construct, thereby
      establishing a prognosis of a disease, determining the status of a disease
      or diagnosing a disease involving MHC recognising cells.
- A method for determining the effectiveness of a medicament against a disease involving MHC recognising cells comprising the steps of
   (a) contacting a sample from a subject receiving treatment with a medicament with a MHC molecule construct as defined above,
      and
   (b) determining any binding of the MHC molecule construct, thereby determining the effectiveness of the medicament.
- A method for manufacturing the composition as defined above comprising the steps of
   (i) providing at least 5 MHC molecules;
   (ii) providing a dextran carrier molecule;
   (iii) providing more than one binding entity;
   (iv) attaching said at least 5 MHC molecules to said dextran carrier via said more than one binding entity; wherein the attachment results in that each binding entity is attached to from 2 to 4 MHC molecules;
   (v) optionally attaching one or more biologically active molecules to said dextran carrier either directly or via said more than one binding entity;
   (vi) optionally attaching one or more labelling compounds to said dextran carrier molecule, or to one or more of said more than one binding entity or to one or more of said at least 5 MHC molecules;
      and thereby manufacturing the composition as defined above.

To exploit the real potentials of multi-valent MHC compounds, novel MHC molecule constructs were generated, which have been shown to possess numerous advantages and thus the expectations to these novel constructs are high. The constructs of the present invention can potentially express very high numbers of MHC molecules (poly-ligands) being well-defined TCR specific ligands.

It is shown herein (cf. the Examples) that these MHC molecules constructs bind with much higher avidity to antigen specific T-cells than the known prior art tetramers under comparable conditions. The unexpected and surprisingly increased binding energy, derived through a higher valence of the compounds, allows specific and efficient staining of even subtle T-cell populations in e.g. peripheral blood cells e.g. using flow cytometry (cf. the Examples).

Thus, in a first aspect, the present invention relates to novel MHC molecule constructs comprising a carrier molecule having attached thereto one or more MHC molecules, said MHC molecules being attached to the carrier molecule either directly or via one or more binding entities.

The MHC molecule constructs of the invention may be provided in non-soluble or soluble form, depending on the intended application.

The MHC molecule constructs of the present invention have numerous uses and are a valuable and powerful tool e.g. in the fields of diagnosis, prognosis, monitoring, stratification, and determining the status of diseases or conditions as well as in therapy. Thus, the MHC molecule constructs may be applied in the various methods involving the detection of MHC recognising cells.

Furthermore, the present invention relates to compositions comprising the MHC molecule constructs in a solubilising medium. The present invention also relates to compositions comprising the MHC molecule constructs immobilised onto a solid or semi-solid support.

The MHC molecule constructs are very suitable as detection systems. Thus, the present invention relates to the use of the MHC molecule constructs as defined herein as detection systems.

In another aspect, the present invention relates to the general use of MHC molecules and multimers of such MHC molecules in various sample-mounted methods. These methods include diagnostic methods, prognostic methods, methods for determining the progress and status of a disease or condition, and methods for the stratification of a patient.

The MHC molecule constructs of the present invention are also of value in testing the expected efficacy of medicaments against or for the treatment of various diseases. Thus, the present invention relates to methods of testing the effect of medicaments or treatments, the methods comprising detecting the binding of the MHC molecule constructs to MHC recognising cells and establishing the effectiveness of the medicament or the treatment in question based on the specificity of the MHC recognising cells.

As mentioned above, the present invention also relates generally to the field of therapy. Thus, the present invention relates per se to the MHC molecule construct as defined herein for use as medicaments, and to the MHC molecule constructs for use in in vivo and ex vivo therapy.

The present invention relates to therapeutic compositions comprising as active ingredients the MHC molecule constructs as defined herein.

An important aspect of the present invention is therapeutic compositions comprising as active ingredients effective amounts of MHC recognising cells obtained using the MHC molecule constructs as defined herein to isolate relevant MHC recognising cells, and expanding such cells to a clinically relevant number.

The present invention further relates to methods for treating, preventing or alleviating diseases, methods for inducing anergy of cells, as well as to methods for up-regulating, down-regulating, modulating, stimulating, inhibiting, restoring, enhancing and/or otherwise manipulating immune responses.

The invention also relates to methods for obtaining MHC recognising cells using the MHC molecule constructs as described herein.

Also encompassed by the present invention are methods for preparing the therapeutic compositions of the invention.

### BRIEF DESCRIPTION OF THE FIGURES

Below is given a brief description of the Figures.
Figures 1-24, and 49-57 illustrate some embodiments of the MHC molecule constructs of the present invention. Figures 49 and 50 illustrate in particular some embodiments of the MHC molecule constructs are provided in immobilised form, althouth the embodiments shown in Figure 1-24 can also be provided in immobilised form. It should be recognised that the embodiments are examples of suitable MHC molecule constructs and should in no way be limiting on the scope of the invention.
Figure 25-33 illustrate some of applications of the MHC molecule constructs including specificity, effect of size, time of incubation, dependency of concentration, time course for dissociation of binding, inhibition of binding by antibodies, effect of HLA (human MHC)-to-dextran ratio at ligation on the specific binding, as well as ability to stain T-cell subpopulations, cf. the Examples. Thus, the usefulness of the MHC molecule constructs in practical clinical applications is demonstrated.
Figures 34-37 show interesting HLA (human MHC) Class I and Class II binding motifs, interesting HIV/SIV protein, interesting tumour-associated antigens recognised by T-lymphocytes, and HIV CTL epitopes.
Figures 38-45 illustrate the usefulness of the MHC molecule constructs in practical applications, cf. the Examples.
Figures 46-48 illustrate in a schematic way how the MHC molecules of the constructs of the present invention mimic the real life situation, where a receptor on a cell binds to a MHC molecule presented on e.g. an infected cell.

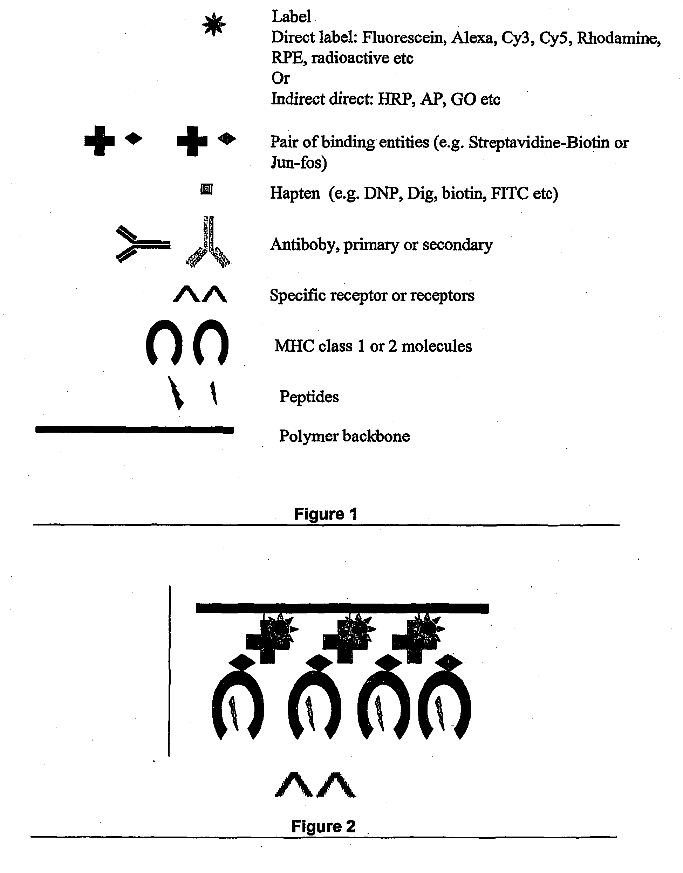

Figure 1. Summary of symbols used in the Figures 2-24. Direct or indirect labels, pair of binding entities, haptens, antibodies, specific receptors, MHC molecules, peptides and polymer.
Figure 2. MHC molecule construct comprising multiple MHC molecules, each filled with a peptide, attached via labelled binding entities to a carrier molecule.

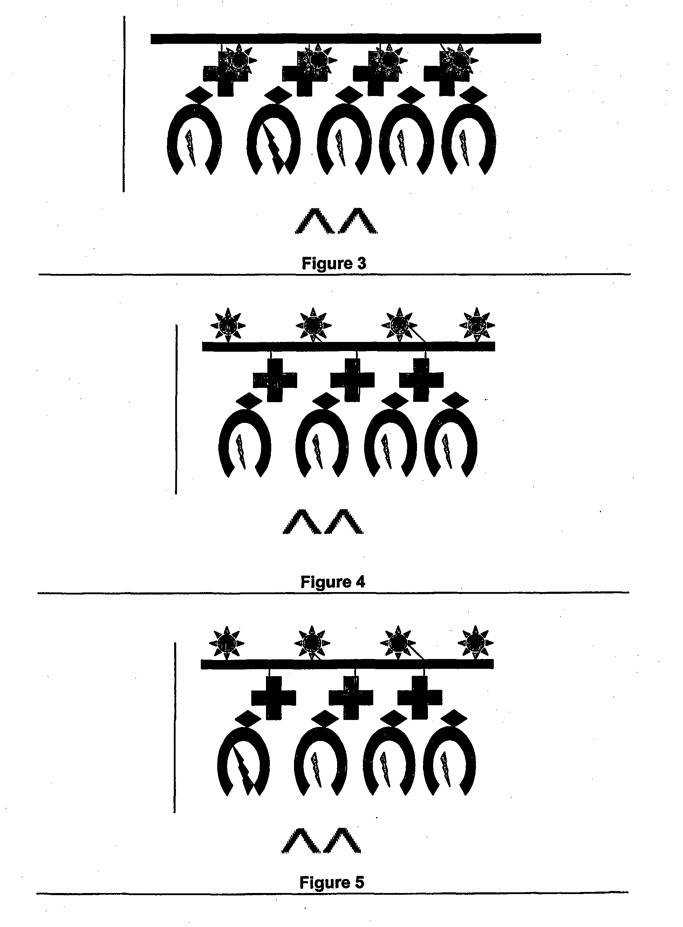

Figure 3. MHC molecule construct comprising different MHC molecules, filled with different peptides, attached via binding entities to a carrier molecule.
Figure 4. MHC molecule construct comprising multiple MHC molecules, each filled with peptide, attached via the binding entities to a labelled carrier molecule.
Figure 5. MHC molecule construct comprising different MHC molecules, filled with different peptides, attached via binding entities to a labelled carrier molecule.

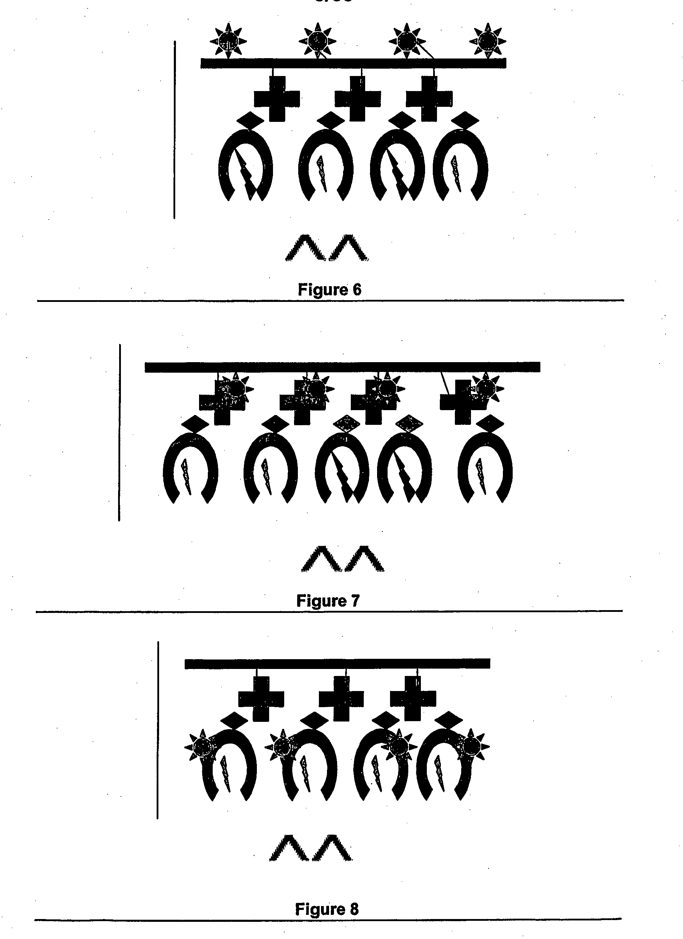

Figure 6. MHC molecule construct comprising multiple MHC molecules, filled with different peptides, attached via binding entity to a labelled carrier molecule.
Figure 7. MHC molecule construct comprising different MHC molecules, filled with different peptides, and attached via different binding entities to a carrier molecule. The binding entities are labelled.
Figure 8. MHC molecule construct comprising multiple MHC molecules, each filled with peptide, attached via binding entities to a carrier molecule. The MHC molecules are labelled.

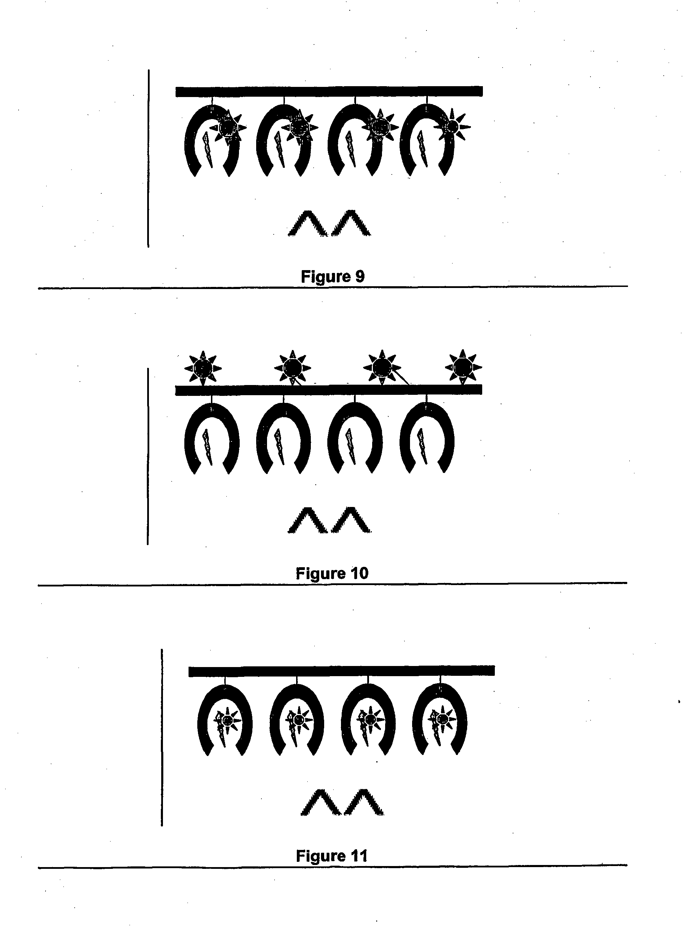

Figure 9. MHC molecule construct comprising multiple MHC molecules, each filled with peptide, attached directly to a carrier molecule. The MHC molecules are labelled.
Figure 10. MHC molecule construct comprising multiple MHC molecules, each filled with peptide, attached directly to a carrier molecule. The carrier molecule is labelled.
Figure 11. MHC molecule construct comprising multiple MHC molecules, each filled with peptide, attached directly to a carrier molecule. The peptides are labelled.

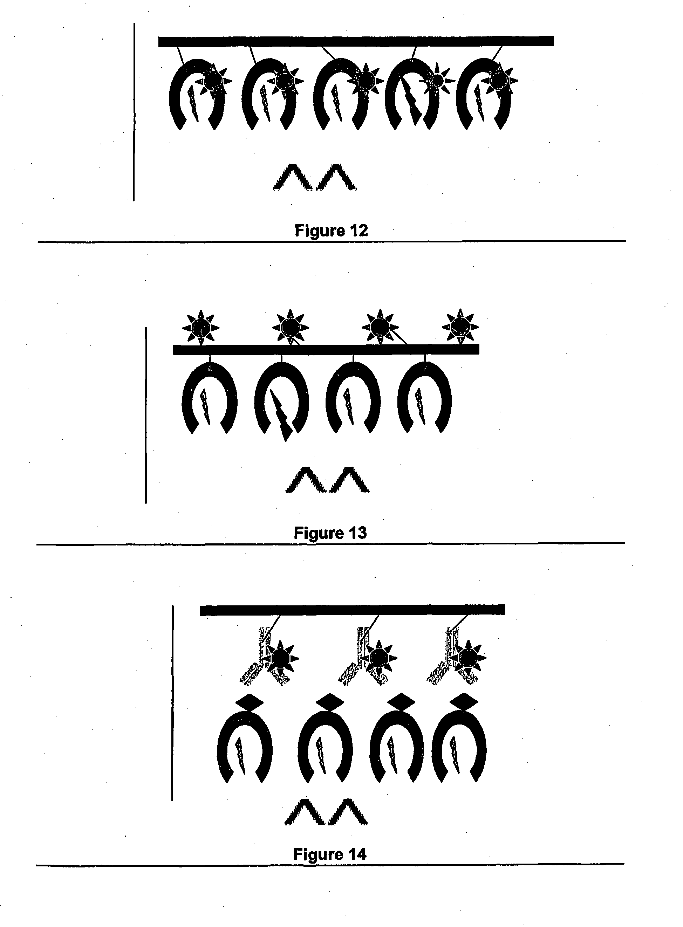

Figure 12. MHC molecule construct comprising MHC molecules, filled with different peptides, attached directly to a carrier molecule. The MHC molecules are labelled.
Figure 13. MHC molecule construct comprising different MHC molecules, filled with different peptides, attached directly to a carrier molecule. The carrier molecule is labelled.
Figure 14. MHC molecule construct comprising multiple MHC molecules, each filled with peptide, attached via binding entities being specific antibodies and/or antigens to a carrier molecule. The antibodies/antigens are labelled.

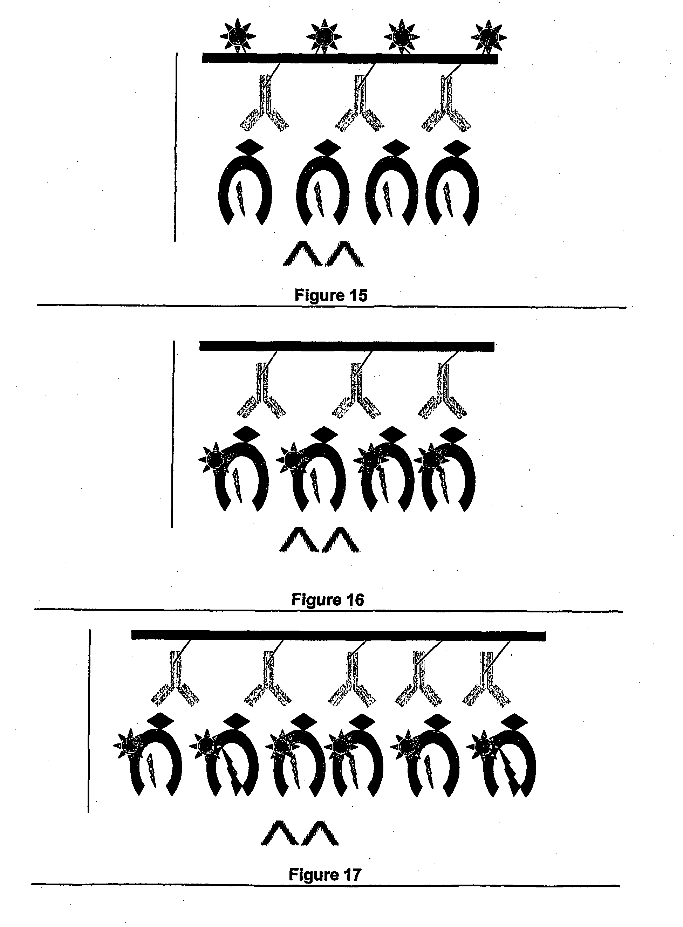

Figure 15. MHC molecule construct comprising multiple MHC molecules, each filled with peptide, attached via binding entities being specific antibodies and/or antigens to a carrier molecule. The carrier molecule is labelled.
Figure 16. MHC molecule construct comprising multiple MHC molecules, each filled with peptide, attached via binding entities being specific antibodies and/or antigens to a carrier molecule. The MHC molecules are labelled.
Figure 17. MHC molecule construct comprising different MHC molecules, filled with different peptides, attached via binding entities being specific antibodies and/or antigens to a carrier molecule. The MHC molecules are labelled.

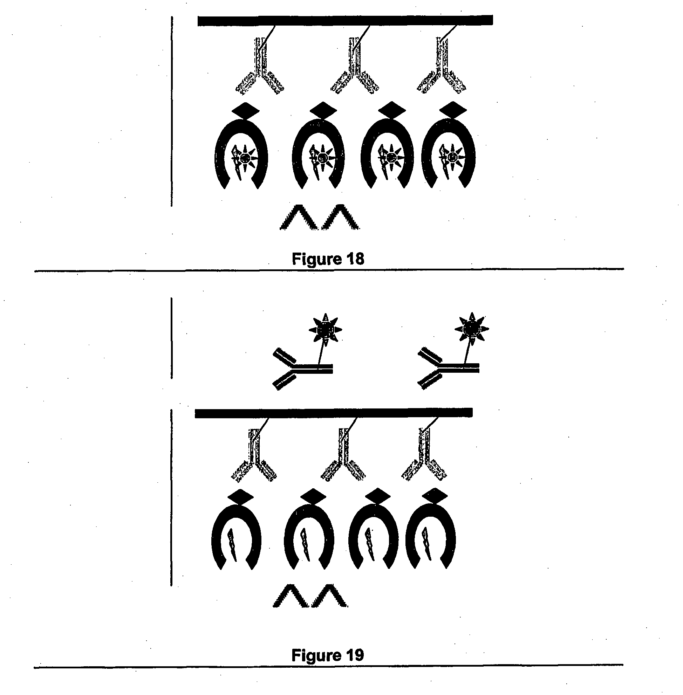

Figure 18. MHC molecule construct comprising multiple MHC molecules, each filled with labelled peptide, attached via binding entities being specific antibodies and/or antigens to a carrier molecule.
Figure 19. MHC molecule construct comprising multiple MHC molecules, each filled with peptide, attached via binding entities being specific antibodies and/or antigens to a carrier molecule. Labelled secondary antibodies/antigens are binding to the antibodies/antigens of the binding entity.

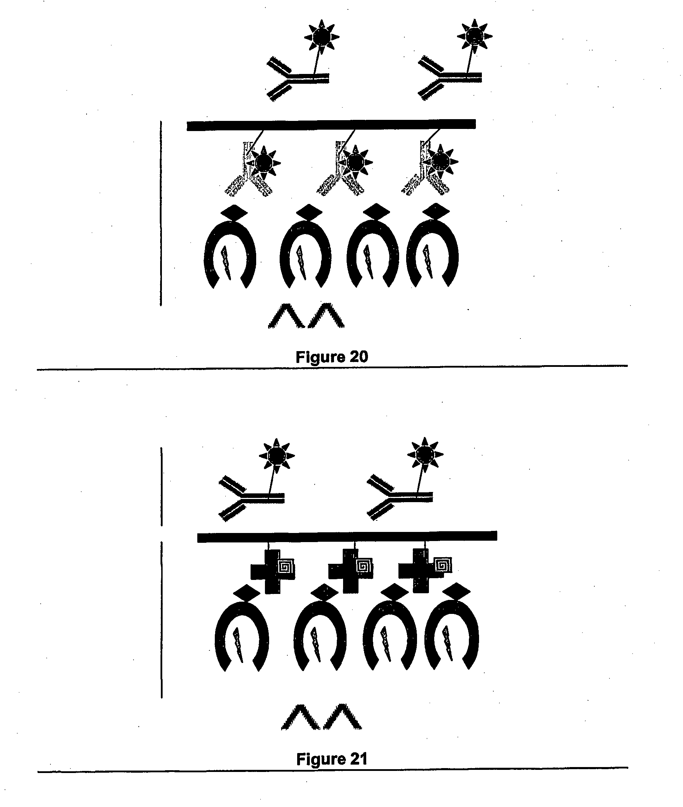

Figure 20. MHC molecule construct comprising multiple MHC molecules, each filled with peptide, attached via binding entities being specific and labelled antibodies and/or antigens to a carrier molecule. Labelled secondary antibodies/antigens are binding to the antibodies/antigens of the binding entity. The label may be the same or different.
Figure 21. MHC molecule construct comprising multiple MHC molecules, each filled with peptide, attached via hapten labelled binding entities, to a carrier molecule. Labelled antibodies are binding to the haptens on the binding entities.

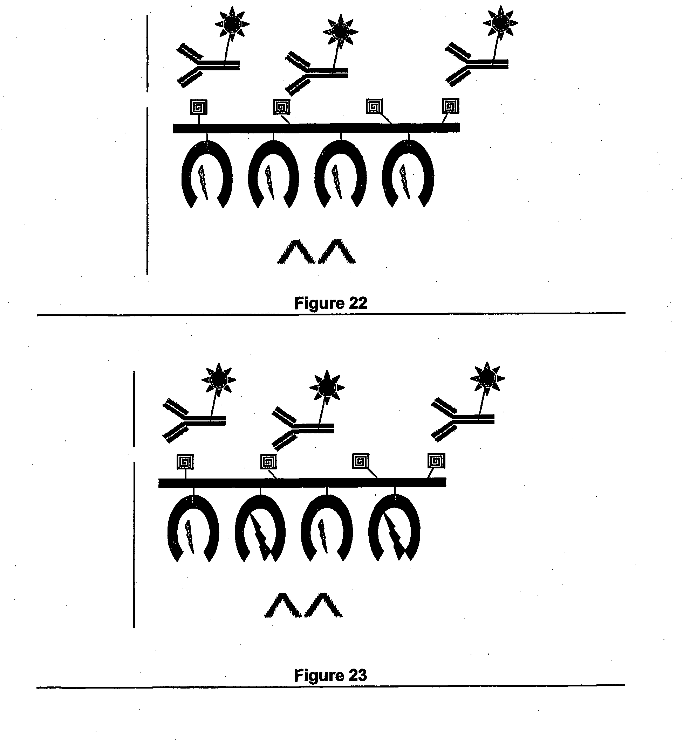

Figure 22. MHC molecule construct comprising multiple MHC molecules, each filled with peptide, attached directly to a hapten labelled carrier molecule. Labelled secondary antibodies are binding to the haptens on the carrier molecule.
Figure 23. MHC molecule construct comprising multiple MHC molecules, filled with different peptides, attached directly to a hapten labelled carrier molecule. Labelled secondary antibodies are binding to the haptens on the carrier molecule.

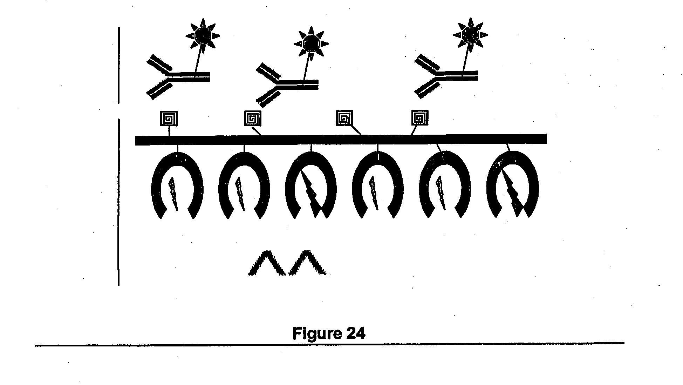

Figure 24. MHC molecule construct comprising multiple and different MHC molecules, each filled with peptide, attached directly to a hapten labelled carrier molecule. Labelled secondary antibodies are binding to the haptens of the binding entity.

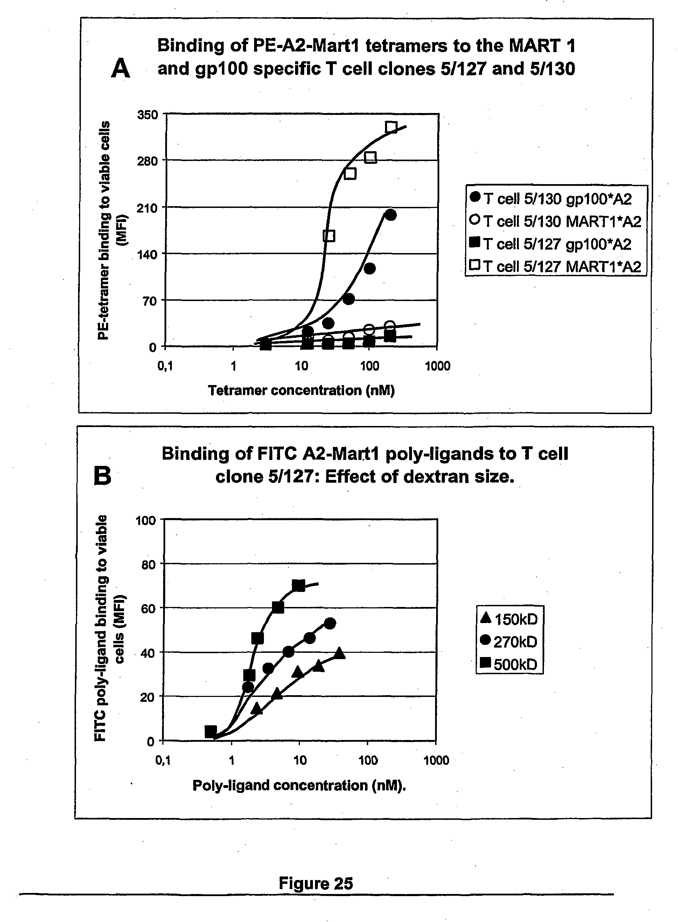

Figure 25. Flow cytometric analysis of the binding of MART-1 specific poly-ligand MHC molecule constructs of the invention and MART-1 specific MHC molecule tetramers to specific T-cell clones. Data are presented as staining intensity of viable T-cells as a function of increasing concentrations of poly-ligand MHC molecule constructs and MHC molecule tetramers, respectively. Figure 25A shows the specificity in the binding of PE-A2-MART-1 tetramers to the MART-1 and gp100 specific T-cell clones 5/127 and 5/130. Figure 25B shows the effect of carrier molecule size on the binding of FITC A2-MART-1 poly-ligand MHC molecule constructs to T-cell clone 5/127.

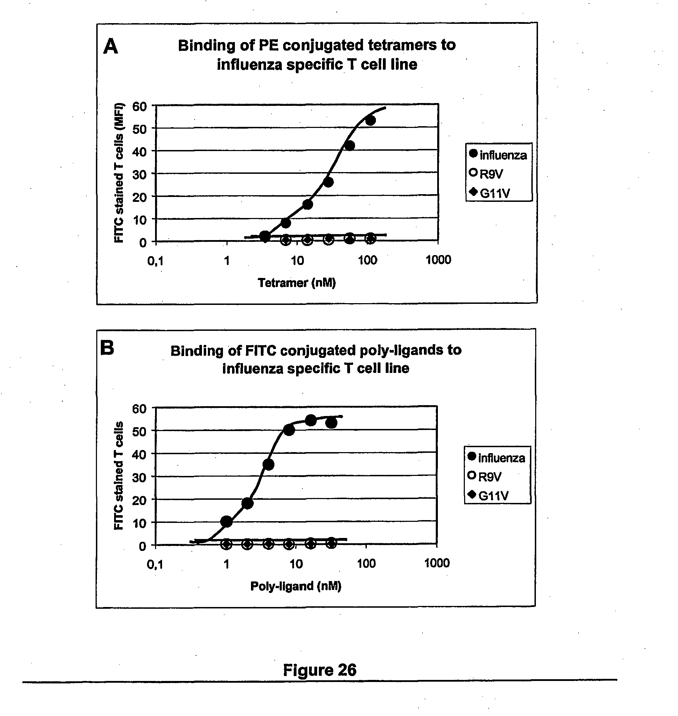

Figure 26. Flow cytometric analysis of the binding of poly-ligand MHC molecule constructs and MHC molecule tetramers to an influenza specific T-cell line illustrating specific and dose dependent staining. Figure 26A: Binding of PE-labelled tetramers to an influenza specific T-cell line. Figure 26B: Binding of FITC-labelled poly-ligand MHC molecule constructs to an influenza specific T-cell line.

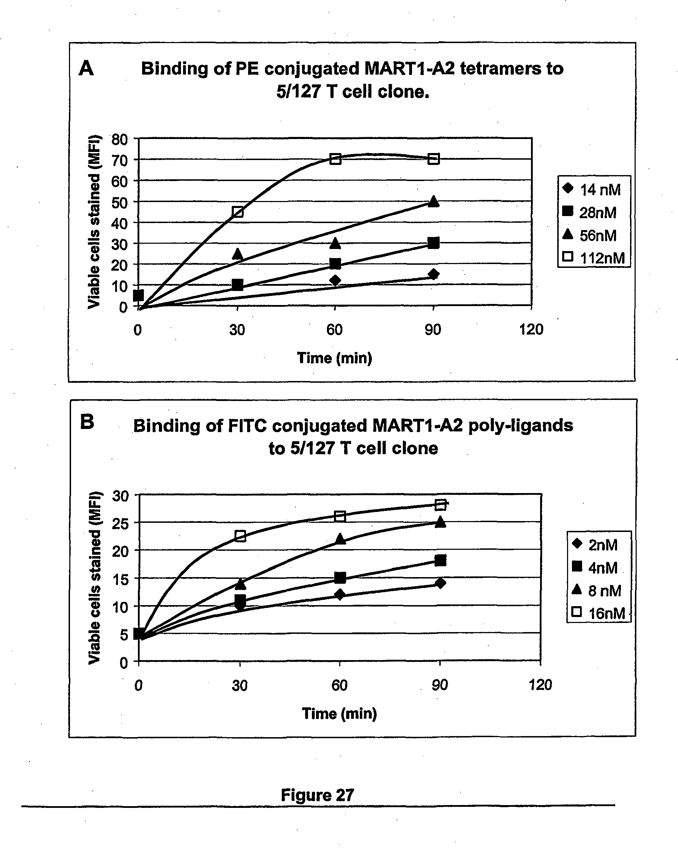

Figure 27. Comparative flow cytometric analysis of time and concentration dependent staining by poly-ligand MHC molecule constructs of the invention and MHC molecule tetramers to the 5/127 T-cell clone. Figure 27A: Time dependent binding of PE-labelled MART1-A2 tetramers at tetramer concentrations from 14 to 112 nM. Figure 27B: Time dependent binding of FITC-labelled MART1-A2 poly-ligand MHC molecule constructs at concentrations from 2 to 16 nM.

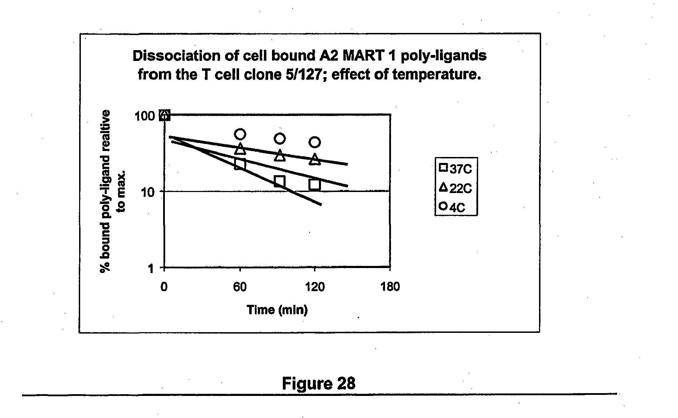

Figure 28. Flow cytometric analysis of the temperature effect on the dissociation of cell bound A2-MART-1 poly-ligand MHC molecule constructs of the invention from the T-cell clone 5/127. The effect was studied at 4°C, 22°C and 37°C, respectively.

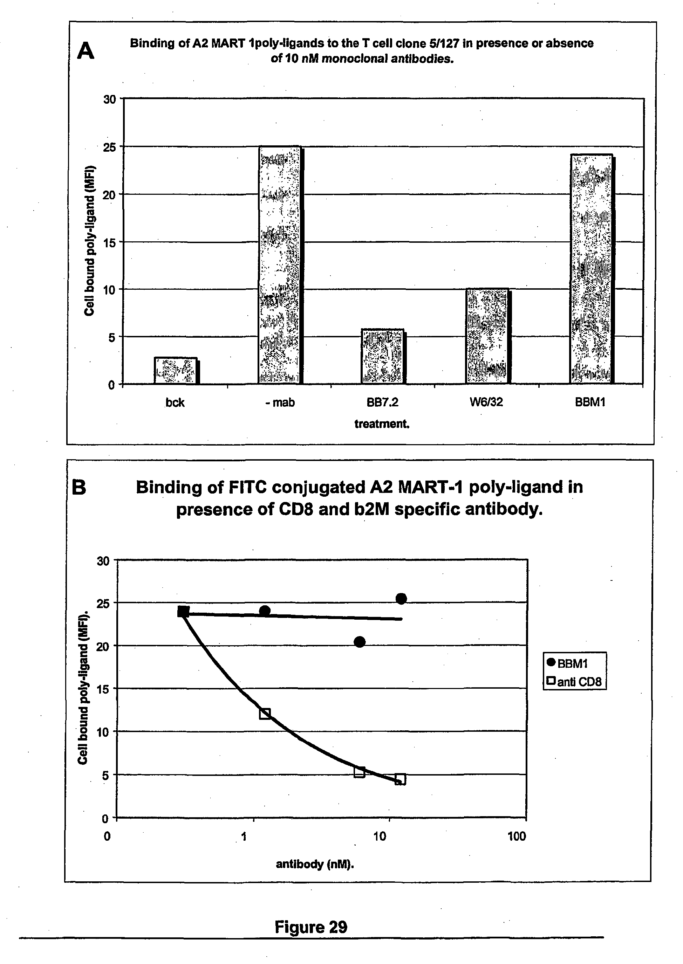

Figure 29. Flow cytometric analysis of the inhibition of A2-MART-1 poly-ligand MHC molecule construct binding to the T-cell clone 5/127 by monoclonal antibodies directed against the proximity of the peptide-binding site. Figure 29A: The effect of the following antibodies were tested: BB7.2, HLA A0201 specific; W6/32, HLA A, B, C pan specific; BBM1, β₂m specific. All antibodies were used at a concentration of 10 nM. bck: Background value, - mab: without antibody treatment. Figure 29B: Test of increasing concentrations of the two monoclonal antibodies: BBM1 (β₂m specific) antibody and mouse anti human T-cell CD8 antibody.

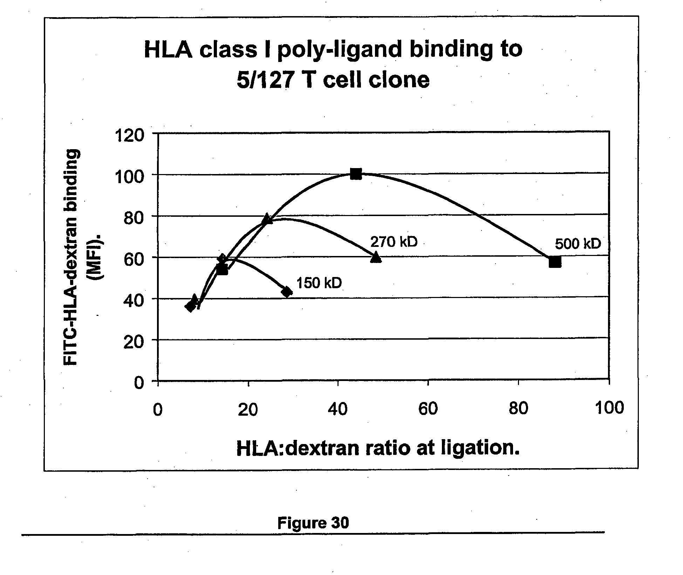

Figure 30. Flow cytometric analysis of HLA Class I poly-ligand construct binding to the to the T-cell clone 5/127 showing the effect of variations in the HLA to dextran ratio in the ligation process. Dextrans of 150, 270 and 500 kDa were tested.

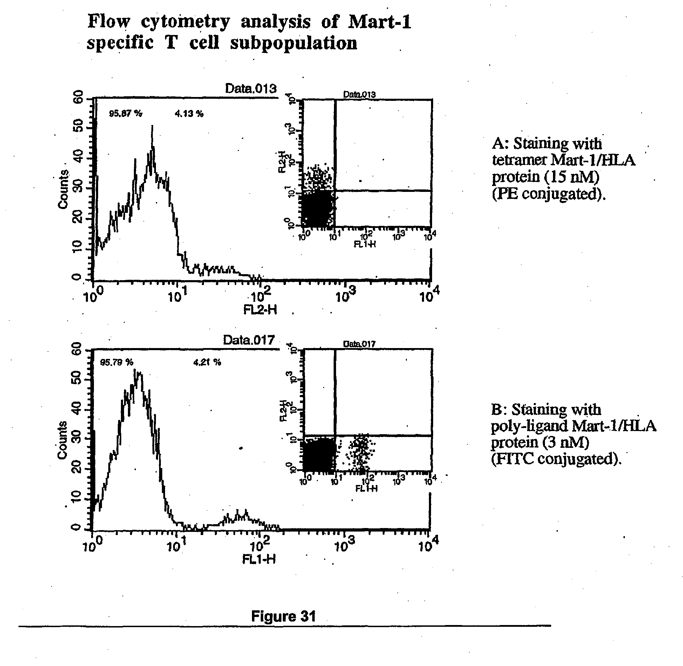

Figure 31. Flow cytometric diagram showing the analysis of an A2-MART-1 peptide specific T-cell subpopulation of 5% stained with: (A) PE-labelled tetramer MART-1/HLA molecule at a concentration of 15 nM. (B) FITC-labelled poly-ligand MART-1/HLA molecule construct at a concentration of 3 nM. The T-cell population was obtained by mixing the two T-cell clones 5/127 (MART-1 specific) and the 5/130 (gp100 specific).

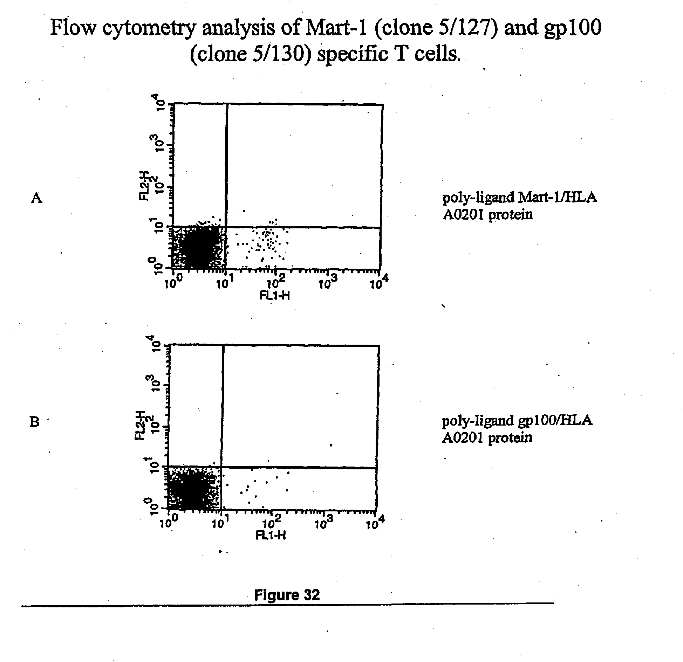

Figure 32. Flow cytometric diagram showing poly-ligand MHC molecule construct staining of (A) MART-1 (clone 5/127) and (B) gp100 (clone 5/130) specific T-cells. In each case only 1% of the T-cell population was positive mimicking the level of positive T-cells typically found in patient samples. The T-cell population was obtained by mixing the two T-cell clones 5/127 and 5/130 at the relevant ratios.

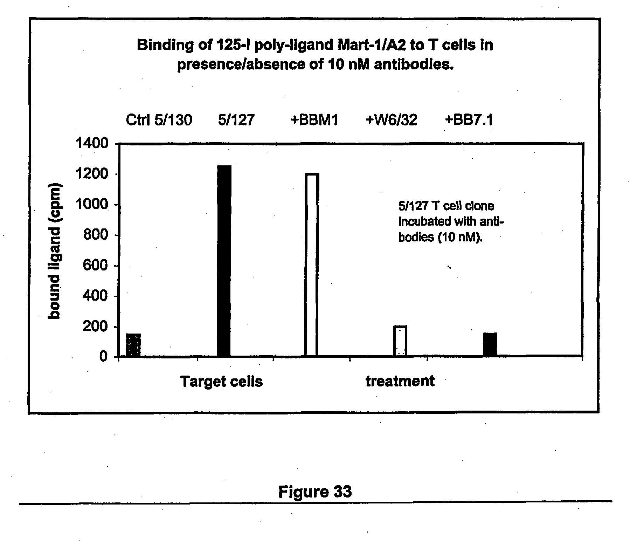

Figure 33. Binding assay showing the effect of the presence/absence of 10 nM monoclonal antibodies on the binding of iodinated (¹²⁵I-labelled β₂m) poly-ligand MHC molecule construct displaying A2-MART-1 to T-cell clone 5/127 (MART-1 specific). The following antibodies were used: BBM1 (human β₂m specific); W6/32 HLA A, B, C panspecific); and BB7.2 (HLA-A0201 specific). Ctrl 5/130: Negative control cell line 5/130.

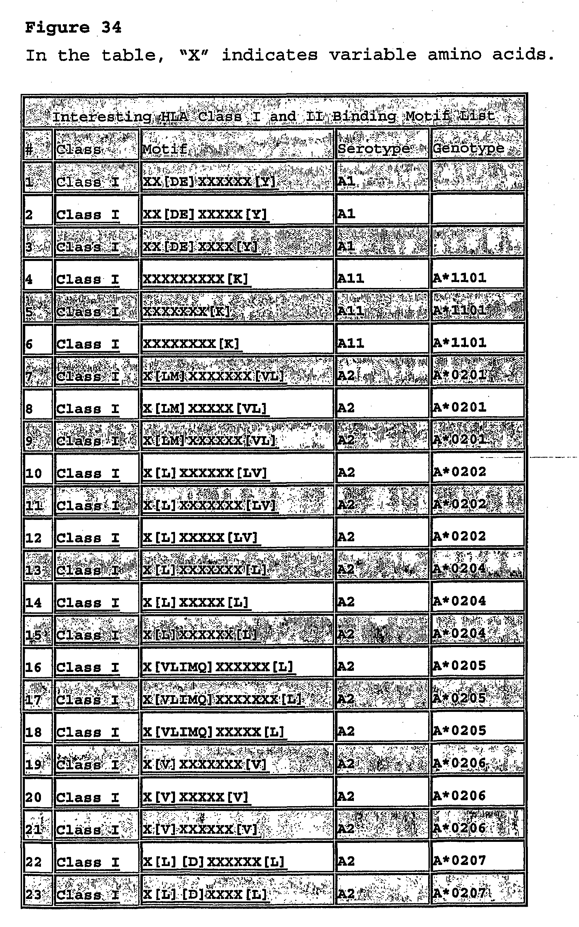

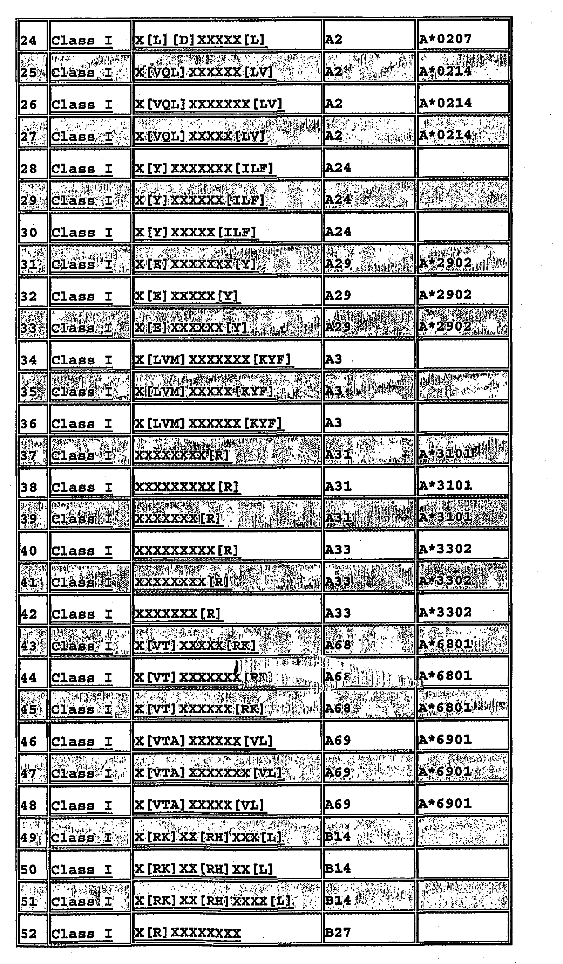

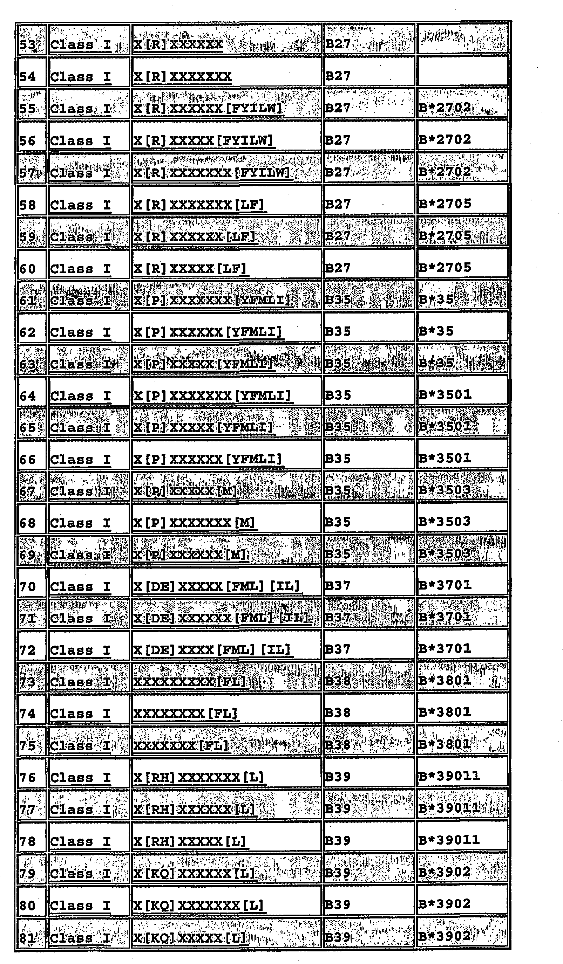

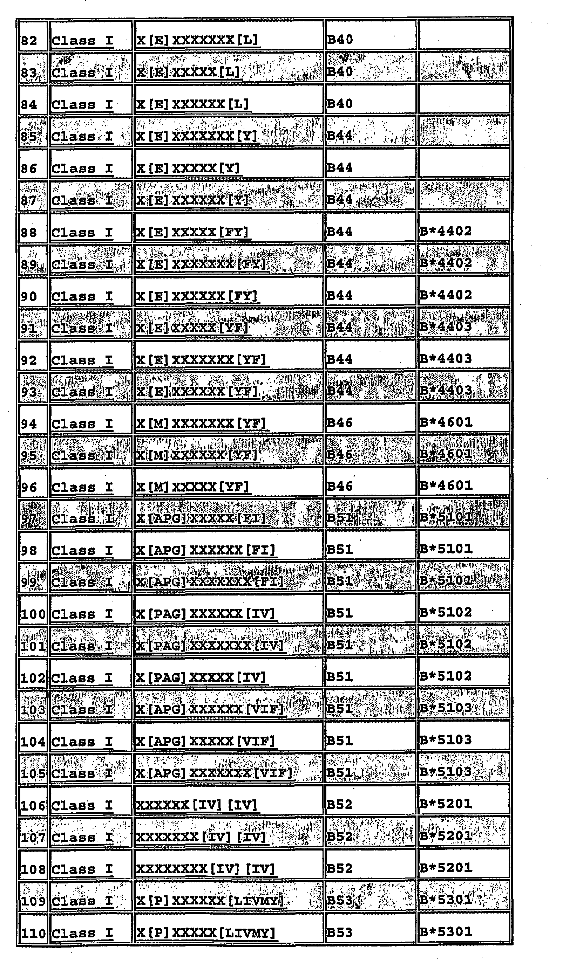

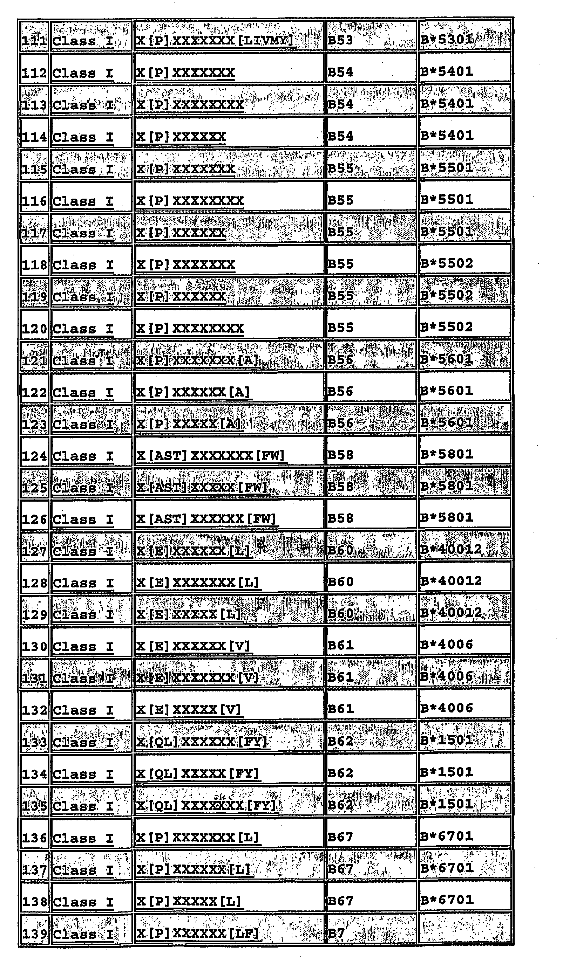

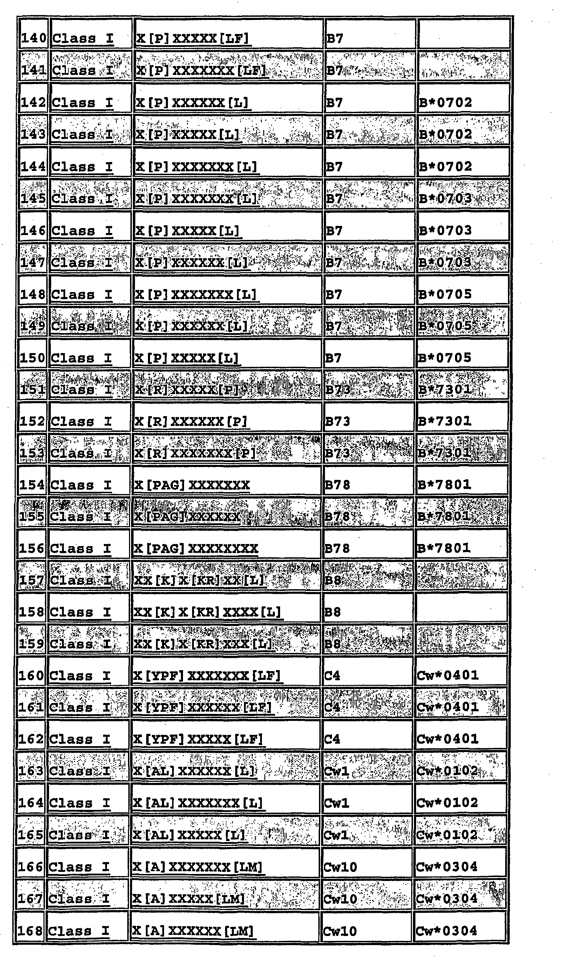

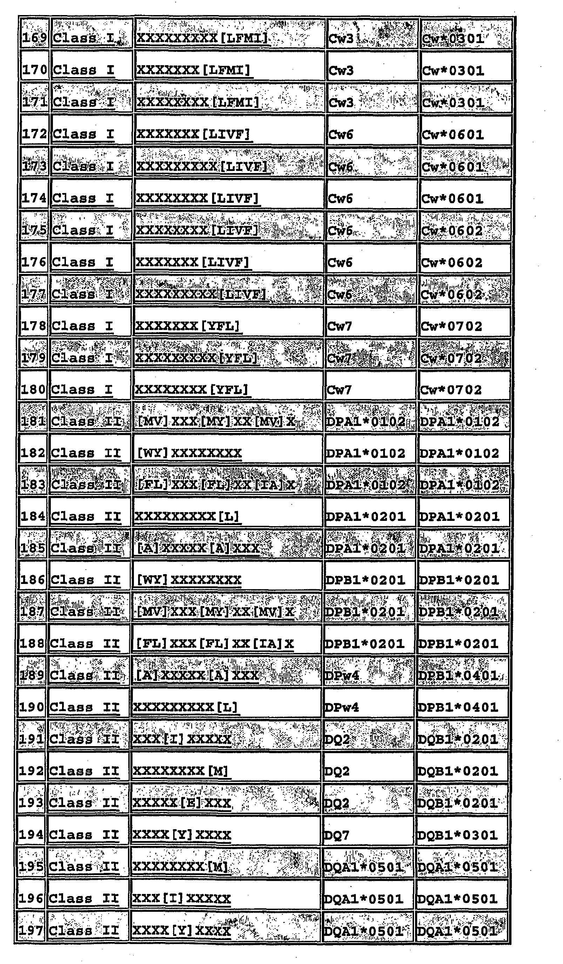

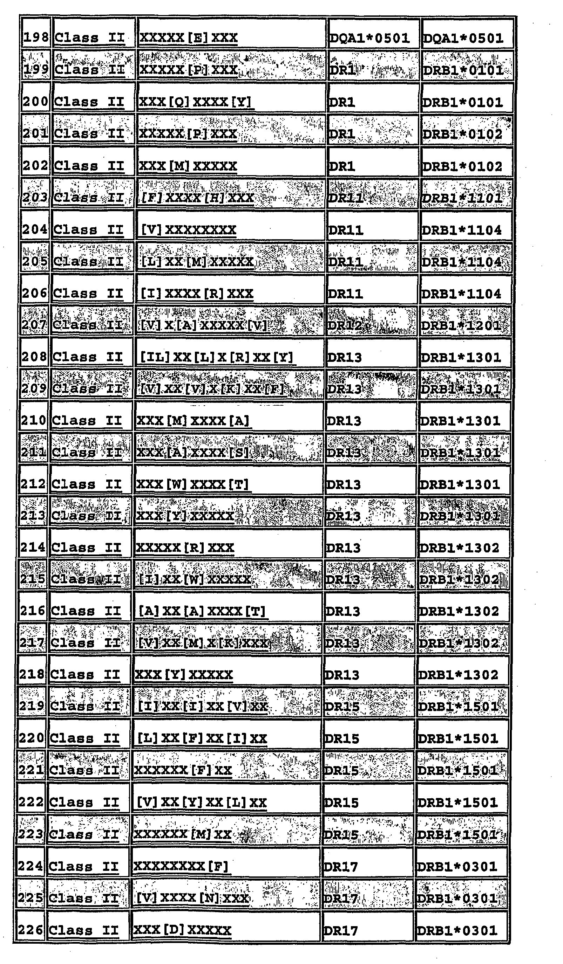

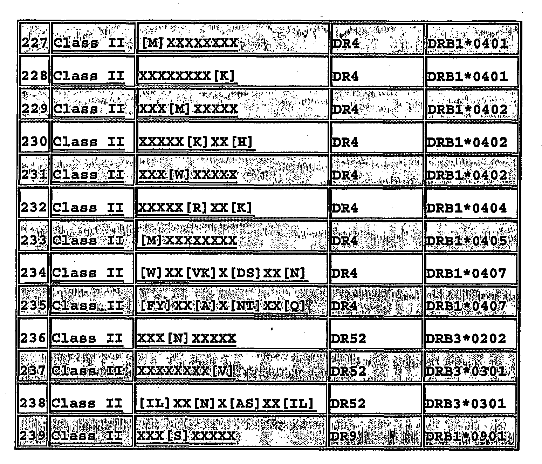

Figure 34 indicates interesting HLA Class I and Class II molecule binding motifs.

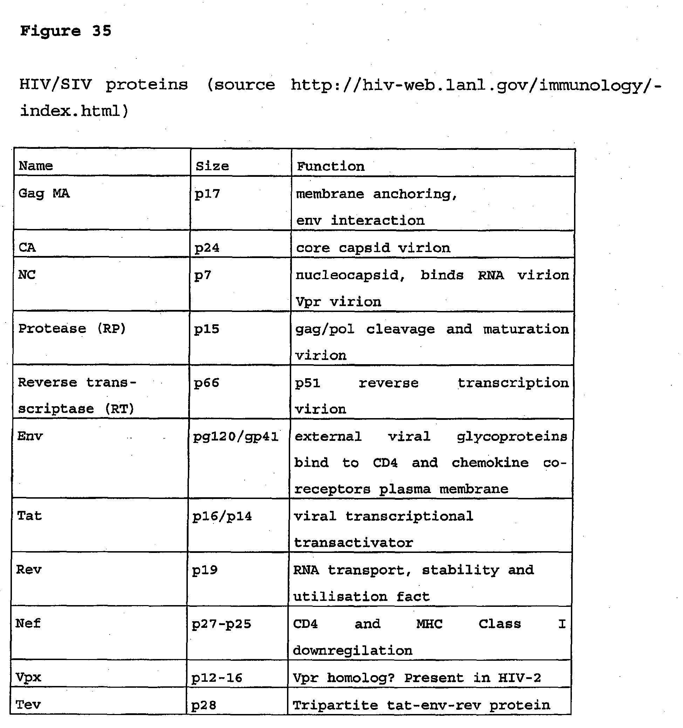

Figure 35 indicates interesting HIV/SIV proteins.

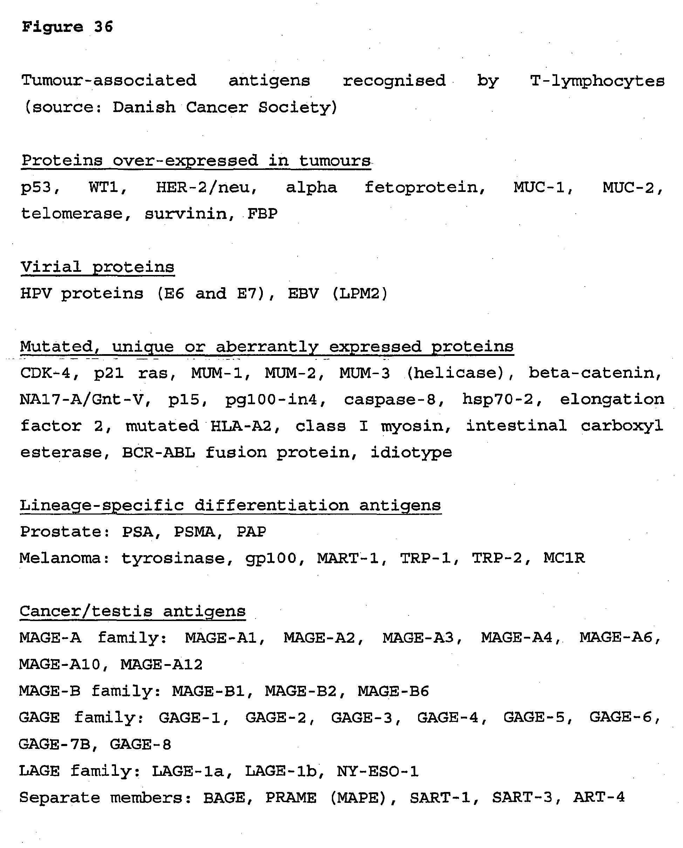

Figure 36 indicates interesting tumour-associated antigens recognised by T-lymphocytes.

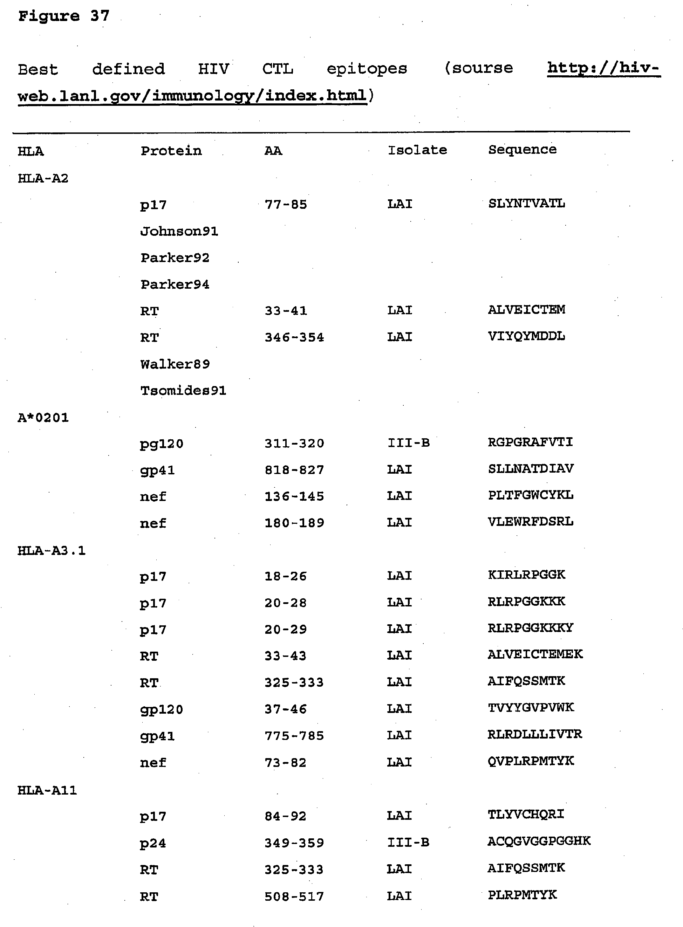

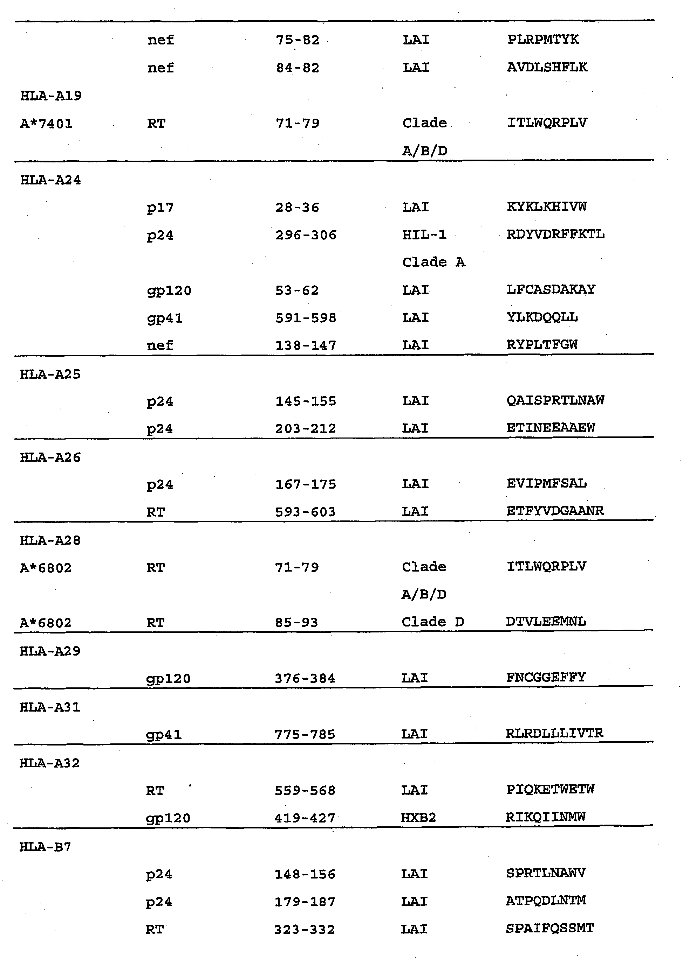

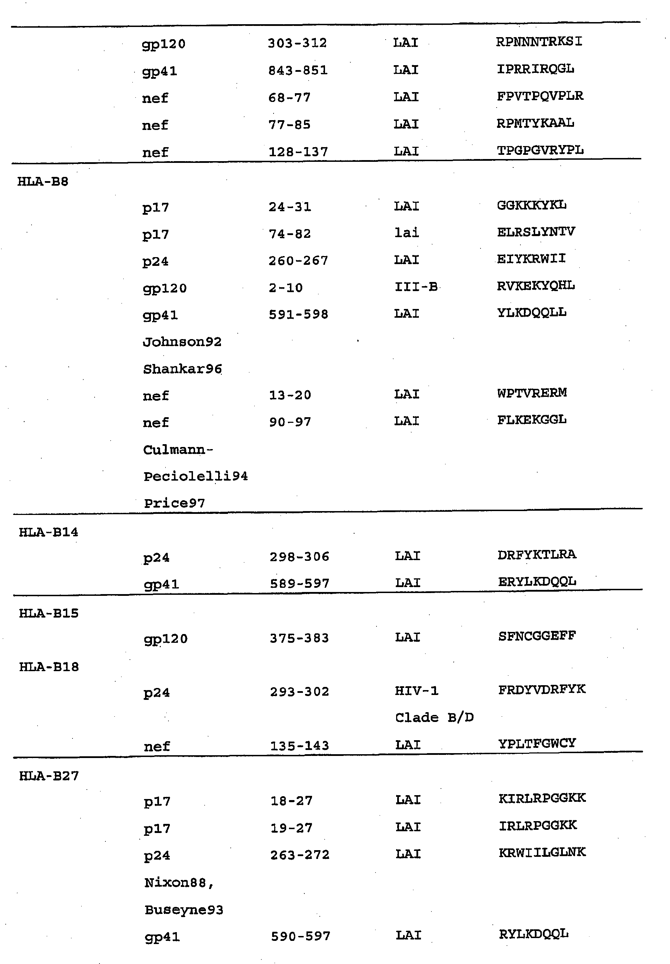

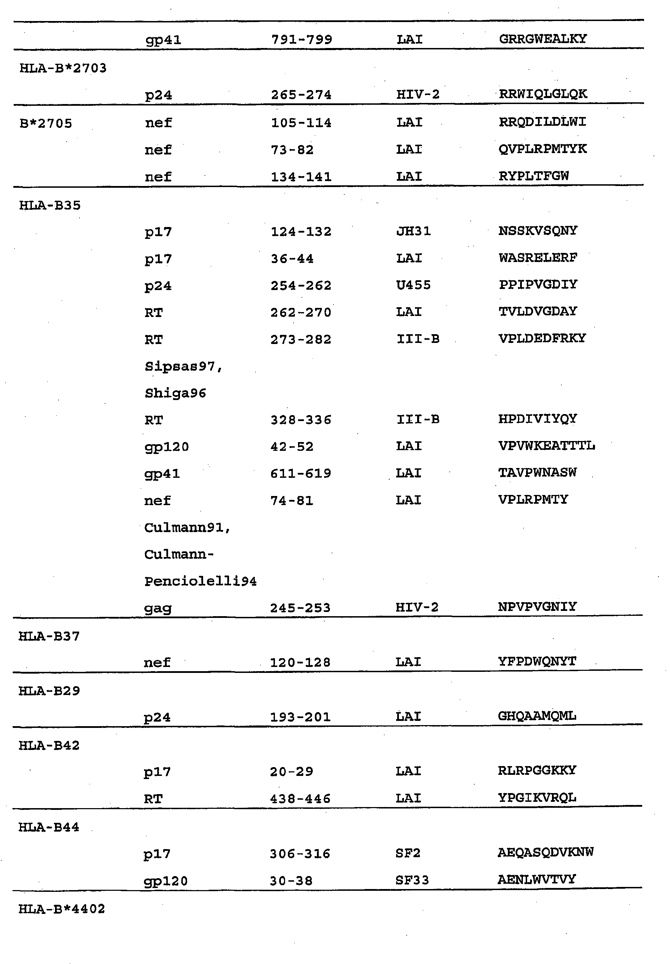

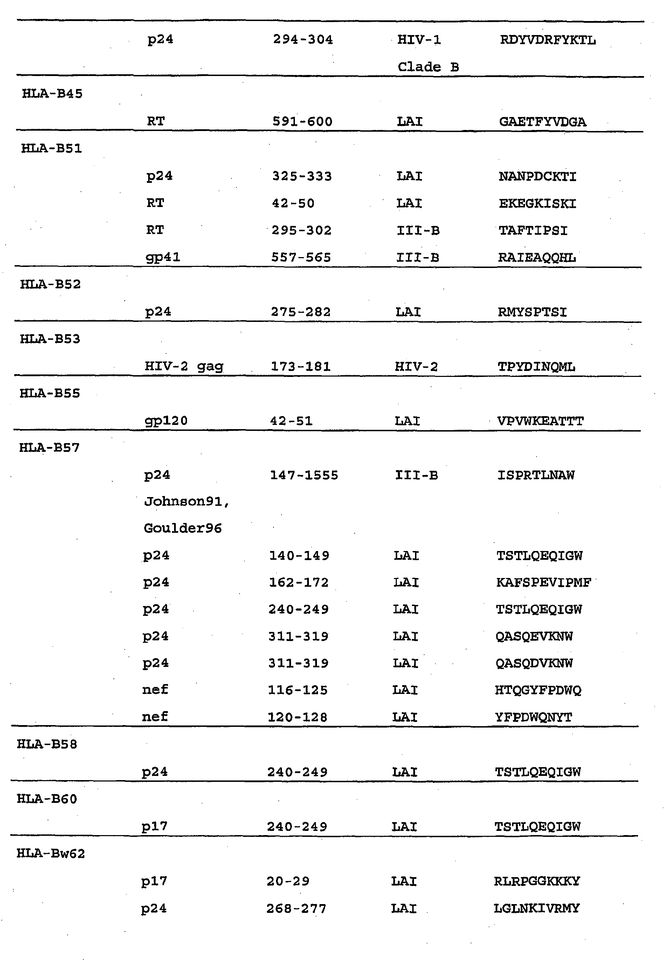

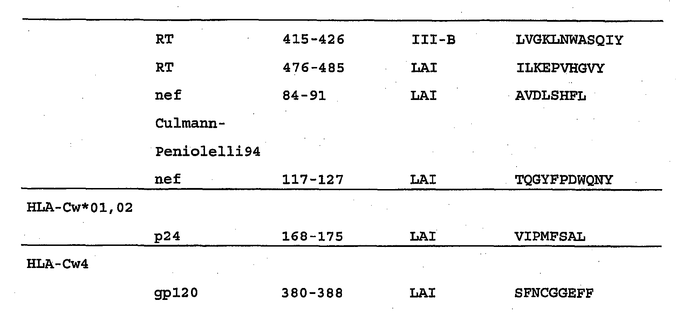

Figure 37 indicates interesting HIV CTL epitopes.

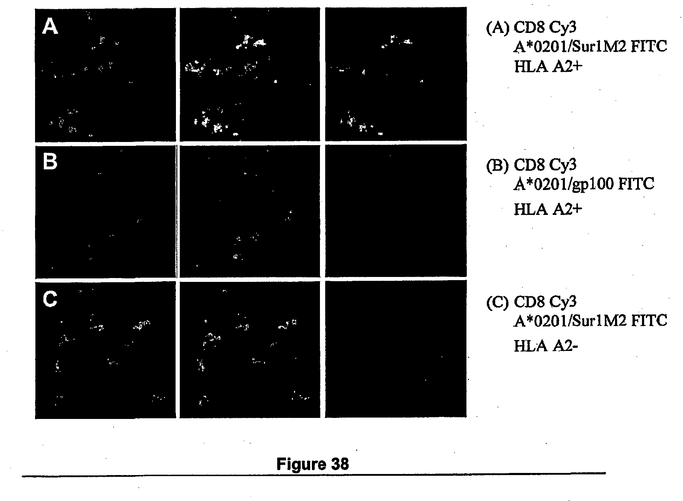

Figure 38. Fluorescent in situ staining of T-cells in biopsies, taken from different breast cancer lesions, with labelled CD8 antibody and poly-ligand MHC molecule constructs. Figure 38A: Staining with Cy3-labelled CD8 antibody (left picture), fluorescein-labelled survivin analogue peptide poly-ligand HLA A0201 molecule construct (right picture) on biopsies of HLA A2 positive patient, and the merged pictures (middle picture). Figure 38B: Staining with Cy3-labelled CD8 antibody (left picture), fluorescein-labelled non-sense gp100 analogue peptide poly-ligand HLA A0201 molecule construct (right picture) on biopsies of HLA A2 positive patient, and the merged pictures (middle picture). Figure 38C: Staining with Cy3-labelled CD8 antibody (left picture), fluorescein-labelled survivin analogue peptide poly-ligand HLA A0201 molecule construct (right picture) on biopsies of HLA A2 negative patient, and the merged pictures (middle picture).

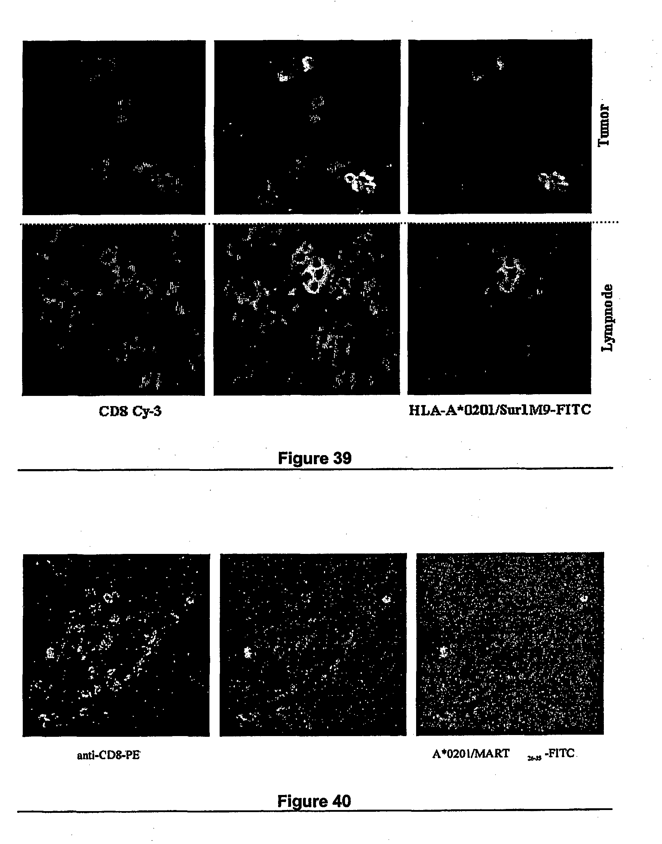

Figure 39. Fluorescent in situ staining of T-cells in biopsies, taken from melanoma lesions and lymph nodes, with labelled CD8 antibody and poly ligand MHC molecule constructs. Top lane: Staining with Cy3-labelled CD8 antibody (left picture), fluorescein-labelled survivin analogue peptide poly-ligand HLA A0201 molecule construct (right picture) of melanoma lesions from HLA A2 positive patient, and the merged pictures (middle picture). Bottom lane: Staining with Cy3-labelled CD8 antibody (left picture), fluorescein-labelled survivin analogue peptide poly-ligand HLA A0201 molecule construct (right picture) of lymph node from HLA A2 positive patient, and the merged pictures (middle picture).
Figure 40. Fluorescent in situ staining of T-cells with PE-labelled CD8 antibody (left picture), fluorescein-labelled MART-1 peptide poly-ligand HLA A0201 molecule construct (right picture) of melanoma lesions from HLA A2 positive patient, and the merged picture (middle picture).

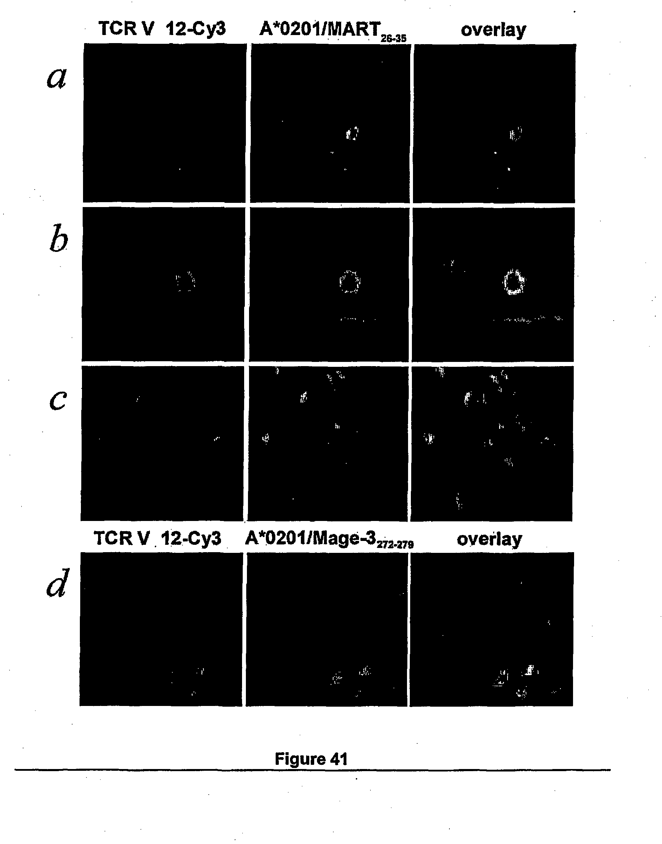

Figure 41. Fluorescent in situ staining of different T-cell populations in skin biopsies from immunisation injection site with labelled TCR VB12 antibody and MART-1 or MAGE-3 peptide poly ligand MHC molecule constructs. Figures 41A, 41B and 41C: Staining with Cy3-labelled TCR BV12 antibody (left pictures), fluorescein-labelled MART-1 peptide poly-ligand HLA A0201 molecule construct (middle pictures), and the merged pictures (right pictures). Figure 41D: Staining with Cy3-labelled TCR VB12 antibody (left picture), fluorescein-labelled MAGE-3 peptide poly-ligand HLA A0201 molecule construct (middle picture), and the merged pictures (right picture).

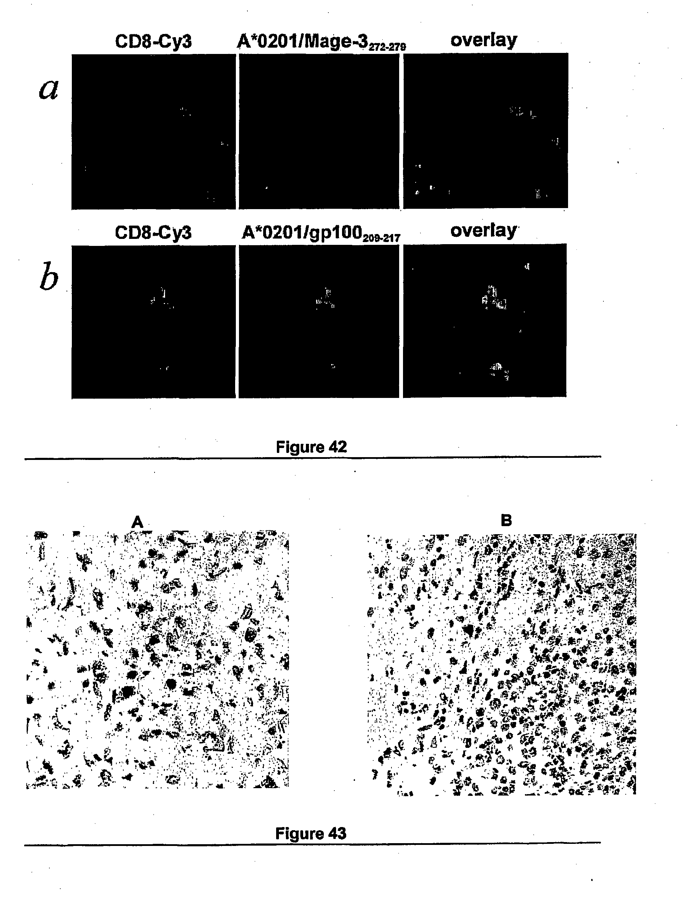

Figure 42. Fluorescent in situ staining of T-cells in skin biopsies from gp100 peptide epitope immunisation injection site with CD8 antibody and poly ligand MHC molecule constructs. Figure 42A: Staining with Cy3-labelled CD8 antibody (left picture), fluorescein-labelled non-sense MAGE-3 peptide poly-ligand HLA A0201 molecule construct (middle picture), and the merged pictures (right picture). Figure 42B: Staining with Cy3-labelled CD8 antibody (left picture), fluorescein-labelled gp100 peptide poly-ligand HLA A0201 molecule construct (middle picture), and the merged pictures (Right picture).
Figure 43. Bright field microscope picture of an AEC chromogen in situ staining of T-cells in HLA A0201 positive melanoma tissue with MART-1 peptide analogue (ELAGIGILTV) poly-ligand HRP-labelled MHC molecule construct. Different endogenous peroxidase blocking reagents were used. Figure 43A: peroxide/methanol solution, and Figure 43B: DAKO peroxidase blocking solution. Positive cells are coloured red.

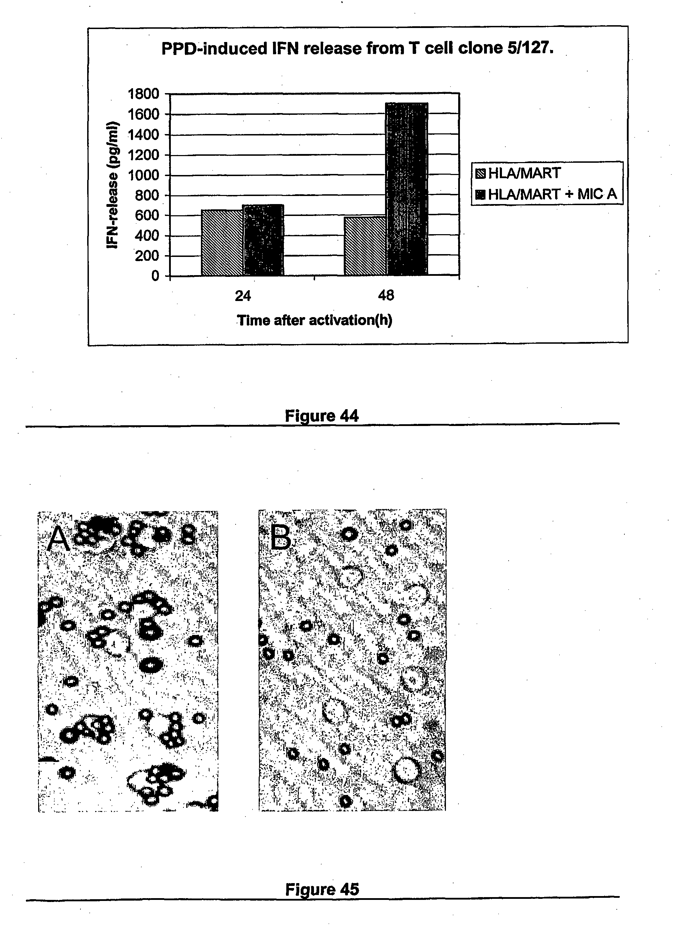

Figure 44. Effect of poly-ligand MHC molecule constructs without or combined with MIC A protein on release of IFN-gamma from T-cells. Cells were incubated with MHC molecule construct for the indicated periods before measurement of INF-gamma in supernatants. The T-cell clone did not secrete INF-gamma when incubated with MHC molecule construct displaying an irrelevant peptide.
Figure 45. Bright field microscope picture of Survivin reactive cytotoxic T-lymphocytes (CTL) isolated from a melanoma infiltrated lymph node using magnetic beads. Figure 45A: The Survivin peptide reactive CTLs were isolated/rosetted with Dynabeads^{®} coated with a recombinant HLA A0201/Survivin peptide-complex. Figure 45B: Dynabeads^{®} coated with a recombinant HLA A0201/influenza peptide-complex are used as negative control.

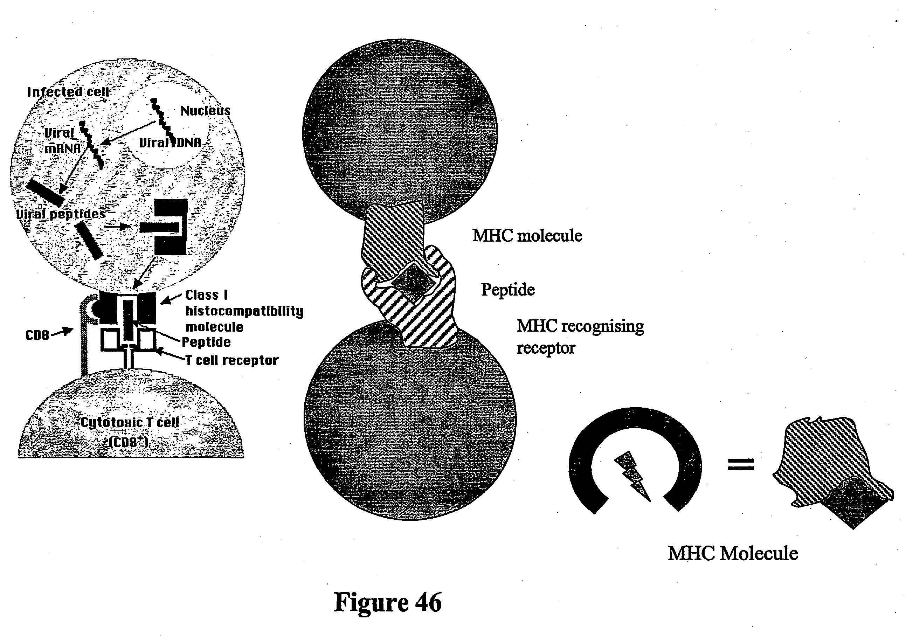

Figure 46. Foreign peptides and proteins are presented to the immune apparatus through MHC molecules displayed on the surface of cells. Some cells recognise and bind to the MHC molecule in complex with a peptide presented at the surface of the cells. Such cells (MHC recognising cells) "taste" the different MHC molecule/peptide combinations. If the receptor on the MHC recognising cell can bind to the MHC molecule/peptide complex, then a cascade of cellular responses may be induced.

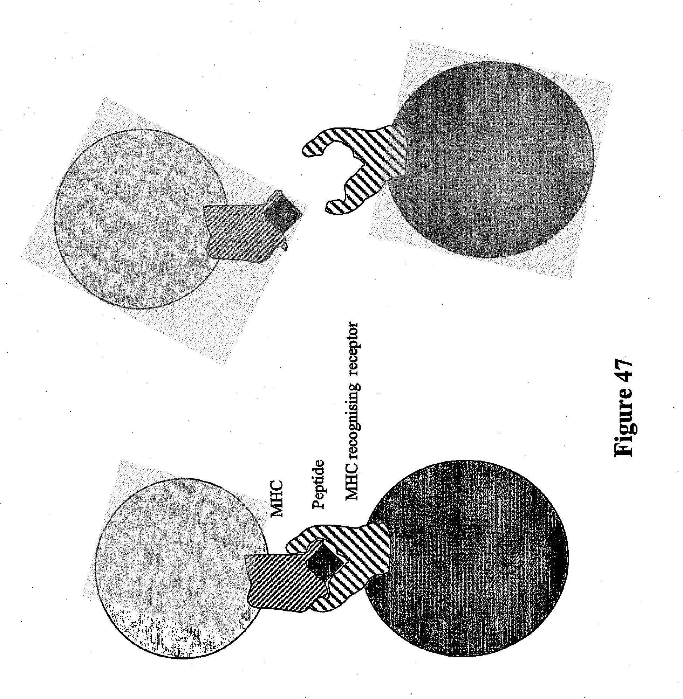

Figure 47 illustrates how the MHC molecule in complex with a peptide fits into a receptor by a cell. Each MHC molecule of the MHC constructs of the invention acts like the cell displaying a MHC molecule/peptide complex. Thus, the presence of specific cells, namely those that have a receptor recognising the specific MHC molecule/peptide combination, can be identified.

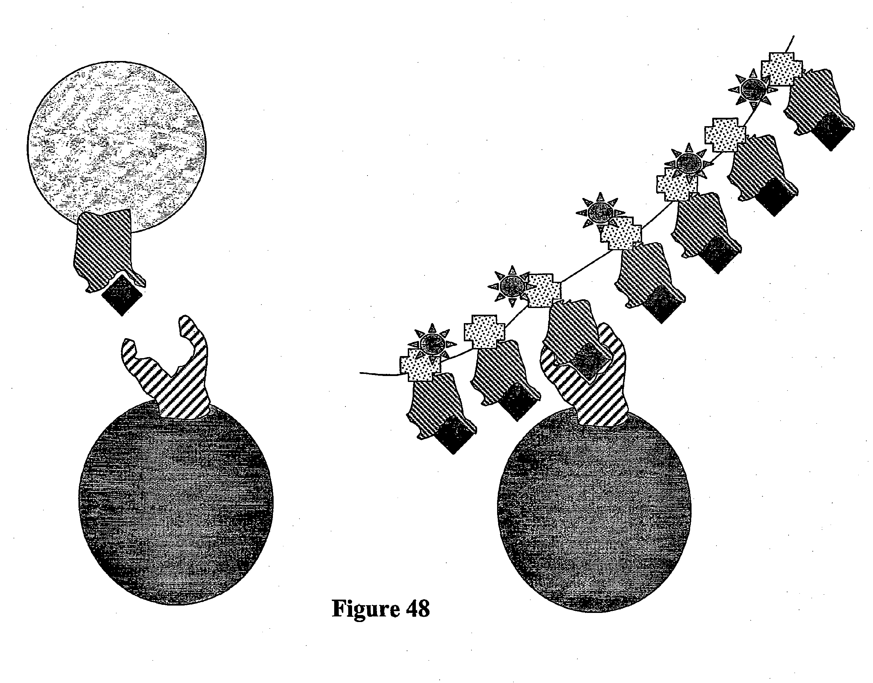

Figure 48 illustrates how the MHC molecules of the MHC molecule constructs of the invention may be viewed as a "string of pearls". The MHC molecule constructs bind with high avidity to receptors on the MHC recognising cells.

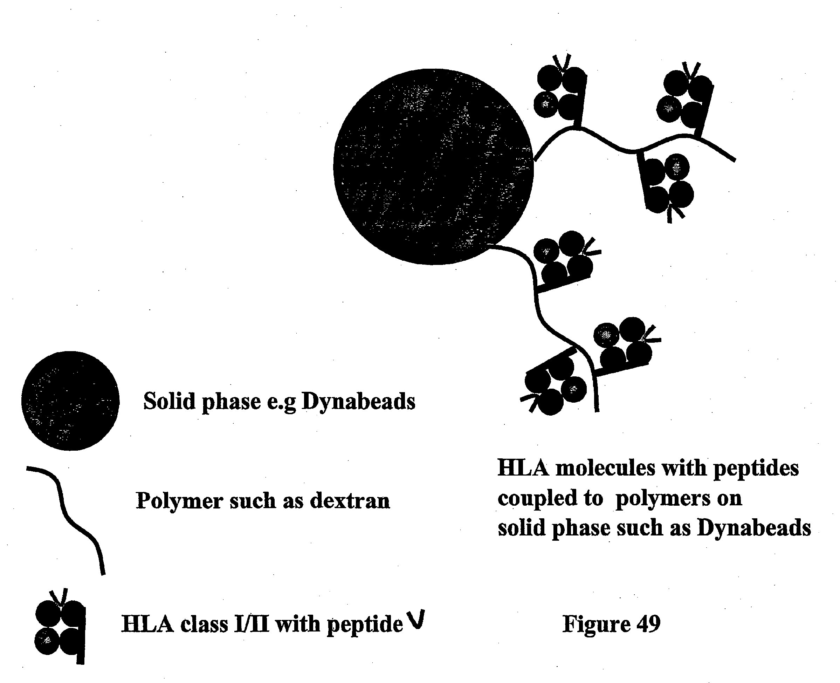

Figure 49 shows an embodiment of the present invention where the MHC molecule construct of the invention comprises peptide filled HLA molecules. Here the MHC molecule construct is coupled (or immobilised) directly onto a solid or semi-solid support in this case beads or particles.

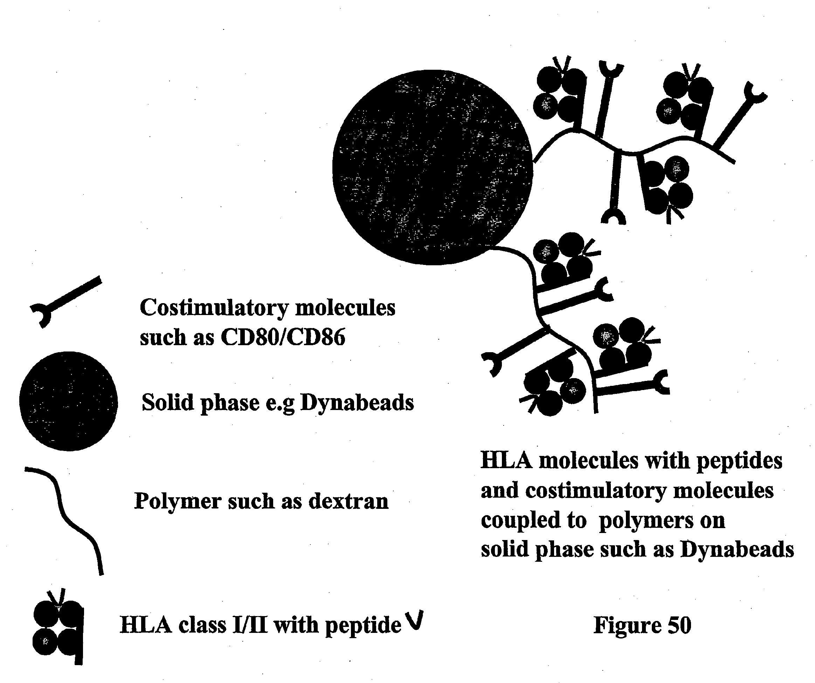

Figure 50 shows an embodiment of the present invention where the MHC molecule construct of the invention comprises peptide filled HLA molecules as well as co-stimulatory molecule e.g. CD80 or CD86. Here the MHC construct of the present invention is coupled (or immobilised) directly onto a solid or semi-solid support in this case beads or particles.

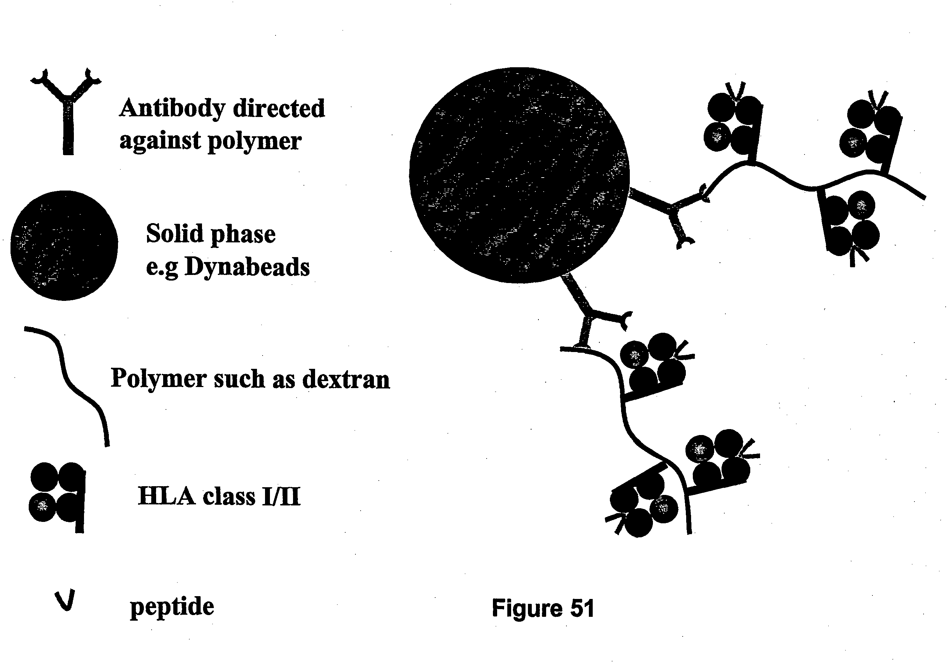

Figure 51 shows an embodiment of the present invention where the MHC molecule construct of the invention comprises peptide filled HLA molecules. Here the MHC construct of the present invention is coupled (or immobilised) onto a solid or semi-solid support in this case beads or particles using an antibody binding to the carrier molecule.

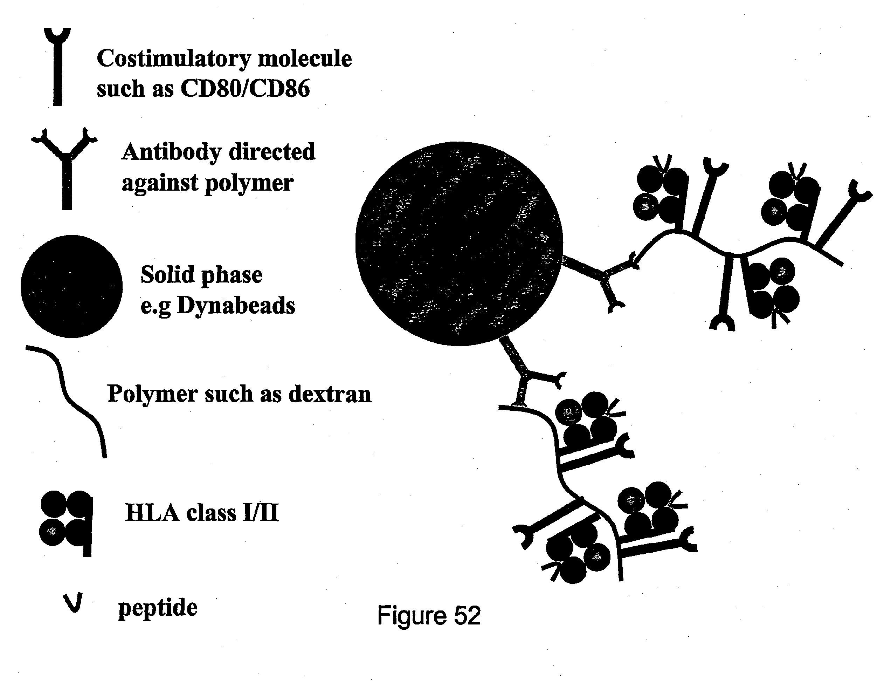

Figure 52 shows an embodiment of the present invention where the MHC molecule construct of the invention comprises peptide filled HLA molecules and co-stimulatory molecules. Here the MHC construct of the present invention is coupled (or immobilised) onto a solid or semi-solid support in this case beads or particles using an antibody binding to the carrier molecule.

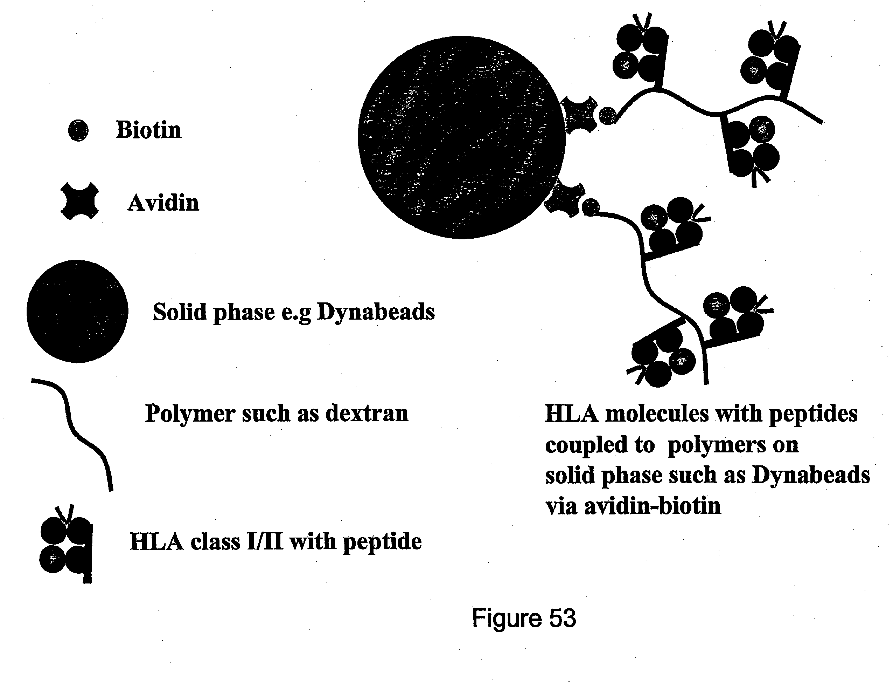

Figure 53 shows an embodiment of the present invention where the MHC molecule construct of the invention comprises peptide filled HLA molecules. Here the MHC construct of the present invention is coupled (or immobilised) onto a solid or semi-solid support, in this case, beads or particles by a biotin-avidin binding pair.

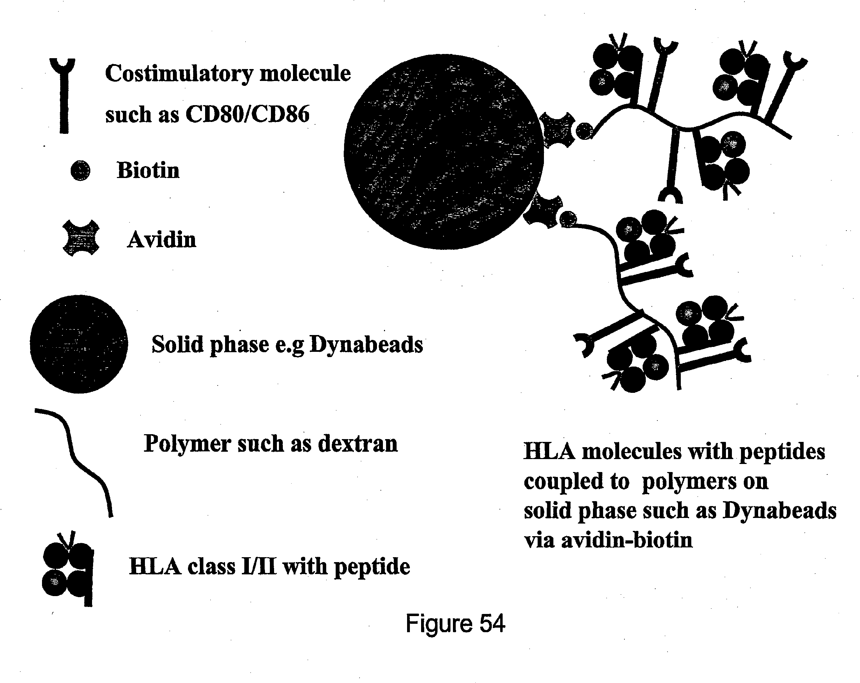

Figure 54 shows an embodiment of the present invention where the MHC molecule construct of the invention comprises peptide filled HLA molecules and co-stimulatory molecules. Here the MHC construct of the present invention is coupled (or immobilised) onto a solid or semi-solid support, in this case, beads or particles via biotin-avidin.

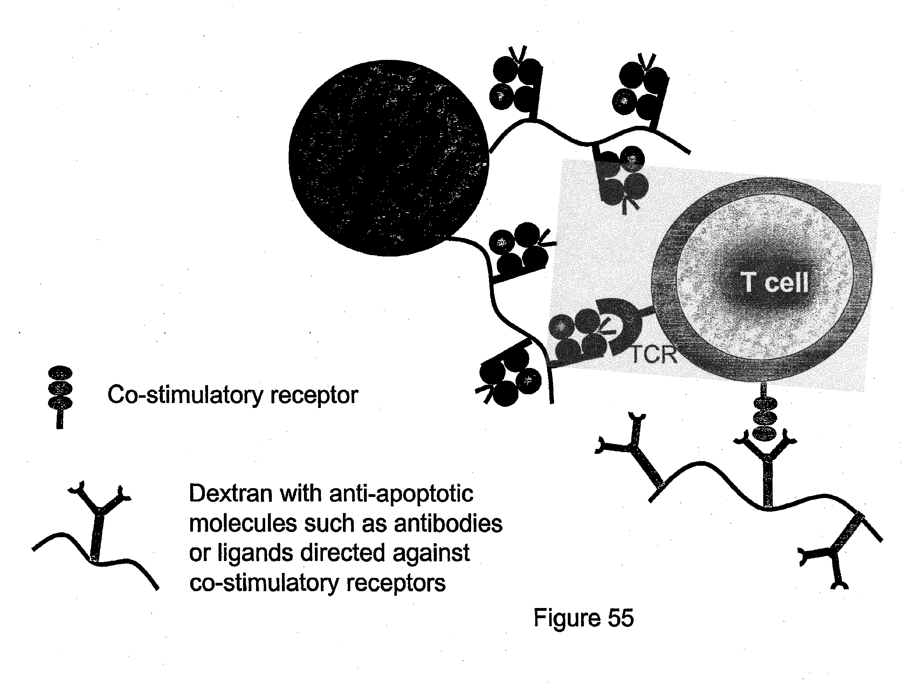

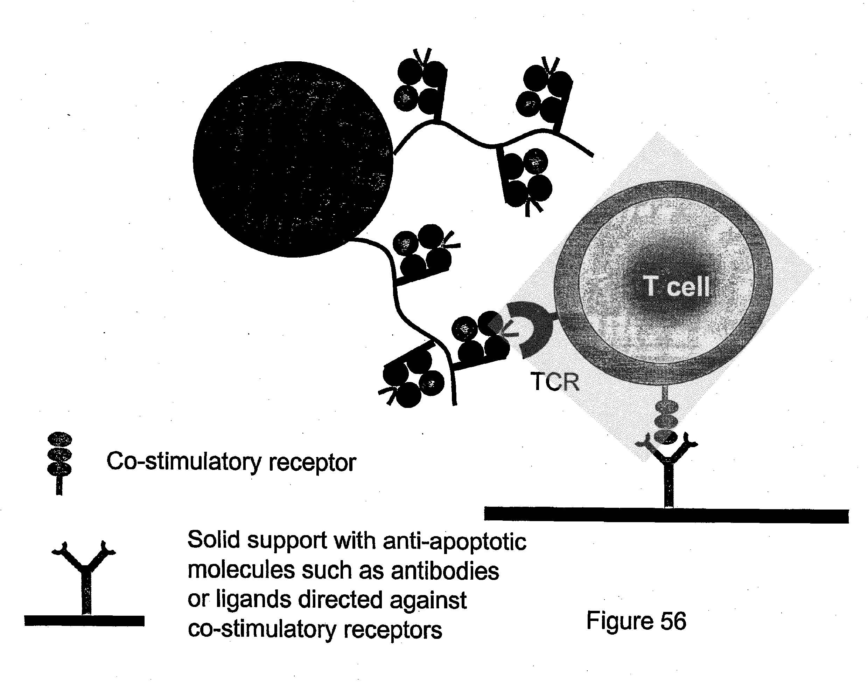

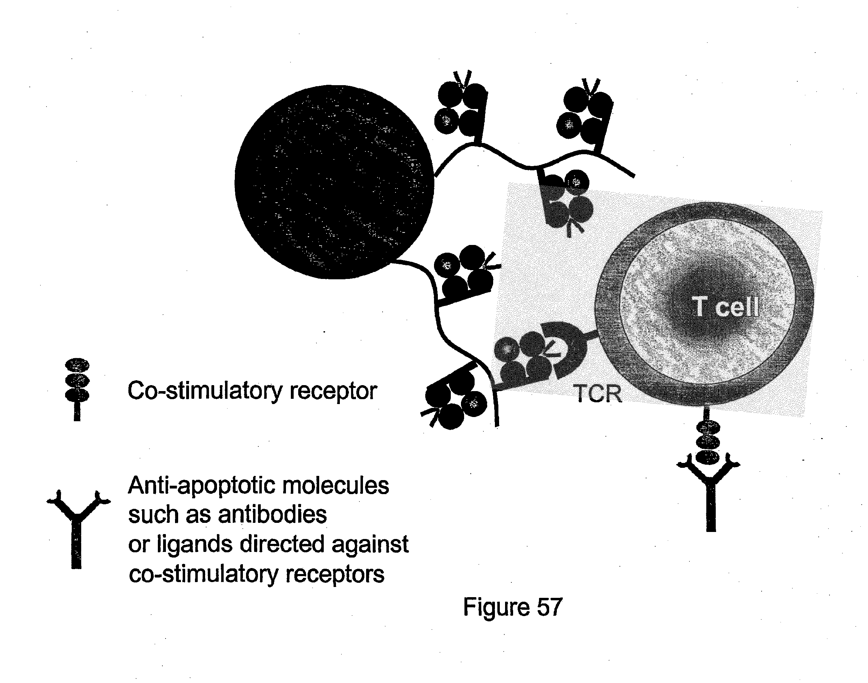

Figures 55, 56, and 57. In the case T-cells only receive one signal through the TCR/CD3 complex, this signal can induce apoptosis of the T-cells. In principle, this can occur during the separation procedure where T-cells may be contacted by magnetic beads carrying MHC molecule be contacted by magnetic beads carrying MHC molecule constructs. Apoptosis can be avoided or reduced by providing the T-cells with a co-stimulatory signal during the cell isolation procedure. Poly-ligand constructs according with both MHC molecules and co-stimulatory molecules would compromise the specificity of cell isolation. In order to avoid or reduce this, T-cell isolation can be performed with MHC molecule constructs immobilised on beads in the presence of co-stimulatory antibodies or ligands conjugated to soluble MHC molecule constructs (Figure 55), in the presence of co-stimulatory antibodies or ligands conjugated to another solid or semi-solid phase (Figure 56), or in the presence of soluble co-stimulatory antibodies or ligands (Figure 57).

### DETAILED DESCRIPTION OF THE INVENTION

In order to better explain the present invention, a description of some important characteristics of the immune system and MHC molecules are given below.

### The immune system and MHC molecules

The immune system can be viewed as one of natures major bioinformatic systems. It evaluates any substance that enters into the internal environment, determines its nature and decides whether to take action against it. Proteins and peptides are the most important means of obtaining and conveying such immune information. From this point of view, MHC molecule encodes the most essential group of molecules required for recognition of immunogenic antigens. MHC molecules are sampling the entire protein metabolism for peptide information and make this information available for the central recognition unit of the immune system, the T-cell. However, these effector cells require substantial interaction between the MHC molecule and TCRs to sustain that at least two signals are required for activation of naive T-cells: One signal emerges by the molecular interaction between MHC molecules and TCR, whereas the other signal is derived from ligation of co-stimulatory ligands e.g. B7-1 and their counter receptors e.g. CD28. It has also been shown that other molecules e.g. NK receptors may regulate the activation threshold of T-cells. Thus, full activation of T-cells and other immune competent effector cells require an orchestrated action of multiple ligands; one could say that immune responses are under control of poly-ligand compounds.

The function of the immune system is to protect the body against foreign invaders or aberrant self-molecules (e.g. parasites, bacteria, viruses and cancer). Such threats can normally be eliminated or neutralised efficiently by the immune system. To administrate this potential, the immune system must discriminate normal molecules in the healthy body from the presence of foreign or aberrant self-molecules, which may be expressed during genesis of diseases e.g. cancer. Ideally, foreign or aberrant molecules should be eliminated, while the body itself should be left unharmed. One major hallmark of the immune system is therefore one of specificity i.e. the ability to discriminate between various targets and in particular to distinguish between self and non-self. The specific - or adaptive - immune system involve a large number of different cell types. Immune responses develop from an orchestral interplay of antigen-processing/presenting cells and effector cells. The central effector cells are lymphocytes, with a major subdivision into B- and T-cells representing humoral and cellular responses, respectively. Both cell populations use receptors, which in their genome are encoded in many bits and pieces allowing enormous recombinatorial receptor diversity. Each B- or T-cell carries one, and only one, of these receptors which recognise their tiny but unique fragments of the universe. All human lymphocytes combined divide the entire universe into two major groups of targets: a group of self-antigens that are tolerated by the immune system and a group of non-self or aberrant antigens that may elicit a response. The overall specificity of the immune system is generated, regulated and co-ordinated through processes controlling individual lymphocytes. Deleting, or inactivating a lymphocyte clone removes the corresponding specificity from the repertoire. Activation and propagation of a lymphocyte clone enhances the corresponding specificity - and allows the immune system to respond quickly and strongly toward the antigen.

### The immune system cells

The cells of the immune system include the following: Lymphocytes are a type of white blood cells found in the blood and many other parts of the body. Types of lymphocytes include B-cells, T-cells, and Natural Killer (NK) cells.

The B- and T-cells recognise and respond specifically to aberrant substances, thus being a part of the specific immune system.

B-cells (B-lymphocytes) mature into plasma cells that secrete antibodies (immunoglobulins), the proteins that recognise and attach to foreign substances known as antigens. Each type of B-cell produces one specific antibody, which recognises one specific epitope on the antigen.

The T-cells recognise and respond towards aberrant substances by interaction with antigen presenting cells (APC) that display antigens in form of "non-self" (or aberrant) peptides in context of MHC molecules. Each T-cell clone expresses one unique specificity of T-cell receptors (TCR), which recognise one specific peptide/MHC epitope.

T-cells comprise two major subpopulations. Cytolytic T-cells directly attack infected, foreign, or cancerous cells displaying foreign or aberrant forms of endogenous peptides in context of MHC Class I molecules (described below). "Helper" T-cells that are activated by foreign exogenous peptides in MHC Class II molecules, contribute to regulation of the immune response by signalling other immune system defenders. T-cells also work by producing proteins called lymphokines.

NK cells produce powerful chemical substances that bind to and kill any foreign invader. They attack without first having to recognise a specific antigen, thus being an immune cell type that also relate to the innate immune system.

Monocytes are white blood cells that are able to swallow and digest microscopic organisms and particles in a process known as phagocytosis and antigen processing. Dendritic cells (DC) are of particular interest as they present peptide epitopes in a "professional way" which leads to effective activation of T-cells. The professional APC express a variety of co-stimulatory molecules that ligate with a variety of counter receptors expressed on the T-cells.

Cells in the immune system secrete two types of proteins, namely antibodies and cytokines. Specific antibodies match epitopes on specific antigens, fitting together much the way a key fits a lock. Conventional vaccine approaches, in particular, work through activation of helper T-cells and B-cells leading to secretion of antigen specific antibodies.

Cytokines are substances produced by some immune cells to communicate with other cells. Types of cytokines include lymphokines, interferons, interleukins, and colony-stimulating factors.

### Antigen-recognition by B- and T-cells

B- and T-cells use entirely different mechanisms to recognise their targets. B-cells recognise soluble antigens, and since they can secrete their receptors as antibodies, they can potentially interact with antigen throughout the fluid phase of the extra-cellular space. In sharp contrast, the T-cell receptor is always membrane bound and it only recognises antigen, which is presented on the membrane of an antigen-presenting cell (APC). In other words, T-cell recognition involves a direct physical interaction between two cells, a T-cell and an APC. B- and T-cells also differ with respect to what they recognise. B-cells can recognise organic substances of almost any kind, whereas T-cells predominantly recognise proteins (as a biological target, proteins are particularly important since they constitute the structural and functional basis of all life). B-cells recognise the three-dimensional structure of proteins as illustrated by their ability to distinguish between native and denatured proteins. In contrast, T-cells cannot distinguish between native and denatured proteins. Today, we know that T-cells only recognise antigenic peptides presented in association with MHC molecules on the surface of APC's. In general, cytotoxic T-cells recognise short peptides (corresponding in general to 8-11 residues), the amino and carboxy-termini of which are deeply embedded within the MHC Class I molecule (i.e. the peptide length is restricted). In comparison, helper T-cells tend to recognise longer peptides (corresponding in general to 13-30 residues) with amino and carboxy terminal ends extending out of the MHC Class II molecule.

### MHC restriction and T-cell immunity

T-cells determine the reactivity and specificity of the adaptive immune system, including the activity of B-cells. It is therefore appropriate to focus the attention on these cells. T-cells can only recognise a given antigen, when it is presented in the context of a particular MHC molecule. They are "educated" during ontogeny and further activated during the first priming in processes designed to develop T-cells carrying receptors specific for a particular antigen-MHC molecule combination. These T-cells are subsequently only able to recognise the same antigen-MHC molecule combination. This phenomenon is known as "MHC restriction". Another immune phenomenon - that of "responder status"- is also determined by the MHC molecules. Individuals of one MHC haplotype will respond to some antigens, and not to others. Other individuals with other MHC haplotypes will respond differently. These two phenomena are of obvious importance for any rational immune manipulation. As mentioned, we now know that MHC molecules control them both. These molecules have specifically evolved for the purpose of antigen presentation. Our current understanding of antigen presentation can be summarised as follows. Firstly, the foreign substance, the antigen, is taken up by APC's. An intracellular pool of antigenic peptides is generated through proteolytic fragmentation of all the protein available to the cell (which may include both normal cell proteins ("self-proteins") and antigens ("non-self proteins") from infective organisms.

This pool of peptides is offered to the MHC molecules of the individual and sampled according to length and sequence; some are bound, while others are ignored (the MHC molecule is said to perform "determinant selection"). Subsequently, MHC molecules protect the selected peptides against further degradation, transport them to the surface of the APC and display them for T-cell scrutiny. Antigenic peptides from "non-self proteins" are, in contrast to peptides from "self-proteins", recognised by T-cells that consequently may become activated.

### A plurality of receptors are involved in antigen specific activation of immune cells

Several ligand-receptor interactions related to control this network of cells are complex, in comparison to more "conventional" ligand-receptor models comprising simple hormone-receptor interaction e.g. insulin and IR.

For example, full activation of T-cells acquires simultaneous signalling through a variety of receptors in addition to TCR signalling. The binding energy yielded from ligation of multiple membrane molecules expressed on APC and T-cells, ensure a close physical contact between the involved cells. One of the most important additional receptors related to activation of T-cells is CD28 molecules, which bind proteins of the B7 family expressed on professional APCs. Other known examples of regulatory receptors expressed on T-cells are a variety of NK receptors (NKR), which comprise both inhibitory and activating isoforms. The balance between expressed forms of activating and inhibiting NKRs is believed to determine a threshold for activation of specific T-cells.

It has recently been reported that molecular interactions between many of the receptors and ligands involved in this cellular interplay, including TCR and MHC molecules, are unstable i.e. of low affinity. By example, it has been measured that monovalent MHC molecule-TCR interaction has an affinity constant of K_{D} = 10 µM with a dissociation constant less than a minute. Molecular interaction of CD28 and B7 protein has an affinity constant of same level. In comparison, the stability and affinity of complexes formed by high-affinity interactions e.g. hormone ligand-receptor binding (insulin/IR) and antibody-antigen binding, are significantly higher (affinity constant K_{D} in the range of 0.1-10 nM).

The plurality of proteins related to activation of T-cells do, however, not only stabilise cellular contact between APC and T-cells, they also contribute to a variety of signalling events required for activation of T-cells. It is orchestrated actions of these signalling events that determine the activation of T-ucells. For example, it has been shown that naïve cytolytic T-cells require at least two signals for activation. The first signal is delivered through ligation of MHC molecules (expressed on APCs) to TCRs on T-cells. The second signal is delivered through co-stimulatory molecules from e.g. B7 protein family, which ligate with the CD28 receptor on T-cells.

### MHC molecule and antigen presentation: a port to the specific immune system

The genes located in the human MHC locus (HLA locus) encode a set of highly polymorph membrane proteins that sample peptides in intracellular compartments and present such peptide epitopes on surfaces of antigen presenting cells (APC) to scrutinising T-cells. The extensive genetic polymorphism of the MHC locus is the background for the unique genetic finger print of the immune system in individuals and defines the repertoire of antigenic peptide epitopes which the human population is capable of recognising and respond to.

Two subtypes of MHC molecules exist, MHC Class I and II molecules. These subtypes correspond to two subsets of T-lymphocytes: 1) CD8+ cytotoxic T-cells, which usually recognise peptides presented by MHC Class I molecules, and kill infected or mutated T-cells, and 2) CD4+ helper T-cells, which usually recognise peptides presented by MHC Class II molecules, and regulate the responses of other cells of the immune system. MHC Class I molecules consist of a 43,000 MW transmembrane glycoprotein (the α chain) non-covalently associated with a 12,000 MW non-glycosylated protein (the light (β) chain, also known as β₂-microglobulin). MHC Class II molecules have an overall structure similar to MHC Class I molecules although the domain distribution is different. The MHC Class II molecule consists of two non-covalently associated transmembrane glycoproteins of approximately 34,000 and 29,000 MW. The detailed structures of MHC Class I and II molecules have been solved at the X-ray crystallography level by e.g. Björkman et. al. (ref. 8). The most interesting part of the MHC molecule structure is the "upper" part that shows a unique peptide-binding groove consisting of two alpha helixes forming the walls of the groove and eight beta-pleated sheaths forming the floor of the groove.

The peptides are the essential target structures in recognition of "non-self" by the adaptive immune system and, one could say, the group of MHC molecules comprises a port to the immune system, thus being a major player in determining penetrance and spreading of human diseases. MHC molecules of other higher vertebrate species exert identical biological functions as those of HLA in man.

The MHC locus is extremely polymorphic i.e. many different versions (alleles, allotypes) exist in the population, but each individual has only inherited two of these (one from the father and one from the mother). It is also polygenic i.e. several MHC encoding loci exist in the genome allowing for simultaneous expression of several isotypes. Importantly, the majority of the polymorphic residues points towards the peptide binding groove affecting its size, shape and functionality as described by e.g. Matsumura et al (ref. 9). Peptide-MHC interactions are specific, albeit broad, allowing the binding of many unrelated peptides to each MHC allotype. The polymorphism dictates the specificity of peptide binding and the biological consequence of this is that each individual in the population educates and shapes a unique T-cell repertoire.

A variety of relatively invariant MHC Class I molecule like molecules have been identified. This group comprise CD1d, HLA E, HLA G, HLA H, HLA F, MIC A, MIC B, ULBP-1, ULBP-2, and ULBP-3. These non-classical molecules have a tissue distribution and functions distinct from HLA A, B and C. Some of them comprise only a heavy chain protein i.e. do not associate with β₂m molecules and peptides. As described previously, the immune responses develop from an orchestral interplay of antigen-processing/presenting cells and effector cells.

Monomer and soluble forms of cognate as well as modified MHC molecules e.g. single chain protein with peptide, heavy and light chains fused into one construct, have been produced in bacteria as well as eucaryotic cells. Recent advances in recombinant technology and *in vitro* protein folding methods have provided efficient protocols for large-scale production of multimeric MHC molecules, which bind with high avidity to appropriate T-cell receptors.

### NK cells and MHC molecules

NK cells remained mysterious until recently. These cells were defined by their ability to lyse certain tumours in the absence of prior stimulation. Recent reports have however shown that NK cell activity is regulated by a number of ligands including MHC molecule (ref. 5). NK cells recognise MHC Class I molecules through surface receptors that deliver an inhibitory signal. Thus, NK cells may lyse target cells that have lost expression of MHC molecules. It is well known that tumour cells often reduce or loose their expression of MHC molecules presumably due to a selective pressure from cytotoxic T-cells that recognise tumour associated antigens (peptides) in context of MHC molecules. The ability of NK cells to discriminate between normal and tumour cells is then explained by the "missing-self hypothesis" by Ljunggren et.al (ref. 6). However, NK cells are not simply equipped with receptors that recognise a broad spectrum of MHC molecules. The complexity of NK receptors is also reflected by expression of different isoforms, some of which are activating whereas others are inhibitory. Interestingly, 5-10% of the (alpha beta) T-cells also express different NK receptors such as KIR, ILT or CD94/NKG2, which belong to the inhibitory-receptor superfamily. Such receptors may serve to raise the activation threshold for cellular immune responses (ref. 7).

The balances between stimulating and inhibitory receptors presumably control the activation of T- and NK cells. Some of the different NK receptors expressed on NK and T-cells recognise broader specificity of MHC Class I molecules, whereas others recognise more rarely expressed allelic determinants. Thus, the MHC molecules may be involved in both stimulation and inhibition of specific immune responses.

### The T-cell receptor

The T-cell receptor, a member of the immunoglobulin superfamily, consists of two non-covalently associated transmembrane glycoproteins ("α" and "β" chains) of approximately 30,000 MW; each comprising two extra-cellular domains. The two chains form a dimmer, which associate with a larger protein complex, CD3. The detailed structures of TCR in association with MHC Class I molecules have been solved at the X-ray crystallography level. Recombinant forms of soluble TCRs (consisting of extracellular domains) have been produced in bacteria and eucaryotic cells.

The specific interplay of specific TCR ligands i.e. immunogenic peptide/HLA complexes and specific T-cell receptors results in ligand induced formation of a signalosome composed by the TCR/CD3 complex and its interplay with intracellular pools of tyrosine kinases (lck, Fyn, Syk, Zap-70) and adaptors (LAT, TRIM and Grp2) (ref. 4). As described above, the TCRs are expressed clonally and only appropriate peptide specific MHC complexes can elicit an immune response.

To summarise, one could say that individual T-cell clones "taste" on tiny fragments from the outer and inner world through interplay of specific T-cell receptors and their natural ligands; the peptides in context of HLA molecules. The T-cells require, however, additional ligands (one could say compounded ligands) which - in a concerted action with HLA molecules - provide appropriate signals for T-cell activation.

### Co-stimulatory molecules

The adaptive immune responses require two signals for initial activation: one signal provided through the binding of peptide-MHC on the antigen presenting cell (APC) to the T-cell receptor (TCR), and a second antigen-independent signal called co-stimulation. CD28 is a membrane receptor on T-cells that provides co-stimulatory function when T-cells encounter APCs that express CD28 ligands, B7-1 (CD80) of B7-2 (CD86). The functions of CD28 are predominantly to influence signals initiated through the TCR, which results in qualitative and quantitative changes in the cascade of events leading to proliferation, cytokine production, and cell survival. Triggering of naive T-cells without the co-stimulatory signal may render the T-cells functionally unresponsive (anergy, apoptosis). CD28 induces greater proliferation of CD4+ T-cells compared with CD8+ T-cells. Other members of the CD28 immunoglobulin (Ig) superfamily such as includes inducible co-stimulator (ICOS) provides co-stimulatory signals on activated CD4+ and CD8+ T-cells to enhance their proliferation.

Lymphocyte responses are regulated by inhibitory as well as activating signals. CTLA-4 and PD-1 mediated such inhibitory signals. CTLA-4 has higher affinity for shared ligands B7-1 and B7-2 compared with CD28, and it is up-regulated upon TCR-CD28 engagement. PD-1 appears to mediate an inhibitory signal, and it is widely expressed on hematopoietic-derived tissues and on activated T-cells. Interleukin-2 and co-stimulatory signals are the two most important factors required for maintenance of continuous cell division.

Although CD28 provides a critical co-stimulatory signal on naive T-cells, other co-stimulatory molecules in the tumour necrosis receptor (TNFR) superfamily, such as 4-1BB (CD137), CD27 and OX40 (CD134), provides co-stimulatory signals on activated T-cells to orient the quality of T-cell response towards cell survival or apoptosis.

Some CD8+ effector T-cells lack CD28 expresion. However, these cells the lectin-like NKG2D homo-dimer, a receptor for the MHC Class I-like molecules called MIC. NKG2D serves as a co-stimulatory molecule for CD28-CD8+ T-cells and with combined triggering of TCR/CD3 complexes induced IL-2 and T-cell proliferation. Expression and function of NKG2D are selectively up-regulated by the cytokine IL-15. Human NKG2D is expressed on gamma,delta T-cells, CD8+ T-cells, NK cells, and a small subset of CD4+ T-cells. The stress-induced MIC A and MIC B molecules are expressed in the intestinal epithelium as well as in diverse tumours of epithelial origin. NK cells are able to reject tumours expressing MHC Class I molecules if the tumour expresses a ligand for NKG2D, i.e. MIC A or MIC B. A family of receptors (NKp46, NKp30, NKp44) termed natural cytotoxicity receptors (NCR) expressed on NK cells are involved in NK-mediated lysis of various tumours.

### Cytokines

As mentioned above, cytokines are play an important role in the communication between cells. The group of cytokines comprise

Interferons (IFN): Interferons are types of cytokines that occur naturally in the body. There are three major types of interferons; interferon-alpha, interferon-beta, and interferon-gamma. Interferons stimulate NK cells, T-cells, and macrophages, boosting the immune system's function.

Interleukins: Like interferons, interleukins are cytokines that occur naturally in the body and can be made in the laboratory. Many interleukins have been identified; interleukin-2 (IL-2) has been the most widely studied in cancer treatment. IL-2 stimulates the growth and activity of many immune cells, such as lymphocytes. Colony-stimulating factors (CSFs) (sometimes called hematopoietic growth factors) usually stimulate bone marrow cells to divide and develop into white blood cells, platelets, and red blood cells. Bone marrow is critical to the body's immune system because it is the source of all blood cells.

### Therapy and vaccine approaches for manipulation of immune responses

Biological therapy (immunotherapy, biotherapy, or biological response modifier therapy) is a relatively new type of treatment and based on knowledge of cellular and molecular mechanism underlying activation of the human immune system. For example, the immune system may recognise the difference between healthy cells and cancer cells in the body and work to eliminate those that become cancerous. Biological therapies use the body's immune system, either directly or indirectly, to fight cancer or to lessen the side effects that may be caused by some cancer treatments. An important goal of such immunotherapy is boosting of killing power of immune system cells by stimulation of appropriate effector cells, such as T-cells.

Cancer "vaccines" are a form of biological therapy. Conventional vaccines for infectious diseases, such as measles, mumps, and tetanus, are effective because they expose the immune system to weakened versions of the disease. This exposure causes the immune system to respond by producing antibodies. Once the immune system has created antibodies, some of the activated immune cells remember the exposure. Therefore, the next time the same antigen enters the body, the immune system can respond more readily to destroy it.

For cancer treatment, researchers are developing vaccines that may encourage the immune system to recognise cancer cells. These vaccines may help the body reject tumours and prevent cancer from recurring. In contrast to vaccines against infectious diseases, cancer vaccines are designed to be injected after the disease is diagnosed, rather than before it develops. By example, it has been shown that immunisation with DCs loaded with appropriate peptides from tumour associated antigens (TAAs) stimulate "tumour specific" T-cells, which in some patients prevent further progression of the disease and eventually lead to regression of the disease. This approach takes advantage of the "professional pathway" of antigen processing/- presentation performed by DCs. In contrast, injection of soluble tumour TAAs or soluble MHC molecules comprising appropriate peptides from TAAs have only proven a limited or no effect, presumably due to low efficacy of antigen stimulation from soluble antigens. The low affinity as well as insufficient stimulation of specific T-cells may explain poor protection obtained by immunisation with soluble peptide/MHC molecule. Indeed, the low intrinsic affinity of essential ligand-receptor interactions has implied limited utilisation of soluble recombinant proteins for stimulation of specific T-cells.

### The present invention

As evident from the above, MHC molecules play a very important role, and thus detection of cells recognising MHC molecules is of major importance and value. Likewise, several attempts have been made to manipulate the immune system in a controllable, efficient and consistent way. However, the success has been limited. Thus, finding substances for immunotherapy that actually works would be a major step forward for the fight against a number of serious diseases for which we do not at present have a cure.

By the present invention, powerful tools are provided. The present invention introduces certain poly-ligand compounds, which will be efficient i.a. in the field of diagnosis and therapy.

The present invention is related to the major undertaking to generate compounds comprising MHC molecules to detect and analyse receptors on MHC recognising cells such as epitope specific T-cell clones or other immune competent effector cells. It is shown herein that the increased valences of compounds of the invention produce surprisingly higher avidity in comparison to oligo-valent complexes (tetramers) known from the prior art. This allows for quantitative analysis of even small cell populations by e.g. flow cytometry.

The tetramers mentioned above are e.g. known from US 5,635,363 by Altman et al. (ref. 10). In US 5,635,363, multimeric binding complexes comprising two or more MHC molecules with peptides attached to a multivalent entity are described. The number of MHC molecules is preferably four, thus, forming a tetramer. The multivalent entity is preferably streptavidin or avidin.

The potential and value of the present invention is obvious, as several reports have demonstrated lack of correlation between T-cell reactivity in peripheral blood and the course of neoplastic diseases (e.g. ref. 11). For instance, analysis of T-cell activity in tumour tissues as well as lymphatic tissues may provide better insights on immunity toward solid tumours.

Combining the growing genome databases of primary protein sequences of humans and parasites with the knowledge of how the immune system handles the molecular information provided by appropriate ligands will lead to new and powerful strategies for development of curative vaccines. This in turn will improve the possibilities for directed and efficient immune manipulations against diseases caused by tumour genesis or infection by pathogenic agent like viruses and bacteria. HIV is an important example. The ability to generate and ligate recombinant MHC molecules or a variety of mixed ligands to carrier molecules as envisaged by the present invention, will enable a novel analytical tool for monitoring immune responses and contribute to a rational platform for novel therapy and "vaccine" applications. Therapeutic compositions ("vaccines") that stimulate specific T-cell proliferation by peptide-specific stimulation is indeed a possibility within the present invention. Thus, quantitative analysis and ligand-based detection of specific T-cells that proliferate by the peptide specific stimulation should be performed simultaneously to monitor the generated response. Effective methods to produce a variety of molecules from the group of highly polymorphic human HLA encoded proteins would lead to advanced analyses of complex immune responses, which may comprise a variety of peptide epitope specific T-cell clones. The high avidity-based flow cytometry and tissue-staining approaches from the state of art technology disclosed herein will add significantly to the development of such advanced analysis of T-cell responses.

### The MHC molecule constructs of the present invention

One of the benefits of the MHC molecule constructs of the present invention is clearly that the MHC molecule constructs overcome low intrinsic affinities of monomer ligands and counter receptors. It should be noted, however, that such MHC molecule constructs may have a large variety of applications that include targeting of high affinity receptors (e.g. hormone peptide receptors for insulin) on target cells. Taken together poly-ligand binding to target cells does have practical, clinical and scientifically uses of immediate commercial interest.

Thus, the present invention provides constructs of MHC molecules, which present multi-valent binding sites for MHC recognising cells. The constructs of the present invention have highly advantageous properties and are an important tool with a broad range of valuable uses.

Thus, in a first aspect, the present invention relates to MHC molecule construct comprising a carrier molecule having attached thereto one or more MHC molecules, said MHC molecules being attached to the carrier molecule either directly or via one or more binding entities.

"One or more" as used everywhere herein is intended to include one and a plurality.

This applies i.a. to the MHC molecule and the binding entity. The carrier molecule may thus have attached thereto a MHC molecule or a plurality of MHC molecules, and/or a binding entity or a plurality of binding entities.

"A plurality" as used everywhere herein should be interpreted as two or more.

This applies i.a. to the MHC molecule and the binding entity.

When a plurality of MHC molecules is attached to the carrier molecule, the number may only be limited by the capacity of the carrier molecule or the binding entity, as the case may be. The number of binding entities may only be limited by the capacity and nature of the carrier molecule.

Depending on the use of the MHC molecule constructs of the present invention, the construct as such can be provided in soluble or non-soluble form by carefully selecting the carrier molecule. Both the soluble and the non-soluble format display the advantageous properties.

As used everywhere herein, the term "a", "an" or "the" is meant to be one or more, i.e. at least one.

"MHC molecule construct" and "MHC constructs" may be used interchangably herein.

By the term "MHC molecule" as used everywhere herein is meant such molecule, which is capable of performing at least one of the functions attributed to said molecule. The term includes both classical and non-classical MHC molecules. The meaning of "classical" and "non-classical" in connection with MHC molecules is well known to the person skilled in the art. Non-classical MHC molecules are subgroups of MHC-like molecules. The terms "MHC molecule", and "MHC" are used interchangeably herein. Thus, term "MHC molecule" is further intended to include MHC Class I molecules, MHC Class II molecules, as well as MHC-like molecules (both Class I and Class II), including the subgroup non-classical MHC Class I and Class II molecules.

A "MHC Class I molecule" as used everywhere herein is defined as a molecule which comprises 1-3 subunits, including a heavy chain, a heavy chain combined with a light chain (β₂m), a heavy chain combined with a light chain (β₂m) through a flexible linker, a heavy chain combined with a peptide, a heavy chain combined with a peptide through a flexible linker, a heavy chain/β₂m dimer combined with a peptide, and a heavy chain/β₂m dimer with a peptide through a flexible linker to the heavy or light chain. The MHC molecule chain may be changed by substitution of single or by cohorts of native amino acids or by inserts, or deletions to enhance or impair the functions attributed to said molecule. By example, it has been shown that substitution of XX with YY in position nn of human β₂m enhance the biochemical stability of MHC Class I molecule complexes and thus may lead to more efficient antigen presentation of subdominant peptide epitopes.

A "MHC Class II molecule" as used everywhere herein is defined as a molecule which comprises 2-3 subunits including an α-chain and a β-chain (α/β-dimer), an α/β dimer with a peptide, and an α/β dimer combined with a peptide through a flexible linker to the a or β chain, an α/β dimer combined through an interaction by affinity tags e.g. jun-fos, an α/β dimer combined through an interaction by affinity tags e.g. jun-fos and further combined with a peptide through a flexible linker to the α or β chain. The MHC molecule chains may be changed by substitution of single or by cohorts of native amino acids or by inserts, or deletions to enhance or impair the functions attributed to said molecule.

MHC Class I like molecules (including non-classical MHC Class I molecules) include CD1d, HLA E, HLA G, HLA F, HLA H, MIC A, MIC B, ULBP-1, ULBP-2, and ULBP-3.

MHC Class II like molecules (including non-classical MHC Class II molecules) include HLA DM, HLA DO, I-A beta2, and I-E beta2.

A "peptide free MHC Class I molecule" as used everywhere herein is meant to be a MHC Class I molecule as defined above with no peptide.

A "peptide free MHC Class II molecule" as used everywhere herein is meant to be a MHC Class II molecule as defined above with no peptide.

Such peptide free MHC Class I and II molecules are also called "empty" MHC Class I and II molecules.

The MHC molecule may suitably be a vertebrate MHC molecule such as a human, a mouse, a rat, a porcine, a bovine or an avian MHC molecule. Such MHC molecules from different species have different names. E.g. in humans, MHC molecules are denoted HLA. The person skilled in the art will readily know the name of the MHC molecules from various species.

In general, the term "MHC molecule" is intended to include alleles. By way of example, in humans e.g. HLA A, HLA B, HLA C, HLA D, HLA E, HLA F, HLA G, HLA H, HLA DR, HLA DQ and HLA DP alleles are of interest, and in the mouse system, H-2 alleles are of interest. Likewise, in the rat system RT1-alleles, in the porcine system SLA-alleles, in the bovine system BoLA, in the avian system e.g. chicken-B alleles, are of interest.

The definition of the MHC molecule construct of the present invention enables various valuable possibilities as regards the MHC molecules. Thus, examples of valuable MHC molecule constructs are such
wherein at least two of the MHC molecules are different, wherein the MHC molecules are the same,
wherein at least two of the peptides harboured by the MHC molecules are different,
wherein the peptides harboured by the MHC molecules are the same,
wherein the peptides harboured by the MHC molecules are chemically modified or synthesised to contain not natural amino acids, or to contain hydrophilic or hydrophobic groups,
wherein the peptides harboured by the MHC Class I molecules are linked to the MHC Class I heavy chain by a flexible linker,
wherein the peptides harboured by the MHC Class I molecules are linked to the MHC Class I light chain (β₂m) by a flexible linker,
wherein the peptides are harboured by MHC Class I molecules comprising MHC Class I heavy chain in association with a light chain (β₂m) by a flexible linker,
wherein the peptide harboured by the MHC Class II molecules are linked to the alpha-chain by a flexible linker,
wherein the peptide harboured by the MHC Class II molecules are linked to the β-chain by a flexible linker, wherein the MHC Class I molecules are mutated,
wherein the MHC Class II molecules are mutated.

The above list is not exhaustive in any way, but outlines a number of valuable possibilities.

In particular, if the peptides harboured by a plurality of MHC molecules are different from each other, such may be used to detect several types of MHC recognising cells simultaneously. This can be achieved either by employing one MHC molecule construct with MHC molecules filled with different peptides, or by employing several MHC molecule constructs, where each MHC molecule construct have MHC molecules with the same type of peptide, e.g. one MHC molecule construct displaying one peptide, and another MHC molecule construct displaying another peptide.

In one embodiment of the MHC molecule constructs of the present invention, the one or more MHC molecules are attached to the carrier molecule directly. In another embodiment, the one or more MHC molecules are attached to the carrier molecule via one or more binding entities.

When the MHC molecules are attached via one or more binding entities, each binding entity suitably has attached thereto from 1 to 10, such as from 1 to 8, from 1 to 6, from 1 to 4, from 1 to 3, or 1 or 2 MHC molecules. However, it is to be understood that the possible number of MHC molecules depends on the binding entity in question (i.e. how many MHC molecules that can be attached). Thus, by choosing the binding entity carefully, it may be possible to attach more than 10 MHC molecules to each binding entity. However, it is to be understood that this number may be the average number of MHC molecules attached to each binding entity. Thus, the number of MHC molecules may be evenly or unevenly distributed on the binding entity as the MHC molecule constructs are most often made and purified with a certain desired weight distribution. Thus, the average number needs not be an integer, but but can be anything between two integers (i.e. a decimal number), e.g. 2.8, 4.7 or 5.3, to mention a few, non-limiting examples.

The total number of MHC molecules of the MHC molecule construct is in principle unlimited. Thus, the total number of MHC molecules of the construct may suitably be at least 4, at least 5, at least 6, at least 7, at least 8, at least 9, at least 10, at least 12, at least 16, at least 20, at least 24, at least 28, at least 32, or at least 64. In particular, the total number of MHC molecules of the construct may be within from 1 to 100, within from 1 to 95, within from 1 to 90, within from 1 to 85, within from 1 to 80, within from 1 to 75, within from 1 to 70, within from 1 to 65, within from 1 to 60, within from 1 to 55, within from 1 to 50, within from 1 to 45, within from 1 to 40, within from 1 to 35, within from 1 to 30, within from 1 to 25, within from 1 to 20, within from 1 to 15, within from 1 to 10, within from 1 to 5, within from 1 to 4, within from 1 to 3, or 1 or 2. It is to be understood that the term "total number" is intended to include MHC molecules attached to the carrier molecule via one or more binding entities as well as MHC molecules attached directly to the carrier molecule. However, it is to be understood that total this number may be the average number of MHC molecules attached. Thus, the number of MHC molecules may be evenly or unevenly distributed among a plurality of MHC molecule constructs. Thus, the average number needs not be an integer, but can be any number between two integers (i.e. a decimal number), e.g. 28.4, 44.5 or 57.2, to mention a few, non-limiting examples.

The binding entity is any such suited for attachment of the MHC molecules, while rendering the MHC molecules capable of binding to MHC recognising cells. Examples of suitable binding entities are streptavidin (SA) and avidin and derivatives thereof, biotin, immunoglobulins, antibodies (monoclonal, polyclonal, and recombinant), antibody fragments and derivatives thereof, leucine zipper domain of AP-1 (jun and fos), hexa-his (metal chelate moiety), hexa-hat GST (glutathione S-tranferase) glutathione affinity, Calmodulin-binding peptide (CBP), Strep-tag, Cellulose Binding Domain, Maltose Binding Protein, S-Peptide Tag, Chitin Binding Tag, Immunoreactive Epitopes, Epitope Tags, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, lectins that mediate binding to a diversity of compounds, including carbohydrates, lipids and proteins, e.g. Con A (*Canavalia ensiformis*) or WGA (wheat germ agglutinin) and tetranectin or Protein A or G (antibody affinity). Combinations of such binding entities are also comprised. Non-limiting examples are streptavidin-biotin and junfos. In particular, when the MHC molecule is tagged, the binding entity may be an "anti-tag". By "anti-tag" is meant an antibody binding to the tag and any other molecule capable of binding to such tag.

The number, density, and nature of the binding entities can vary for each carrier molecule. It is to be understood that the binding entity may be attached to the carrier molecule by a linker. Suitable linkers include Calmodulin-binding peptide (CBP), 6xHIS, Protein A, Protein G, biotin, Avidine, Streptavidine, Strep-tag, Cellulose Binding Domain, Maltose Binding Protein, S-Peptide Tag, Chitin Binding Tag, Immuno-reactive Epitopes, Epitope Tags, GST tagged proteins, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope.

The one or more MHC molecules may suitably be attached to the binding entity by tags. Examples of tags are given above under the difinition of suitable binding entities. Thus, MHC molecules being recombinantly tagged or chemically tagged bind specifically to the binding entity due to high affinity. The recombinant tags of MHC molecules furthermore allow regio-specific attachment sites in the constructs.

The tags may be located at any part of the MHC molecule, but it is presently believed that the tags should preferably be located away from the cell binding part of the MHC molecule.

By way of example, a tagged MHC molecule could be a recombinant MHC fusion molecule consisting of MHC Class I heavy chain molecule and a C-terminal target peptide sequence for enzymatic mono-biotinylation. The C-terminal location of the affinity tag allows optimal exposure of the N-terminal cell binding part of the MHC molecule. It is presently believed that target sequences for biotinylation also may be located at β₂m molecules. Chemically biotinylated MHC protein binds to a streptavidin binding entity. Biotinylated MHC molecule binds to streptavidin with high affinity. The ratio of MHC molecules per streptavidin is theoretically 4:1 due to four biotin-binding sites in streptavidin complexes.

In many applications, it will be advantageous that the MHC molecule construct further comprises one or more biologically active molecules. By the term "biologically active" is meant that the compound may affect the binding characteristics or the effects of the MHC molecule construct. As regards the terms "one or more", "a plurality", "a", "an", and "the", reference is made to the definitions above. Thus, the MHC molecule construct may comprise several biologically active molecules which may be the same or different.

Such biologically active molecules may in particular be selected from proteins, co-stimulatory molecules, cell modulating molecules, receptors, accessory molecules, adhesion molecules, natural ligands, and toxic molecules, as well as antibodies and recombinant binding molecules to any of the foregoing, and combinations thereof.

"Recombinant binding molecules" is intended to mean molecules such as peptide fragment prepared by recombinant technology, and which have the ability to mimic the activity (e.g. up-regulation or down-regulation) of natural molecules, or to inhibit or block the activity of natural molecules.

The biologically active molecule may suitably be attached to the carrier molecule either directly or via one or more of the binding entities.

In particular, the biologically active molecule may be selected from

proteins such as MHC Class I-like proteins like MIC A, MIC B, CD1d, HLA E, HLA F, HLA G, HLA H, ULBP-1, ULBP-2, and ULBP-3,
co-stimulatory molecules such as CD2, CD3, CD4, CD5, CD8, CD9, CD27, CD28, CD30, CD69, CD134 (OX40), CD137 (4-1BB), CD147, CDw150 (SLAM), CD152 (CTLA-4), CD153 (CD30L), CD40L (CD154), NKG2D, ICOS, HVEM, HLA Class II, PD-1, Fas (CD95), FasL expressed on T and/or NK cells, CD40, CD48, CD58, CD70, CD72, B7.1 (CD80), B7.2 (CD86), B7RP-1, B7-H3, PD-L1, PD-L2, CD134L, CD137L, ICOSL, LIGHT expressed on APC and/or tumour cells,
cell modulating molecules such as CD16, NKp30, NKp44, NKp46, NKp80, 2B4, KIR, LIR, CD94/NKG2A, CD94/NKG2C expressed on NK cells, IFN-alpha, IFN-beta, IFN-gamma, IL-1, IL-2, IL-3, IL-4, IL-6, IL-7, IL-8, IL-10, IL-11, IL-12, IL-15, CSFs (colony-stimulating factors), vitamin D3, IL-2 toxins, cyclosporin, FK-506, rapamycin, TGF-beta, clotrimazole, nitrendipine, and charybdotoxin, accessory molecules such as LFA-1, CD11a/18, CD54 (ICAM-1), CD106 (VCAM), and CD49a,b,c,d,e,f/CD29 (VLA-4),
adhesion molecules such as ICAM-1, ICAM-2, GlyCAM-1, CD34, anti-LFA-1, anti-CD44, anti-beta7, chemokines, CXCR4, CCR5, anti-selectin L, anti-selectin E, and anti-selectin P,
toxic molecules selected from toxins, enzymes, antibodies, radioisotopes, chemiluminescent substances, bioluminescent substances, polymers, metal particles, and haptens, such as cyclophosphamide, methrotrexate, Azathioprine, mizoribine, 15-deoxuspergualin, neomycin, staurosporine, genestein, herbimycin A, Pseudomonas exotoxin A, saporin, Rituxan, Ricin, gemtuzumab ozogamicin, Shiga toxin, heavy metals like inorganic and organic mercurials, and FN18-CRM9, radioisotopes such as incorporated isotopes of iodide, cobalt, selenium, tritium, and phosphor, and haptens such as DNP, and digoxiginin,
and combinations of any of the foregoing, as well as antibodies (monoclonal, polyclonal, and recombinant) to the foregoing, where relevant. Antibody derivatives or fragments thereof may also be used.

In order to enable easy detection of the binding of the MHC molecule construct to MHC recognising cells, the construct may be labelled. Thus, in another aspect, the present invention relates to a MHC molecule construct as defined above further comprising one or more labelling compounds. The definition of the terms "one or more", "a plurality", "a", "an", and "the" given above also apply here. A plurality of labelling compounds should everywhere be interpreted as two or more labelling compounds which may be the same or different.

In particular,
one or more labelling compounds may be attached to the carrier molecule, or
one or more labelling compounds may be attached to one or more of the binding entities, or
one or more labelling compounds may be attached to one or more of the MHC molecules, or
one or more labelling compounds may be attached to the carrier molecule and/or one or more of the binding entities and/or one or more of the MHC molecules, or
one or more labelling compounds may be attached to the peptide harboured by the MHC molecule.

In some applications, it may be advantageous to apply different MHC molecule constructs, either as a combination or in individual steps. Such different MHC molecule constructs can be differently labelled (i.e. by labelling with different labelling compounds) enabling visualisation of different target MHC recognising cells. Thus, if several different MHC molecule constructs with different labelling compounds are present, it is possible simultaneously to identify more than one specific receptor, if each of the MHC molecule constructs present a different peptide.

The labelling compound is preferably such which is directly or indirectly detectable.

The labelling compound may be any labelling compound suitable for directly or indirectly detection. By the term "directly" is meant that the labelling compound can be detected per se without the need for a secondary compound, i.e. is a "primary" labelling compound. By the term "indirectly" is meant that the labelling compound can be detected by using one or more "secondary" compounds, i.e. the detection is performed by the detection of the binding of the secondary compound(s) to the primary compound.

The labelling compound may further be attached via a suitable linker. Linkers suitable for attachment to labelling compounds would be readily known by the person skilled in the art.

Examples of such suitable labelling compounds are fluorescent labels, enzyme labels, radioisotopes, chemiluminescent labels, bioluminescent labels, polymers, metal particles, haptens, antibodies, and dyes.

The labelling compound may suitably be selected
from fluorescent labels such as 5-(and 6)-carboxy-fluorescein, 5- or 6-carboxyfluorescein, 6-(fluorescein)-5-(and 6)-carboxamido hexanoic acid, fluorescein isothiocyanate (FITC), rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeston Red, Green fluorescent protein (GFP) and analogues thereof, and conjugates of R-phycoerythrin or allophycoerythrin and e.g. Cy5 or Texas Red, and inorganic fluorescent labels based on semiconductor nanocrystals (like quantum dot and Qdot™ nanocrystals), and time-resolved fluorescent labels based on lanthanides like Eu3+ and Sm3+,
from haptens such as DNP, biotin, and digoxiginin,
from enzymic labels such as horse radish peroxidase (HRP), alkaline phosphatase (AP), beta-galactosidase (GAL), glucose-6-phosphate dehydrogenase, beta-N-acetylglucosaminidase, ß-glucuronidase, invertase, Xanthine Oxidase, firefly luciferase and glucose oxidase (GO),
from luminiscence labels such as luminol, isoluminol, acridinium esters, 1,2-dioxetanes and pyridopyridazines, and
from radioactivity labels such as incorporated isotopes of iodide, cobalt, selenium, tritium, and phosphor.

Radioactive labels may in particular be interesting in connection with labelling of the peptides harboured by the MHC molecules.

As defined above, the MHC molecule constructs of the invention comprise a carrier molecule. The carrier molecule may be a soluble carrier molecule or a not soluble carrier molecule. The carrier molecule may be any such which enables attachment of the MHC molecules, the binding entities, and/or the biologically active compounds, while providing the advantageous properties of the construct. Examples of suitable carrier molecules are
polysaccharides including dextrans, carboxy methyl dextran, dextran polyaldehyde, carboxymethyl dextran lactone, and cyclodextrins,
pullulans, schizophyllan, scleroglucan, xanthan, gellan, O-ethylamino guaran, chitins and chitosans indlucing 6-O-carboxymethyl chitin and N-carboxymethyl chitosan,
derivatised cellolosics including carboxymethyl cellulose, carboxymethyl hydroxyethyl cellulose, hydroxyethyl cellulose, 6-amino-6-deoxy cellulose and O-ethylamine cellulose,
hydroxylated starch, hydroxypropyl starch, hydroxyethyl starch, carrageenans, alginates, and agarose,
synthetic polysaccharides including ficoll and carboxymethylated ficoll,
vinyl polymers including poly(acrylic acid), poly(acryl amides), poly(acrylic esters), poly(2-hydroxy ethyl methacrylate), poly(methyl methacrylate), poly(maleic acid), poly(maleic anhydride), poly(acrylamide), poly(ethyl-co-vinyl acetate), poly(methacrylic acid), poly(vinylalcohol), poly(vinyl alcohol-co-vinyl chloroacetate), aminated poly(vinyl alcohol), and co block polymers thereof,
poly ethylene glycol (PEG) or polypropylene glycol or poly(ethylene oxide-co-propylene oxides) containing polymer backbones including linear, comb-shaped or StarBurst™ dendrimers,
poly amino acids including polylysines, polyglutamic acid, polyurethanes, poly(ethylene imines), pluriol.
proteins including albumins, immunoglobulins, and virus-like proteins (VLP), and
polynucleotides, DNA, PNA, LNA, oligonucleotides and oligonucleotide dendrimer constructs.

Also included in this definition of the carrier molecule is mixed forms, i.e. a carrier molecule composed of one or more of the above examples.

The choice of carrier molecule depends i.a. on the application of the MHC molecule construct. Of course, several parameters can be varied in the above-given examples of carrier molecules, including the length and branching. Furthermore, the carrier molecules may carry various substitutents, including such, which can be protected and/or activated, enabling further derivatisation.

It is to be understood that the MHC molecule construct of the invention may further comprise one or more additional substituents. The definition of the terms "one or more", "a plurality", "a", "an", and "the" also apply here. Such biologically active molecules may be attached to the construct in order to affect the characteristics of the constructs, e.g. with respect to binding properties, effects, MHC molecule specificities, solubility, stability, or detectability. For instance, spacing could be provided between the MHC molecules, one or both chromophores of a Fluorescence Resonance Energy Transfer (FRET) donor/acceptor pair could be inserted, functional groups could be attached, or groups having a biological activity could be attached.

The MHC molecule construct of the invention is preferably provided in soluble form. By "soluble form" is meant that the construct is soluble in a suitable solvent ("solubilising medium"). However, the MHC molecule construct may also be provided in non-soluble form, e.g. dispersable form, in a suitable solvent. By "dispersable" is meant that the MHC molecule construct is dispersed, but solubilised, in the solvent.

Examples of suitable solvents are water and various buffers such as acetate, ammonium sulphate, sodium chloride, CAPS, CHES, immidazole, PIPES, TAPS, TES, triethanolamine, MOPS, MES, HEPES, PBS, carbonate, TRIS, borate containing buffers, as well as mixtures thereof. Other suitable solvents include aqueous mixtures containing ethylene glycol, propylene glycol, NMP, DMSO, or DMF.

Providing the MHC molecule construct in a solubilising medium makes the handling and storage easy. Furthermore, providing the MHC molecule construct in a solubilising medium facilitates the application of the MHC molecule constructs since the constructs can be prepared in a "ready-to-use" format for many applications. Also, when applied in therapy, it may be advantageous that the MHC molecule construct is readily soluble in the body fluid, or is already solubilised prior to administration.

In a number of applications, it may be advantageous immobilise the MHC molecule construct onto a solid or semi-solid support. Such support may be any which is suited for immobilisation, separation etc. Non-limiting examples include particles, beads, biodegradable particles, sheets, gels, filters, membranes (e. g. nylon membranes), fibres, capillaries, needles, microtitre strips, tubes, plates or wells, combs, pipette tips, micro arrays, chips, slides, or indeed any solid surface material. The solid or semi-solid support may be labelled, if this is desired. The support may also have scattering properties or sizes, which enable discrimination among supports of the same nature, e.g. particles of different sizes or scattering properties, colour or intensities.

Conveniently the support may be made of glass, silica, latex, plastic or any polymeric material. The support may also be made from a biodegradable material.

Generally speaking, the nature of the support is not critical and a variety of materials may be used. The surface of support may be hydrophobic or hydrophilic. Preferred are materials presenting a high surface area for binding of the MHC molecule constructs. Such supports may be for example be porous or particulate e.g. particles, beads, fibres, webs, sinters or sieves. Particulate materials like particles and beads are generally preferred due to their greater binding capacity. Particularly polymeric beads and particles may be of interest.

Conveniently, a particulate support (e.g. beads or particles) may be substantially spherical. The size of the particulate support is not critical, but it may for example have a diameter of at least 1 µm and preferably at least 2 µm, and have a maximum diameter of preferably hot more than 10 µm and more preferably not more than 6 µm. For example, particulate supports having diameters of 2.8 µm and 4.5 µm will work well.

An example of a particulate support is monodisperse particles, i.e. such which are substantially uniform in size (e. g. size having a diameter standard deviation of less than 5%). Such have the advantage that they provide very uniform reproducibility of reaction. Monodisperse particles, e.g. made of a polymeric material, produced by the technique described in US 4,336,173 (ref. 25) are especially suitable.

Non-magnetic polymer beads may also be applicable. Such are available from a wide range of manufactures, e.g. Dynal Particles AS, Qiagen, Amersham Biosciences, Serotec, Seradyne, Merck, Nippon Paint, Chemagen, Promega, Prolabo, Polysciences, Agowa, and Bangs Laboratories.

Another example of a suitable support is magnetic beads or particles. The term "magnetic" as used everywhere herein is intended to mean that the support is capable of having a magnetic moment imparted to it when placed in a magnetic field, and thus is displaceable under the action of that magnetic field. In other words, a support comprising magnetic beads or particles may readily be removed by magnetic aggregation, which provides a quick, simple and efficient way of separating out the beads or particles from a solution. Magnetic beads and particles may suitably be paramagnetic or superparamagnetic. Superparamagnetic beads and particles are e.g. described in EP 0 106 873 (Sintef, ref. 26). Magnetic beads and particles are available from several manufacturers, e.g. Dynal Biotech ASA (Oslo, Norway, previously Dynal AS, e.g. Dynabeads^{®}).

The support may suitably have a functionalised surface. Different types of functionalisation include making the surface of the support positively or negatively charged, or hydrophilic or hydrophobic. This applies in particular to beads and particles. Various methods therefore are e.g. described in US 4,336,173 (ref. 25), US 4,459,378 (ref. 27) and US 4,654,267 (ref. 28).

The MHC molecule constructs of the present invention can be attached (immobilised) to the solid or semi-solid support by any method known in the art for attachment (or immobilisation) to supports. In particular, the MHC molecule constructs may be immobilised to the support by way of linkers, spacers or antibodies, or any combination thereof. Examples of suitable linkers include Calmodulin-binding peptide (CBP), 6xHIS, Protein A, Protein G, biotin, Avidine, Streptavidine, Strep-tag, Cellulose Binding Domain, Maltose Binding Protein, S-Peptide Tag, Chitin Binding Tag, Immuno-reactive Epitopes, Epitope Tags, GST tagged proteins, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, "zero length cross-linkers" such as 1-ethyl-3(3-dimethylaminopropyl)-carbodiimide hydrochloride (EDAC), homobifunctional cross-linkers such as glutaric dialdehyde, disuccinimidyl suberate (DSS) dimethyl adipimidate dihydrochloride (DMA), divinylfulfone (DVS), or bismaleimidohexane, and heterobifunctional cross-linkers such as 4-(N-maleimidomethyl)cyclohexane-1-carboxyl hydrazide hydrochloride (M2C2H), succinimidyl-4-(N-maleimidomethyl) (SMCC), N-succinimidyl-3-(2-pyridyldithio) propionate (SPDP), and N-(gamma-maleimidobutyryloxy)succinimide (GMBS). Examples of suitable spacers include multifunctional molecules such as diamino alkanes, dicarboxyls and dihydroxyls. The spacers may additionally include functionalities such as e.g. ethers, amides, and amines. Examples of suitable antibodies (polyclonal, monoclonal, recombinant) include antibodies directed against the carrier molecule and antibodies against the binding entity. It is to be understood that the MHC molecule constructs may be attached covalently or reversibly. By "reversibly" is meant that the attachment may be reversed such that the MHC molecule constructs can be liberated from the support. Examples of possible reversible linkers (e.g. molecules having an inserted amino acid sequence comprising an elastomeric peptide) are described in WO 99/11661 (ref. 29).

By way of example, if the carrier molecule is a dextran molecule, the MHC molecule construct may be immobilised using anti-dextran antibodies. By way of example, a PNA could be attached to the MHC molecule construct, and an anti-PNA antibody could be used for immobilisation.

It is to be understood that several or only one type of support may be applied at the same time. Likewise, a support may have immobilised thereto one or more MHC molecule constructs. As regards the definitions of "one or more", "a plurality", "a", "an", and "the", cf. above. The MHC molecule constructs immobilised onto the support may be the same or different. E.g. on type of MHC molecule construct may be immobilised to one type of support, and another type of MHC molecule construct to another type of support. In principle, the number of different MHC molecule construct is unlimited.

Uses in which the MHC molecule constructs of the invention may suitably be provided in solubilised form include radio immune assay (RIA), cell bound radioactive ligand assay, flow cytometry and ELISA. Such assays are readily known to the person skilled in the art as are the procedures, by which such are carried out.

Uses in which the MHC molecule constructs of the invention may suitably be provided immobilised onto a solid or semi-solid support include flow cytometry, immunomagnetic separation techniques, ex vivo stimulation of cultured cells, aggregation techniques, lateral flow devices, ELISA, RIA and cell bound radio ligand assays.

Thus, the present invention relates in particular to MHC molecule constructs as defined above for use in flow cytometric methods, histochemical methods, and cytochemical methods. Accordingly, the MHC molecule constructs of the invention are suited as detection systems.

### Methods employing the MHC molecule constructs of the invention

The MHC molecule constructs of the invention are a powerful tool in a broad range of in vitro or ex vivo methods.

Thus, the present invention relates to methods for detecting the presence of MHC recognising cells in a sample comprising the steps of
(a) providing a sample suspected of comprising MHC recognising cells,
(b) contacting the sample with a MHC molecule construct as defined above, and
(c) determining any binding of the MHC molecule construct, which binding indicates the presence of MHC recognising cells.

Such methods are a powerful tool in diagnosing various diseases. Establishing a diagnosis is important in several ways. A diagnosis gives information about the disease, thus the patient can be offered a suitable treatment regime. Also, establishing a more specific diagnosis may give important information about a subtype of a disease for which a particular treatment will be beneficial (i.e. various subtypes of diseases may involve display of different peptides which are recognised by MHC recognising cells, and thus treatment can be targeted effectively against a particular subtype). In this way, it may also be possible to gain information about aberrant cells, which emerge through the progress of the disease or condition, or to investigate whether and how T-cell specificity is affected. The binding of the MHC molecule construct makes possible these options, since the binding is indicative for the presence of the MHC recognising cells in the sample, and accordingly the presence of MHC molecules displaying the peptide.

The present invention also relates to methods for monitoring MHC recognising cells comprising the steps of
(a) providing a sample suspected of comprising MHC recognising cells,
(b) contacting the sample with a MHC molecule construct as defined above, and
(c) determining any binding of the MHC molecule construct, thereby monitoring MHC recognising cells.

Such methods are a powerful tool in monitoring the progress of a disease, e.g. to closely follow the effect of a treatment. The method can i.a. be used to manage or control the disease in a better way, to ensure the patient receives the optimum treatment regime, to adjust the treatment, to confirm remission or recurrence, and to ensure the patient is not treated with a medicament which does not cure or alleviate the disease. In this way, it may also be possible to monitor aberrant cells, which emerge through the progress of the disease or condition, or to investigate whether and how T-cell specificity is affected during treatment. The binding of the MHC molecule construct makes possible these options, since the binding is indicative for the presence of the MHC recognising cells in the sample, and accordingly the presence of MHC molecules displaying the peptide.

The present invention also relates to methods for establishing a prognosis of a disease involving MHC recognising cells comprising the steps of
(a) providing a sample suspected of comprising MHC recognising cells,
(b) contacting the sample with a MHC molecule construct as defined above, and
(c) determining any binding of the MHC molecule construct, thereby establishing a prognosis of a disease involving MHC recognising cells.

Such methods are a valuable tool in order to manage diseases, i.a. to ensure the patient is not treated without effect, to ensure the disease is treated in the optimum way, and to predict the chances of survival or cure. In this way, it may also be possible to gain information about aberrant cells, which emerge through the progress of the disease or condition, or to investigate whether and how T-cell specificity is affected, thereby being able to establish a prognosis. The binding of the MHC molecule construct makes possible these options, since the binding is indicative for the presence of the MHC recognising cells in the sample, and accordingly the presence of MHC molecules displaying the peptide.

The present invention also relates to methods for determining the status of a disease involving MHC recognising cells comprising the steps of
(a) providing a sample suspected of comprising MHC recognising cells,
(b) contacting the sample with a MHC molecule construct as defined above, and
(c) determining any binding of the MHC molecule construct, thereby determining the status of a disease involving MHC recognising cells.

Such methods are a valuable tool in managing and controlling various diseases. A disease could, e.g. change from one stage to another, and thus it is important to be able to determine the disease status. In this way, it may also be possible to gain information about aberrant cells which emerge through the progress of the disease or condition, or to investigate whether and how T-cell specificity is affected, thereby determining the status of a disease or condition. The binding of the MHC molecule construct makes possible these options, since the binding is indicative for the presence of the MHC recognising cells in the sample, and accordingly the presence of MHC molecules displaying the peptide.

The present invention also relates to methods for the diagnosis of a disease involving MHC recognising cells comprising the steps of
(a) providing a sample suspected of comprising MHC recognising cells,
(b) contacting the sample with a MHC molecule construct as defined above, and
(c) determining any binding of the MHC molecule construct, thereby diagnosing a disease involving MHC recognising cells.

Such diagnostic methods are a powerful tool in the diagnosis of various diseases. Establishing a diagnosis is important in several ways. A diagnosis gives information about the disease, thus the patient can be offered a suitable treatment regime. Also, establishing a more specific diagnosis may give important information about a subtype of a disease for which a particular treatment will be beneficial (i.e. various subtypes of diseases may involve display of different peptides which are recognised by MHC recognising cells, and thus treatment can be targeted effectively against a particular subtype). Valuable information may also be obtained about aberrant cells emerging through the progress of the disease or condition as well as whether and how T-cell specificity is affected. The binding of the MHC molecule construct makes possible these options, since the binding is indicative for the presence of the MHC recognising cells in the sample, and accordingly the presence of MHC molecules displaying the peptide.

The present invention also relates to methods of correlating cellular morphology with the presence of MHC recognising cells in a sample comprising the steps of
(a) providing a sample suspected of comprising MHC recognising cells,
(b) contacting the sample with a MHC molecule construct as defined above, and
(c) determining any binding of the MHC molecule construct, thereby correlating the binding of the MHC molecule construct with the cellular morphology.

Such methods are especially valuable as applied in the field of histochemical methods, as the binding pattern and distribution of the MHC molecule constructs can be observed directly. In such methods, the sample is treated so as to preserve the morphology of the individual cells of the sample. The information gained is important i.a. in diagnostic procedures as sites affected can be observed directly.

The present invention also relates to methods for determining the effectiveness of a medicament against a disease involving MHC recognising cells comprising the steps of
(a) providing a sample from a subject receiving treatment with a medicament,
(b) contacting the sample with a MHC molecule construct as defined herein, and
(c) determining any binding of the MHC molecule construct, thereby determining the effectiveness of the medicament.

Such methods are a valuable tool in several ways. The methods may be used to determine whether a treatment is effectively combating the disease. The method may also provide information about aberrant cells which emerge through the progress of the disease or condition as well as whether and how T-cell specificity is affected, thereby providing information of the effectiveness of a medicament in question. The binding of the MHC molecule construct makes possible these options, since the binding is indicative for the presence of the MHC recognising cells in the sample, and accordingly the presence of MHC molecules displaying the peptide.

The present invention also relates to methods for manipulating MHC recognising cells populations comprising the steps of
(a) providing a sample comprising MHC recognising cells,
(b) contacting the sample with a MHC molecule construct immobilised onto a solid support as defined above,
(c) isolating the relevant MHC recognising cells, and
(d) expanding such cells to a clinically relevant number, with or without further manipulation.

Such ex vivo methods are a powerful tool to generate antigen-specific, long-lived human effector T-cell populations that, when re-introduced to the subject, enable killing of target cells and has a great potential for use in immunotherapy applications against various types of cancer and infectious diseases.

In the above methods, the term "a MHC molecule construct" is intended to include one or more MHC molecule constructs. Reference is made to the definitions of "a plurality", "a", "an", and "the", and the intended meanings given above.

As used everywhere herein, the term "MHC recognising cells" are intended to mean such which are able to recognise and bind to MHC molecules. The intended meaning of "MHC molecules" is given above. Such MHC recognising cells may also be called MHC recognising cell clones, target cells, target MHC recognising cells, target MHC molecule recognising cells, MHC molecule receptors, MHC receptors, MHC peptide specific receptors, or peptide-specific cells. The term "MHC recognising cells" is intended to include all subsets of normal, abnormal and defect cells, which recognise and bind to the MHC molecule. Actually, it is the receptor on the MHC recognising cell that binds to the MHC molecule.

As described above, in diseases and various conditions, peptides are displayed by means of MHC molecules, which are recognised by the immune system, and cells targeting such MHC molecules are produced (MHC recognising cells). Thus, the presence of such MHC protein recognising cells is a direct indication of the presence of MHC molecules displaying the peptides recognised by the MHC protein recognising cells. The peptides displayed are indicative and may involved in various diseases and conditions.

For instance, such MHC recognising cells may be involved in diseases of inflammatory, auto-immune, allergic, viral, cancerous, infectious, allo- or xenogene (graft versus host and host versus graft) origin.

In particular, the MHC recognising cells may be involved in a chronic inflammatory bowel disease such as Crohn's disease or ulcerative colitis, sclerosis, type I diabetes, rheumatoid arthritis, psoriasis, atopic dermatitis, asthma, malignant melanoma, renal carcinoma, breast cancer, lung cancer, cancer of the uterus, cervical cancer, prostatic cancer, brain cancer, head and neck cancer, leukaemia, cutaneous lymphoma, hepatic carcinoma, colorectal cancer, bladder cancer, rejection-related disease, Graft-versus-host-related disease, or a viral disease associated with hepatitis, AIDS, measles, pox, chicken pox, rubella or herpes.

In one embodiment, the MHC recognising cells are selected from subpopulations of CD3+ T-cells, gamma,delta T-cells, alpha,beta T-cells, CD4+ T-cells, T helper cels, CD8+ T-cells, Suppressor T-cells, CD8+ cytotoxic T-cells, CTLs, NK cells, NKT cells, LAK cells, and MAK.

In the above-described methods, the sample is preferably selected from histological material, cytological material, primary tumours, secondary organ metastasis, fine needle aspirates, spleen tissue, bone marrow specimens, cell smears, exfoliative cytological specimens, touch preparations, oral swabs, laryngeal swabs, vaginal swabs, bronchial lavage, gastric lavage, from the umbilical cord, and from body fluids such as blood (e.g. from a peripheral blood mononuclear cell (PBMC) population isolated from blood or from other blood-derived preparations such as leukopheresis products), from sputum samples, expectorates, and bronchial aspirates. Such samples may be used as they are, or they may be subjected to various purification, decontamination, filtration, or concentration methods, and/or methods to isolate parts of the sample like immunomagnetic separation. The sample or part thereof (sample constituents) may further be treated so as to preserve morphology or arrest the cells of the sample. Such methods for sample treatment are readily known by the person skilled in the art. The term "cells of the sample" as used everywhere herein is intended to mean the sample as such or isolated parts thereof, whether or not various treatments are applied.

The MHC molecule construct employed in the methods of the invention may, as mentioned above, be directly or indirectly labelled so as to facilitate observation of binding. Thus, the MHC molecule construct may suitably be labelled so as to enable observation by inspection in a microscope, by light, by fluorescence, by electron transmission, or by flow cytometry.

It is to be understood that one MHC molecule construct may be employed in the methods as well as several (a plurality of) MHC molecule constructs (i.e. one or more), depending on the information desired. The total number of MHC molecule constructs as well as actual combination of MHC molecules, peptides, optionally biologically active compounds, and optionally labelling compounds are in principle unlimited.

The methods of the invention described above may suitably be such, wherein the sample to be analysed is mounted on a support. The support may suitably be a solid or semi-solid surface. Suitable solid and semi-solid surfaces are readily known in the art, and include glass slides, beads, particles, membranes, filters, filter membranes, polymer slides, polymer membranes, chamber slides, backings, settings, dishes, and petridishes.

Below, a brief discussion of specific procedures for carrying out the methods of the invention is given.

### Cytological and histological methods

The methods described herein may be performed as cytological and histological methods (sample-mounted methods).

By the term "mounted" is meant placed on or attached to a substantially planar support. Included is placing the tissue or cell sample on a support, e.g. for viewing on a microscope slide. The sample can be attached to further prevent it from falling or sliding off during handling of the support. The method of attachment to the support includes relying on the physical, capillary attraction, adhesives and chemically binding. The sample may be fixed or not fixed.

As mentioned above, the sample may be purified or concentrated, or cells may be isolated prior to analysis. The sample may also be embedded into paraffin and sectioned prior to analysis. Such procedures are readily known to the person skilled in the art.

In particular, the support may be a glass slide, a membrane, a filter, a polymer slide, a chamber slide, a dish, or a petridish.

The sample or parts thereof may suitably be grown or cultured directly on the support prior to analysis. Examples of suitable culture media includes culture media of biological origin such as blood serum or tissue extract; chemically defined synthetic media; or mixtures thereof. Cell cultures are usually grown either as single layers of cells on e.g. a glass or plastic surface, in flasks or on chamber slides, or as a suspension in a liquid or semisolid medium. The cells can be transferred to and mounted onto a more suitable support, e.g. a glass slide. If grown on a chamber slide, which is suitable for e.g. viewing in a microscope, the cells can potentially remain on the support.

However, the cells need not be grown or cultured prior to analysis. Often the sample will be analysed directly without culturing. It is to be understood that samples for direct analysis may undergo the processing procedures described above.

Thus, the sample may, either directly or after having undergone one or more processing steps, be analysed in primarily two major types of methods, in situ methods (in situ analyses) and in vitro methods (in vitro analyses).

In this context, in situ methods (in situ analyses) are to be understood as assays, in which the morphology of the sample cells is essentially preserved. By "essentially preserved" is meant that the overall morphology is preserved, making it possible to identify some or all of the structural compositions of the tissue or cells. Examples are analysis of smears, biopsies, touch preparations and spreading of the sample onto the support. Samples may be subjected to i.a. fixation, permeabilisation, or other processing steps prior to analysis.

In vitro methods are to be understood as methods, in which the overall morphology is not preserved. In the case of in vitro methods, the sample is subjected to a treatment, which disrupts the morphology of the cell structure. Such treatments are known to the person skilled in the art and include treatment with organic solvents, treatment with strong chaotropic reagents such as high concentrations of guanidine thiocyanate, enzyme treatment, detergent treatment, bead beating, heat treatment, sonication and/or application of a French press.

Histological and cytological materials include biopsies and other tissue samples. In general, cytology is the study of the structure of all normal and abnormal components of cells and the changes, movements, and transformations of such components. Cytology disciplines include cytogenics, cytochemistry, and microscopic anatomy. Cells are studied directly in the living state or are killed (fixed) and prepared by e.g. embedding, sectioning, or staining for investigation in bright field or electron microscopes.

One well-known cytology procedure is the Papanicolaou test medical procedure used to detect cancer of the uterine cervix. A scraping, brushing, or smear, is taken from the surface of the vagina or cervix and is prepared on a slide and stained for microscopic examination and cytological analysis. The appearance of the cells determines whether they are normal, suspicious, or cancerous.

By histology is generally understood the study of groups of specialised cells called tissues that are found in most multi-cellular plants and animals.

Histologists study the organisation of tissues at all levels, from the whole organ down to the molecular components of cells. Animal tissues are for example classified as epithelium, connective, muscle and nerve tissue. Blood and lymph are sometimes commonly classified separately as vascular tissue.

These tissue types are combined in different ways in the organism to form characteristic organs. The way cells are connected and organised is sometimes called the morphology of the tissue and gives valuable information about the state of the cells and the tissue.

A variety of techniques are used for histological studies, including tissue culture, use of various fixatives and stains, the use of a microtome for preparing thin sections, light microscopy, electron microscopy, and X-ray diffraction. The histology field also includes histochemistry, which is the study of the chemical composition of tissue structures.

Histological investigation includes study of tissue death and regeneration and the reaction of tissue to injury or invading organisms. Because normal tissue has a characteristic appearance, histologic examination is often utilised to identify diseased tissue.

The term "morphology" is used with regard to both individual cells and tissues.

There are in general, two categories of histological materials. The most common is a fixed, paraffin-embedded tissue specimen, often archive material. These specimens are fixed, usually using a formalin-based fixative, dehydrated to xylene, embedded in paraffin or plastic (e.g. Epon, Araldite, Lowicryl, LR White or polyacrylamide), sectioned onto a slide, deparaffinised or otherwise treated, re-hydrated, and stained.

The second category includes preparations, which are fresh tissues and/or cells, which generally are not fixed with aldehyde-based fixatives. Such specimens are either placed directly on a slide or cover slip, or frozen and sectioned onto slides. Such specimens are then fixed, usually with an alcohol- or acetone-based fixative, and stained. These specimens commonly include biopsy materials, which may be analysed while the surgical procedure is in progress (frozen sections), cytological preparations (including e. g. touch preparations and blood smears), and tissues, which are to be histochemically analysed.

The method of viewing the stained specimens includes bright field microscopes or scanners, fluorescent microscopes or scanners, transmission electron microscope (TEM) or scanning electron microscope (SEM).

Immunostaining requires a series of treatment steps conducted on a tissue section mounted on a slide to highlight by selective staining certain morphological indicators of disease states. Typical steps include pre-treatment of the tissue section to reduce non-specific binding, contacting with specific reagent, and various visualisation techniques, optionally separated by washing steps. Counterstaining with e.g. hematoxylin, Ehrlich staining, Sirius red, Methyl green, methylene blue, and the like, may also be applied. Incubations at room temperature or at slightly elevated temperatures, usually around 40°C, may be applied, and the tissue must be continuously protected from dehydration.

In the following, some of the individual steps in a staining procedure are described.

Fixatives are needed to preserve cells and tissues in a reproducible and life-like manner. To achieve this, tissue blocks, sections, or smears are immersed in a fixative fluid, or in the case of smears, are dried. Fixatives stabilise cells and tissues thereby protecting them from the rigors of processing and staining techniques.

Types of fixative include formalin (aqueous formaldehyde) and neutral buffered formalin (NBF) is among the most commonly used. Other fixatives include glutaraldehyd, acrolein, carbodiimide, imidates, benzoequinone, osmic acid and osmium tetraoxide.

Fresh biopsy specimens, cytological preparations (including touch preparations and blood smears), frozen sections and tissues for immunohistochemical analysis are commonly fixed in organic solvents, including ethanol, methanol and/or acetone.

The methods for attaching or mounting sections to slides include using clean slides and relying on the capillary attraction and no adhesive. Other techniques include glues like egg-white glycerine, glycerine-gelatine mixtures, polyvinyl acetate glue, chrome-alum gelatine and poly lysine coating. Heating or "burning" of the section as a means of facilitating mounting of the section should be used with caution, as the tissue can be destroyed.

To facilitate the specific recognition in fixed tissue, it is often necessary to retrieve or unmask the targets through pre-treatment of the specimens to increase reactivity of the majority of targets.

Target retrieval includes a variety of methods by which the availability of the target for interaction with a specific detection reagent is maximised. The most common techniques are enzymatic digestion with a proteolytic enzyme (e.g. Protinease, pronase, pepsin, papain, trypsin or neuraminidase) in an appropriate buffer or heat induced epitope retrieval (HIER) using microwave irradiation, heating in a regular oven, autoclaving or pressure-cooking in an appropriately pH stabilised buffer, usually containing EDTA, Tris-HCl, citrate, urea, glycin-HCl or boric acid.

The penetration of reagents through the tissue section may be increased using detergents during pre-treatment of sections or cytological preparations, or as additives to dilution media and rinsing buffers.

Additionally, the signal-to-noise ratio may be increased by different physical methods, including application of vacuum and ultrasound, or freezing and thawing of the sections before or during incubation of the reagents.

Endogenous biotin binding sites or endogenous enzyme activity (e.g. phosphatase, catalase or peroxidase) can be removed as a step in the staining procedure.

Similarly, blocking of unspecific binding sites with inert proteins like, HSA, BSA, ovalbumine, fetal calf serum or other sera, or detergents like Tween20, Triton X-100, Saponin, Brij or Pluronics is widely used. Blocking unspecific binding sites in the tissue or cells with unlabelled and target non-specific versions of the specific reagents.

The standard visualisation techniques utilised in immunocytochemistry may not be used directly for staining of the receptors, as the binding relies on the low binding strength of MHC molecules and not the high avidity antibodies or DNA probes normally used. Also, the polymorph and somewhat sensitive nature of the MHC molecule distinguishes it from e.g. the monoclonal antibodies used in immunocytochemistry. On the other hand, in order to be of practical use, specific receptor staining procedures and methods used should resemble current methods.

The present invention surprisingly makes it possible to stain specific receptors using a methodology which resembles routine immunocytochemistry procedures.

For a general introduction to different Immunocytochemistry visualization techniques, see e.g. Lars-Inge Larsson (ref. 23).

The most commonly used detection methods in immuno-histochemistry are direct visualisation of fluorescence or gold particles and enzyme mediated colorimetric detection.

For direct fluorescent studies, the labels can e.g. be 5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetra-methylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeston Red, Green fluorescent protein (GFP) and analogues thereof, and conjugates of R-phycoerythrin or allophycoerythrin and e.g. Cy5 or Texas Red, and and inorganic fluorescent labels based on semiconductor nanocrystals (like quantum dot and Qdot™ nanocrystals), and time-resolved fluorescent labels based on lanthanides like Eu3+ and Sm3+.

Colloidal gold or silver can be used as direct labels for immunocytochemical studies for electron microscopy and light microscopy. Amplification of the signal can be obtained by further silver enhancement of the colloidal gold particles.

The general enzymatic methods use labelled avidin or streptavidin-biotin (LAB), avidin or streptavidin-biotin complex (ABC), enzyme anti-enzyme complex (e.g. PAP and APAAP), direct dextran polymer based antibody-enzyme complex (e.g. DAKO's EPOS); indirect dextran polymer based antibody-enzyme complex (e.g. DAKO's Envision) or double bridge enzyme anti-enzyme complex.

The enzymatic staining uses enzymatic labels such as horse radish peroxidase (HRP), alkaline phosphatase (AP), beta-galactosidase (GAL), glucose-6-phosphate dehydrogenase, beta-N-acetylglucosaminidase, invertase, Xanthine Oxidase, firefly luciferase and glucose oxidase (GO).

Examples of commonly used substrates for horse radish peroxidase include 3,3'-diaminobenzidine (DAB), diaminobenzidine with nickel enhancement, 3-amino-9-ethylcarbazole (AEC), Benzidine dihydrochloride (BDHC), Hanker-Yates reagent (HYR), Indophane blue (IB), tetramethylbenzidine (TMB), 4-chloro-1-naphtol (CN), α-naphtol pyronin (α-NP), o-dianisidine (OD), 5-bromo-4-chloro-3-indol-ylphosphate (BCIP), Nitro blue tetrazolium (NBT), 2-(p-iodophenyl)-3-p-nitrophenyl-5-phenyl tetrazolium chloride (INT), tetranitro blue tetrazolium (TNBT), 5-bromo-4-chloro-3-indoxyl-beta-D-galactoside/ferro-ferricyanide (BCIG/FF).

Examples of commonly used substrates for Alkaline Phosphatase include Naphthol-AS-B1-phosphate/fast red TR (NABP/FR), Naphthol-AS-MX-phosphate/fast red TR(NAMP/FR), Naphthol-AS-B1-phosphate/fast red TR (NABP/FR), Naphthol-AS-MX-phosphate/fast red TR (NAMP/FR), Naphthol-AS-B1-phosphate/new fuschin (NABP/NF), bromochloroindolyl phosphate/nitroblue tetrazolium (BCIP/NBT), 5-Bromo-4-chloro-3-indolyl-b-d-galactopyranoside (BCIG).

One of the most potent detection systems is the catalysed reporter deposition (CARD); this amplification method is based on the deposition of labelled tyramide on tissue through the enzymatic action of HRP. After HRP-immunostaining, labelled tyramide is applied and bound near the site of HRP-activity. The bound and labelled tyramide is then visualised by traditional fluorescence or colorimetric enzyme mediated detection.

Automated staining systems have been introduced to reduce cost, increase uniformity of slide preparation, reduce laborious routine work and most significantly reduce procedural human errors.

The current automated systems can handle any immunochemical assay including assays relying on immunofluorescence, indirect immunoassay procedures, enzyme or gold staining methods. They perform all steps of the immunohistochemical assay irrespective of complexity or their order, at the prescribed time and temperature.

Immunocytochemistry techniques have traditionally used specific antibodies for identification and visualisation of specific antigens. The technique is complex, many steps and molecules with high affinities for specific staining are needed.

By the present invention, immunocytochemistry techniques have been improved to allow identification of the minute quantity of delicate receptors, which is not based on antibody-antigen interactions.

Staining of MHC peptide specific receptors in tissue mounted on e.g. slides will be a very potent diagnostic tool, enabling identification of MHC peptide specific receptors, optionally combined with morphological information, if desired.

By further combining morphological information with double staining of specific cells and specific receptors, additional useful diagnostic information can be obtained.

### Flow cytometric method

The MHC molecule constructs of the invention are suitably used as labelled reagents to identify MHC recognising cells by flow cytometry. This further allows for the analysis of additional surface markers like e.g. antibody epitopes expressed by CD8, CD4, CD3, CD94/NKG2-A/C and KIRs.

A further advantage of the MHC molecule constructs of the invention is that by coupling flow cytometric analysis with high-speed cell sorting, functional assays can be performed on sorted cells without the need for in vitro expansion of the cells to be analysed.

In the flow cytometer, different cells can be identified by their distinct cell morphology like density, shape and size. Tissue morphology as such is not visible from the data obtained from flow cytometer as the cells are broken up.

Flow cytometry is a system for measuring cells, beads or particles as they move in a liquid stream, in the so-called flow cell, through a laser or light beam past a sensing area. The relative light scattering and colour discriminated fluorescence of the particles is measured.

A flow cytometer consists in general of a light source, flow cell, optics to focus light of different colours onto a detector, signal amplifier and processor and a computer to record and analyse data.

Lasers are used as the preferred light source in modern flow cytometers. The most common laser used is the argonion laser. This produces a major line at 488 nm, which gives a source of blue light for excitation of e.g. fluorescein, phycoerythrin, and tandem conjugates and for propium iodide used in DNA measurements. In the flow cell, cells are aligned by hydrodynamic focusing, so that they pass through the laser beams one at a time.

Light scatter is utilised to identify the cell or particle population of interest, while the measurement of fluorescence intensity provides specific information about individual cells.

Individual cells held in the stream of fluid are passed through one or more laser beams. The cells scatter the laser light, which at the same time make fluorescent dyes emit light at various frequencies. Photomultiplier tubes (PMT) convert light to electrical signals and cell data is collected.

What makes flow cytometry such a powerful technique is its ability to measure several parameters on many thousands of individual cells in a very short time, by measurement of their fluorescence and the way in which they scatter light. As an example, using blue light for excitation, it is possible to measure red, green and orange fluorescence and the amount of light scattered, both forward and at right angles to the beam, on each cell in a population of thousands.

Many instruments can measure at least five different parameters. As all the parameters cannot be combined for display simultaneously in a correlated fashion, a system called gating is employed. Regions of interest - or "Gates"- are defined, enabling selection of specific cell populations for display of further parameters. A flow cytometer can be used to analyse sub-populations of cells, which have been fluorescently labelled, with speed and accuracy. Sorting on the basis of other features, e.g. size, is also possible.

Flow cytometry instruments simultaneously generate three types of data: 1) Forward scatter (FSc) gives the approximate cell or particle size, 2) Side or Orthogonal scatter (SSc) gives the cell or particle complexity or granularity, and 3) fluorescent labelling is used to investigate e.g. cell structure and function.

Forward and side scatter are used for preliminary identification of cells. In a peripheral blood sample, for example, lymphocyte, monocyte, and granulocyte populations can be defined on the basis of forward and side scatter. Forward and side scatter are used to exclude debris and dead cells. Particles, for example, can be identified by their size and/or their fluorescence.

Cell or particle populations may be represented on single or dual parameter histograms. Light scatter and fluorescence signals may be analysed after linear or logarithmic amplification. Once the population of cells or particles to be analysed has been identified, the fluorescence associated with bound antibodies or dyes is determined after the background fluorescence has been established.

Some flow cytometers are able to physically sort cells or particles into specific populations. This is most commonly done by electrostatic deflection of charged droplets containing a cell. The flow cell is vibrated and causes the liquid stream to break up into small droplets as it leaves the exit nozzle. At the moment a cell or particle of interest is inside the droplet currently being formed, the flow cell is charged - thus charging the droplet. The stream of droplets then passes through a pair of electrically charged plates, and droplets that are charged (containing the cells or particles of interest) are deflected into a collection vessel.

The electric field created between the plates can direct the cells or particles towards one of several user-specified collection receptacles. Uncharged droplets flow into a waste vessel.

Analysis of concentrations of cells or subsets of cells, often referred to as "absolute counting", can be of further interest for medical diagnostics or monitoring the status of cells in cell cultures or other biotechnological processes.

The flow cytometer is able to rapidly screen large numbers of cells far beyond the capacity of traditional pathological or cytological methods. The information obtained aids in the diagnosis, classification, and prognosis of a variety of diseases.

The applications to which flow cytometry can be applied have expanded rapidly from cell sorting, to measurement of cell surface antigens, and analysis of DNA to aid the interpretation of malignant disorders.

Common uses for flow cytometry in the routine clinical laboratory include immunophenotyping of hematopoietic neoplasms, immune status evaluation, especially quantificaation of CD4+ T-cells in HIV positive patients, and DNA cell cycle analysis of solid tumours.

Different cell populations that compose the hematopoietic system express distinctly different cell surface antigens at various stages of maturation. By detecting and measuring these expressed antigens, flow cytometry can aid in the classification of the cell lineage of leukaemia and lymphoma.

Although not intended to be an independent diagnostic modality, flow cytometry is often able to sub-classify haematopoietic malignancies beyond the capabilities of traditional morphologic and cytochemical techniques.

The most common routine uses of flow cytometry have been measurement of surface antigens (markers) by immunofluorescent labelling using monoclonal antibodies. The markers commonly used are total B-cells, total T-cells and subsets of T-cells. The markers for total T-cells, Helper T-cells and suppressor T-cells have been assigned the cluster differentiation (CD) categories of CD3, CD4, and CD8, respectively. This spectrum of markers, of which there are more than 45 in all, are used for clinical classification of immunodeficiency states, lymphoid leukaemias, autoimmune diseases and for monitoring their response to therapy.

For example, CD4 and CD8 measurements are especially useful to monitor the progression of AIDS, as the CD4+ cells are depleted by infection by HIV, whereas the CD8+ cells persist. The absolute number of CD4+ cells is also a marker of progression of HIV infection to more overt AIDS. The CD4/CD8 ratio can also be used to assess the success of immunosuppressive therapy with cyclosporin A in transplant patients.

For immune status evaluation, typically sub-populations of lymphocytes are identified and quantified by the flow cytometer by utilising monoclonal antibodies to various cell surface antigens. Patients with acquired or congenital immunodeficiency disease and patients on immunosuppressive drug therapy exhibit characteristic alterations in lymphocyte populations.

The typical direct staining procedure for flow cytometry may include one or several of the following steps besides washing and mixing steps:

Fixation of the cells with e.g. buffered formaldehyde, permeabilisation, addition of fluorescently labelled target specific reagent, incubation, centrifugation, aspiration of the supernatant from the cell pellet, resuspension, dilution and analysis on flow cytometer.

Several examples on flow cytometry based detection and quantitative analysis of proliferating immune subpopulations of in vitro expanded T-cells in blood samples from patients have been reported. Well-known examples on antigenic TAA peptides recognised by T-cell that have been monitored in patients undergoing tumour specific immune therapy are MART-1 (27-35), gp100 (154-162), and NY-ESO (157-165). Other interesting MHC molecules include HLA A, HLA B, HLA C, H-2, DR-alleles and HLA E to detect a variety of receptors with low intrinsic affinities e.g. peptide specific TCRs and NK receptors like CD94/NKG2-A/C and KIRs.

The interaction between peptide/MHC molecule and the specific counter receptor is driven by a relatively high affinity, which is essential for the staining function. Thus, low affinity MHC recognising cell clones, interacting with sub-dominant peptide/MHC molecule complexes, may potentially "escape" analysis by flow cytometry. This has indeed been observed in the case of the prior art tetramers in flow cytometric procedures. However, by the construct of the present invention, this disadvantage in flow cytometric procedures is eliminated.

The poly-ligand MHC molecule constructs of the invention bind more tightly to receptors of specific MHC recognising cells as compared to the prior art tetramers, which is needed for reliable flow cytometric procedures. The constructs of the invention are therefore in particular useful for flow cytometric analysis of even subtle subpopulations of MHC recognising cells. The increased binding avidity of MHC molecule constructs of the invention allows detection of MHC recognising cells expressing low affinity receptors. The augmented interactions also allow detection of even very small MHC recognising cell populations in blood samples without the need for in vitro expansion. It is therefore envisioned that MHC molecule constructs of the invention are useful for direct monitoring by flow cytometry of all types of MHC recognising cells in blood samples.

The poly-ligand MHC molecule constructs of the invention also allow better separation of specific and unspecific MHC recognising cells and, thus, augment utilisation of fast flow cell sorting of antigen specific MHC recognising cells.

### Other techniques

Also, it is believed that the MHC molecule constructs of the invention may suitably be applied in the so-call "free-floating" techniques.

In staining procedures using the so-called "free floating techniques", a tissue section is brought into contact with different reagents and wash buffers in suspension or freely floating in appropriate containers, e.g. micro centrifuge tubes.

The tissue sections can be transferred from tube to tube with different reagents and buffers during the staining procedure using e.g. a "fishing hook like" device, a spatula or a glass ring.

The different reagents and buffer can also be changed by gentle decantation or vacuum suction. Alternatively, containers with the tissue sections can be emptied into a special staining net, like the Corning "Netwells" and the tissue section washed before being transferred back into the tube for the next staining step.

All the individual staining procedure steps, including e.g. fixation, antigen retrieval, washing, incubation with blocking reagents, immuno-specific reagents and e.g. the enzymatic catalysed development of the coloured stains, are done while the tissue section is floating freely or withheld on nets. After development of the stain, the tissue section is mounted on slides, dried, before being counterstained and cover slipped before being analysed in e.g. a microscope.

Occasionally, the tissue section is mounted on slides following the critical incubation with the immuno-specific reagents. The rest of the staining process is then conducted on the slide mounted tissue sections.

The free-floating method has been used mainly on thick tissue sections. It is important that sections never dry out during the staining process.

Advantages of the free-floating method include even and good penetration of the immunohistochemical staining reagents. The free-floating method allows for high concentrations of reagents and good mixing.

### Compositions comprising MHC molecule constructs

Compositions (kits) comprising MHC molecule constructs are also an important embodiment of the present invention. Such compositions may be formulated in a way making them ready-to-use in hospitals and laboratories. They may also be formulated so as to enable the user to modify or use as desired.

It is to be understood that the composition may include one MHC molecule construct or several MHC molecule constructs, depending on the intended use. The total number of MHC molecule constructs as well as actual combination of MHC molecules and peptides are in principle unlimited.

Thus, the present invention relates to compositions comprising a MHC molecule construct as defined above, and optionally other components such as buffers and/or visualisation means. The MHC molecules of the MHC molecule construct may be peptide filled or peptide free MHC molecules as defined above, or a mixture thereof. The MHC molecule construct and optionally other components may be provided in separate containers or in the same container.

As used throughout the present context, the terms "one or more", "a plurality", "a", "an", and "the" have the meaning indicated above.

In one embodiment, the composition of the invention comprises a MHC molecule construct as defined above in a solubilising medium. The composition may be such, wherein the MHC molecule construct comprises peptide filled MHC molecules, or such, wherein the MHC molecule construct comprises peptide free MHC molecules. In the latter case, the composition may be such, wherein peptides to fill the peptide free MHC molecules, and the MHC molecule construct comprising peptide free MHC molecules are provided separately.

In another embodiment, the composition of the invention comprises a MHC molecule construct as defined above, wherein the MHC molecule construct is immobilised onto a solid or semi-solid support. Suitable solid and semi-solid supports are indicated above. The composition may be such, wherein the MHC molecule construct comprises peptide filled MHC molecules, or such, wherein the MHC molecule construct comprises peptide free MHC molecules. In the latter case, the composition may be such, wherein peptides to fill the peptide free MHC molecules are provided separately.

In particular, the MHC molecule constructs may be provided in a form, wherein the MHC molecules are filled with low affinity peptides. Thus, it may be possible to exchange these low affinity peptides with higher affinity peptides for a particular use. This application may particularly be valuable when providing the compositions (kits). Filling the MHC molecules with low affinity peptides has the advantage of stabilising the MHC molecules, while providing the benefits of peptide free MHC molecules.

In the following, the production of MHC molecules, peptides, and MHC molecule constructs is described.

### Production of MHC molecules, peptides and MHC molecule constructs

Herein the production of MHC molecules and i.a. their usage for well-defined MHC molecules organised as poly-ligand compounds on a carrier molecule (MHC molecule constructs) to achieve specific binding of the MHC molecule to immune competent target cells (MHC recognising cells) expressing appropriate T-cell receptors and NK cell receptors are described.

### Production of MHC molecule

Some MHC molecules have proven very difficult to obtain from natural sources i.e. eukaryotic cells as they are contaminated or pre-occupied with undesired peptides during the cellular biosynthesis.

Recent technological progress allows production of peptide empty but functional MHC Class I as well as MHC Class II using appropriate cDNA ligated into a bacterial expression vector. A recently developed in vitro folding procedure Oxidized Protein Folding (OPF) allows production of well-defined MHC Class I molecules, cf. WO 2000/15665 (ref. 31). The method may be of use in any protein production scheme (be it in prokaryotes or eucaryotes) where the protein (e.g. inclusion bodies) at some point during the production is solvated in chaotrophic (e.g. urea) at conditions that do not disrupt established and appropriate disulphide bonds. Briefly, the OPF method takes advantage of pre-formed disulphide bonds that guide the denatured MHC molecule through an efficient and fast folding pathway in buffers with appropriate conditions (like pH, salinity). By example, peptide empty and relatively stable MHC Class I molecule is instantly formed by dilution of denatured heavy chain molecule with appropriate disulphide bonds in a buffer containing excess of functional β₂m. This intermediate state of de *novo* folded MHC Class I heavy chain is strictly controlled by the presence of β₂m. Subsequent addition of peptide induces molecular changes in the heavy chain molecule and lead to formation of stable and functional MHC Class I molecules. Thus, the OPF method allows production of MHC Class I molecule in two distinct forms, namely a) as a peptide filled molecule, which is extremely stable and T-cell binding, and b) as a partially mature, peptide free molecule ("empty" MHC molecules), which is reasonably stable and readily peptide receptive. In comparison, conventional folding of bacterial produced MHC molecule requires presence of both β₂m and peptide and leads, consequently, only to stable peptide filled MHC molecules.

Oxidised states of e.g. MHC Class I subunits can, only, be obtained by biochemical purification, e.g. size exclusion and ion-exchange chromatography of individual subunits from urea solubilised bacterial inclusion bodies or from denatured MHC Class I molecules produced in eukaryotic cells e.g. CHO cells.

The MHC molecules can also be generated by recombinant technology to obtain well defined and highly purified components tagged with an appropriate moiety (e.g. a biotinylation site) for ligation to the carrier molecule via a binding entity, like e.g. streptavidin. MHC molecules and MHC-like molecules are only obtained with difficulty from natural sources, as they are loaded with many different peptides in intracellular compartments during biosynthesis. Efficient methods for production of MHC molecules are also prerequisites to overcome the extreme polymorphism of the MHC locus. In the human population more than 400 different HLA A, HLA B and HLA C alleles exist, and more that 200 HLA D alleles exist. This molecular diversity has as stated above an immunological purpose, but is a practical obstacle to MHC production because many different MHC molecules need be generated and individually optimised, validated, characterised, stored etc. Recombinant MHC molecules that present well-defined peptides can, however, be obtained with high efficacy by in vitro folding of denatured and pre-oxidised subunits (i.e. heavy and light β₂m of MHC Class I molecules, and α, β chains of MHC Class II molecules) from MHC molecules that have been produced in bacteria or eukaryotic cells.

MHC molecules can be obtained by cloning of cDNA encoding the various molecules of interest following standard procedures, e.g. as described in Molecular Cloning (Sambrook, Fritsch and Maniatis, Cold Spring Harbor Press, 1989, ref. 13). Briefly, cDNA is synthesised from appropriate cell lines using commercial cDNA synthesis kits (in casu from Pharmacia). For instance, in the case of human cells, the cells can be derived from the panel of HLA expressing EBV transformed human B-cell lines from the 12 International Histocompatibility Workshop Cell Lines Panel Database ("HLA: Genetic diversity of HLA. Functional and Medical Implication", Ed. Dominique Charron, EDK Press, 1997 (ref. 12). E.g. in the case of HLA A*0201, an appropriate cell line would be the IHW 9012. The nucleotide sequence corresponding to a desired MHC (HLA) molecule can be found at public available databases. Using the appropriate sequence information oligonucleotide primers can be designed to amplify by the PCR reaction the coding region encompassing of the relevant mature MHC (HLA) molecule from the appropriate cDNA. The relevant forward and backward primer set for the purpose of amplifying is inserted into the NcoI and HindIII restriction sites of an appropriate expression vector. Suitable expression vectors are e.g. obtainable from Novagen (Novagen, Inc, Madison, WI, USA).

### Peptides associated with MHC Class I and MHC Class II molecules

The peptides (or peptide antigens) to fill the MHC molecules may be any length. The peptides should be at least 8-10 amino acid residues long when associated to MHC Class I molecules. The length of the peptides associated to MHC Class II molecules are usually longer than the peptides associated to MHC Class I molecules and may be e.g. as much as 50 amino acid residues, however, usually less than about 20 amino acid residues such as less than about 17 amino acid residues. However, it is to be understood that the above-indicated lengths are by way of example, and should, thus, not be limiting.

Since antigenic molecules or tissues are known for a number of immunopathologies, suitable peptides can be selected using this information. By way of example, a panel of antigenic peptides from tumour-associated antigens recognised by specific cytotoxic T-cells have been identified.

The amino acid composition can also be obtained by iterative procedures or by molecular modelling. The rapid and reliable identification of MHC Class I- and Class II-restricted T-cell epitopes is essential in various fields of medical research including the definition of new tumour antigens, auto-antigens or with respect to infectious diseases. A prerequisite therefore is the exact knowledge about the molecular interactions within the MHC-peptide-TCR complex. By means of synthetic combinatorial peptide libraries a large number of MHC peptide binding motifs have been revealed with in the last ten years. A common feature of MHC peptide binding motifs is the presence of anchor residues of the peptide and pockets of the binding site, which control the strength of peptide binding to the MHC molecule. More than 500 different MHC molecules have been found, each of them comprising different peptide binding motifs. From existing biodatabases containing information of binding motifs, a number of algorithms have developed to predict MHC binding motifs deduced from known sequences of antigens. One well-known example is the DATABASE OF MHC LIGANDS AND PEPTIDE MOTIFS "SYFPEITHI", a database comprising approximately 2000 peptide sequences known to bind Class I and Class II MHC molecules. The entries are compiled from published reports. The databases provide a strong tool for identification of MHC binding motifs in molecules involved in a variety of infections and cellular transformations. For example HIV/SIV antigens and tumour-associated antigens have been identified. From the known sequences of these antigens a number of MHC binding motifs have been predicted and subsequently verified by peptide binding analyses. Similar deductions of MHC binding peptides are available for a variety of disease-associated antigens in e.g. cancer, malaria and tuberculosis.

More recent approaches to improve prediction of suitable MHC Class I peptide epitopes are based on knowledge to digest patterns of proteosomes that generate the MHC Class I bound peptides in ER. By example, the NetChop WWW server produces neural network predictions for cleavage sites of the human proteasome (http://www.cbs.dtu.dk/-services/NetChop/). Since the proteasome structure is quite conserved, it is likely that the server is able to produce reliable predictions for at least the other mammalian proteasomes. A similar WWW server is available at http://www.uni-tuebingen.de/uni/kxi/ (see also C. Kuttler, A.K. Nussbaum, T.P. Dick, H.-G. Rammensee, H. Schild, K.P. Hadeler, An algorithm for the prediction of proteasomal cleavages, J. Mol. Biol. 298 (2000), 417-429) (ref. 24).

Analysis by trained artificial networks enables identification of additional motifs and characteristics that promote or inhibit cleavage. The tools also enable, in combination with a predictor of MHC binding capacity, a more complete prediction of the generation and presentation of peptides on MHC Class I molecules.

Peptides can be obtained by solid phase synthesis methods. The first stage of the technique firstly introduced by Merrifield (refs. 14 and 15) consists of peptide chain assembly with protected amino acid derivatives on a polymeric support. The second stage of the technique is the cleavage of the peptide from the support with the concurrent cleavage of all side chain protecting groups to give the crude free peptide. To achieve larger peptides, these processes can be repeated sequentially.

For a review of this methodology, including the different chemical protection schemes and solid and soluble supports, see for example G. Barany and Fields (refs. 16 and 17).

A large number of peptides, so-called peptide libraries, can be obtained by combinatorial peptide synthesis; see e.g. Gordon et al., R.A. Houghten et al. and G. Jung et al. (refs. 18, 19 and 20). These collections of peptides can contain both natural, unnatural amino acids and amino acid mimics in the sequences. The libraries are useful for screening a large number of peptides.

Other methods for obtaining peptides include enzymatic fragment ligation, genetic engineering techniques as e.g. site-directed mutagenesis. Alternatively, the peptides can be obtained after isolation from natural sources.

### Production of MHC molecule constructs of the invention

By the present invention, it is possible to bind low affinity soluble MHC molecules stably to their specific counter receptors. The process making this possible is described in the following and comprises associating the MHC molecule to a carrier molecule (which may be chosen to be soluble or non-soluble, depending on the intended use) to form the MHC molecule construct, which thus is a poly-ligand (i.e. poly-valent) compound.

The plurality of low affinity MHC molecules organised in this way as multi- or poly-valent molecular complexes compensates for intrinsic high off-rates related to binding of individual MHC molecules.

The MHC molecule constructs of the present invention expressing multiple low affinity MHC molecules bind to specific receptors on MHC recognising cells with high avidity. For instance, the monomer form of soluble HLA Class I dissociates rapidly, whereas a MHC molecule construct of the invention comprising HLA Class I has proven far more stable.

Thus, the present invention further relates to a process for preparing a MHC molecule construct.

The process of the invention comprises the steps of
(a) providing a MHC molecule or a MHC molecule subunit, and
(b) associating the MHC molecule or the MHC molecule subunit to a suitable carrier molecule as described herein, or a suitable carrier molecule and a suitable binding entity as described herein, thereby obtaining a MHC molecule construct.

As mentioned, the carrier molecule may be chosen so as to be soluble or non-soluble.

More specifically, the process of the present invention comprises the steps of
(a) providing a prokaryotic or eukaryotic cell comprising one or more genes coding for a tagged or untagged MHC molecules or MHC molecule subunits, the gene or genes being expressible in said cell,
(b) cultivating the cell under conditions where the gene is expressed,
(c) isolating the MHC molecules or MHC subunits from the cell under conditions which allow subsequent purification of the MHC molecules or MHC molecule subunits generated by the cell, and
(d) optionally subjecting the isolated MHC molecule subunits to a folding treatment prior to or during a process of association to a carrier molecule as described herein, or a binding entity and a carrier molecule as described herein, thereby obtaining the MHC molecule construct.

The MHC molecules may be generated in the same cell or in different cells. In the latter case, the MHC molecules (which may very well be two different kinds of molecules, e.g. a heavy chain of a MHC Class I molecule and a β₂m) may be combined prior to or during the time of association to the carrier molecule (with or without a binding entity).

Host cells comprising appropriate expression vectors can be prokaryotic or eukaryotic.

Particularly preferred for production of MHC molecules used herein is the versatile and high expressive bacterial expression. By way of example, an E.coli strain e.g. lysogene BL21(DE3) can easily be transformed with a cDNA encoding expression vector of interest and induced to expression of large amounts of.molecules.

The host cells comprising expression plasmids encoding the MHC molecules may be of prokaryotic origin (bacteria) or of eukaryotic origin (yeast, insect or mammalian cells).

A preferred bacterial production is such which yields high amounts of denatured subunit molecule e.g. heavy chain and β₂m molecule. Functional MHC molecules can be obtained by in vitro folding following standard procedures known by persons skilled in the art. For example, a conventional method describes that denatured and fully reduced MHC Class I heavy chain molecule obtained from bacteria regenerates in presence of β₂m and appropriate peptide at physical and chemical conditions that allow formation of disulphide bonds and establishment of secondary and tertiary formation of denatured polypeptide chains (ref. 21). Both peptide and β₂m are added in excess in comparison to the amount of folding heavy chain to compensate for the low affinities of subunit molecules in the early folding phases. The peptide of interest should comprise appropriate anchor residues to ensure a sufficient loading into the peptide-binding site formed by the heavy chain.

A more recently developed and preferred method "Oxidised Folding Protein" (OPF) takes advantage of heavy chain molecules with pre-formed disulphide bonds, which direct a fast and more efficient folding of denatured molecules. This method describes folding of MHC Class I heavy chains in presence of β₂m alone. A peptide empty and bio-chemically stable MHC Class I molecule is formed by association of heavy- and light chain. Subsequent addition of peptides comprising appropriate anchor residues leads to fast formation of the functional and stable MHC-peptide complexes.

Well-defined MHC molecules or MHC molecule subunits can also be produced in cells encoding appropriate cDNAs. Expression vectors comprising such cDNA can be introduced into host cells using any technique known in the art. These techniques include electroporation, calcium-phosphate mediated transfection, transferrin-polycation mediated DNA transfer, transfection with naked or encapsulated nucleic acids, liposome mediated cellular fusion, intracellular transportation of DNA-coated latex beads, protoplast fusion and viral infection.

By way of example, peptide-binding motifs, which would be interesting to study in connection with the present invention, are shown in Figures 34-37.

### Therapy

As mentioned above, the present invention relates in general to the field of therapy. MHC molecule constructs of the invention are a powerful tool in various therapeutic applications. In particular, the MHC molecule constructs of the invention are applicable in in vivo and ex vivo therapeutic applications as will be apparent from the following.

The present invention is also based on the recognition that it is possible to design a poly-ligand MHC molecule construct so as to (I) target specific MHC recognising cells, and (II) induce a response as desired, in specific target MHC recognising cells by addressing receptors on such cells. It was further recognised that with such design of MHC molecule/peptide complexes with a given specificity, it is possible to "add" other stimuli to the therapeutic composition by incorporating other molecules, which will affect the activity of the MHC recognising cells. Thus, it is possible to modulate the activity of specifically targeted MHC recognising cell clones, while leaving other MHC recognising cell clones unaffected. Furthermore, it is also possible to specifically modulate the activity of more than one MHC recognising cell clone by choosing the before-mentioned other molecules appropriately. The inventions is further based on the recognition that it is possible to obtain specific MHC recognising cells using the MHC molecule constructs described herein, to modulate such ex vivo, whereby such cells can be used for in vivo treatment.

Accordingly, the present invention provides for methods of up-regulating, down-regulating, modulate, restoring, enhancing, and/or stimulate the immune system, as well as methods of inducing anergy of cells. This can in accordance with the present invention in general be accomplished in two ways, namely in vivo or ex vivo. By "in vivo" is meant that an effective amount of an active substance or ingredient is administered to a subject by any suitable route, the active substance or ingredient excerting its effect in the subject. By "ex vivo" (may also be termed "in vitro") is meant that cells withdrawn from a subject are in some way affected outside the subject, and then re-introduced to the subject, thereby achieving a desired response.

It should be emphasised that all and any definitions given above both with respect to the MHC molecule constructs and other terms apply equally to the following. It is to be understood that the therapeutic compositions of the present invention may comprise one or more MHC molecule constructs as defined above. For the definition of "one or more" as well as "a", "an", "a plurality", and "the", cf. above. As also described above, the MHC molecules of the construct may be peptide filled, peptide empty or mixtures thereof. The MHC molecules of each construct may be the same or different. Likewise, the peptides of the MHC molecules may be the same or different. Likewise, the MHC molecule construct may comprise one or more biologically active molecules, which may be the same or different. These expressions are described in the foregoing.

In particular, the inclusion of biologically active molecules may be important to initiate a response as desired. As mentioned above, the immune system is dependent on several signalling pathways, and thus inclusion of biologically active molecules, either as part of the MHC construct or alone, may be an excellent way to control or guide the immune system.

Thus, the present invention relates generally to the MHC molecule constructs per se as defined above for use as therapeutic compositions or medicaments. The present invention also relates to the MHC molecule constructs as defined herein for use in in vivo therapy and for use in ex vivo therapy.

In one aspect, the present invention relates to therapeutic compositions comprising as an active ingredient a MHC molecule construct as defined herein.

In another aspect, the present invention relates to therapeutic compositions comprising as active ingredient an effective amount of MHC recognising cells, the MHC recognising cells being obtainable by

bringing a sample from a subject comprising MHC recognising cells into contact with a MHC molecule construct as described herein, whereby the MHC recognising cells become bound to the MHC molecule construct,
isolating the bound MHC molecule construct and the MHC recognising cells, and
expanding such MHC recognising cells to a clinically relevant number.

The therapeutic compositions may suitably comprise one or more adjuvants and/or excipients.

As used herein, the term "adjuvant" refers to an immunological adjuvant. By this is meant a compound that is able to enhance or facilitate the immune system's response to the ingredient in question, thereby inducing a immune response or series of immune responses in the subject. The adjuvant may facilitate the effect of the therapeutic composition by forming depots (prolonging the half-life of the ingredient), provide additional T-cell help and stimulate cytokine production. Facilitation of antigen survival and unspecific stimulation by adjuvants may, in some cases, be required if MHC molecule epitopes are the only feature in the therapeutic composition recognised by the immune system.

Included in the term "immune response" is specific humoral, i.e. antibody, as well as cellular immune responses, the antibodies being serologic as well as secretory and pertaining to the subclasses IgM, IgD, IgG, IgA and IgE as well as all isotypes, allotypes, and subclasses thereof. The term is further intended to include other serum or tissue components. The cellular response includes Type-1 and Type-2 T-helper lymphocytes, cytotoxic T-cells as well NK cells.

Examples of suitable adjuvant are those mentioned above, i.e. saponins such as Quil A and Qs-21, oil in water emulsions such as MF59, MPL, PLG, PLGA, aluminium salts, calcium phosphate, water in oil emulsions such as IFA (Freund's incomplete adjuvant) and CFA (Freund's complete adjuvant), interleukins such as IL-1β, IL-2, IL-7, IL-12, and INFy, Adju-Phos^{®}, glucan, antigen formulation, biodegradable microparticles, Cholera Holotoxin, liposomes, DDE, DHEA, DMPC, DMPG, DOC/Alum Complex, ISCOMs^{®}, muramyl dipeptide, monophosphoryl lipid A, muramyl tripeptide, and phospatidylethanolamine In a preferred embodiment, the adjuvant is selected from saponins such as Quil A and Qs-21, MF59, MPL, PLG, PLGA, calcium phosphate, and aluminium salts. Examples of suitable excipients are those mentioned above, i.e. diluents, buffers, suspending agents, wetting agents, solubilising agents, pH-adjusting agents, dispersing agents, preserving agents, and/or colorants. In particular a PBS buffer without calcium ions and magnesium ions may be suited.

The therapeutic compositions of the invention may suitably be applied in the treatment, prevention, stabilisation, or alleviation of various diseases. Diseases of relevance are those mentioned above, i.e. diseases of of inflammatory, auto-immune, allergic, viral, cancerous, infectious, allo- or xenogene (graft versus host and host versus graft) origin. In particular, the disease may be a chronic inflammatory bowel disease such as Crohn's disease or ulcerative colitis, sclerosis, type I diabetes, rheumatoid arthritis, psoriasis, atopic dermatitis, asthma, malignant melanoma, renal carcinoma, breast cancer, lung cancer, cancer of the uterus, prostatic cancer, brain cancer, head and neck cancer, leukaemia, cutaneous lymphoma, hepatic carcinoma, colorectal cancer, bladder cancer, rejection-related disease, Graft-versus-host-related disease, or a viral disease associated with hepatitis, AIDS, measles, pox, chicken pox, rubella or herpes.

More specifically, the disease may be
of inflammatory/auto-immune origin, including asthma, hypersensitivity pneumonitis, interstitial lung disease, sarcoidosis, idiopathic pulmonary fibrosis, interstitial lung disease associated with Crohn's Disease or ulcerative colitis or Whipple's disease, interstitial lung disease associated with Wegeners granulomatosis or hypersensitivity vasculitis,
vasculitis syndromes, Hennoch-Schönleins purpura, Goodpastures syndrome, Wegeners granulomatosis,
renal diseases such as antibody mediated glomerulopathia as in acute glomerulonephritis, nephritis associated with systemic lupus erythematosus, nephritis associated with other systemic diseases such as Wegeners granulomatosis and Goodpastures syndrome and mixed connective tissue disease, chronic interstitial nephritis, chronic glomerulonephritis,
gastrointestinal diseases such as Crohn's Disease, Ulcerative colitis, coeliac disease, Whipple's disease, collagenous colitis, eosinophillic colitis, lymphatic colitis,
hepatobilliary diseases such as auto-immune hepatitis, alcohol induced hepatitis, periportal fibrosis, primary billiary cirrhosis, sclerosing colangitis,
disorders of the central or peripheral nervous system such as demyelinating disease as multiple sclerosis, acute disseminated encephalomyelitis, sub-acute sclerosing panencephalitis,
skin disease such as psoriasis, atopic dermatitis, eczema, allergic skin disease, progressive systemic sclerosis (scleroderma), exfoliating dermatitis, pemphigus vulgaris,
joint diseases such as rheumatoid arthritis, ankylosing spondylitis, arthritis associated with psoriasis or inflammatory bowel disease,
muscoloskelletal diseases such as myastenia gravis, polymyositis,
endocrine diseases such as insulin dependent diabetes mellitus, auto-immune thyroiditis (Hashimoto), thyreotoxicosis, Graves,
diseases of the hematopoetic system such as auto-immune anaemia, auto-immune thrombocytopenia,
cardiovascular diseases such as cardiomyopathia, vasculitis, cardiovascular disease associated with systemic diseases as systemic lupus erythematosus, polyarthritis nodosa, rheumatoid arthritis, scleroderma, sarcoidosis,
diseases of cancerous origin, including malignant melanoma, Sezary's syndrome, cutaneous T-cell lymphoma, renal cell carcinoma, colorectal cancer, breast cancer, ovarian cancer, cancer of the uterus, prostatic cancer, hepatic carcinoma, lung cancer, and sarcoma,
diseases, disorders or conditions of allergic origin.

The most common allergens, to which allergic reactions occur, include inhalation allergens originating i.a. from trees, grasses, herbs, fungi, house dust mites, storage mites, cockroaches and animal hair, feathers, and dandruff. Important pollen allergens from trees, grasses and herbs are such originating from the taxonomic orders of *Fagales, Oleales* and *Pinales* including i.a. birch (Betula), alder (*Alnus*), hazel *(Corylus),* hornbeam (*Carpinus*) and olive (Olea), the order of *Poales* including i.a. grasses of the genera *Lolium, Phleum, Poa, Cynodon, Dactylis* and *Secale,* the orders of *Asterales* and *Urticales* including i.a. herbs of the genera *Ambrosia* and *Artemisia.* Important inhalation allergens from fungi are i.a. such originating from the genera *Alternaria* and *Cladosporium.* Other important inhalation allergens are those from house dust mites of the genus *Dermatophagoides,* storage mites from the genus *Lepidoglyphys destructor,* those from cockroaches and those from mammals such as cat, dog, horse, cow, and bird. Also, allergic reactions towards stinging or biting insects such as those from the taxonomic order of Hymenoptera including bees, wasps, and ants are commonly observed. Specific allergen components are known to the person skilled in the art and include e.g. *Bet v* 1 (B. *verrucosa,* birch), *Aln g* 1 *(Alnus glutinosa,* alder), *Cor* a 1 (*Corylus avelana,* hazel) and *Car b* 1 (*Carpinus betulus,* hornbeam) of the *Fagales* order. Others are *Cry j* 1 (*Pinales*), *Amb* a 1 and 2, Art *v* 1 (*Asterales*), *Par j* 1 (*Urticales*), *Ole* e 1 (*Oleales*), *Ave* e 1, *Cyn d* 1*, Dac g 1, Fes p* 1, *Hol l* 1*, Lol p* 1 and 5, *Pas* n 1, *Phl p* 1 and 5, Poa *p* 1, 2 and 5, *Sec c* 1 and 5, and *Sor h* 1 (various grass pollens), *Alt* a 1 and *Cla h* 1 (fungi), *Der f* 1 and 2, *Der p* 1 and 2 (house dust mites, D. *farinae* and D. *pteronyssinus,* respectively), *Lep d* 1, *Bla g* 1 and 2, Per a 1 (cockroaches, *Blatella germanica* and *Periplaneta* americana, respectively), *Fel d* 1 (cat), *Can f* 1 (dog), *Equ* c 1, 2 and 3 (horse), *Apis m* 1 and 2 (honeybee), *Ves g* 1, 2 and 5, *Pol* a 1, 2 and 5 (all wasps) and *Sol i* 1, 2, 3 and 4 (fire ant), to mention the most common.

The therapeutic compositions of the invention may be formulated in any suitable way, i.a. depending on the route of administration, and the amount of active ingredient to be administered. In particular, the therapeutic compositions of the invention may be formulated for parenteral administration, including intravenous, intramuscular, intraarticular, subcutaneous, intradermal, epicutantous/transdermal, and intraperitoneal administration, for infusion, for oral administration, for nasal administration, for rectal administration, or for topic administration.

The MHC molecule construct may suitably be immobilised onto a solid or semi-solid support. Examples of solid and semi-solid support are those mentioned above, i.e. particles, beads, biodegradable particles, sheets, gels, filters, membranes, fibres, capillaries, needles, microtitre strips, tubes, plates or wells, combs, pipette tips, micro arrays, and chips. In particular, the solid support may be selected from particles and beads, preferably particles and beads, which are polymeric, magnetic or superparamagnetic. For in vivo therapy, biodegradable particles will be especially preferred, while for ex vivo therapy, biodegradable, polymeric, magnetic, paramagnetic or superparamagnetic particles will be especially preferred.

As mentioned, the MHC molecule constructs used in the therapeutically compositions are very interesting molecule poly-ligand compounds possessing highly appropriate properties for modulation of MHC recognising cells both in vivo and ex vivo.

The poly-ligand MHC molecule constructs can, due to the carrier molecule, be loaded with a plurality of peptide-displaying MHC molecules to ensure high avidity binding to specific counter receptors.

It is to be understood that such responses include inducing anergy leading to apoptosis, up-regulating a response, down-regulating a response, stimulating a response, modulating a response, enhancing a response, inhibiting a response, and in any other way manipulating a response. In this connection, it should be understood that the MHC molecules/peptides of the construct may be chosen so as to induce a number of other responses, or activate signal pathways which results in the production of various signalling substances which may have a beneficial influence on the disease to be treated, prevented or alleviated.

To accomplish this, the MHC molecule construct used in the composition may suitably comprise heterogeneous or homogeneous MHC molecules (e.g. displaying different peptides) to elicit one or more or more functions in the target MHC recognising cells in vivo and ex vivo.

Furthermore, this desired effect of the MHC molecule construct may be enhanced, reduced, inhibited, stimulated or combined with other effects by the further attachment of biologically active compounds as described above, thereby addressing specific MHC recognising cell clones. For this, a specific combination of MHC molecule and peptide may be selected. By way of example, loading co-stimulatory molecules e.g. B7.1 onto a poly-ligand MHC molecule construct thus leads to formation of a bifunctional poly-ligand MHC molecule construct, which (I) is directed to the peptide specific MHC recognising cell clones of interest and (II) facilitate appropriate stimulation implying two mandatory signals to initiate an immune response. Another example, is compositions for the treatment of auto-immune diseases wherein a recombinant toxin, e.g. PE-38, is also attached to the MHC molecule constructs used.

By this, it is possible to modulate the response in any way it would be desired. Therefore, the present invention also relates to a method of designing a MHC molecule/peptide combination, resulting in a desired target cell response both in vivo and ex vivo.

A variety of diseases, including cancer cause immunosuppression in the patients. Immunotherapy is an attempt to stimulate the patient' s own immune system to recognise and destroy cancer cells. The tumour can exert its suppressive influence over the immune system through several different mechanisms. Although tumour cells can prime the immune system, tumour escape mechanisms can induce immunological tolerance to the tumour. There are several known mechanisms of tumour escaping immune surveillance. For instance, tumour cells are often inefficient in presenting tumour antigens to effector T-cells. This can be the result of tumour cell down-regulation or mutation of MHC molecules, or down-regulation of co-stimulatory molecules such as B7, or other molecules, such as TAP, that are important in the antigen presenting pathway.

Furthermore, tumour cells have been shown to induce tolerance in T-cells by down-regulating the expression of CD3-zeta chain in T-cells. Tumour cells can also suppress T-cell activation by release of inhibitory cytokines, and induce apoptosis in T-cells through Fas-Fas ligand interaction. Tumour cells also have the ability to suppress the immune system through release of cytokines such as IL-12 that inhibits the maturation of immature dendritic cells into fully mature antigen presenting cells. Inhibitory factors released by tumour cells have been shown to suppress granulocyte activation, thus avoiding the killing of tumour cells by activated granulocytes.

Common treatment regimes such as chemotherapy and radiation therapy also suppress immunity in a more general way.

A variety of different prior art strategies have been employed in an attempt to restore or enhance the patient's immune response to tumours, including treatment with monoclonal antibodies, cancer vaccines, cytokine therapy and adoptive cellular immunotherapy using dendritic cells or T-cells. Cellular immunotherapy involving T-cells include CD8+ cytotoxic effector cells that have the capacity to kill tumour cells. Furthermore, it has been shown that CD4+ cytokine producing T-cells also play an important role in maintaining a sustainable anti-tumour cell activity of cytotoxic CD8 cells. The success, however, of the prior art methods have been limited.

In accordance with the above, interesting the MHC molecule constructs of the therapeutic composition may be such,
wherein at least two of the MHC molecules of the MHC molecule construct used are different,
wherein the MHC molecules of the MHC molecule construct used are the same,
wherein at least two of the peptides harboured by a plurality MHC molecules of the MHC molecule construct used are different,
wherein the peptides harboured by the MHC molecules of the MHC molecule construct used are the same,
wherein the peptides harboured by the MHC molecules of the MHC molecule construct used are chemically modified or synthesised to contain not natural amino acids, hydrophilic or hydrophobic groups,
wherein the peptides harboured by the MHC Class I molecules of the MHC molecule construct used are linked to the MHC Class I heavy chain by a flexible linker, wherein the peptides harboured by the MHC Class I molecules of the MHC molecule construct used are linked to the MHC Class I light chain (β₂m) by a flexible linker,
wherein the peptides are harboured by MHC Class I molecules of the MHC molecule construct used comprising of MHC Class I heavy chain in association with a light chain (β₂m) by a flexible linker,
wherein the peptide harboured by the MHC Class II molecules of the MHC molecule construct used are linked to the alfa-chain by a flexible linker,
wherein the peptide harboured by the MHC Class II molecules of the MHC molecule construct used are linked to the β-chain by a flexible linker,
wherein the MHC Class I molecules of the MHC molecule construct used are mutated,
wherein the MHC Class II molecules of the MHC molecule construct used are mutated,
wherein the MHC molecules of the MHC molecule construct used are peptide free MHC molecules.

As mentioned above, the MHC molecule construct may comprise one or more biologically active molecules. Such are defined above. Particularly preferred biologically compounds will be selected from MIC A, MIC B, CD1d, ULBP-1, ULBP-2, ULBP-3, CD2, CD3, CD4, CD5, CD8, CD9, CD27, CD28, CD30, CD69, CD134 (OX40), CD137 (4-1BB), CD147, CDw150 (SLAM), CD152 (CTLA-4), CD153 (CD30L), CD40L (CD154), NKG2D, ICOS, HVEM, HLA Class II, PD-1, Fas (CD95), FasL, CD40, CD48, CD58, CD70, CD72, B7.1 (CD80), B7.2 (CD86), B7RP-1, B7-H3, PD-L1, PD-L2, -CD134L, CD137L, ICOSL, LIGHT, CD16, NKp30, NKp44, NKp46, NKp80, 2B4, KIR, LIR, CD94/NKG2A, and CD94/NKG2C.

As mentioned the present invention relates to methods for the treatment of an animal, including a human being, which methods comprise administering a therapeutic composition as described herein in an effective amount. The treatment may be such which involves up-regulation, down-regulation, modulation, stimulation, inhibition, restoration, enhancement and/and otherwise manipulation of immune responses. This can indeed be accomplished by the compositions of the present invention. The present invention also relates to methods of inducing anergy in a cell, by which methods a therapeutic composition as described herein is administered.

In a further aspect, the present invention relates to methods of performing adoptive immunotherapy, which methods comprise administrating to an animal, including a human being, a therapeutic composition as described herein.

### In vivo therapy

As mentioned above, the therapeutic composition of the invention is suited for in vivo therapy.

Therapeutic compositions for in vivo therapy may suitably comprise from 1 to 10 different MHC molecule constructs. Thus, the inclusion of two, three, four, five, six or more different MHC molecule constructs are contemplated and believed to be advantageous in some cases. Also, it may be advantageous to include a MHC molecule construct carrying MHC molecules harbouring different peptides. The amount of each MHC molecule construct depends on the MHC molecule construct or combination of MHC molecule constructs in question. Furthermore, the affinity of the MHC molecule should be taken into consideration. High-affinity, as well as low affinity peptides may be harboured by the MHC molecules. This, however, is expected to affect the amount necessary to generate the desired response and the strength of the desired response. It is contemplated that the amount of MHC molecule construct required to induce a systemic immune response will typically be in the range of from 0.0001 to 10000 µg/kg/dose, such as from 0.01 to 1000 µg/kg/dose, from 0.1 to 100 µg/kg/dose, or from 1 to 10 µg/kg/dose. In general, the MHC molecules used should be synergenic with the receiving subject to avoid or minimise the risk of alloreactions.

The administration of the composition of the invention may be as single doses or as several doses. In certain cases, administration only once may be sufficient. In general, several doses should be given with intervals of a day, a week, two weeks, a month, or several months, etc. For example, a single dose may be given once, or a dose may be given as a primer, followed by one or more administration, or a continuous administration regime like up to four doses per week, followed by one month without administrations, followed by up to four doses per week (optionally with increasing amount of the MHC molecule construct), etc. Optionally different adjuvants or combinations of adjuvants may be used in the different administrations. These are all examples, and the optimal administration regime depends on the MHC molecule construct in question and several other factors. The person skilled in the art will readily know how to optimise this.

Of course, other medicaments may be administered simultaneously in order to enhance or support the treatment.

In particular, one or more MHC molecule constructs without MHC molecules attached, but with biologically active molecules attached may be administered together with the MHC molecule construct to sitmulate, up-regulate, down-regulate, inhibit or enhance other MHC recognising cell clones than the MHC recognising cell clones addressed by the MHC molecule construct of the composition. Such may also be added to promote response to the cell clone addressed. In particular, such biologically active molecules may be part of the MHC molecule construct as described in the foregoing.

Containers for mixing and storage of the therapeutic composition of the invention may be made of glass or various polymeric materials. The containers chosen should not adsorb the product stored. The containers may suitably be ampoules or capped vials for mono- or multidosage.

The invention further relates to methods for producing the therapeutic compositions of the invention, which methods comprise
providing a MHC molecule construct as described herein, and
solubilising or dispersing the MHC molecule construct in a medium suitable for therapeutic substances, and optionally adding other adjuvants and/or excipients.

### Ex vivo therapy

As mentioned above, the compositions of the present invention are suited for ex vivo therapy.

Thus, the present invention relates in particular to therapeutic compositions comprising as an active ingredient an effective amount of MHC recognising cells, the MHC recognising cells being obtained by
isolating from a subject MHC recognising cells using a MHC molecule construct as defined herein, and
expanding such MHC recognising cells to a clinically relevant number.

Once the MHC recognising cells have been isolated they may, if needed, be genetical or in any other appropraite way modified or manipulated before they are expanded.

The MHC recognising cells can be isolated in a number of ways, which are described in more detail in the following.

In particular:
1) One or more MHC molecule constructs as defined herein may be brought into contact with a sample from a subject, whereby the MHC molecule constructs are allowed to bind to MHC recognising cells in the sample. The MHC molecule constructs may then be recovered from the sample, whereafter the MHC recognising cells may be liberated from the MHC molecule constructs and subsequently expanded.
2) One or more MHC molecule constructs as defined herein may be brought into contact with a sample from a subject, whereby the MHC molecule constructs are allowed to bind to MHC recognising cells in the sample. The MHC molecule constructs may then be recovered from the sample, whereafter the MHC recognising cells may be liberated from the MHC molecule construct and subsequently expanded in the presence of one or more other MHC molecule constructs.
3) One or more MHC molecule constructs as defined herein immobilised onto a solid or semi-support as defined herein may be brought into contact with a sample from a subject, whereby the MHC molecule constructs are allowed to bind to MHC recognising cells in the sample. The MHC molecule constructs may then be recovered from the sample, whereafter the MHC recognising cells may be liberated from the MHC molecule constructs and subsequently expanded.
4) One or more MHC molecule constructs as defined herein immobilised onto a solid or semi-support as defined herein may be brought into contact with a sample from a subject, whereby the MHC molecule constructs are allowed to bind to MHC recognising cells in the sample. The MHC molecule constructs may then be recovered from the sample, whereafter the MHC recognising cells may be liberated from the MHC molecule constructs and subsequently expanded in the presence of one or more other MHC molecule constructs.
5) One or more labelled MHC molecule constructs as defined herein immobilised onto a solid or semi-support as defined herein may be brought into contact with a sample from a subject, whereby the MHC molecule constructs are allowed to bind to MHC recognising cells in the sample. The MHC molecule constructs may then be recovered from the sample, whereafter the MHC recognising cells may be liberated from the MHC molecule constructs and subsequently expanded.
6) One or more labelled MHC molecule constructs as defined herein immobilised onto a solid or semi-support as defined herein may be brought into contact with a sample from a subject, whereby the MHC molecule constructs are allowed to bind to MHC recognising cells in the sample. The MHC molecule constructs may then be recovered from the sample, whereafter the MHC recognising cells may be liberated from the MHC molecule constructs and subsequently expanded in the presence of one or more other MHC molecule constructs.
7) One or more MHC molecule constructs as defined herein may be brought into contact with a sample from a subject, whereby the MHC molecule constructs are allowed to bind to MHC recognising cells in the sample. Then the sample containing the MHC recognising cells bound MHC molecule constructs may be brought into contact with a solid or semi-solid support as defined herein having immobilised thereon one or more molecules which are able to bind to the any part of the MHC recognising cells or MHC molecule construct, whereby the MHC molecule construct with the bound MHC recognising cells will become bound to the support. The thus bound MHC molecule constructs with MHC recognising cells may then be recovered from the sample, whereafter the MHC recognising cells may be liberated from the MHC molecule constructs and subsequently expanded.
8) One or more labelled MHC molecule constructs as defined herein may be brought into contact with a sample from a subject, whereby the MHC molecule constructs are allowed to bind to MHC recognising cells in the sample. Then the sample containing the MHC recognising cells bound MHC molecule constructs may be brought into contact with a solid or semi-solid support as defined herein having immobilised thereon one or more molecules which are able to bind to the any part of the MHC recognising cells or MHC molecule construct, whereby the MHC molecule construct with the bound MHC recognising cells will become bound to the support. The thus bound MHC molecule constructs with MHC recognising cells may then be recovered from the sample, whereafter the MHC recognising cells may be liberated from the MHC molecule constructs and subsequently expanded in the presence of one or more other MHC molecule constructs.

The above list is not exhaustive in any way.

It is to be understood that the time of contact between the MHC molecule constructs and the sample will depend on several factors, i.a. the MHC molecule constructs in question and the conditions under which the contacting take place. The contact time will be any such sufficiently to enable binding to the MHC recognising cells to the MHC molecule construct. The person skilled in the art will readily how to optimise this. In general, the sample may and the MHC molecule construct may be brought into contact for 10 minutes to 2 hours, such as from 20-45 minutes, at a temperature of from 4°C to 20°C.

It is to be understood that the MHC recognising cells are those indicated above.

It is to be understood that "sample" has the meaning defined above. In particular, the sample may be peripheral blood mononuclear cells (PBMC) or other blood-derived preparations such as leukopheresis products, or bone marrow, spleen or umbilical cord. Samples may be used as they are, or they may be subjected to various purification, decontamination, filtration, or concentration methods, and/or methods to isolate or remove parts of the sample like immunomagnetic separation.

The MHC molecule construct with bound MHC recognising cells may suitably be isolated by use of a magnetic field (if the support are magnetic particles or beads) (i.e. an immunomagnetic separation technique), or by use of a cell sorter device such as a flow cytometer. For the latter isolation procedure, the MHC molecule constructs may suitably be labelled. If the immobilisation to the solid or semi-solid support is carried out following contact between the MHC molecule construct and sample, it is to be understood that the MHC molecule construct with the MHC recognising cells may be immobilised using a compound capable of binding thereto. Such, which are suitable, are indicated above. For immunomagnetic isolation of MHC recognising cells from larger sample volumes, disposable blood bags may be applied. Examples of equipment suited for such purpose are the Isolex® 300i or MaxSep® equipment from Baxter Healthcare Corp, or the CliniMACS from Miltenyi Biotech.

The MHC recognising cells can be liberated from the MHC molecule construct by procedures such as DNA-linker digested by DNase, temperature-sensitive Elastin-linker, as described in WO 99/11661 (ref. 29), detaching antibodies as described in WO 91/15766 (ref. 30), release by incubation and other release mechanisms known to persons skilled in the art.

The MHC molecule construct may in accordance with the definitions above comprise one or more biologically active molecules. Such can be included e.g in order to attract the MHC recognising cells desired, and to lower or prevent potential induction of apoptosis resulting from the isolation procedure.

It is to be understood that the expansion of the cells may be carried out in the presence of one or more MHC molecule constructs as defined above. The such used MHC molecule constructs may be the same as used for capturing the MHC recognising cells or may be different. Such may in accordance with the definitions above comprise one or more biologically active molecules. Such biologically active molecules will further facilitate multiple interactions with the TCR and co-stimulatory molecules on MHC recognising cells and produce efficient ex vivo stimulation of MHC recognising cells. The binding affinity of e.g. co-stimulatory molecules and their ligands is in the same range as the binding affinity of MHC molecule-peptide complexes and the TCR (10 µM range). The inclusion of such biologically active molecules facilitates the use of natural molecules as a replacement for stimulatory antibodies during expansion, since they compensate for low affinity by introducing multiple binding interactions. In particular, the MHC recognising cells may be expanded in the presence of one or more MHC molecule constructs without MHC molecules attached, but with biologically active molecules attached to stimulate, up-regulate, down-regulate, inhibit or enhance 1) other MHC recognising cell clones than the MHC recognising cell clones of interest as well as 2) the MHC recognising cell clones of interest. The expansion may further be carried out in the presence of feeder cells such as dendritic cells or stroma cells.

It is to be understood that the expansion of the cells may additionally be carried out in the presence of further compounds, e.g. such which promote or stimulate expansion of the cells, inhibit growth of non-relevant cells, or select for the desired cells. Such can e.g. be one or more biologically active molecules as described above. Such further compounds may also be selected from cytokines such as lymphokines, interferons, interleukins, growth factors, and colony-stimulating factors. For example, Il-2 may be added to enhance proliferation of cells, and other cytokines may be added to induce particular differenciation patterns, if required. For example, IL-4 triggers differentiation of T-cell populations into the Th2 subpopulation, and INF-gamma triggers differentiation into the Th1 subpopulation. Of course a suitable culturing medium and suitable conditions have to be used to expand and maintain cells. Expansion time is usually between 3 and 10 days, but can be as long as 14 to 20 days, or even longer providing viability and continued proliferation of cells are maintained.

In a special aspect, the present invention relates to methods of obtaining MHC recognising cells comprising, which methods comprise bringing into contact a MHC molecule construct as described herein and a sample suspected of comprising MHC recognising cells under conditions whereby the MHC recognising cells bind to the MHC molecule construct, and isolating the bound MHC molecule construct and MHC recognising cells.

Such methods are e.g. suited for the obtaining the MHC recognising cells of therapeutic compositions of the invention. Such methods are further believed to be of value for identifying new disease-associated peptides, in that random peptides can be applied as part of the MHC molecule construct, and their binding effect to MHC recognising cells can be tested. The methods can suitably be carried out by immunomagnetic separation techniques or by flow cytometry.

The invention further relates to methods for producing the therapeutic compositions of the invention, which methods comprise
obtaining MHC recognising cells using a MHC molecule construct as described herein,
expanding such MHC recognising cells to a clinically relevant number,
formulating the obtained cells in a medium suitable for administration, and
optionally adding adjuvants and/or excipients.

The invention further relates to kits for obtaining the MHC recognising cells. In one embodiment, such kits comprise one or more MHC protein constructs as defined herein, optionally immobilised on to a solid or semi-solid support as defined herein. In another embodiment, such kits comprise one or more MHC protein constructs as defined herein and means for immobilisation of the MHC molecule constructs(s) prior to or following binding to the MHC recognising cells.

The invention further in general relates to the use of the MHC molecule construct described herinfor ex vivo expansion of MHC recognising cells. For such ex vivo expansion of cells, the MHC molecule construct may be provided in soluble form. The MHC molecule construct may also be provided immobilised onto a solid or semi-solid support. The solid and semi-solid supports are those mentioned above. Beads and particles are especially preferred, in particular polymeric, magnetic or superparamagnetic particles or beads. In particular, the MHC molecule construct may comprise one or more of the biologically active compounds as described above.

In the following, more specific procedures are described in the context of cancerous diseases, but the procedures apply equally well to other diseases.

It is believed that one strategy to overcome the suppressive effect of e.g. tumour cells on the immune system would be to remove the immune relevant cells from a subject through standard apheresis procedures, and to expand and modify these immune cells ex vivo before re-infusion to the patients. This would not only remove the suppressive pressure of tumour cells, but also allow for the rescue of immunocompetent cells prior to immunosuppressive treatment regimens including chemotherapy and radiation therapy.

Pheripheral blood T-cells can be removed from a subject, and placed into culture under conditions that allow the T-cells to proliferate. Such conditions include growing T-cells with mitogens or antigens in the presence of cytokines (IL-2) and dendritic cells as antigen presenting cells. Antigen can be introduced in the cultures either as protein extracts from tumours, defined protein antigens or peptides, or as tumour mRNA or DNA transfected into the dendritic cells. Alternatively, T-cell expansion protocols have been developed that include the use of stimulatory antibodies such as anti-CD3 and anti-CD28 antibodies, either in soluble form or coupled to a solid phase. The advantage of such antibody-based expansion protocols is that they circumvent the need for feeder cells and tumour antigens that may not be readily available. Following ex vivo T-cell-expansion, often in the order of 100-1000 fold, the T-cells are re-infused to the patients in order to restore or enhance the immune function towards the tumour.

It is well known that T-cells of the immune system express a multitude of specificities, and only a limited number of the available T-cell clones express specificities that are relevant for the recognition and killing of the tumour cells.

Most prior art protocols for T-cell expansion do not take into consideration the antigen specificity of the T-cells for tumour antigens, and result in a polyclonal expansion of T-cells which include a multitude of irrelevant T-cell specificities. Although the expanded T-cells would help restore the immune function of the patients through production of cytokines, it is expected that only a fraction of the re-infused T-cells can recognise and kill tumour cells directly. In addition, polyclonal expansion of T-cells increases the risk of expanding T-cell clones with autoimmune specificities.

The present invention relates in particular to adoptive immunotherapy using T-cells with known specificities for tumour antigens. In this aspect of immunotherapy, CD4 and CD8 T-cells specific for pre-determined tumour antigens can be isolated from peripheral blood, apheresis products, bone marrow, lymph nodes, primary tumours, secondary organ metastasis and other tissues by an immunomagnetic separation procedure or by a flow cytometric procedure. After optional purification, the cells can be expanded ex vivo and re-infused to the patients. Such expanded antigen specific T-cells will have the ability to target tumour cells directly, and thus be more efficient than polyclonally expanded T-cells. In addition, the use of antigen specific T-cells would decrease the potential danger of re-infusing T-cells clones with autoimmune specificities.

By the present invention, a support as defined above, preferably in the form of beads or particles as defined above, having MHC molecule constructs as defined herein immobilised thereon, may be applied to aid manipulation and separation of relevant cells from a sample. Thus, a support comprising magnetic particles may readily be removed by magnetic aggregation, which provides a quick, simple and efficient way of ex vivo separating bound cells.

The magnetic particles or beads with the specific T-cells attached may be removed ex vivo from a sample onto a suitable solid or semi-solid support by application of a magnetic field e.g. using a permanent magnet. It is usually sufficient to apply a magnet to the side of the vessel containing the sample mixture to aggregate the particles to the wall of the vessel and to pour away the remainder of the sample.

Especially preferred are superparamagnetic particles, as magnetic aggregation and clumping of the particles during reaction can be avoided. Dynabeads^{®} (Dynal Biotech ASA, Oslo, Norway) are one particularly suited example.

In a convenient embodiment, the MHC molecule constructs may be attached to the support, prior to contact with the sample. Such attachment may readily be achieved by methods (e. g. coupling chemistries) well known in the art, and conveniently the MHC molecule constructs are bound directly to the solid support, for example by coating. However, the MHC molecule constructs may also be attached via a spacer, a linker, or an antibody as described above. The MHC molecule constructs may be covalently or reversibly attached according to choice.

Alternatively, as mentioned above, the MHC molecule constructs may first be brought into contact with the sample, to bind to the T-cells before being attached to the solid support. In this case, the solid support may conveniently carry or be provided with a molecule as described above capable of binding to the MHC molecule construct thereby capturing the MHC molecule construct. Non-limiting examples include antibodies against the binding entity, antibodies against the carrier molecule, streptavidin or derivatives thereof for use with biotinylated carrier molecules, and anti-leucocyte antibodies.

Where more than one type of MHC molecule construct is used they may be attached to the same or different solid supports. Such a system using different solid supports is applicable particularly in the case of a particulate support such as beads or particles. Thus, different MHC molecule constructs may be attached to different beads or particles. Such beads or particles may suitably have difference sizes or properties, which thus enable separation according to the different MHC molecule constructs.

In an embodiment where more than one different type of MHC molecule construct is used, appropriate amounts or ratios at which the different types of MHC molecule constructs may be used will be readily determined by a person skilled in the art.

As mentioned above, cell separation techniques based on solid phase affinity binding (e.g. immunomagnetic separation (IMS)) are well known in the art and conditions to achieve this may readily be determined by the skilled worker in this field. Thus, for example a solid support carrying anti-leukocyte antibodies may be brought into contact with the sample. A particulate solid support may, for example, be added to the sample contained (e.g. suspended) in an appropriate medium (e. g. a buffer). The support may then be left in contact with the sample (e. g. incubated) for a length of time to enable binding to the cells to occur. Conditions during the step are not critical, and the sample-support mixture may be incubated at e.g. 4°C to 20°C for 10 minutes to 2 hours e.g. 20-45 minutes.

Antigen specific T-cells usually occur at very low frequencies. The low frequency of antigen specific T-cells makes these cells difficult targets for immunomagnetic isolation. In addition, the binding affinity between MHC-peptide complexes and the TCR is relatively low (in the order of 10 µM) compared to the binding affinity of antibodies and their target molecules (in the range of 10-0.1 nM). The low binding affinity of the MHC-peptide complexes can be compensated by introducing multiple binding sites between the solid phase used for cell isolation and the TCR on the T-cell surface. In the present invention, this is achieved by conjugating multiple MHC-peptide complexes to poly-ligand molecules. By coupling multiple such poly-ligand molecules to the solid phase, the avidity of binding to T-cells is increased to facilitate efficient isolation of the target T-cells.

Efficient isolation of antigen specific T-cells can be achieved by coupling the MHC-conjugated polymer molecules to the beads or particles prior to the cell isolation step, or indirectly by mixing soluble MHC molecule constructs with the sample, before introducing beads or particles which have a binding affinity for the a part of the MHC molecule construct.

Following isolation, the T-cells can be cultivated under conditions that facilitate proliferation and expansion. T-cell activation and proliferation depends, as described above, on two different signals delivered by antigen presenting cells. The first signal is the antigen specific signal delivered by MHC-peptide complexes to the TCR. The second signal is an antigen unspecific signal delivered by co-stimulatory molecules on the antigen presenting cells. Such co-stimulatory molecules include B7-1 and B7-2 which interact with interact with the CD28 molecule on T-cells, and other co-stimulatory molecules such as LFA-3, CD3, CD40, ICOS, NKG2D, OX40 and CD137.

As mentioned above, administration of the expanded cells may be by any convenient route. Typically, the number of cell for each administration should be about 10⁹-10¹¹ cells. The cells may suitably be administered in a volume of from about 50 ml to about 1 litre, such as about 50 ml to about 500 ml, about 50 ml to about 250 ml, about 50 ml to about 150 ml, or about 50 ml to about 100 ml, depending on the route of administration and the disease to be treated. The cells may be administered in a single dose or as several doses. In certain cases, administration only once may be sufficient. In general, several doses should be given with intervals of a day, a week, two weeks, a month, or several months, etc. For example, a single dose may be given once, or a dose may be given as a primer, followed by one or more administration, or a continuous administration regime like up to four doses per week, followed by one month without administrations, followed by up to four doses per week (sometimes with increasing or decreasing amount of the cells), etc. Optionally different adjuvants or combinations of adjuvants may be used in the different administrations. These are all examples, and the optimal administration regime depends on the cells in question and several other factors. The person skilled in the art will readily know how to optimise this. Of course, other medicaments may be administered simultaneously in order to enhance or support the treatment.

In particular, a MHC molecule construct as defined herein, or one or more MHC molecule constructs without MHC molecules attached, but with biologically active molecules attached may be administered together with the therapeutic composition to sitmulate, up-regulate, down-regulate, inhibit or enhance other MHC recognising cell clones than the MHC recognising cell clones addressed by the MHC molecule construct of the composition. Such constructs may optionally be immobilised onto biodegradable particles.

Containers for mixing and storage of the therapeutic composition of the invention may be made of glass or various polymeric materials. The containers chosen should essentially not affect the product stored. The containers may suitably be ampoules or capped vials for mono- or multidosage.

### Uses of MHC molecules

In a special aspect, the present invention relates to
uses of MHC molecules in histological methods, and
uses of MHC molecules in cytological methods.

Such methods are sample-mounted methods. The terms "histological" and "cytological" are as defined above. The term "mounted" is as defined above (sample-mounted methods).

This aspect of the invention is based on the surprising recognition that although MHC molecules or multimers of MHC molecules per se may not be very suited for some applications due to low intrinsic affinity, they perform unexpectedly well in sample-mounted applications.

All definitions, explanations, and interpretations given above also apply mutadis mutandis to this aspect of the invention.

Thus, in one embodiment, the present invention relates to the use of MHC molecules in a method for determining the presence of MHC recognising cells in a sample, wherein the MHC recognising cells of the sample are mounted on a support.

Such methods are a powerful tool in diagnosing various diseases. Establishing a diagnosis is important in several ways. A diagnosis gives information about the disease, thus the patient can be offered suitable treatment. Also, establishing a more specific diagnosis may give important information about a subtype of a disease for which a particular treatment will be beneficial (i.e. various subtypes of diseases may involve display of different peptides which are recognised by MHC recognising cells, and thus treatment can be targetted effectively against a particular subtype). In this way, it may also be possible to gain information about aberrant cells, which emerge through the progress of the disease or condition, or to investigate whether and how cell specificity is affected. The binding of the MHC molecule makes possible these options, since the binding is indicative for the presence of the MHC recognising cells in the sample, and accordingly the presence of MHC molecules displaying the peptide.

In another embodiment, the present invention relates to the use of a MHC molecule in a method for monitoring the presence of MHC recognising cells in a sample, wherein the MHC recognising cells of the sample are mounted on a support.

Such methods are a powerful tool in monitoring the progress of a disease, e.g. to closely follow the effect of a treatment. The method can i.a. be used to manage or control the disease in a better way, to ensure the patient receives the optimum treatment, to adjust the treatment, to confirm remission or recurrence, and to ensure the patient is not treated with a medicament which does not cure or alleviate the disease. In this way, it may also be possible to monitor aberrant cells which emerge through the progress of the disease or condition, or to investigate whether and how T-cell specificity is affected. The binding of the MHC molecule makes possible these options, since the binding is indicative for the presence of the MHC recognising cells in the sample, and accordingly the presence of MHC molecules displaying the peptide.

In yet another embodiment, the present invention relates to the use of a MHC molecule in a method for determining the status of a disease involving MHC recognising cells, in which method the MHC recognising cells of the sample are mounted on a support.

Such methods are a valuable tool in managing and controlling various diseases. A disease could, e.g. change from stage to another, and thus it is important be be able to determine the disease status. In this way, it may also be possible to gain information about aberrant cells which emerge through the progress of the disease or condition, or to investigate whether and how cell specificity is affected, thereby determining the status of a disease or condition. The binding of the MHC molecule makes possible these options, since the binding is indicative for the presence of the MHC recognising cells in the sample, and accordingly the presence of MHC molecules displaying the peptide.

In still another embodiment, the present invention relates to the use of a MHC molecule in a method for establishing a prognosis of a disease involving MHC recognising cells, in which method the MHC recognising cells of the sample are mounted on a support.

Such methods are a valuable tool in order to manage diseases, i.a. to ensure the patient is not treated without effect, to ensure the disease is treated in the optimum way, and to predict the chances of survival or cure. In this way, it may also be possible to gain information about aberrant cells, which emerge through the progress of the disease or condition, or to investigate whether and how T-cell specificity is affected, thereby being able to establish a prognosis. The binding of the MHC molecule makes possible these options, since the binding is indicative for the presence of the MHC recognising cells in the sample, and accordingly the presence of MHC molecules displaying the peptide.

The present invention also relates to the use of a MHC molecule in methods for the diagnosis of a disease involving MHC recognising cells, in which method the MHC recognising cells of the sample are mounted on a support.

Such diagnostic methods are a powerful tool in the diagnosis of various diseases. Establishing a diagnosis is important in several ways. A diagnosis gives information about the disease, thus the patient can be offered suitable treatment. Also, establishing a more specific diagnosis may give important information about a subtype of a disease for which a particular treatment will be beneficial (i.e. various subtypes of diseases may involve display of different peptides which are recognised by MHC recognising cells, and thus treatment can be targeted effectively against a particular subtype). Valuable information may also be obtained about aberrant cells emerging through the progress of the disease or condition as well as whether and how T-cell specificity is affected. The binding of the MHC molecule makes possible these options, since the binding is indicative for the presence of the MHC recognising cells in the sample, and accordingly the presence of MHC molecules displaying the peptide.

The present invention also relates to the use of a MHC molecule in methods of correlating cellular morphology with the presence of MHC recognising cells in a sample. Such methods are especially valuable as applied in the field of histological methods, as the binding pattern and distribution of the MHC molecule constructs can be observed directly. In such methods, the sample is treated so as to preserve the morphology of the individual cells of the sample. The information gained is important i.a. in diagnostic procedures as sited affected can be viewed directly.

As mentioned above, the use of the MHC molecule is in sample-mounted methods. Thus, the sample is mounted on a support. The support is selected from a solid or semi-solid surface. In particular, the support is selected from glass slides, membranes, filters, polymer slides, chamber slides, dishes, and petridishes.

The sample may suitably be selected from histological material, cytological material, primary tumours, secondary organ metastasis, fine needle aspirates, spleen tissue, bone marrow specimens, cell smears, exfoliative cytological specimens, touch preparations, oral swabs, laryngeal swabs, vaginal swabs, bronchial lavage, gastric lavage, from the umbilical cord, and from body fluids such as blood (e.g. from a peripheral blood mononuclear cell (PBMC) population isolated from blood or from other blood-derived preparations such as leukopheresis products), from sputum samples, expectorates, and bronchial aspirates. Such may be subjected to various treatments. Reference is made to the definitions given above, which also apply here.

The MHC molecule to be used may be
a MHC Class I molecule selected from the group consisting of a heavy chain, a heavy chain combined with a β₂m, a heavy chain combined with a peptide, and a heavy chain/β₂m dimer with a peptide;
or a MHC Class II molecule selected from the group consisting of an α/β dimer, an α/β dimer with a peptide, α/β dimer combined through an affinity tag and a α/β dimer combined through an affinity tag with a peptide;
or a MHC Class I like molecule or a MHC Class II like molecule.

The definitions given above with respect to the terms "MHC molecule", "MHC Class I molecule" and "MHC Class II molecule"also apply here.

The MHC molecule may suitably be a vertebrate MHC molecule such as a human, a murine, a rat, a porcine, a bovine or an avian molecule. The explanation to these molecules given above also applies here.

In particular, the MHC molecule to be used may be a human MHC molecule.

The MHC molecule to be used may be a peptide free MHC molecule, or a peptide filled MHC molecule.

The MHC molecule to be used may suitably be attached to a binding entity. Suitable binding entities are those described above, including streptavidin (SA) and avidin and derivatives thereof, biotin, immunoglobulins, antibodies (monoclonal, polyclonal, and recombinant), antibody fragments and derivatives thereof, leucine zipper domain of AP-1 (jun and fos), hexa-his (metal chelate moiety), hexa-hat GST (glutathione S-tranferase) glutathione affinity, Calmodulin-binding peptide (CBP), Strep-tag, Cellulose Binding Domain, Maltose Binding Protein, S-Peptide Tag, Chitin Binding Tag, Immunoreactive Epitopes, Epitope Tags, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, lectins that mediate binding to a diversity of compounds, including carbohydrates, lipids and proteins, e.g. Con A (*Canavalia ensiformis*) or WGA (wheat germ agglutinin) and tetranectin or Protein A or G (antibody affinity). It is to be understood that the binding entity may be a combination of those mentioned above.

Each binding entity may suitably have attached thereto from 1 to 10 MHC molecules, such as from 1 to 9, from 1 to 8, from 1 to 7, from 1 to 6, from 1 to 5, from 1 to 4, from 1 to 3, or 1 or 2 MHC molecules. Such MHC molecules may be the same or different (i.e. from different species). If the MHC molecules are peptide filled, such peptides may be the same or different. The number of MHC molecules attached to the binding entity is only limited by the capacity of the binding entity. When more than one MHC molecules is attached to a binding entity, such is herein termed a MHC molecule multimer, e.g. a dimer (two MHC molecules), a trimer (three MHC molecules), and a tetramer (four MHC molecules). This number should be interpreted as in average, cf. the explanations above. Thus, the average number needs not an integer, but can be any number between two integers (i.e. a decimal number).

For enabling detection of the MHC molecule, the MHC molecule may further comprise a labelling compound. The labelling compound is suitably such which is directly or indirectly detectable. Thus, the labelling compound may be a fluorescent label, an enzyme label, a radioisotope, a chemiluminescent label, a bioluminescent label, a polymer, a metal particle, a hapten, an antibody, or a dye. In particular, the labelling compound may be selected from
5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeston Red, Green fluorescent protein (GFP) and analogues thereof, and conjugates of R-phycoerythrin or allophycoerythrin and e.g. Cy5 or Texas Red, and inorganic fluorescent labels based on semiconductor.nanocrystals (like quantum dot and Qdot™ nanocrystals), and time-resolved fluorescent labels based on lanthanides like Eu3+ and Sm3+,
from haptens such as DNP, biotin, and digoxiginin, or
is selected from enzymatic labels such as horse radish peroxidase (HRP), alkaline phosphatase (AP), beta-galactosidase (GAL), glucose-6-phosphate dehydrogenase, beta-N-acetylglucosaminidase, ß-glucuronidase, invertase, Xanthine Oxidase, firefly luciferase and glucose oxidase (GO), or
is selected from luminiscence labels such as luminol, isoluminol, acridinium esters, 1,2-dioxetanes and pyridopyridazines, or
is selected from radioactivity labels such as incorporated isotopes of iodide, cobalt, selenium, tritium, and phosphor.

A labelling compound may be attached to the MHC molecule, the binding entity, or both to the MHC molecule and the binding entity.

Thus, the present invention relates to methods for detecting the presence of MHC recognising cells in a sample comprising the steps of
(a) providing a sample suspected of comprising MHC recognising cells mounted on a support,
(b) contacting the sample with a MHC molecule as described herein, and
(c) determining any binding of the MHC molecule, which binding indicates the presence of MHC recognising cells.

The invention further relates to methods for monitoring MHC recognising cells comprising the steps of
(a) providing a sample suspected comprising MHC recognising cells mounted on a support,
(b) contacting the sample with a MHC molecule as described herein, and
(c) determining any binding of the MHC molecule, thereby monitoring MHC recognising cells.

The invention also relates to methods for establishing a prognosis of a disease involving MHC recognising cells comprising the steps of
(a) providing a sample suspected comprising MHC recognising cells mounted on a support,
(b) contacting the sample with a MHC molecule as described herein, and
(c) determining any binding of the MHC molecule, thereby establishing a prognosis of a disease involving MHC recognising cells.

Furthermore, the invention relates to methods for determining the status of a disease involving MHC recognising cells comprising the steps of
(a) providing a sample suspected comprising MHC recognising cells mounted on a support,
(b) contacting the sample with a MHC molecule as described herein, and
(c) determining any binding of the MHC molecule, thereby determining the status of a disease involving molecule recognising cells.

The present invention also relates to methods of correlating cellular morphology with the presence of MHC recognising cells in a sample comprising the steps of
(a) providing a sample suspected of comprising MHC recognising cells mounted on a support,
(b) contacting the sample with a MHC molecule as described herein, and
(c) determining any binding of the MHC molecule, thereby correlating the binding of the MHC molecule construct with the cellular morphology.

Also comprised by this invention are methods for diagnosing a disease involving MHC recognising cells, comprising the steps of
(a) providing a sample suspected comprising MHC recognising cells mounted on a support,
(b) contacting the sample with a MHC molecule as described herein, and
(c) determining any binding of the MHC molecule, thereby diagnosing a disease involving MHC recognising cells.

The invention also relates to methods for determining the effectiveness of a medicament against a disease involving MHC recognising cells comprising the steps of
(a) providing a sample from a subject receiving treatment with a medicament mounted on a support,
(b) contacting the sample with a MHC molecule as described herein, and
(c) determining any binding of the MHC molecule, thereby determining the effectiveness of the medicament.

The disease may be of inflammatory, auto-immune, allergic, viral, cancerous, infectious, allo- or xenogene (graft-versus-host and host-versus-graft) origin. In particular, the disease may be a chronic inflammatory bowel disease such as Crohn's disease or ulcerative colitis, sclerosis, type I diabetes, rheumatoid arthritis, psoriasis, atopic dermatitis, asthma, malignant melanoma, renal carcinoma, breast cancer, lung cancer, cancer of the uterus, cervical cancer, prostatic cancer, brain cancer, head and neck cancer, leukaemia, cutaneous lymphoma, hepatic carcinoma, colorectal cancer, bladder cancer, rejection-related disease, Graft-versus-host-related disease, or a viral disease associated with hepatitis, AIDS, measles, pox, chicken pox, rubella or herpes.

The definition of "MHC recognising cells" and examples of MHC recognising cells given above also applies here.

The sample to be subjected to the methods of the invention may suitably be selected from histological material, cytological material, primary tumours, secondary organ metastasis, fine needle aspirates, spleen tissue, bone marrow specimens, cell smears, exfoliative cytological specimens, touch preparations, oral swabs, laryngeal swabs, vaginal swabs, bronchial lavage, gastric lavage, from the umbilical cord, and from body fluids such as blood (e.g. from a peripheral blood mononuclear cell (PBMC) population isolated from blood or from other blood-derived preparations such as leukopheresis products), from sputum samples, expectorates, and bronchial aspirates. Such may be subjected to various treatments. Reference is made to the definitions given above, which also apply here.

### ILLUSTRATIVE EMODIMENTS

### 1. Applicability of the present invention in breast cancer

One example of the utility of the methods of the present invention employing the MHC molecule constructs is diagnosis of breast cancer as well as selection of the suitable therapeutic regime for treating specific types of breast cancer.

Breast cancer is one of the leading causes of cancer in women. New therapeutic approaches consist of administering synthetic peptides to induce cytotoxic T-lymphocyte (CTL) responses to antigens expressed on breast tumour cells. Because CTLs are the immune cells most capable of directly killing tumour cells, vaccines that induce these immune responses are an attractive option of preventing cancer recurrences or inhibiting tumour progression in early stages of the disease.

Tissue samples taken from a patient suspected of suffering from breast cancer can be taken, stained with MHC molecule constructs of the invention, wherein the MHC molecules are loaded with peptides expressed by tumour cells, and insofar binding is observed a diagnosis can be made.

Tissue samples taken from an already diagnosed breast cancer patient can be taken, and stained with MHC molecule constructs of the invention loaded MHC molecules filled with certain peptides to investigate whether a certain peptide is involved in the disease. If binding is observed, the optimal treatment can be selected. For examples, some aggressive breast cancers expresses HER2/neu, others do not. Accordingly, such information will be valuable both in the selection of treatment, in the prognosis of the disease, and in the prediction of the progress of the disease. Other peptides of relevance are MAGE, CEA, and pS3 peptides.

### 2. Applicability of the present invention for specific binding of CTLs

Tissue samples taken from a cancer patient treated with a therapeutic substance can be taken, stained with MHC molecule constructs of the present invention, loaded with the correct MHC allele, and wherein the MHC molecules are loaded with peptides recognising the specific antigen and infiltrating CTLs in the cancer tissue sample. A positive staining of (binding to) the CTLs will indicate the effectiveness of the particular administered therapeutic composition, as induced CTLs are identified at the actual site of the cancer. By double staining procedures, the specific and all the CTLs can be stained. This will provide additional information on the effectiveness of the formulation. For example, a positive staining using one specific peptide will indicate that the corresponding peptide will be particular effective as a component in the therapeutic composition. Thus, the therapeutic composition can be adjusted/altered in accordance with the patient's response based on the identification (binding) of specific infiltrating CTLs in the cancerous tissue.

Furthermore, specific CTLs circulating in the blood stream can be identified and quantified using MHC molecule constructs (with correct allele and peptides) targeting such CTLs specifically, by subsequently analyse in a flow cytometer, whereby binding cells are counted. The less invasive nature of circulating blood analysis makes flow cytometric analysis use for the continuous monitoring of the immune response.

Another approach is a combination of detecting antigen and infiltrating CTLs in cancer tissue samples in double or triple staining procedures to identify various chemokines. The MHC molecule constructs loaded with appropriate peptide filled MHC molecules and optionally other substances (biologically active compounds) are indeed a possibility. The degree of CTL infiltration can then be correlated with certain chemokines and allow for manipulating of specific CTLs to infiltrate and kill the cancer.

Also by the present invention such CTLs can be isolated from a patient by an immunomagnetic separation procedure using MHC molecule constructs, loaded with peptide filled MHC molecules specifically binding to the CTLs. After isolation, the CTLs can be expanded, optionally stimulated, and then re-introduced to the patient, either alone as an effective treatment or as part of a treatment.

### 3. Applicability of the present invention in connection with transplantation

The present invention can also be used to follow the T-cell response against certain vira in transplanted patients. Transplant patients are often susceptible to virus attacks due to the necessary immunosuppressive therapy to avoid graft rejection. By monitoring the post-transplant development of vira-specific T-cells that assist on-coming vira, the immunosuppressive treatment or other preventive medicine can be adjusted to match the immune status of the patient. This can be done using MHC molecule constructs, wherein the MHC molecules are loaded with peptides recognised by such T-cells emerging from on-going infection. Examples of vira, a phycisian may want to monitor, are CMV (cytomegalo virus) and EBV (Epstein-Barr virus).

Another approach will be to isolate and expand to a clinically relevant number cells capable of fighting such infection. Isolation can be done using the appropriate MHC molecule construct capable of binding to such cells, whereafter such bound cells can be separated by an immunomagnetic separation procedure or by flow cytometry. The cells can then be expanded and optionally stimulated or further activated, and then re-introduced to the patient to help the patient's immune system fight the infection.

The present invention can also be used to monitor the T-cell response in transplanted patients towards the graft or host tissue. The appropriate MHC molecule construct is applied, and the binding observed is used to adjust the immunosuppresive treatment to correlate with the immune status of the patient.

### 4. Applicability of the present invention in connection with various infections

The present invention can be used to detect and monitor the T-cell response (or more correctly, the specific T-cell receptors) in connection with complex diseases such as influenza, tuberculosis, malaria, herpes simplex, and chlamydia. MHC molecule constructs, wherein the MHC molecules are loaded with the appropriate peptides can be applied in the detection and monitoring.

The information such obtained can be used to deduce new treatment schemes, or even to develop new medicaments.

Cells capable of fighting the infection can also be obtained using appropriate MHC molecule constructs. Isolation can be done using an immunomagnetic separation procedure or flow cytometry. The so obtained can be expanded under suitable conditions, optionally stimulated or further activated, and then be re-introduced to the patient.

### 5. Preparation of SA poly acrylic amide molecule

A carrier molecule of poly acrylic amide having attached thereto a plurality of steptavidin (SA) as binding entities is prepared according to the following procedure.

Streptavidine (SA, Genzyme) is dialysed overnight (100 mg in 5 ml, against 1000 ml, 0.10 M NaCl, 2-4°C, 10 kDa MwCO, change three times). In the following, all buffers are saturated with nitrogen before use. Acrylic acid N-hydroxysuccinimide ester (Sigma Chemical Co., catalogue number A8060) stored in the freezer is allowed to stand at room temperature for one hour before being opened and dissolved in dry NMP (7 mg/ml) and is slowly added to a stirred solution of SA (in total 0.140 ml NMP solution/ml, 5 mg SA/ml, 0.1 M NaCl, 25 mM carbonate buffer, pH 8.5) and stirred at 30°C under nitrogen atmosphere for 2 hours. Any remaining reactive groups are quenched by addition of 1/10 volume reaction mixture of an ethanol amine-containing buffer (110 mM ethanol amine, 50 mM HEPES, 0.1 M NaCl, pH 7.0) and stirred for 30 minutes at 30°C.

Polymerisation is initiated by addition of a 10% ammonium persulfate solution in water (ammonium persulfate, >98%, Sigma catalogue number A3678) to the solution (0.005 ml per ml), followed by addition of N,N,N",N"-tetramethylethylenediamine (TEMED) (Sigma catalogue number T9281, 0.005 ml per ml). The reaction mixture is stirred for 4 hours at 30°C. The partly inhomogeneous reaction mixture is transferred to a dialysis tube and dialysed against 100 fold excess volume of 0.10 M NaCl (2-4°C, 10 kDa MwCO, change three times during 24 hours). The solution containing the polymeric conjugate is filtered through a 20 my polysulfone filter before being purified from unbound SA by gel filtration (FPLC, Pharmacia, S-500, 0.1 M HEPES, 0.1 M NaCl, pH 7.2). The only fractions clearly separated from the free SA and not in the void peak are collected as the SA poly acrylic amide molecule fraction. The degree of SA incorporation on the poly acrylic amide carrier molecule can be calculated from the UV absorbance at 278 nm. The SA poly acrylic amide carrier molecule is then concentrated to approximately 3.0 mg SA per ml. The molecule can is labelled with label, e.g. FITC or Alexa as described below.

### 6. Preparation of carboxyl-modified dextran and pullulan carrier molecules

Dextran (500 kDa, Pharmacia BioTech, T-500, catalogue number 17-0320-2) or pullulan (Sigma, catalogue number 70051, Mw 400 kDa, polydisparity MW/MN = 1.38) is dissolved in water, cooled on ice and added potassium hydroxide and monochloroacetic acid (Fluka, catalogue number 24510) (in total, 1.0 mg dextran or pullulan/ml, 0.58 mg monochloroacetic acid/ml, ice cold, stirring for 4 hours). The pH in the reaction solution is adjusted to pH 7 by slowly adding dilute hydrochloric acid while stirring on an ice bath. The solution is dialysed extensively against water (100 fold excess volume, room temperature, 10 kDa MwCO, and change six times during 24 hours). The dilute solution is collected and freezer dried over several days to yield a completely dry powder. The degree of carboxyl methyl activation is measured by proton NMR analysis by comparing the methyl group integrals at 4.2 ppm with the carbohydrate backbone integrals. The dry powder is stored in a discicator at room temperature.

### 7. Preparation of rabbit-anti-biotin antibody or SA attached to carboxyl-modified dextran or attached to carboxyl-modified pullulan

Carrier molecules being carboxyl-modified dextran or carboxyl-modified pullulan having attached thereto a number of binding entities being rabbit-anti-biotin antibody or SA can be prepared the following way. The carboxyl-modified dextran or carboxyl-modified pullulan is dissolved and added N-hydroxysuccinimide (Aldrich cat. No. 13067-2) and 1-ethyl-3-(3-dimethylaminopropyl)carbo-diimide hydrochloride (EDAC, Aldrich, catalogue number E6383, 191.7) (2.0 mg carboxyl modified carbohydrate/ml, 1.42 mg NHS-OH/ml, 0.59 mg EDAC/ml, 50 mM 2-(N-morpholino)ethanesulfonic acid ("MES", Aldrich, catalogue number M8250), 100 mM NaCl, pH 6.0) followed by stirring at room temperature for one hour. The dialysed binding protein, fab2 affinity purified rabbit-anti-biotin antibody or SA is added (in total 1.06 mg activated carboxyl modified carbohydrate/ml, 5.3 mg Rabbit anti biotin /mL or 6.4 mg streptavidine/mL, 50 mM MES, 100 mM NaCl, pH 6.0). After standing overnight at room temperature, any remaining reactive groups are quenched by addition of 1/10 volume reaction mixture of an ethanol amine-containing buffer (110 mM ethanol amine, 50 mM HEPES, 0.1 M NaCl, pH 7.0) and stirred for 30 minutes at 30°C. The solution containing the binding entity-conjugated carrier molecule is filtered through a 20 my polysulfone filter before being purified from unbound protein by gel filtration (FPLC, Pharmacia, S-500, 0.1 M HEPES, 0.1 M NaCl, pH 7.2). Only the fraction clearly separated from the free streptavidine and not in the first exclusion peak is collected as the conjugate fraction. The degree of attachment of antibody or SA to the carrier molecule can be calculated from the UV absorbance at 278 nm. The binding entity-conjugated carrier molecule is concentrated to approximately 3.0 mg antibody or protein per ml. The conjugate then is labelled with a plurality of labelling compounds, e.g. FITC or Alexa as described below.

### 8. Preparation of SA N-vinylpyrrolidone/N-acryloamide molecules

Here the carrier molecule is N-vinylpyrrolidone/N-acryloxysuccinimide copolymer and the binding entity is SA.

Activated N-vinylpyrrolidone/N-acryloxysuccinimide copolymer is prepared by copolymerisation of N-vinylpyrrolidone (NVP, Aldrich) and acrylic acid N-hydroxysuccinimide ester (Sigma Chemical Co., catalogue number A8060) according to the standard procedures. N-vinylpyrrolidone/N-acryloxysuccinimide copolymer in NMP (approximately 200 kDa with broad molecular weight distribution, 10 mg/ml NMP) is dropwise added to dialysed SA in solution while stirring (in total, 6.0 mg SA/ml, 1.0 mg copolymer/ml, 100 mM NaCl, 50 mM carbonate, pH 9.0, 30°C, 6 hours). Any remaining reactive groups are quenched by addition of 1/10 volume reaction mixture of an ethanol amine-containing buffer as above, and the hazy polymer conjugate solution filtered through a 20 my polysulfone filter before being purified from unbound SA by gel filtration and concentrated to approximately 3.0 mg SA per ml as described above. The conjugate is labelled with a plurality of labelling compounds, e.g. FITC or Alexa as described below.

### 9. Preparation of MHC molecules constructs

To prepare MHC molecule constructs from the above molecules 5-8, MHC molecules can be attached to the binding entity-conjugated carrier molecules by adding biotinylated MHC molecules to the conjugates in a PBS buffer. The MHC molecules may be selected as desired (e.g. HLA, or various HLA alleles). The MHC molecules may be peptide filled or empty as desired.

### 10. MHC molecule construct, wherein the MHC molecule is attached directly to the carrier molecule

The following example illustrates conjugation of MHC molecules directly to an activated polymer carrier molecule being 500 kDa dextran under mild conditions.

Purified MHC molecule (100 nM heavy chain containing the peptide of interest and β₂m) is added to vinylsulfone-activated dextran (500 kDa, approximately 25% activated, in total 2 ng dextran/ml, 10 ng MHC molecule/ml, 200 mM MES, 100 mM NaCl, pH 6.0). A saturated ammonium sulfate solution is slowly added to the mixture (in total 60% of original volume) while stirring. After 6 hours at 30°C, the mixture is centrifuged (10.000 g), the clear solution removed and the pellet dissolved in water (0.25 ml per mg protein). Any remaining reactive groups are quenched by addition of 1/10 volume reaction mixture of an ethanol amine-containing buffer as described previously, and the polymer conjugate solution filtered through a 20 my polysulfone filter before being purified from unbound protein by gel filtration, giving more than 5 MHC molcule per dextran chain in average, and concentrated to approximately 3.0 mg MHC molecule per ml as described previously. The construct is labelled with a suitable labelling compound, e.g. FITC or Alexa following the procedures below.

### 11. Preparation of MHC molecule constructs, wherein the labelling compound is alkaline phosphatase (AP)

The following example illustrates attachment (conjugation) of MHC molecule constructs wherein a plurality of MHC molecules and a plurality of labelling compounds are attached directly to a carrier molecule. The carrier molecule is dextran, and the labelling compound is AP.

Purified MHC molecule (100 nM heavy chain containing the peptide of interest and β₂m) and dialyzed AP (Roche, dialyzed against 100 mM NaCl) are added to vinylsulfone-activated dextran (500 kDa, approximately 25% activated, in total 2 ng dextral/ml, 10 ng MHC molecule/ml, 20 ng AP/ml, 200 mM MES, 100 mM NaCl, pH 6.0). A saturated ammonium sulphate solution is slowly added to the mixture (in total 60% of original volume) while stirring. After 6 hours at 30°C, the mixture is centrifuged (10.000 g), the clear solution removed and the pellet dissolved in water (0.25 ml per mg MHC molecule). Any remaining reactive groups are quenched by addition of 1/10 volume reaction mixture of an ethanol amine-containing buffer as described previously, and the polymer conjugate solution filtered through a 20 my polysulfone filter before being purified from unbound protein by gel filtration. The conjugate is added protein and enzyme stabilisers.

The invention is further illustrated by the following, non-limiting examples.

### EXAMPLES

Until now, several examples on specific binding of oligomerised e.g. tetramer MHC Class I and II molecules have been used to identify peptide epitope specific T-cell populations. The high avidity of oligomer MHC complexes, e.g. tetramers, has added a new and significant tool to analyse clonal T-cell responses toward pathogenic organisms and antigens. In following examples, the binding of multi-valent MHC molecule constructs of the invention has been addressed.

The examples described below intent to characterise peptide specific and high avidity binding of MHC molecules displaying poly-ligands to different peptide epitope specific T-cell clones and lines when these are presented in the form of constructs according to the invention.

It was surprisingly found that improved binding avidity, generated by the high valence of MHC molecule constructs according to the invention result in improved detection of subtle T-cell populations as compared to the prior art MHC molecule tetramers.

### EXAMPLE 1

### Production of poly-ligand MHC molecule and tetramers

### A. Production of carrier molecules with binding entities

### Vinylsulfon activated dextran

Dextrans of different molecular sizes (150 and 270 kDa from Pharmacosmos, 500 kDa from Pharmacia) were activated with divinylsulfon (Aldrich) according to the description by A. Lihme and T. Boenisch ("Water soluble, polymer based reagents and conjugates comprising moieties derived from divinely sulfone", WO 93/01498, ref. 22) resulting in a degree of vinylsulfone activation of approximately 25% of the monomer units.

FITC-streptavidine-dextran (150, 270, 500 kDa) conjugates Streptavidine (SA, Genzyme) was dialysed overnight (100 mg in 5 ml, against 1000 ml 0.10 M NaCl, 2-4°C, 10 kDa MwCO, changed three times).

A fluorescein isothiocyanate (FITC, Molecular Probes) solution (14.0 mg/ml DMF) was added to a stirred mixture of streptavidine (14.0 mg SA/ml, 0.19 mg FITC/ml, 0.1 M NaCl, 25 mM carbonate buffer, pH 8.5, 30°C).

After 6 hours, the reaction mixture was added to a solution of vinylsulfon-activated dextran (approximately 25% activated) of 150, 270 or 500 kDa (in total 1.6 mg vinylsulfon dextran/ml, 7.7 mg SA/ml, 0.1 M NaCl, 25 mM carbonate buffer, pH 8.5) and stirred at 30°C 18 hours.

Any remaining reactive groups were quenched by addition of 1/10 volume reaction mixture of an ethanolamine-containing buffer (110 mM ethanolamine, 50 mM HEPES, 0.1 M NaCl, pH 7.0) and stirred for 30 minutes at 30°C.

The obtained polymeric conjugate was purified from free fluorescein and unbound streptavidine by gelfiltration (FPLC, Pharmacia, S-200, 0.1 M HEPES, 0.1 M NaCl, pH 7.2).

The degree of fluorescein and streptavidine incorporation could be calculated from the UV absorbance at 278 and 498 nm in the three fractions containing conjugate, unbound streptavidine and unbound fluorescein, respectively. The conjugates were added sodium azide to 15 mM as a preservative.

| Dextran carrier molecule | SA per dextran | FITC per SA | Concentration dextran (mole/l) |
|---|---|---|---|
| 150 | 4.4 | 2.7 | 61.1×10⁻⁸ |
| 270 | 6.9 | 2.6 | 54.7×10⁻⁸ |
| 500 | 13.6 | 2.7 | 31.2×10⁻⁸ |

Unless otherwise stated the FITC conjugated 500, 270 and 150 kDa dextrans used in examples described below were conjugated in average with about 13.6 (in the case of the 500 kDa dextran), 6.9 (in the case of the 270 kDa dextran) and 4.4 (in the case of the 150 kDa dextran) SA complexes per dextran molecule.

### Preparation of HRP-streptavidine-dextran (70, 150, 270 kDa) conjugates

Horseradish peroxidase (HRP, Fairzyme) and streptavidine (SA, Genzyme) were dialysed overnight (100 mg in 5 ml, against 1000 ml 0,10 M NaCl, 2-4°C, 10 kDa MwCO, changed three times) before being concentrated. The conjugation was performed by sequential addition of HRP and streptavidine to activated dextran.

The HRP solution was added to a solution of vinylsulfon activated dextran (approximately 25% activated) of 70, 150 or 270 kDa (totally 40.0 mg HRP/ml, 1.6 mg dextran/ml, 25 mM carbonate, 0.1 M NaCl, pH 8.5) and stirred on a water bath (30°C, 6.0 hours). The streptavidine solution was added to the reaction mixture (totally 9.14 mg streptavidine/ml, 1.06 mg dextran/ml, 26.67 mg HRP/ml 25 mM carbonate, 0.10 M NaCl, pH 8.5) and stirred overnight on a water bath (30°C, 18 hours).

Any remaining reactive groups were quenched by addition of 1/10 volume reaction mixture of an ethanolamine-containing buffer (110 mM ethanol amine, 50 mM HEPES, 0.1 M NaCl, pH 7.0) and stirred for 30 minutes at 30°C.

The conjugate was separated from unconjugated streptavidine and HRP by gelfiltration (FPLC, Pharmacia, S-200, 0.1 M HEPES, 0.1 M NaCl, pH 7.2).

The degree of HRP and streptavidine incorporation could be calculated from the UV absorbance at 280 and 403 nm of the fraction containing the conjugate and the fraction containing streptavidine and HRP. The conjugates were added BSA as protein stabiliser and bronidox as preservative.

| Dextran carrier molecule | SA per dextran | HRP per dextran | Concentration dextran (mole/l) |
|---|---|---|---|
| 70 | 3.0 | 2.3 | 10.5×10⁻⁸ |
| 150 | 5.4 | 3.7 | 7.0×10⁻⁸ |
| 270 | 8.0 | 5.3 | 4.6×10⁻⁸ |

### B. Production of peptide-loaded MHC molecules

The HLA Class I heavy and light (β₂m) chains were produced and partially purified as inclusion bodies from an E.coli strain (BL21 (DE3), Novagen (Novagen, Inc, Madison, WI, USA) following standard procedure.

The isolated inclusion body molecules were solubilised in 8M urea at non-reducing conditions to obtain heavy chain molecule with intact disulphide bonds. The heavy chain molecule was additionally purified by size- and ionexchange chromatography following standard procedure and finally subjected to folding as described below.

Peptide epitope specific HLA Class I complexes were generated in vitro using a "folding by dilution" approach where the higly purified preparations of denatured HLA Class I heavy chain molecule (about 10-20 µM in 8M urea) A0201, 1-275) were renatured by incubation in a 100-fold dilution buffer (final concentration of heavy chain is thus about 100-200 nM) containing the peptide of interest (10 µM) and β₂m (1 µM), for 16 hours at 18°C. Misfolded HLA Class I heavy chain was precipitated by centrifugation prior to purification of *de* novo folded HLA Class I molecule by G75 size exclusion chromatography following standard procedure. The fraction of folded HLA A0201 molecule was ruinously about 40-50% of total amount of HLA A0201 heavy chain molecule added to the folding reaction. The fraction of misfolded heavy chain molecule contained inappropriate disulphide bonds and was not available for renaturation. This folding scheme, described above, was useful for rapid generation of a variety of peptide-loaded monomer MHC Class I complexes encoded by the polymorphic HLA and H-2 gene complexes. The purified complexes were finally enzymatic mono-biotinylated utilising protein ligase BIR A as described by the manufacturer (AVIDITY; Denver, Co, USA).

### C. Production of peptide empty HLA Class I molecules

Peptide empty MHC Class I was produced in a process where functional mono-biotinylated MHC Class I complexes (cf. example 1B) initially were denatured by addition of urea (8M) or guanidine (6M). The chaeotrophic buffers dissociated the structural molecule subunits from the Class I complex, leaving free soluble biotinylated heavy chain and free soluble β₂m molecules available for biochemical purification. The heavy chain molecule was excluded from the dissociated β₂m and peptide by G75 size exclusion chromatography following standard procedure. The purified heavy chain molecule form spontaneously a peptide receptive hetero-dimer complex consisting of heavy and light chain in a folding buffer containing excess β₂m (cf. Example 1:B hereabove). The peptide empty HLA Class I dimer remained stable in excess of β₂M and could be ligated to streptavidin to form peptide empty construct of the invention or peptide empty tetramer. Peptide (1 µM) of interest were be added to during or after the process of ligation with soluble or SA-conjugated dextran to generate TCR-binding MHC molecules in the form of MHC molecule constructs of the invention or MHC molecule tetramers.

### D. Production of poly-ligand MHC molecule constructs of the invention

The preparations of SA conjugated dextrans of different molecular sizes were mixed with amounts of HLA complexes corresponding to a ratio of two biotinylated HLA Class I molecules per SA molecule. The HLA molecule was added directly to a solution of SA-conjugated dextrans. Thus, MHC molecule constructs were formed comprising
(1) a carrier molecule being a 500 kDa dextran having attached thereto 27.2 biotinylated HLA Class I molecules (MHC molecules) via about 13.6 FITC-labelled SA (binding entities) (in average 2 HLA Class I molecules per SA), each SA labelled in average with 2.7 FITC,
(2) a carrier molecule being a 270 kDa dextran having attached thereto about 13.8 biotinylated HLA Class I molecules (MHC molecules) via about 6.9 FITC-labelled SA (binding entities) (in average 2 HLA Class I molecules per SA), each SA labelled in average with 2.6 FITC,
(3) a carrier molecule being a 150 kDa dextran having attached thereto about 8.8 biotinylated HLA Class I molecules (MHC molecules) via about 4.4 FITC-labelled SA (binding entities) (in average 2 HLA Class I molecules per SA), each SA labelled in average with 2.7 FITC,
(4) a carrier molecule being a 70 kDa dextran having attached thereto about 6.0 biotinylated HLA Class I molecules (MHC molecules) via about 3.0 SA (binding entities) (in average 2 HLA Class I molecules per SA), each dextran labelled in average with 2.3 HRP enzymes,
(5) a carrier molecule being a 270 kDa dextran having attached thereto about 10.8 biotinylated HLA Class I molecules (MHC molecules) via about 5.4 SA (binding entities) (in average 2 HLA Class I molecules per SA), each dextran labelled in average with 3.7 HRP enzymes,
(6) a carrier molecule being a 270 kDa dextran having attached thereto about 16.0 biotinylated HLA Class I molecules (MHC molecules) via about 8.0 SA (binding entities) (in average 2 HLA Class I molecules per SA), each dextran labelled in average with 5.3 HRP enzymes.

The attachment of this high number of HLA Class I molecules was possible due to the high affinity between SA and biotin (affinity dissociation constant; K_{D}= 10¹⁵).

By this procedure, the following MHC molecule constructs of the present invention were prepared:
a MHC molecule construct comprising the 500 kDa dextran carrier molecule having attached thereto 27.2 biotinylated HLA A0201 in complex with the MART-1 peptide analogue (ELAGIGILTV) and β₂m via 13.6 FITC labelled SA (in average 2 HLA A0201 molecules per SA, and in average 2.7 FITC per SA) (MHC molecule construct 1),
a MHC molecule construct comprising the 270 kDa dextran carrier molecule having attached thereto 13.8 biotinylated HLA A0201 molecules in complex with the MART-1 peptide analogue (ELAGIGILTV) and β₂m via 6.9 FITC labelled SA (in average 2 HLA A0201 molecules per SA, and in average 2.6 FITC per SA) (MHC molecule construct 2),
a MHC molecule construct comprising the 150 kDa dextran carrier molecule having attached thereto 8.8 biotinylated HLA A0201 molecules in complex with the MART-1 peptide analogue (ELAGIGILTV) and β₂m via 4.4 FITC labelled SA (in average 2 HLA A0201 molecules per SA, and in average 2.7 FITC per SA) (MHC molecule construct 3),
a MHC molecule construct comprising the 500 kDa dextran carrier molecule having attached thereto 27.2 biotinylated HLA A0201 in complex with the influenza matrix protein amino acids 58-66 (GILGFVFTL) and β₂m via 13.6 FITC labelled SA (in average 2 HLA A0201 molecules per SA and in average 2.7 FITC per SA) (MHC molecule construct 4),
a MHC molecule construct comprising the 500 kDa dextran carrier molecule having attached thereto 27.2 biotinylated HLA A0201 in complex with the wild type P53 peptide R9V (RMPEAAPPV) and β₂m via 13.6 FITC labelled SA (in average 2 HLA A0201 molecules per SA and in average 2.7 FITC per SA) (MHC molecule construct 5),
a MHC molecule construct comprising the 500 kDa dextran carrier molecule having attached thereto 27.2 biotinylated HLA A0201 in complex with the wild type P53 peptide G11V (GLAPPQHLIRV) and β₂m via 13.6 FITC labelled SA (in average 2 HLA A0201 molecules per SA and in average 2.7 FITC per SA) (MHC molecule construct 6),
a MHC molecule construct comprising the 500 kDa dextran carrier molecule having attached thereto 27.2 biotinylated peptide empty HLA A0201 via 13.6 FITC labelled SA (in average 2 HLA A0201 molecules per SA and in average 2.7 FITC per SA) (MHC molecule construct 7),
a MHC molecule construct comprising the 500 kDa dextran carrier molecule having attached thereto 27.2 biotin-ylated HLA A0201 in complex with the gp100 peptide KTWGQYWOV and β₂m via 13.6 FITC labelled SA (in average 2 HLA A0201 molecules per SA and in average 2.7 FITC per SA) (MHC molecule construct 8),
a MHC molecule construct comprising the 500 kDa dextran carrier molecule having attached thereto 27.2 HLA A0201 heavy chain in complex with the MART-1 peptide analogue (ELAGIGILTV) and iodinated β₂m via 13.6 SA (in average 2 HLA A0201 molecules per SA), having a radioactivity of 100000 cpm/sample) (MHC molecule construct 9),
a MHC molecule construct comprising the 270 kDa dextran carrier molecule having attached thereto 16.0 biotinylated HLA A0201 in complex with the Mart-1 peptide analogue (ELAGIGILTV) and β₂m via 8.0 SA (in average 2 HLA A0201 molecules per SA) and 5.3 HRP enzymes to the dextran (MHC molecule construct 10),
a MHC molecule construct comprising the 500 kDa dextran carrier molecule having attached thereto 27.2 biotinylated HLA A0201 in complex with the sur1/M2 peptide analogue (LMLGEFLKL) and β₂m via 13.6 FITC labelled SA (in average 2 HLA A0201 molecules per SA, and in average 2.7 FITC per SA) (MHC molecule construct 11),
a MHC molecule construct comprising the 150 kDa dextran carrier molecule having attached thereto 10.8 biotinylated HLA A0201 in complex with the MART-1 peptide analogue (ELAGIGILTV) and β₂m via 5.4 SA (in average 2 HLA A0201 molecules per SA) and 3.7 HRP enzymes to the dextran (MHC molecule construct 12),
a MHC molecule construct comprising the 70 kDa dextran carrier molecule having attached thereto 16.0 biotinylated HLA A0201 in complex with the MART-1 peptide analogue (ELAGIGILTV) and β₂m via 3.0 SA (in average 2 HLA A0201 molecules per SA) and 2.3 HRP enzymes to the dextran (MHC molecule construct 13),
a MHC molecule construct comprising the 500 kDa dextran carrier molecule having attached thereto 27.2 biotinylated HLA A0201 in complex with the MAGE-3 peptide (FLWGPRALV) and β₂m via 13.6 FITC labelled SA (in average 2 HLA A0201 molecules per SA and in average 2.7 FITC per SA) (MHC molecule construct 14),
a MHC molecule construct comprising the 500 kDa dextran carrier molecule having attached thereto 14.1 biotinylated HLA A0201 in complex with the MART-1 peptide analogue (ELAGIGILTV) and β₂m via 13.6 FITC labelled SA (in average 1 HLA A0201 molecules per SA, and in average 2 FITC per SA) (MHC molecule construct 15),
a MHC molecule construct comprising the 500 kDa dextran carrier molecule having attached thereto 14.1 biotinylated HLA A0201 in complex with the MART-1 peptide analogue (ELAGIGILTV) and β₂m and 7.1 MIC A molecules via 13.6 FITC labelled SA (in average 1 HLA A0201 molecules per SA, and in average 2 FITC per SA) (MHC molecule construct 16),
a MHC molecule construct comprising the 500 kDa dextran carrier molecule having attached thereto 14.1 biotinylated HLA A0201 in complex with the gp100 peptide KTWGQYWOV and β₂m via 13.6 FITC labelled SA (in average 1 HLA A0201 molecules per SA and in average 2 FITC per SA) (MHC molecule construct 17),
a MHC molecule construct comprising the 500 kDa dextran carrier molecule having attached thereto 14.1 biotinylated HLA A0201 in complex with the gp100 peptide KTWGQYWOV and β₂m and 7.1 MIC A molecules via 13.6 FITC labelled SA (in average 1 HLA A0201 molecules per SA and in average 2 FITC per SA) (MHC molecule construct 18).

### EXAMPLE 2

### Production of MHC molecule tetramers

The peptide epitope specific HLA molecule used for the tetramers was generated as described in Example 1, B. The tetramers were formed by sequential addition of small amounts of PE-conjugated SA (Molecular Probes, Holland) to a solution of biotinylated HLA complexes. The final amount of HLA complex in the mixture should be four-fold the amount of SA to ensure saturation (four biotin binding sites per SA complex).

By this procedure, the following tetramers were prepared:
A PE-labelled tetramer consisting of four biotinylated HLA A0201 in complex with the modified MART-1 peptide (ELAGIGILTV) and β₂m (tetramer 1),
a PE-labelled tetramer consisting of four biotinylated HLA A0201 in complex with the gp100 peptide (KTWGQYWOV) (tetramer 2) and β₂m,
a PE-labelled tetramer consisting of four biotinylated HLA A0201 in complex with the influenza matrix protein amino acids 58-66 (GILGFVFTL) (tetramer 3),
a PE-labelled tetramer consisting of four biotinylated HLA A0201 in complex with the wild type P53 peptide R9V (RMPEAAPPV) (tetramer 4),
a PE-labelled tetramer consisting of four biotinylated HLA A0201 in complex with the wild type P53 peptide G11V (GLAPPQHLIRV) (tetramer 5),
a PE-labelled peptide empty tetramer consisting of four PE-labelled peptide empty HLA A0201 (tetramer 6).

### EXAMPLE 3

### Dose dependent binding of MHC molecule constructs according to the invention as compared to MHC molecule tetramers to the T-cell is peptide specific

In this experiment, the binding of peptide epitope specific MHC molecule constructs of the invention and MHC molecule tetramers to established T-cell clones was investigated.

Previously established and characterised "in house" T-cell clones, named 5/127 and 5/130, which reacted against melanoma specific tumour antigens, were utilised to analyse binding of the HLA molecule constructs (i.e. MHC molecule constructs) of the invention to TCR on cell surfaces by flow cytometry following a standard flow cytometry protocol. Briefly, 5×10⁵ cells were incubated in 50 *µ*l "FACS-buffer" (phosphate-buffered saline (PBS), 10 mg/ml bovine serum albumin (BSA), 0.2% azide) with either the poly-ligand MHC molecule constructs of the invention or the tetramers, displaying the peptides of interest. Unless otherwise stated, the cells were washed once in the FACS buffer and analysed on a Becton Dickenton FACSCalibur flow cytometer.

The two T-cell clones reacted specifically with HLA A0201 bound peptides from the tumour (melanoma)-associated antigens MART-1 and gp100, respectively.

The following poly-ligand HLA molecule constructs of the invention were used:
MHC molecule construct 1,
MHC molecule construct 2,
MHC molecule construct 3.

The following MHC molecule tetramers were used:
tetramer 1,
tetramer 2.

The PE-labelled tetramers 1 and 2 were used for comparison.

The T-cell clones 5/127 and 5/130 were thawed and grown 24 hours at 37°C in presence of 50U IL-2 and 10% human serum. About 5×10⁵ T-cell clones were incubated 1 hour at 22°C with graded doses of MHC molecule construct of the invention (MHC molecule construct 1: 0-9.36 nM, 2-fold dilutions, cf. Figure 25; MHC molecule construct 2: 0-27.5 nM, 2-fold dilutions, cf. Figure 25; MHC molecule construct 3: 0-37.5 nM, 2-fold dilutions, cf. Figure 25) or PE-labelled tetramers (tetramers 1 and 2; 0-200 nM, 2-fold dilutions, cf. Figure 25). After incubation, the cells were washed only once to avoid dissociation of low avidity bound MHC molecule constructs or tetramer, and analysed by flow cytometry following standard flow cytometry procedures for cell bound MHC molecule construct (results shown in Figure 25B) and PE-labelled tetramer (results shown in Figure 25A).

The MART-1 peptide specific T-cell clone 5/127 (indicated as squares in Figure 25A) bound tetramers that displayed ELAGIGILTV peptide (open squares) with high avidity. Half-maximal staining of the 5/127 T-cells was observed by addition of 20-30 nM of tetramers. In the control experiment, the tetramer preparation that displayed the gp100 peptide KTWGQYWOV (filled squares) did not interact with the 5/127 T-cell clones. In comparison, the gp100 reactive T-cell clone 5/130 was stained with tetramers displaying the gp100 peptide KTWGQYWOV (black circles) and interacted only weakly with high concentrations of tetramers displaying the ELAGIGILTV peptide (open circles). The binding of peptide specific tetramers to the two T-cell lines showed that about 100 nM tetramers almost saturated the 5/127 cell line, whereas the 5/130 cell line was only partially stained due to low avidity binding. Though the peptide specific tetramer preparations clearly bound with different avidity, the data demonstrated clearly that both cell lines bound appropriate peptide-HLA complexes specifically. Thus, it was concluded that both T-cell clones were useful for analysis of the constructs of the invention.

For the subsequent analyses of the binding of different construct of the invention and for with comparison the tetramer constructs, the robust 5/127 T-cell clones were chosen.

The T-cell clone 5/127 was stained as described above with MHC molecule constructs 1, 2 and 3 of the invention. As shown in Figure 25B, all sizes of dextran carrier molecules facilitated a dose dependent staining of the MART-1 specific T-cell clone. In comparison, the larger construct (the 500 kDa dextran carrier molecule) bound more efficiently to the T-cells than the intermediate construct (270 kDa dextran carrier molecule) and the smaller construct (150 kDa dextran carrier molecule). However, as evident from dose dependent staining of the cells shown in Figure 25A, all three constructs stained the 5/127 T-cell clone more efficiently than did the tetramers which had to be added in higher amounts to obtain significant staining of the cells (compare Figure 25A (open squares) with the three curves in Figure 25B). The improved binding avidity of the three constructs of the invention was clearly reflected by the low concentrations of the constructs (2-10 nM) required for half-saturation, whereas the corresponding tetramers required 20-30 nM for half-saturation (cf. the tetramer staining in Figure 25A).

Thus, it was concluded that the constructs of the invention bound dose-dependent to peptide epitope specific T-cells and with higher avidity than corresponding tetramers displaying identical peptides.

### EXAMPLE 4

### Binding of MHC molecule constructs of the invention and tetramers to influenza specific T-cell line

In this experiment, the binding of peptide specific constructs of the invention and tetramers to a T-cell line recognising a conventional non-self peptide presented in context of HLA A0201 molecules was investigated.

Dendritic cells (DC) were generated from freshly isolated PBMC from HLA-A0201 donors following standard protocols, using 250 U/ml hrIL-4 (R&D Systems, Minneapolis, MN, USA) and 500 U/ml hrGM-CSF (Leucomax, Novartis/Schering-Ploug, Basel, Switzerland) for DC culture and 72 hours exposure to hrCD40LT, 1 *µ*g/ml (Immunex Corporation, Seattle, Washington, USA) to induce DC maturation.

On day 10 of culture, DC were isolated by EDTA treatment and loaded with the influenza peptide IMP 58-66 (40 µg/ml) for 1 hour followed by wash and irradiation (3000 Rad). Subsequently, freshly isolated autologous PBMC (2×10⁶/ml) were added to the peptide loaded DC (1-2×10⁵/ml) in 24 well plates in 1 ml AB-medium/well containing 20 U/ml rhIL-4 and 5 ng/ml rhIL-7 (Peprotech EC, London, UK). After 9-11 days, T-cell cultures were depleted for CD4+ cells by Dynabead^{®} separation (according to the manufacturers instructions) and the negatively selected CD8+ cells (4×10⁵/ml) were re-stimulated with peptide pulsed autologous irradiated DC (1-2×10⁵/ml) and irradiated (3000 Rad) autologous PBMC (10⁶/ml) in AB-medium supplemented with rhIL-4 and rhIL-7 in 96 wells U-bottomed plates. Further re-stimulations were performed every 7th day of culture as described above using irradiated (6000 Rad) peptide pulsed HLA-A2⁺ EBV-B-cells (2×10⁵/ml) as stimulators and irradiated (3000 Rad) allogeneic PBMC. rhIL-2 (20 U/ml, Proleukin, Chiron, CA, USA) was added at day 1 after each re-stimulation.

The following MHC molecule constructs of the invention were used:
MHC molecule construct 4,
MHC molecule construct 5,
MHC molecule construct 6.

The following tetramers were used:
tetramer 3,
tetramer 4,
tetramer 5.

The tetramers 3-5 were used for comparison.

5×10⁵ T-cells were incubated in 50 *µ*l FACS-buffer (PBS, 10 mg/ml BSA, 0.2% azide) with the poly-ligand MHC molecule constructs of the invention or the tetramers, all displaying peptides of interest.

The cells were incubated for 90 minutes at 22°C in graded doses of the constructs of the invention (0-32 nM, 2-fold dilutions, cf. Figure 26) or the tetramers (0-112 nM, 2-fold dilutions, cf. Figure 26), washed once and analysed by flow cytometry following standard flow cytometry procedures for cell bound molecule construct of and tetramer, respectively.

As shown in Figure 26, the fraction of peptide specific T-cells (about 55%) in the established cell line was fully stained using low concentrations of the constructs of the invention (<30 nM), whereas the tetramers stained the cells less efficiently. Half-maximal staining was obtained with about 3 nM of the constructs of the invention and 30 nM of the tetramers. In contrast, the constructs of the invention and tetramers expressing wild-type P53 peptides did not stain the T-cells. Thus, it was concluded that the constructs of the invention stained sub-populations of influenza specific T-cells specifically and with higher efficacy than the peptide identical tetramers. As illustrated in Figure 25 and 26, the improved staining efficiency was produced by the higher HLA molecular valence of the constructs as compared to the tetramers.

### EXAMPLE 5

### Time dependence of MHC molecule construct and tetramer binding

In this experiment, it was shown that binding of MHC molecule constructs of the invention appear to be time dependent. The results obtained are shown in Figure 27. For comparison, PE-labelled tetramers displaying the same peptides as the used MHC molecule constructs of the invention were tested in parallel assays.

The following MHC molecule construct of the invention was used:
MHC molecule construct 1.

The following tetramer was used:

### Tetramer 1.

Briefly, 5×10⁵ MART-1 specific T-cell cones (5/127) were incubated in 50 *µ*l FACS-buffer (PBS, 10 mg/ml BSA, 0.2% azide) with the poly-ligand MHC molecule constructs of the invention or the tetramers, all displaying peptides of interest. The T-cell clones were incubated in graded doses (2-fold dilutions, cf. Figure 27) of the construct of the invention or the tetramer, both displaying the MART-1 related peptide analogue ELAGIGILTV. The cells were incubated at room temperature (22°C). Aliquots of cells were taken at different time points (cf. Figure 27) washed and measured by flow cytometry following standard procedures for flow cytometry for cell bound construct (shown in Figure 27B) or tetramer (shown in Figure 27A). As shown in Figure 27A, in case of the tetramer, a steady-state binding was obtained after 1 hour of incubation using high concentrations of tetramer (112 nM), whereas lower concentrations (14-56 nM) did not reach steady-state within the measured time interval. In comparison, the construct of the invention reached a steady-state level within 60 minutes using a significant lower concentration of construct (16 nM).

Thus, it was demonstrated that the association of the constructs of the invention was faster than association of the tetramers, presumably due to a higher valence of the constructs of the invention.

### EXAMPLE 6

### Dissociation of cell bound constructs of the invention

In this experiment, the dissociation of cell bound constructs of the invention was investigated.

The following MHC molecule construct was used:

### MHC molecule construct 1.

T-cell clones (5/127) were incubated with the construct of the invention displaying the MART-1 related peptide analogue ELAGIGILTV. The cells (5×10⁵) were incubated 1 hour at 22°C and washed once and incubated at 4°C, 22°C and 37°C, respectively, in FACS-buffer (PBS, 10 mg/ml BSA, 0.2% azide) containing 50 nM CD8 specific monoclonal antibody to prevent re-binding of dissociating construct. At different time points (0, 60, 90, 120 minutes, respectively), aliquots of cells were taken, washed and analysed for cell bound constructs by flow cytometry following a standard protocol for flow cytometry. The results are shown in Figure 28. At 4°C, the half-life of the construct binding was about 90 minutes, which were reduced to about 50 and 30 minutes at 22°C and 30°C, respectively. The biphasic dissociation of the constructs from cells incubated at 37°C indicated that some degree of internalisation of the construct into the cells took place. Alternatively, biphasic dissociation could explained with complex interaction between the construct and counter receptors on the T-cell surface at 37°C as compared to binding of the same construct at lower temperatures.

Thus, it was concluded that dissociation of cell bound construct was time and temperature dependent.

### EXAMPLE 7

### Binding of construct of the invention: the impact of antibodies

In this experiment, it was shown that cell surface binding of constructs of the invention is affected by HLA Class I specific monoclonal antibodies reacting with HLA Class I epitopes in close proximity of the peptide-binding site.

The following MHC molecule construct of the invention was used:

### MHC molecule construct 1.

The following monoclonal antibodies were used:
BB7.2 (HLA A0201 specific), W6/32 (HLA A,B,C pan specific), BBM1 (human β₂m specific), and mouse anti human T-cell, CD8 Clone DK25 (DAKO Code No. M0707) (CD8-specific antibody).

The MART-1 specific T-cell clone 5/127 was incubated with a mixture of 2 nM construct displaying the MART-1 related peptide epitope with or without 10 nM monoclonal antibody (BB7.2, W6/32, BBM1 and CD8 specific, respectively) as indicated in Figure 29A. The cells were incubated for 90 minutes at 22°C, washed once and analysed flow cytometry following standard procedures for cell bound construct. The monoclonal antibodies B7.1 and W6/32 that reacted with epitopes in close proximity of the peptide binding site of HLA A0201 inhibited as shown in Figure 29A the binding of the construct to a level near the background (the background signal being obtained by incubating..cells with 10 nM FITC-labelled construct with no HLA molecules).

In contrast, the presence of monoclonal antibody BBM1 that bound to the HLA Class I light chain, β₂m, did not affect the binding of the construct.

In a similar experiment (cf. Figure 29B), the impact of a CD8 specific monoclonal antibody was analysed. The T-cells (5/127) were incubated for 60 minutes at 22°C with the MHC molecule construct 1 (cf. Figure 29A) and graded doses of antibody 0-12 nM (cf. Figure 29B). The cells were washed and analysed by flow cytometry following standard flow cytometry procedures for cell bound construct. The CD8 specific antibody strongly inhibited the association of the construct to the T-cells, cf. Figure 29B, suggesting that CD8 molecules on the T-cells contribute significantly to the binding of peptide epitope specific constructs.

Thus, it was concluded that binding of the construct could be blocked by antibodies reacting with the binding site of HLA Class I (BB7.2 and W6/32) displayed by the construct (steric hindrance) and mouse anti human T-cell, CD8 Clone DK25 on the T-cells. It should be noted, however, that none of the inhibitory antibodies used in this study were added in saturating amounts.

### EXAMPLE 8

### Binding of the constructs of the invention: The effect of HLA Class I:dextran ratio during the process of ligation

In this experiment, the more optimal number of HLA Class I molecules per dextran carrier molecule required for maximal cell binding of MHC molecule constructs of the invention was analysed.

The following MHC molecule constructs of the invention were used:
MHC molecule construct 1, however, with different amounts of HLA A0201, cf. below,
MHC molecule construct 2, however, with different amounts of HLA A0201, cf. below,
MHC molecule construct 3, however, with different amounts of HLA A0201, cf. below.

Graded amounts of recombinant biotinylated HLA A0201 complexes displaying the MART-1 peptide analogue (ELAGIGILTV) were added to individual solutions of the constructs comprising different molecular sizes dextran carrier molecules, namely 150, 270 and 500 kDa dextran carrier molecules, respectively. More specifically, a 80 nM solution of 500 kDa dextran (conjugated with 14 SA, each labelled in average with 2 FITC) was incubated in FACS-buffer (PBS, 10 mg/ml BSA, 0.2% azide) with 88-(7040 nM HLA A0201), 44- (3520 nM) and 14- (1121 nM) fold excess of mono-biotinylated HLA A0201 complexes, respectively. The reaction mixture was incubated 60 minutes at 22°C to obtain steady-state between the HLA A0201 and the SA molecules conjugated to the dextran. From the ratios of HLA A0201 and dextran (88, 44, 14 HLA molecules to one dextran molecules) ratios of HLA to SA during ligation corresponding to 6.5, 3.25 and 1, respectively, could be calculated. Due to the high affinity of SA and biotinylated MHC, it was expected that the ligation between HLA A0201 and SA per dextran resulted in fully saturated (88-fold excess of HLA), nearly saturated (44-fold excess of HLA) and partly saturated (14-fold excess HLA) MHC molecule constructs. Assuming 4 binding sites per SA, the molecule constructs are thus loaded with 54.4, 44.2 and 14.1 HLA A0201 molecules per dextran. The solutions were diluted 4-fold (final MHC molecule construct concentration of 20 nM) prior to usage for T-cell staining by flow cytometry following a standard protocol.

In a similar procedure, a 145 nM SA/dextran preparation (270 kDa, 7 SA per dextran, each SA labelled in average with 2 FITC) was ligated with biotinylated HLA A0201. The concentrations of HLA A0201 used for the ligation process were 48.5, 24.2 and 8.1 fold excess of the dextran concentration. The ratio of HLA:SA could be calculated to 7, 3.5 and 1.1, respectively. Assuming 4 binding sites per SA, the molecule constructs were thus loaded with 27.6, 24.2 and 7.6 HLA A0201 molecules per dextran. The solutions were diluted to 20 nM prior to T-cell staining following a standard procedure.

In a similar procedure, a 244 nM SA/dextran preparation (150 kDa, 4.4 SA per dextran, each SA labelled in average with 2 FITC) was ligated with biotinylated HLA A0201. The concentrations of HLA A0201 used for the ligation process corresponded to 28.8 (7040 nM HLA A0201), 14.4 (3520 nM HLA A0201) and 7.2 (1760 nM HLA A0201) fold excess of the dextran concentration. The ratio of HLA:SA could be calculated to 6.5, 3.3 and 1.6, respectively. Assuming 4 binding sites per SA, the molecule constructs were thus loaded with 17.6, 14.5 and 7.0 HLA A0201 molecules per dextran. The solutions were diluted to 20 nM prior to T-cell staining following a standard procedure.

The T-cell clones were incubated for 60 minutes at 22°C prior to staining of the T-cell clone (5/127). The T-cell clones were incubated 60 minutes at room temperature with 20 nM solutions of the MHC molecule constructs loaded with different amounts of HLA A0201. The cells were subsequently washed once and analysed for cell bound construct by flow cytometry following standard flow cytometry procedure. All of the constructs bound as expected specifically to clone 5/127 (results shown Figure 30).

The construct comprising the larger (500 kDa) dextran carrier molecules was - in average - conjugated with 13,6 streptavidin molecules with a theoretical number of binding sites for biotinylated HLA molecules about 54 per dextran molecule (assuming 4 biotin binding sites per SA). In comparison, the construct comprising the 270 kDa and the 150 kDa dextran carrier molecules were conjugated with 6,9 and 4.4 streptavidin molecules per dextran, respectively, with a theoretical number of biotin binding sites corresponding to 42 and 17 biotinylated HLA Class I molecules, respectively.

As shown in Figure 30, the constructs comprising the 500 kDa dextran carrier molecule bound optimally to the T-cells when loaded with a total of 44 HLA Class I molecules per dextran carrier molecule. In comparison, the constructs comprising the 270 and the 150 kDa dextran carrier molecules, respectively, bound optimally when loaded with totally 24.2 and 14.5 HLA Class I molecules per dextran (corresponding to about 4 bound HLA molecules per SA, respectively). The observed density of HLA Class I/dextran corresponded to well to 4 HLA Class I molecules/SA molecule conjugated to the dextran molecule. The observed reduction of T-cell staining using MHC molecule constructs generated in excess HLA molecules could be due to inhibition by unbound monomer HLA molecules. Thus, it was concluded that the MHC molecule constructs of the invention bound optimally to peptide specific T-cells when all available biotin binding sites of the carrier molecule were saturated during the process of ligation. Excess of unbound monomeric MHC molecule inhibited, however, the interaction between MHC molecule constructs and specific TCRs. Thus, the ligation process should, consequently, be performed with 1:1 ratio of MHC molecules to binding sites.

### EXAMPLE 9

### Binding of MHC molecule constructs and tetramers to small populations of T-cells

In this experiment, it was shown that binding of MHC molecule constructs of the invention provided improved detection of minor populations of specific T-cells as compared to tetramers.

The following MHC molecule construct of the invention was used:

### MHC molecule construct 1.

The following tetramer was used:

### Tetramer 1.

The tetramer was used for comparison.

The T-cell clones 5/127 recognising the peptide analogue from MART-1 and 5/130 recognising the peptide from gp100, respectively, were mixed in a ratio of 1:20 T-cell clone 5/127 to T-cell clone 5/130 and used for analysis of MHC molecule constructs of the invention and tetramers. The tetramer was used in 5-fold higher concentration to compensate for the lower binding avidity (cf. the findings of Example 3) as compared to the peptide-corresponding poly-ligand MHC molecule construct of the invention (cf. Figures 25 and 26).

The cell solution was incubated with 3 nM MHC molecule construct or 15 nM tetramer for 1 hour at room temperature. The cells were washed once and analysed by flow cytometry following standard flow cytometry procedures. As shown in Figure 31, both the construct of the invention and the tetramer stained about 5% of the cells corresponding to the MART-1 specific sub-population of 5/127 T-cell clones. The staining of cells by the construct of the invention provided, however, a clear distinction between positive and negative T-cells.

In comparison, the staining by the tetramer provided a less clear distinction between the two T-cell populations cf. Figure 31.

Consequently, it was concluded that the constructs of the invention provide better staining, thus, improved capacity for detection by flow cytometry of minor T-cell populations in comparison to the prior art tetramers.

### EXAMPLE 10

### Pre-formed peptide empty MHC molecule constructs bind to specific T-cells after loading with appropriate peptides

The surprising and sensitive capacity of the MHC molecule constructs of the invention in the detection of small T-cell specificities (cf. Example 9) in mixed cell samples was further investigated using samples containing about 1% T-cell clone 5/127 ("high avidity T-cell clone", cf. Figure 25) and 1% 5/130 ("low avidity T-cell clone", cf. Figure 25. The percentage of 1 was chosen as a variety of studies have shown that a sub-population of proliferating T-cells frequently comprises approximately 1% of total number of T-cells in blood samples and within a range of 0.1 to 10% in immune responding patients.

The following peptide empty MHC molecule construct of the invention was generated from peptide empty HLA A0201 molecule construct of invention:

### MHC molecule construct 7.

Also, MHC molecule construct 1 and MHC molecule construct 8 was used.

In this experiment a peptide specific peptide HLA A0201 molecule construct was further generated.

The following tetramer was produced from peptide empty HLA A0201 ligated to streptavidin labelled with PE:

### Tetramer 6.

Also, tetramer 1 and tetramer 2 was used.

The peptide empty HLA molecule construct of the invention was formed by incubation of 60 pmol mono-biotinylated heavy chain (2 *µ*l stock solution with 30 *µ*M heavy chain molecule obtained as described in Example 1.C.) with 1 nmol β₂m in 198 *µ*l dilution buffer (20 mM tris, pH 6.8, 150 mM NaCl) for 2 hours at 18°C. The formed dimer (approximately 270 nM) was stable in this buffer for several days when stored at 4°C. The peptide empty HLA molecule construct of the invention was formed by addition of streptavidin dextran carrier molecules (500 kDa, 13.6 SA/dextran, added to a final concentration of 10 nM) to 100 *µ*l solution.

The peptide-displaying HLA molecule construct (construct 1) was formed by adding the MART-1 peptide analogue ELAGIGILTV to the solution of HLA dimers and dextran molecules to a final concentration of 10 *µ*M and incubating over night at 18°C.

In a similar approach, MHC molecule construct 8 of the invention displaying a gp100 peptide was generated by addition of the peptide KTWGQYWOV.

The tetramers were generated in a similar approach except that PE-labelled SA was added sequentially to a final concentration of 70 nM, to ensure a ratio of 1:4 between SA and HLA molecules, prior to addition of the MART-1 peptide analogue ELAGIGILTV or gp100 peptide KTWGQYWOV. The tetramer was used for comparison.

Prior to the staining, the solutions with MHC molecule constructs of invention and the tetramers were diluted twice in FACS buffer described above containing 2 mg/ml BSA and 0,2% azide. The concentrations of molecule of the constructs and the tetramers used for staining of T.cells were thus 5 and 35 nM, respectively.

The two T-cells clones 5/127 and 5/130, respectively, were mixed in ratios of 1:100, i.e. one cell sample contained about 1% 5/127 and 99% 5/130 T-cells and an other cell sample contained 1% 5/130 and 99% 5/127. The mixed cell solutions were tested for binding of construct of the invention or tetramers displaying peptides recognised by the 1% subpopulations of T-cells.

For flow cytometry, the cells (5×10⁵) were centrifuged at 300g for 5 minutes, and re-suspended in 50 *µ*l solution with MHC molecule construct of the invention or tetramer and incubated for 60 minutes at room temperature. Subsequently, the cells were washed once and immediately analysed by flow cytometry following standard procedures.

As shown in Figure 32, the MART-1 peptide (ELAGIGILTV)-displaying construct of the invention provided a clear distinction between positive T-cells (1,2% positively stained cells) whereas the construct displaying the gp100 peptide (KTWGQYWOV) stained about 0,4%. Although utilised in 7-fold higher concentration neither of the corresponding tetramers were able to stain the T-cells (data not shown).

Thus, it was concluded that it was indeed possible to generate peptide empty MHC molecule constructs of the invention. By subsequently loading with appropriate peptides, the resulting MHC molecule constructs were capable of staining minor populations of T-cells. In contrast to the tetramers, the MHC molecule constructs of invention were recognised by both low and high avidity T-cell clones using flow cytometry.

### EXAMPLE 11

### Binding of radio-labelled MHC molecule construct displaying the MART-1 peptide

In this experiment, the cell binding of a radio-labelled MHC molecule construct of invention was investigated. The molecule construct comprised as MHC molecule, HLA/peptide complexes folded in the presence of iodinated β₂m. The construct was prepared according to Example 1, however, with the folding taking place in the presence of iodinated β₂m.

The following MHC molecule construct of the invention was used:
a MHC molecule construct comprising the 500 kDa dextran carrier molecule having attached thereto 27.2 HLA A0201 heavy chain in complex with the MART-1 peptide analogue (ELAGIGILTV) and iodinated β₂m via 13.6 SA.

The β m were iodinated according to standard procedures and used for folding of fully biotinylated and active heavy chain as described above (cf. Example 1.C.). The de novo generated HLA A0201 complex comprising peptide, heavy chain and a radio-labelled β₂m molecule was purified from the excess of β₂m and peptide by G50 chromatography following standard protocol. The radioactivity was counted using a COBRA gamma counter prior to ligation of purified HLA A0201 complexes to SA conjugated dextrans.

Samples of the MART-1 or gp100 specific T-cell (5×10⁵) clones 5/127 and 5/130 in 100 *µ*l PBS with 1% BSA, were incubated with the radio-labelled MHC molecule construct of the invention (100000 cpm/sample at 18°C for 1 hour with or without the variety of antibodies as described in Example 7. The cells were washed 5 times and transferred to fresh tubes prior to counting of cell bound radioactivity.

As shown in Figure 33, the 5/127 T-cells bound radio-labelled MHC molecule construct of the invention (MHC molecule construct 1), whereas the gp100 peptide specific T-cell clone 5/130 as expected did not bind.

Furthermore, it was observed that the antibodies BB7.2 and W6/32 but not BBM1 inhibited binding of the construct of the invention in agreement with the findings of Example 7.

Thus, it was concluded that the MHC molecule constructs of the invention comprising a radio-labelled β₂m were capable of binding to specific T-cells. Furthermore, the binding of this type of labelled MHC molecule construct was comparable to the binding of differently labelled MHC molecule constructs.

Another important feature was that labelling of β₂m represents in this context a versatile alternative to labelling of the heavy chain or the peptide, since the β₂m is a common subunit, which facilitates folding of a variety of different HLA molecules.

### EXAMPLE 12

### Staining of tumour specific T-cells

In this example, the ability of poly-ligand MHC molecule constructs to label specific T-cells in breast cancer lesions was tested. The test was performed on acetone-fixed, frozen biopsies from human skin, lymph nodes and tumour lesions, respectively, mounted on slides.

The following MHC molecule constructs were used:
MHC molecule construct 8
MHC molecule construct 11.

In Figure 38, the staining of specific T-cells in HLA A2 positive biopsies taken from breast cancer lesions are shown. The staining was performed by poly-ligand MHC molecule constructs (500 kDa) displaying maximal amount of HLA-A0201 in association with the peptide analogue (SUR1M2) (LMLGEFLKL) from survivin, a recently identified tumour associated antigen.

The frozen tissue was sectioned and collected on glass slides (Superfrost Plus Gold Slides, Erie Scientific Co, Portsmouth, New Hampshire), air dried over-night and fixed in cold acetone for 5 minutes.

All the following procedure steps were performed at room temperature and in the dark. Between each step the slides was washed 3 times 10 minutes with a Phosphate Buffered Saline (PBS) buffer (pH 7.6).

The slides were firstly incubated (45 minutes) with (i) the primary antibody; anti-CD8 (anti-CD8 clone HIT8a, cat. No 550372, Pharmingen, San Diego, CA, USA, 1:100 dilution in PBS buffer), followed by (ii) Cy3-conjugated goat anti-mouse (Jackson ImmunoResearch Laboratories, Inc., West Grove, PA, USA, diluted 1:500 in PBS) for 45 minutes and finally (iii) incubated with the poly-ligand MHC molecule construct for 75 minutes (100 *µ*ml, 20 10-9 M construct in PBS).

Finally, the stained slides were mounted with coverglass in antifade solution (Vectashield, Vector labs, Burlingame, CA, USA) and kept in the refrigerator until analysis under the confocal microscope.

The entire population of cytotoxic TILS in tumour biopsies could be visualised by Cy3 conjugated anti-CD8 specific antibodies (Figure 25, left lanes) whereas the SUR1M2 specific T-cell clones could be visualised with FITC conjugated poly-ligand MHC molecule construct (right lane at top). Double staining of CD8 positive and peptide epitope specific T-cells were revealed in the merged pictures in the middle lanes (top). Another HLA A0201 binding peptide, the melanoma associated gp100 antigen displayed by the MHC molecule construct did not stain TILS in the examined breast cancer tissue (right lane, middle). In a second control, the SUR1M2 poly-ligand MHC molecule construct did not stain T-cells in breast cancer biopsies from A2 negative patients (right, bottom).

Thus, it was concluded that peptide specific poly-ligand MHC molecule bound specifically to a subtle target T-cell population and thus allowed in *situ* analyses of T-cell expression in biopsies from breast cancer patients.

### EXAMPLE 13

### In situ staining of melanoma and lymph node tissues with SUR1M2 poly-ligand MHC molecule construct

In this example the ability of poly-ligand MHC molecule constructs to stain specific T-cells in melanoma and lymph node tissue from HLA-A2 positive patient material was tested.

The following MHC molecule construct was used:

### MHC molecule construct 11.

The experimental details of this Example are similar to those given in Example 12 except that tissue originated from a melanoma patient.

In Figure 39, the left lane Cy-3 staining of CD8+ T-cells in tissue samples from tumour (top) and lymph node (bottom) are shown. The right lane shows the localised staining by the FITC A2-SUR1M2 MHC molecule construct. Double staining of CD8 positive and SUR1M2 peptide specific T-cells are depicted in the middle lane showing the merged pictures.

In conclusion; it was shown that specific binding *in situ* of SUR1M2 peptide displaying poly-ligand MHC molecule construct to CD8+ T-cells in biopsies from melanoma lesions and lymph nodes could be detected.

### EXAMPLE 14

### In situ staining of CD8+ T-cells in melanoma tissue with MART-1 peptide displaying poly-ligand MHC molecule construct

In this example specific staining of melanoma tissue from a HLA-A 0201 positive patient by an A2-MART-1 MHC molecule construct was investigated.

The following MHC molecule construct was used:

### MHC molecule construct 1.

The experimental details of this Example were similar to those given in Example 12, except that tissue was from a melanoma patient and that the poly-ligand MHC molecule construct displayed the MART-1 peptide analogue (ELAGIGILTV).

The result of the experimental staining of a melanoma biopsy is given in Figure 40. The left picture shows the localisation of PE-stained CD-8 positive cells and the right picture the presence of FITC stained MART-1 specific T-cells. Double stained MART-1/CD-8 positive cells are seen in the merged middle picture.

In conclusion, using an approach similar to Example 12, it was shown that specific binding of poly-ligand MHC molecule construct displaying the MART-1 peptide analogue (ELAGIGILTV) to CD8 T-cells in a lesion from an HLA A0201 positive melanoma patient could be detected in *situ.*

### EXAMPLE 15

### In situ staining of BV12 reactive and non-reactive T-cells in skin biopsies from injection sites using MART-1 and MAGE-3 peptide displaying poly-ligand MHC molecule constructs

Attempts to develop curative immune therapy comprise strategies where soluble peptide candidates e.g. SUR1M2 and/or dendritic cells (DC) loaded with peptides or tumour lysates are injected in the patient. Whereas cellular immune responses are initiated by the interaction of T-cells and antigen presenting cells e.g. DC in secondary lymphoid organs, the therapeutic vaccinations may lead to another scenario namely local expansion and accumulation of antigen specific T-cells. Using poly-ligand MHC molecule constructs displaying tumour associated peptides, it was investigated whether peptide specific T-cells are over-represented at the injection site.

The following MHC molecule constructs were used:
MHC molecule construct 1
MHC molecule construct 14.

Using an experimental approach similar to the one used in Example 12 in situ double staining analysis with Cy-3 labelled TCR VB12 antibody and FITC labelled poly-ligand MHC molecule constructs displaying the MART-1 or MAGE-3 peptide were performed on skin biopsies from injection sites. The TCR VB12 antibody only reacts with a subset of the T-cells as it is specific for T-cell receptors expressing the variable ß-chain family 12 region.

The results are shown in Figure 41. Left lanes reveals three distinct populations of T-cells. The populations include a BV12-/MART-1 reactive (A), BV12+/MART-1 reactive (B) as well as BV12+/MART-1 non-reactive cells (C). Thus, a non-specific interaction of MART-1/HLA A0201 poly-ligand MHC molecule construct with all members of the BV12 family could be excluded. Moreover, the MAGE-3 peptide recognising cells were found in small clusters, suggesting a local expansion of this T-cell specificity (D).

Thus in conclusion, using the poly-ligand MHC molecule constructs recognising specific T-cells it was possible to demonstrate the in situ presence of populations of specific T-cells at the injection site 48 hours after s.c. injection of tumour lysate pulsed DCs suggesting a local expansion of antigen specific T-cells.

### EXAMPLE 16

### In situ staining of CD8 reactive T-cells in skin biopsies from injection site using gp-100 and MAGE-3 peptide displaying poly-ligand MHC molecule constructs

In this experiment the accumulation of peptide antigen specific T-cells were further investigated using the same approach as in Example 15.

The following MHC molecule constructs were used:
MHC molecule construct 8
MHC molecule construct 14.

The experimental results are shown in Figure 42. Left and middle lanes show the staining with anti-CD8 antibodies and peptide specific poly-ligands, respectively. The merged pictures are shown in the right lane.

It was concluded that that immunisation of patients with DC pulsed with a gp100 peptide epitope led to infiltration of specific T-cells that recognised the peptide displayed by HLA A0201 poly-ligand MHC molecule construct (Figure 42B) but not a MAGE-3 epitope (Figure 42A).

### EXAMPLE 17

### Chromogen in situ staining of CD8+ T-cells in melanoma tissue with MART-1 peptide displaying poly-ligand MHC molecule construct

It was studied if specific binding of poly-ligand MHC molecule construct displaying the MART-1 peptide analogue (ELAGIGILTV) to CD8 T-cells in a lesion from an HLA A0201 positive melanoma patient, could be visualised by HRP-mediated chromogen staining using two different peroxide blocking methods.

The following MHC molecule construct was used:

### MHC molecule construct 13.

The frozen melanoma lesions were sectioned (5 *µ*m) and collected on glass slides (Superfrost^{®} Plus Gold Slides, Erie Scientific Co, Portsmouth, New Hampshire), air dried for 30 minutes and fixed in cold anhydrous reagent grade acetone (Aldrich, Milwaukee, WI, USA) for 5 minutes.

All the following procedure steps were performed at room temperature. Between each step the slides was washed batch vice 3 times 10 minutes with a PBS buffer (pH 7.6).

Endogenous peroxidase was blocked following two different reagent strategies:

A peroxide/methanol solution (50 ml 3% H₂0₂ plus 200 ml methanol) (Figure 43A) or peroxidase blocking solution (code S2023, DAKO A/S, Glostrup, Denmark) (Figure 43B).

After washing, the slides were incubated for 30 minutes with the indicated HLA-peptide dextran 270 HRP constructs (100 ml, 2.9 10-9 M in PBS).

After two washes, bound complexes were visualised using 3-amino-9-ethylcarbazol (AEC)-substrate (DAKO AEC Substrate System, DAKO A/S, Glostrup, Denmark). The reaction was terminated after 25 minutes.

The slides were counter stained with Mayer's hematoxylin (Code S330930, DAKO A/S, Glostrup, Denmark, 15 seconds) and washed in PBS buffer until slightly blue (about 30 seconds). Finally, the slides were coverslip mounted using Aquamont (DAKO Corporation, Carpenteria, CA, USA) and analysed using a bright field microscope (Zeiss) with photographic capacities.

In conclusion successful staining was achieved irrespective of the strategy used for blocking endogenous peroxidase.

### EXAMPLE 18

### T-cell activation induced by MHC molecule construct: the impact of co-stimulatory molecules

In this experiment, it was shown that activation of T-cell clones incubated with MHC construct is affected by the presence of co-stimulatory molecules attached to the MHC molecule construct, which bind to activating isoforms of NKRs.

The following MHC molecule constructs were used:
MHC molecule construct 15,
MHC molecule construct 16,
MHC molecule construct 17,
MHC molecule construct 18.

MHC molecule constructs 17 and 18 were used as controls.

Sub-optimal amounts of recombinant biotinylated HLA A0201 complexes displaying the MART-1 peptide anloug (ELAGIGILTV) or the gp100 peptide (KTWGQYWOV) were added to a solution of the construct comprising 500 kD dextran carrier molecules. More specifically, a 80 nM solution of dextran (conjugated with 13.6 SA, each labelled in average with 2 FITC) was incubated in PBS with 1121 nM mono-biotinylated HLA A0201 complexes with or without 510 nM mono-biotinylated MIC A protein. It can be stipulated from Example 8 that the molecule constructs comprising only HLA complexes are loaded with 14.1 HLA A0201 molecules per dextran. The molecule constructs comprising HLA and MIC A protein are loaded with 14.1 HLA A0201 molecules per dextran and 7.1 MIC A molecules per dextran, respectively.

The MART-1 peptide/HLA A0201 specific T-cell clones 5/127 (5×10⁵) was grown in media and incubated with 5 nM molecule constructs displaying either MART-1 or gp100 peptides with or without MIC A proteins. The cells were incubated for 24 and 48 hours, respectively, at 37 C, prior to measuring IFN-gamma in supernatants by ELISA following standard procedure.

As shown in Figure 44, the construct comprising HLA A0201 displaying MART-1 peptide combined with or without MIC A stimulated the 5/127 T-cell clone to IFN-gamma release after 24 hours, suggesting that both constructs were capable of binding and induce some signalling. In contrast, only the molecule construct comprising HLA and MIC A was capable of further stimulation as indicated by the increase amount of IFN-gamma. In comparison, the IFN-gamma release remained unchanged by stimulation of MHC molecule construct comprising only MART-1 peptide/HLA complexes. None of the MHC molecule constructs displaying gp100 peptide were capable of stimulation in this experiment (data not shown).

It was concluded that MHC molecule molecule constructs displaying appropriate peptide was able to stimulate T-cells upon binding to peptide specific TCR (cf. Figures 25B and 30). However, only molecule constructs comprising appropriate peptide-HLA complexes and MIC A protein stimulated the T-cells upon prolonged incubation. This feature can be explained by induction of T-cell anergy when stimulated with molecule construct without co-stimulatory proteins. This construct was capable of initial stimulation followed by inactivation of the T-cells, which is characteristic feature of anergy. MHC molecule construct with HLA and MIC A was capable of continuous stimulation.

### EXAMPLE 19

### Preparation of carrier molecules having attached thereto a plurality of binding entities

Various carrier molecules (as exemplified by 150, 270 and 500 kDa dextrans, respectively) having attached thereto a plurality of binding entities (as exemplified by streptavidine (SA)) were prepared according to the procedure described below. MHC molecules and/or biologically active compounds can be attached subsequently. The theoretical number of coupling sites to each SA is 4, meaning that the loading capacity of each SA-dextran molecules is 22.4 (4x5.6), 41.2 (4x10.3) and 68 (4x17.0). Additionally MHC molecules/biologically compounds can be attached to the dextran molecule directly, thus, making the loading capacity even greater.

### SA-Dextran (150, 270, 500 kDa)

Streptavidine (SA, Genzyme) was dialysed overnight (100 mg in 5 ml, against 1000 ml 0.10 M NaCl, 2-4°C, 10 kDa MwCO, changed three times). After UV absorbance measurement the concentration was calculated.

The SA solution was added to a solution of vinylsulfon-activated dextran (approximately 25% activated) of 150, 270 or 500 kDa (in total 1.6 mg vinylsulfon dextran/ml, 7.7 mg SA/ml, 0.1 M NaCl, 25 mM carbonate buffer, pH 8.5) and stirred at 30°C for 18 hours. Any remaining reactive groups were quenched by addition of 1/10 volume reaction mixture of an ethanol amine-containing buffer (110 mM ethanolamine, 50 mM HEPES, 0.1 M NaCl, pH 7.0) and stirred for 30 minutes at 30°C. The so obtained polymeric molecules (SA-dextran) was purified from unbound SA by gel filtration (FPLC, Pharmacia, S-200, 0.1 M HEPES, 0.1 M NaCl, pH 7.2).

The degree of SA incorporation per dextran molecule were calculated from the UV absorbance at 278 nm. The incorporation of SA was in average 5.6 (for the 150 kDa dextran), 10.3 (for the 270 kDa dextran) and 17.0 (for the 500 kDa dextran), respectively. The molecules were concentrated to the equivalent of 3.0 mg SA/mL using a Millipore filter centrifuge device.

### EXAMPLE 20

### Preparation of carrier molecules having attached thereto a plurality of labelled binding entities

By the procedures described below, various carrier molecules (as exemplified by 150, 270 and 500 kDa dextrans, respectively) having attached thereto a plurality of binding entities (as exemplified by streptavidine (SA)) labelled with a plurality of labelling compounds (as exemplified by Alexa 647) were prepared. MHC molecules and/or biologically active compounds can be attached subsequently. The theoretical number of coupling sites to each SA is 4, meaning that the loading capacity of each SA-dextran molecules is 22.4 (4x5.6), 41.2 (4x10.3) and 68 (4x17.0). Additionally MHC molecules/biologically compounds can be attached to the dextran molecule directly, thus, making the loading capacity even greater.

### Alexa 647 labelled SA-Dextran (150, 270, 500 kDa)

The SA dextran molecules obtained from Example 19 were labelled with Alexa 647 according to the general guidelines given by the manufacturer of the Alexa Fluor 647 Protein labelling Kit (Molecular Probes, product number A-20173). The reaction conditions were 1 vial Alexa 647, 2.0 mg SA/mL, 0.10 M NaCl, 50 mM carbonate, pH 8.0, 0.500 mL in total volume, 30°C, in the dark for one hour). Any remaining reactive groups were quenched by addition of 0.050 mL volume reaction mixture to an ethanol amine containing buffer (110 mM ethanol amine, 50 mM HEPES, 0.1 M NaCl, pH 7.0), and stirred for 30 minutes at 30°C. The so obtained fluorescently labelled polymeric molecules were purified from unbound dye by dialysis (against 1000 ml 0.10 M NaCl, 2-4°C, 10 kDa MwCO, changed three times), 0.1 M HEPES, 0.1 M NaCl, pH 7.2).

The degree of SA incorporation per dextran molecule, and Alexa 647 incorporation per SA, as well as molecule concentration were calculated from the UV absorbance at 278 and 650 nm. The molecules were added sodium azide to 15 mM as a preservative. The results are shown below.

| Dextran carier molecule | SA per dextran (in average | Alexa 647 per SA (in average) | Concentration of dextran (mole/l) |
|---|---|---|---|
| 150 | 5.6 | 2.7 | 60×10⁻⁸ |
| 270 | 10.3 | 2.6 | 50×10⁻⁸ |
| 500 | 17.0 | 2.7 | 20×10⁻⁸ |

### EXAMPLE 21

### Preparation of carrier molecules having attached thereto a plurality of binding entities

By the procedures described below, various carrier molecules (as exemplified by 150, 270 and 500 kDa dextrans, respectively) having attached thereto a plurality of binding entities (as exemplified by rabbit-anti-biotin antibody) were prepared. MHC molecules and/or biologically active compounds can be attached subsequently as desired.

### Rabbit-anti-biotin dextran (150, 270, 500 kDa)

Rabbit-anti-biotin antibody (affinity purified, Fab2, approximately 100 kDa, DAKO code number DM0069) was dialysed overnight (100 mg antibody in 5 ml, against 1000 ml 0.10 M NaCl, 2-4°C, 10 kDa MwCO, changed three times). After UV absorbance measurement, the concentration was calculated. The antibody solution was added to a solution of vinylsulfon-activated dextran (approximately 25% activated) of 150, 270 or 500 kDa (in total 0.680 mL, 1.07 mg vinylsulfon dextran/ml, 15.25 mg antibody/ml, 0.1 M NaCl, 25 mM carbonate buffer, pH 8.5), respectively, and stirred at 30°C for 18 hours. Any remaining reactive groups were quenched by addition of 1/10 volume reaction mixture of an ethanol amine-containing buffer (110 mM ethanolamine, 50 mM HEPES, 0.1 M NaCl, pH 7.0) and stirred for 30 minutes at 30°C. The obtained polymeric molecules were purified from unbound antibody by gel filtration (FPLC, Pharmacia, S-200, 0.1 M HEPES, 0.1 M NaCl, pH 7.2).

The degree of antibody incorporation per dextran was calculated from the UV absorbance at 278 nm. The number of antibodies per dextran was in average 8.4 (for the 150 kDa dextran), 19.5 (for the 270 kDa dextran) and 34.4 (for the 500 kDa dextran). The molecules were concentrated to the equivalent of 3.9, 3.4 and 3.4 mg antibody/mL, respectively, using a Millipore filter centrifuge device.

In another preperation using the same conditions, the incorporation of antibodies per dextran was 7.2 (for the 150 kDa dextran) and 11.2 (for the 500 kDa dextran). These molecules were concentrated to the equivalent of 3.5 mg antibody/mL respectively using a Millipore filter centrifuge device.

### EXAMPLE 22

### Preparation of carrier molecules having attached thereto a plurality of labelled binding entities

By the procedures described below, various carrier molecules (as exemplified by 150 and 270 kDa dextrans, respectively) having attached thereto a plurality of binding entities (as exemplified by rabbit-anti-biotin antibody) labelled with a plurality of labelling compounds (as exemplified by Alexa 532 or Alexa 647) were prepared. MHC molecules and/or biologically active compounds can be attached subsequently as desired.

### Preparation of Alexa 532 or 647 labelled rabbit-anti-biotin dextran

The rabbit-anti-biotin dextran molecules obtained in Example 21 were labelled with Alexa 532 or Alexa 647 according to the general guidelines given by the manufacturer of the Alexa Fluor 532 Protein labelling Kit (Molecular Probes, product number A-10236) or Fluor 647 Protein labelling Kit (Molecular Probes, product number A-20173). The reaction conditions were 1 vial Alexa dye, equivalent of 2.0 mg antibody/mL, 0.10 M NaCl, 50 mM carbonate, pH 8.0, 0.500 mL in total volume, 30°C, in the dark for one hour). Any remaining reactive groups were quenched by addition of 0.050 mL volume reaction mixture to an ethanol amine containing buffer (110 mM ethanol amine, 50 mM HEPES, 0.1 M NaCl, pH 7.0) and stirred for 30 minutes at 30°C. The four different fluorescently labelled polymeric molecules were purified from unbound dye by dialysis (against 1000 ml 0.10 M NaCl, 2-4°C, in the dark, 10 kDa MwCO, changed three times), 0.1 M HEPES, 0.1 M NaCl, pH 7.2).

The degree of Alexa 532 incorporation per antibody, and antibody incorporation per dextran, as wells as concentration were calculated from the UV absorbance at 278 and 530 nm. The molecules were added sodium azide to 15 mM as a preservative.

The degree of Alexa 647 incorporation per antibody, and antibody incorporation per dextran, as well as concentration were calculated from the UV absorbance at 278 and 650 nm. The molecules were added sodium azide to 15 mM as a preservative.

| Dextran carrier molecule | Antibody per dextran (in average) | Alexa 532 per antibody (in average) | Concentration dextran (mole/l) |
|---|---|---|---|
| 150 | 8.4 | 3.0 | 165.5×10⁻⁸ |
| 270 | 19.5 | 2.9 | 69.4×10⁻⁸ |

| Dextran carrier molecule | Antibody per dextran (in average) | Alexa 647 per antibody (in average) | Concentration dextran (mole/l) |
|---|---|---|---|
| 150 | 7.21 | 2.7 | 150×10⁻⁸ |
| 270 | 11.2 | 2.6 | 65×10⁻⁸ |

### EXAMPLE 23

### Preparation of carrier molecules having attached thereto a plurality of labelled binding entities

By the procedures described below, various carrier molecules (as exemplified by 150 and 270 kDa dextrans, respectively) having attached thereto a plurality of binding entities (as exemplified by rabbit-anti-biotin antibody) labelled with a plurality of labelling compounds (as exemplified by FITC) were prepared. MHC molecules and/or biologically active compounds can be attached subsequently as desired.

### Preparation of FITC labelled Rabbit-anti-biotin dextrans

The rabbit-anti-biotin dextrans from Example 21 (150 kDa and 270 kDa dextrans) were used for FITC labelling). The FITC vial (FITC (fluorescein isothiocyanate), Molecular Probes, product number F-1906) storred in the freezer was allowed to stand at room temperature for one hour before being opened. A FITC solution (10.1 mg/ml NMP) was added to stirred mixtures of rabbit-anti-biotin dextran molecules (in total 0.750 ml, molecule concentration equivalent to 1.5 mg antibody/ml, 0.0487 mg FITC/ml, equivalent to approximately 8.3 FITC per antibody, 0.1 M NaCl, 200 mM carbonate buffer, pH 8.5, 30°C, one hour in the dark). Any remaining reactive groups were quenched by addition of 1/10 volume reaction mixture of an ethanol amine-containing buffer (110 mM ethanol amine, 50 mM HEPES, 0.1 M NaCl, pH 7.0) and stirred for 30 minutes at 30°C. The two different FITC labelled rabbit-anti-biotin polymeric molecules were purified from unbound fluorescein by dialysis in a float-a-lyzer (against 500 ml 0.10 M NaCl, 2-4°C, in the dark, 10 kDa MwCO, changed three times), 0.1 M HEPES, 0.1 M NaCl, pH 7.2).

The degree of fluorescein incorporation per antibody, and antibody incorporation per dextran was calculated from the UV absorbance at 278 and 498 nm. The molecules were added sodium azide to 15 mM as a preservative.

| Dextran carier molecule | Antibody per dextran (in average | Fluorescein per antibody (in average) | Concentration of dextran (mole/l) |
|---|---|---|---|
| 150 | 8.4 | 1.7 | 125.2×10⁻⁸ |
| 270 | 19.5 | 1.8 | 41.6×10⁻⁸ |

### EXAMPLE 24

### Isolation of CTLs using the MHC molecules in an immunomagnetic separation procedure

In this experiment, it was shown that antigen reactive cytotoxic T lymphocytes (CTL) could be isolated from an HLA-A0201 positive patient lymph node sample by the use of MHC molecules immobilised on magnetic beads.

Single cell suspensions from melanoma infiltrated lymph node biopsy material were obtained after homogenisation and centrifugation to remove cellular debris. The cell isolation was performed by using magnetic beads (Dynabeads^{®}, with streptavidin) coated with biotinylated MHC molecules displaying HLA A0201 in association with the peptide analogue (SUR1/M2) (LMLGEFLKL) from survivin, a recently identified tumour associated antigen. The magnetic beads with immobilised MHC molecule were added to the cell suspension and incubated for 30 minutes at 30°C to allow the beads to bind to the cells. After binding, rosetted cells were isolated by using a magnet. In Figure 45, the results are shown. In Figure 45A, the bright field microscopy picture of the so isolated survivin reactive CTLs bound to the MHC molecule construct-coated beads are shown.

In the same experiment, magnetic beads coated with a biotinylated recombinant HLA A0201/influenza peptide was used as negative control. As shown in Figure 45B, magnetic beads coated with the HLA A0201/influenza peptide complexes did not bind to CTL cells from the melanoma infiltrated lymph node biopsy material.

Thus, it is expected that the high avidity of the MHC molecule constructs of the invention will result in even better specific binding to cells of interest, and accordingly that such cells are obtainable using the MHC molecule constructs of the invention.

### REFERENCES

1. Altman JD, Moss PA, Goulder PJ, Barouch DH, McHeyzer-Williams MG, Bell JI, McMichael AJ, and Davis MM, Science 274 (5284), 94-96 (October 1996), "Phenotypic analysis of antigen-specific T lymphocytes". Erratum in Science 280(5371), 1821 (19 June 1998).
2. Dal Porto, J, Johansen TE, Catipovic B, Parfiit DJ, Tuveson D, Gether U, Kozolowski S, Fearon DT, Schneck JP, PNAS USA 90(14), 6671-6675 (15 July 1993), "A soluble divalent Class I major histocompatibility complex molecule inhibits allowreactive T-cells at nanomolar concentrations".
3. Viola A, and Lanzavecchia A., APMIS 107(7), 615-623 (July 1999), "T-cell activation and the dynamic world of rafts".
4. Zhang W, Sommers CL, Burshtyn DN, Stebbins CC, DeJarnette JB, Trible RP, Grinberg A, Tsay HC, Jacobs HM, Kessler CM, Long EO, Love PE, and Samelson LE, Immunity 10(3), 323-332 (March 1999), "Essential role of LAT in T-cell development".
5. Ugolini S, and Vivier E., Curr. Opin. Immunol. 12(3), 295-300 (June 2000), "Regulation of T-cell function by NK cell receptors for classical MHC class I molecules".
6. Ljunggren HG, Stam NJ, Ohlen C, Neefjes JJ, Hoglund P, Heemels MT, Bastin J, Schumacher TN, Townsend A, Karre K, et al., Nature 346(6283), 476-480 (2 August 1990), "Empty MHC class I molecules come out in the cold".
7. Coles MC, McMahon CW, Takizawa H, and Raulet DH., Eur. J. Immunol. 30(1), 236-244 (January 2000), "Memory CD8 T lymphocytes express inhibitory MHC-specific Ly49 receptors".
8. Bjorkman PJ, Saper MA, Samraoui B, Bennett WS, Strominger JL, and Wiley DC., Nature 329(6139), 512-518 ( 8-14 October 1987), "The foreign antigen binding site and T-cell recognition regions of class I histocompatibility antigens".
9. Matsumura M, Fremont DH, Peterson PA, and Wilson IA, Science 257(5072), 927-934 (14 August 1992), "Emerging principles for the recognition of peptide antigens by MHC class I molecules".
10. US 5,635,363 (Altman).
11. Marchand M, Weynants P, Rankin E, Arienti F, Belli F, Parmiani G, Cascinelli N, Bourlond A, Vanwijck R, Humblet Y, et al., Int. J. Cancer 63(6),883-885 (11 December 1995), "Tumor regression responses in melanoma patients treated with a peptide encoded by gene MAGE-3".
12. "HLA: Genetic diversity of HLA. Functional and Medical Implication", Ed. Dominique Charron, EDK Press 1997.
13. "Molecular Cloning" (Sambrook, Fritsch and Maniatis), Cold Spring Harbor Press, 1989.
14. Merrifield RB, Stewart JM, et al., Anal. Chem. 38, 1905-1914 (1966), "Instument for Automated Synthesis of Peptides".
15. Merrifield B., Science 232, 341-347 (1986), "Solid Phase Synthesis".
16. Barany G, Kneib-Cordonier N, et al., Int.. Peptide Protein Res. 30, 705-739 (1987), "Solid-phase peptide synthesis: A silver anniversary report".
17. Fields GB, and Noble RL, Int. J. Peptide Protein Res. 35, 161-214 (1990), "Solid phase peptide synthesis utilizing 9-fluorenylmethoxycarbonyl amino acids".
18. Gordon EM, Barrett RW, et al., J. Med. Chem. 37(10), 1385-1401 (1994), "Applications of Combinatorial Technologies to Drug Discovery. 2. Combinatorial Organic Synthesis, Library Screening Strategies, and future directions".
19. Houghten RA, Pinilla C, et al., Nature 354, 84-86 (1991), "Generation and use of synthetic peptide combinatorial libraries for basic research and drug discovery".
20. Jung G, and Beck-Sickinger AG, Angew. Chem. Int. Engl. 31(4), 367-384 (1992), "Multiple Peptide Synthesis Methods and Their Applications".
21. Garboczi DN, Hung DT, and Wiley DC, PNAS USA 8(8), 3429-3433 (15 April 1992), "HLA-A2-peptide complexes: refolding and crystallization of molecules expressed in Escherichia coli and complexed with single antigenic peptides".
22. WO 93/01498 (A. Lihme and T. Boenisch, "Water soluble, polymer based reagents and conjugates comprising moieties derived from divinely sulfone").
23. Larsson, Lars-Inge, "Immunocytochemistry: Theory and Practice", CRC Press inc., Boca Raton, Florida, (1988), Chapter 3, page 77-146.
24. Kuttler, C., Nussbaum, A.K., Dick, T.P., Rammensee, H.-G., Schild, H., Hadeler, K.P., An algorithm for the prediction of proteasomal cleavages, J. Mol. Biol. 298 (2000), 417-429
25. US 4,336,173
26. EP 0 106 873 (Sintef)
27. US 4,459,378
28. US 4,654,267
29. WO 99/11661
30. WO 91/15766
31. WO 2000/15665

## Claims

1. A MHC molecule construct in soluble form in a solubilizing medium or immobilized onto a solid or semi-solid support, said MHC molecule construct comprising a soluble dextran carrier molecule attached to at least 5 MHC molecules, wherein said MHC molecules are attached to the dextran carrier molecule via more than one binding entity, wherein each binding entity is attached to from 2 to 4 MHC molecules.

2. A MHC molecule construct in soluble form in a solubilizing medium or immobilized onto a solid or semi-solid support, said MHC molecule construct comprising a soluble dextran carrier molecule attached to at least 5 MHC molecules, wherein said MHC molecules are directly attached to the dextran carrier molecule and said MHC molecule construct comprises one or more labels.

3. The MHC molecule construct according to any one of claims 1 to 2, wherein the MHC molecule is a peptide free MHC molecule or a peptide filled MHC molecule.

4. The MHC molecule construct according to any one of claims 1 to 3, wherein the MHC molecules are the same or at least two of the MHC molecules are different.

5. The MHC molecule construct according to any one of claims 1 to 4, wherein the peptides harboured by the MHC molecules are the same or at least two of the peptides harboured by the MHC molecules are different.

6. The MHC molecule construct according to any one of claims 1 and 3 to 5, wherein the MHC molecules are attached to the carrier molecule via two or more identical binding entities, or combinations of one or more different binding entities.

7. The MHC molecule construct according to any of the claims 1 and 3 to 6, wherein the binding entity is selected from streptavidin, and derivatives thereof, avidin and derivatives thereof, biotin, immunoglobulins, monoclonal antibodies, polyclonal antibodies, recombinant antibodies, antibody fragments and derivatives thereof, leucine zipper domain of AP-1, jun, fos, hexa-his, hexa-hat glutathione S-tranferase, glutathione affinity, Calmodulin-binding peptide, Strep-tag, Cellulose Binding Domain, Maltose Binding Protein, S-Peptide Tag, Chitin Binding Tag, Immuno-reactive Epitopes, Epitope Tags, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, lectins that mediate binding to a diversity of compounds, including carbohydrates, lipids and proteins, preferably Con A or WGA and tetranectin or Protein A and Protein G.

8. The MHC molecule construct according to any one of claims 1 to 7, further comprising one or more biologically active molecules.

9. The MHC molecule construct according to claim 8, wherein the biologically active molecules is attached to the carrier molecule either directly or via one or more of the binding entities.

10. The MHC molecule construct according to any of claims 8 to 9, wherein the biologically active molecule is selected from
(a) Proteins from the group of MHC Class I-like proteins like MIC A, MIC B, CD1d, HLA E, HLA F, HLA G, HLA H, ULBP-1, ULBP-2, and ULBP-3;
(b) co-stimulatory molecules from the group of CD2, CD3, CD4, CD5, CD8, CD9, CD27, CD28, CD30, CD69, CD134, CD137, CD147, CDw150, CD152, CD153, CD40L, NKG2D, ICOS, HVEM, HLA Class II, PD-1, Fas, FasL expressed on T and/or NK cells, CD40, CD48, CD58, CD70, CD72, B7.1, B7.2, B7RP-1, B7-H3, PD-LI, PD-L2, CD134L, CD137L, ICOSL, LIGHT expressed on APC and/or tumor cells;
(c) cell modulating molecules from the group of CD16, NKp30, NKp44, NKp46, NKp80, 2B4, KIR, LIR, CD94/NKG2A, CD94/NKG2C expressed on NK cells, IFN-alpha, IFN-beta, IFN-gamma, IL-1, IL-2, IL-3, IL-4, IL-6, IL-7, IL-8, IL-10, IL-11, IL-12, IL-15, colony-stimulating factors, vitamin D3, IL-2 toxins, cyclosporin, FK-506, rapamycin, TGF-beta, clotrimazole, nitrendipine, and charybdotoxin;
(d) accessory molecules from the group LFA-1, CD11a/18, CD54, CD106, and CD49a,b,c,d,e,f/CD29;
(e) adhesion molecules from the group of ICAM-1 ICAM-2, GlyCAM-1, CD34, anti-LFA-1, anti-CD44, anti-beta7, chemokines, CXCR4, CCR5, anti-selectin L, anti-selectin E, and anti-selectin P;
(f) toxic molecules from the group of cyclophosphamide, methrotrexate, Azathioprine, mizoribine, 1 5-deoxuspergualin, neomycin, staurosporine, genestein, herbimycin A, Pseudomonas exotoxin A, saporin, Rituxan, Ricin, gemtuzumab ozogamicin, Shiga toxin, heavy metals, inorganic mercurials, organic mercurials, FN18-CRM9, radioisotopes, incorporated isotopes of iodide, incorporated isotopes of cobalt, incorporated isotopes of selenium, incorporated isotopes of tritium, incorporated isotopes of phosphor, haptens, DNP, and digoxiginin; and antibodies to any molecule in (a) - (f), derivatives of said antibodies and fragments of said antibodies, and combinations thereof and
(g) proteins, co-stimulatory molecules, cell modulating molecules, receptors, accessory molecules, adhesion molecules, natural ligands, and toxic molecules, and antibodies and recombinant binding molecules thereto, and combinations thereof.

11. The MHC molecule construct according to any of claims 1 to 10 further comprising one or more labeling compounds.

12. The MHC molecule construct according to claim 11, wherein one or more labeling compounds are attached to the carrier molecule and/or one or more of the binding entities and/or one or more MHC molecules.

13. The MHC molecule construct according to claim 11 or 12, wherein the labeling compound is directly or indirectly detectable.

14. The MHC molecule construct according to any of claims 11 to 13, wherein the labeling compound is
(a) selected from fluorescent labels from the group of 5-and 6-carboxyfluorescein, 5- or 6-carboxyfluorescein, 6-fluorescein-5-and 6-carboxamido hexanoic acid, fluorescein isothiocyanate, rhodamine, tetramethylrhodamine, dyes, Cy2, Cy3, Cy5, optionally substituted coumarin, AMCA, PerCP, phycobiliproteins, R-phycoerythrin, allophycoerythrin, Texas Red, Princeston Red, Green fluorescent protein and analogues thereof, conjugates of R-phycoerythrin, conjugates of allophycoerythrin and Cy5 or Texas Red, inorganic fluorescent labels based on semiconductor nanocrystals, quantum dot, and time-resolved fluorescent labels based on lanthanides, time-resolved fluorescent labels based on Eu3+, and timeresolved fluorescent labels based on Sm3+;
(b) selected from haptens from the group of DNP, biotin, and digoxiginin;
(c) selected from enzymatic labels from the group of horse radish peroxidase, alkaline phosphatase, beta-galactosidase, glucose-6-phosphate dehydrogenase, beta-N-acetylglucosaminidase, _-glucuronidase, invertase, Xanthine Oxidase, firefly luciferase and glucose oxidase;
(d) selected from luminiscence labels from the group of luminol, isoluminol, acridinium esters, 1 ,2-dioxetanes and pyridopyridazines; and
(e) selected from radioactivity labels from the group of incorporated isotopes of iodide, incorporated isotopes of cobalt, incorporated isotopes of selenium, incorporated isotopes of tritium, and incorporated isotopes of phosphor and
(f) selected from a fluorescent label, an enzyme label, a radioisotope, a chemiluminescent label, a bioluminescent label, a polymer, a metal particle, a hapten, an antibody, or a dye.

15. The MHC molecule construct according to any of claims 1 to 14, immobilised directly to the solid or semi-solid support.

16. The MHC molecule construct according to any of claims 1 to 15, immobilised to the solid or semi-solid support via a linker, a spacer, or an antibody, an antibody derivative or a fragment thereof.

17. The MHC molecule construct according to any one of claim 15 to 16, wherein the support is selected from the group consisting of particles, beads, biodegradable particles, sheets, gels, filters, membranes, nylon membranes, fibers, capillaries, needles, microtiter strips, tubes, plates or wells, combs, pipette tips, micro arrays, chips, slides, the beads and particles are polymeric beads, polymeric particles, magnetic beads, magnetic particles, supermagnetic beads, or supermagnetic particles.

18. The MHC molecule construct according to any of claims 1 to 17 for use in a flow cytometric method, a histological method or a cytological method.

19. A composition comprising a MHC molecule construct according to any one of claims 1 to 18.

20. The composition according to claim 19, wherein the MHC molecule construct comprises peptide filled MHC molecules or peptide free MHC molecules; and wherein said peptides to fill the peptide free MHC molecules, and the MHC molecule construct comprising peptide free MHC molecules are provided separately.

21. The composition according to claim 19 further comprising one or more biologically active molecules and/or excipients.

22. The composition according to any of claims 19 to 21 for use in treating, preventing, stabilising, or alleviating disease involving MHC recognising cells.

23. The composition according to claim 22, wherein the disease is of inflammatory, auto-immune, allergic, viral, cancerous, infectious, allogene, xenogene, graft versus host, or host-versus-graft origin, chronic inflammatory bowel disease, Crohn's disease, ulcerative colitis, sclerosis, type I diabetes, rheumatoid arthritis, psoriasis, atopic dermatitis, asthma, malignant melanoma, renal carcinoma, breast cancer, lung cancer, cancer of the uterus, prostatic cancer, brain cancer, head and neck cancer, leukemia, cutaneous lymphoma, hepatic carcinoma, colorectal cancer, bladder cancer, rejection-related disease, graft-versus-host-related disease, a viral disease associated with hepatitis, AIDS, measles, pox, chicken pox, rubella or herpes.

24. The composition according to any one of claims 19 to 23 formulated for parenteral administration, including intravenous, intramuscular, subcutaneous, intradermal, epicutantous/transdermal, and intraperitoneal administration, and for infusion.

25. Use of the composition of any one of the claims 19 to 24 for the manufacture of a medicament for up-regulating, down-regulating or modulating an immune response in an animal, for inducing anergy of a cell or for adoptive cellular immunotherapy.

26. A method of obtaining MHC recognising cells comprising bringing into contact a MHC molecule construct according to any one of claims 1 to 18 and a sample suspected of comprising MHC recognising cells under conditions whereby the MHC recognising cells bind to the MHC molecule construct, and isolating the bound MHC molecule construct and MHC recognising cells.

27. The method according to claim 26, wherein the isolation is carried out by applying a magnetic field or by flow cytometry.

28. A method for producing a composition according to any one of claims 19 to 24, comprising providing a MHC molecule construct as defined in claims 1 to 18, solubilising or dispersing the MHC molecule construct in a medium suitable for therapeutic substances, and optionally adding other excipients.

29. A method for detecting or monitoring the presence of MHC recognising cells in a sample comprising the steps of
(a) contacting a sample suspected of comprising MHC recognising cells with a MHC molecule construct according to any one of claims 1 to 18, and
(b) determining any binding of the MHC molecule construct, which binding indicates the presence of MHC recognising cells.

30. A method for establishing a prognosis of a disease, determining the status of a disease or diagnosing a disease involving MHC recognising cells comprising the steps of
(a) contacting a sample suspected of comprising MHC recognising cells with a MHC molecule construct according to claims 1 to 18, and
(b) determining any binding of the MHC molecule construct, thereby establishing a prognosis of a disease, determining the status of a disease or diagnosing a disease involving MHC recognising cells.

31. A method for determining the effectiveness of a medicament against a disease involving MHC recognising cells comprising the steps of
(a) contacting a sample from a subject receiving treatment with a medicament with a MHC molecule construct according to claims 1 to 18, and
(b) determining any binding of the MHC molecule construct, thereby determining the effectiveness of the medicament.

32. The method according to any one or claims 29 to 31, wherein the MHC recognizing cells are involved in a disease of inflammatory, auto-immune, allergic, viral, cancerous, infectious, allogene, xenogene, host-versus-graft, graft-versus-host origin or a chronic inflammatory bowel disease, Crohn's disease, ulcerative colitis, sclerosis, type I diabetes, rheumatoid arthritis, psoriasis, atopic dermatitis, asthma, malignant melanoma, renal carcinoma, breast cancer, lung cancer, cancer of the uterus, cervical cancer, prostatic cancer, brain cancer, head and neck cancer, leukemia, cutaneous lymphoma, hepatic carcinoma, colorectal cancer, bladder cancer, rejection-related disease, graft-versus-host-related disease, viral disease associated with hepatitis, AIDS, measles, pox, chicken pox, rubella or herpes.

33. The method according to claim 29 to 32, wherein the MHC recognising cells are selected from subpopulations of CD3+ T-cells, gamma, delta T-cells, alpha, beta T cells, CD4+ T-cells, T helper cells, CD8+ T-cells, Suppressor T-cells, CD8+ cytotoxic T cells, CTLs, NK cells, NKT cells, LAK cells, and MAK.

34. The method according to any one of claims 29 to 33, wherein the sample is selected from the group consisting of histological material, cytological material, primary tumors, secondary organ metastasis, fine needle aspirates, spleen tissue, bone marrow specimens, cell smears, exfoliative cytological specimens, touch preparations, oral swabs, laryngeal swabs, vaginal swabs, bronchial lavage, gastric lavage, an umbilical cord sample, a body fluid sample, a blood sample, a sample from a peripheral blood mononuclear cell population isolated from blood, a sample from a blood-derived preparation, a sample from a leukopheresis product, sputum samples, expectorates, bronchial aspirates, biopsies and sections of biopsies.

35. The method according to any one of claims 29 to 34, wherein the determination of the binding is carried out by inspection in a microscope, by light, by fluorescence, by electron transmission, or by flow cytometry.

36. The method according to any of claims 29 to 35, wherein the sample is mounted on a support, a solid or semi-solid support or on glass slides, microtiter plates having one or more wells, beads, particles, membranes, filters, filter membranes, polymer slides, polymer membranes, chamber slides, dishes, and petridishes.

37. A method for manufacturing the composition according to claim 19 comprising the steps of
(i) providing at least 5 MHC molecules;
(ii) providing a dextran carrier molecule;
(iii) providing more than one binding entity;
(iv) attaching said at least 5 MHC molecules to said dextran carrier via said more than one binding entity; wherein the attachment results in that each binding entity is attached to from 2 to 4 MHC molecules;
(v) optionally attaching one or more biologically active molecules to said dextran carrier either directly or via said more than one binding entity;
(vi) optionally attaching one or more labelling compounds to said dextran carrier molecule, or to one or more of said more than one binding entity or to one or more of said at least 5 MHC molecules;
and thereby manufacturing the composition according to claim 19.

38. The method according to claim 26, the MHC recognising cells is obtained by bringing a sample from a subject comprising MHC recognising cells into contact with a MHC molecule construct according to any one of claims 1 to 18, whereby the MHC recognising cells become bound to the MHC molecule construct, isolating the bound MHC molecule construct and the MHC recognising cells, and optionally expanding ex vivo such MHC recognising cells to a clinically relevant number and wherein the isolated MHC recognising cells are liberated from the MHC molecule construct prior to expansion.

39. The method according to any of claims 26 or 38, wherein the MHC molecule construct is immobilised onto the solid or semi-solid support prior to or following contact with the sample.

40. The method according to any of claims 38 to 39, wherein the expansion is carried out in the presence of one or more MHC molecule constructs, optionally one or more biologically active molecules and optionally feeder cells such as dendritic cells or feeder cells.

## Patentansprüche

1. MHC-Molekülkonstrukt in löslicher Form in einem lösenden Medium oder immobilisiert auf einem festen oder halbfesten Träger, wobei das MHC-Molekülkonstrukt ein lösliches Dextranträgermolekül umfasst, das an wenigstens 5 MHC-Moleküle gebunden ist, wobei die MHC-Moleküle über mehr als eine Bindungseinheit an das Dextranträgermolekül gebunden ist, wobei jede Bindungseinheit an 2 bis 4 MHC-Moleküle anbindet.

2. MHC-Molekülkonstrukt in löslicher Form in einem lösenden Medium oder immobilisiert auf einem festen oder halbfesten Träger, wobei das MHC-Molekülkonstrukt ein lösliches Dextranträgermolekül umfasst, das an wenigstens 5 MHC-Moleküle gebunden ist, wobei die MHC-Moleküle unmittelbar an das Dextranträgermolekül anbinden und das MHC-Molekülkonstrukt eine oder mehrere Markierungen umfasst.

3. MHC-Molekülkonstrukt nach einem der Ansprüche 1 bis 2, wobei das MHC-Molekül ein peptidfreies MHC-Molekül oder ein peptidgefülltes MHC-Molekül ist.

4. MHC-Molekülkonstrukt nach einem der Ansprüche 1 bis 3, wobei die MHC-Moleküle gleich sind oder wenigstens zwei der MHC-Moleküle unterschiedlich sind.

5. MHC-Molekülkonstrukt nach einem der Ansprüche 1 bis 4, wobei die Peptide, die von den MHC-Molekülen aufgenommen sind, gleich sind oder wenigstens zwei der Peptide, die von den MHC-Molekülen aufgenommen sind, unterschiedlich sind.

6. MHC-Molekülkonstrukt nach einem der Ansprüche 1 und 3 bis 5, wobei die MHC-Moleküle über zwei oder mehr identische Bindungseinheiten oder Kombinationen von einer oder mehreren unterschiedlichen Bindungseinheiten an dem Trägermolekül angebunden sind.

7. MHC-Molekülkonstrukt nach einem der Ansprüche 1 und 3 bis 6, wobei die Bindungseinheit ausgewählt ist aus Streptavidin und Derivativen davon, Avidin und Derivativen davon, Biotin, Immunglobulinen, monoklonalen Antikörpern, polyklonalen Antikörpern, rekombinanten Antikörpern, Antikörperfragmenten und Derivativen davon, der Leucin-Zipper-Domäne von AP-1, jun-, fos-, hexa-his-, hexa-hat-Glutathion-S-Tranferase, glutathionaffinem, calmodulinbindendem Peptid, Strep-Tag, Zellulosebindungsdomäne, Maltosebindungsprotein, S-Peptid-Tag, Chitinbindungs-Tag, immunreaktiven Epitopen, Epitop-Tags, E2Tag, HA-Epitop-Tag, Myc-Epitop, FLAG-Epitop, AU1- und AU5-Epitop, Glu-Glu-Epitop, KT3-Epitop, IRS-Epitop, Btag-Epitop, Proteinkinase-C-Epitop, VSV-Epitop, Lektinen, die die Bindung an eine verschiedene Verbindungen vermitteln, einschließlich Kohlenhydraten, Lipiden und Proteinen, vorzugsweise Con A oder WGA und Tetranectin oder Protein A und Protein G.

8. MHC-Molekülkonstrukt nach einem der Ansprüche 1 bis 7, ferner umfassend ein oder mehrere biologisch aktive Moleküle.

9. MHC-Molekülkonstrukt nach Anspruch 8, wobei die biologisch aktiven Moleküle am Trägermolekül entweder unmittelbar oder über eine oder mehrere der Bindungseinheiten anbinden.

10. MHC-Molekülkonstrukt nach einem der Ansprüche 8 bis 9, wobei das biologisch aktive Molekül ausgewählt ist aus
(a) Proteinen aus der Gruppe MHC-Klasse-1-artige Proteine wie MIC A, MIC B, CD1d, HLA E, HLA F, HLA G, HLA H, ULBP-1, ULBP-2 und ULBP-3;
(b) kostimulatorischen Molekülen aus der Gruppe CD2, CD3, CD4, CD5, CD8, CD9, CD27, CD28, CD30, CD69, CD134, CD137, CD147, CDw150, CD152, CD153, CD40L, NKG2D, ICOS, HVEM, HLA Klasse II, PD-1, Fas, FasL exprimiert an T- und/oder NK-Zellen, CD40, CD48, CD58, CD70, CD72, B7.1, B7.2, B7RP-1, B7-H3, PD-LI, PD-L2, CD134L, CD137L, ICOSL, LIGHT exprimiert an APC- und/oder Tumorzellen;
(c) zellmodulierenden Molekülen aus der Gruppe CD16, NKp30, NKp44, NKp46, NKp80, 2B4, KIR, LIR, CD94/NKG2A, CD94/NKG2C exprimiert an NK-Zellen, IFN-alpha, IFN-beta, IFN-gamma, IL-1, IL-2, IL-3, IL-4, IL-6, IL-7, IL-8, IL-10, IL-11, IL-12, IL-15, kolonstimulierende Faktoren, Vitamin D3, IL-2-Toxinen, Cyclosporin, FK-506, Rapamycin, TGF-beta, Clotrimazol, Nitrendipin und Charybdotoxin;
(d) akzessorischen Molekülen aus der Gruppe LFA-1, CD11a/18, CD54, CD106 und CD49a,b,c,d,e,f/CD29;
(e) Adhäsionsmoleküle aus der Gruppe ICAM-1 ICAM-2, GlyCAM-1, CD34, Anti-LFA-1, Anti-CD44, Anti-beta7, Chemokine, CXCR4, CCR5, Anti-Selectin L, Anti-Selectin E und Anti-Selectin P;
(f) toxische Moleküle aus der Gruppe Cyclophosphamid, Methrotrexat, Azathioprin, Mizoribin, 1 5-Deoxuspergualin, Neomycin, Staurosporin, Genestein, Herbimycin A, Pseudomonas-Exotoxin A, Saporin, Rituxan, Ricin, Gemtuzumab-Ozogamicin, Shiga-Toxin, Schwermetalle, anorganische Quecksilber, organische Quecksilber, FN18-CRM9, Radioisotope, aufgenommene Isotope von Jod, aufgenommene Isotope von Kobalt, aufgenommene Isotope von Selen, aufgenommene Isotope von Tritium, aufgenommene Isotope von Phosphor, Haptene, DNP und Digoxiginin; und Antikörper für eines der Moleküle aus (a) - (f), Derivative der Antikörper und Fragmente der Antikörper und Kombinationen davon und
(g) Proteinen, kostimulatorischen Molekülen, zellmodulierenden Molekülen, Rezeptoren, akzessorischen Molekülen, Adhäsionsmolekülen, natürlichen Liganden und toxischen Molekülen und Antikörpern und rekombinanten daran bindenden Molekülen und Kombinationen davon.

11. MHC-Molekülkonstrukt nach einem der Ansprüche 1 bis 10, ferner umfassend eine oder mehrere Markierungsverbindungen.

12. MHC-Molekülkonstrukt nach Anspruch 11, wobei eine oder mehrere Markierungsverbindungen am Trägermolekül und/oder einer oder mehreren der Bindungseinheiten und/oder einem oder mehreren MHC-Molekülen anbinden.

13. MHC-Molekülkonstrukt nach Anspruch 11 oder 12, wobei die eine oder mehrere Markierungsverbindung direkt oder indirekt nachweisbar ist.

14. MHC-Molekülkonstrukt nach einem der Ansprüche 11 bis 13, wobei die eine oder die mehreren Markierungsverbindungen
(a) ausgewählt ist/sind aus fluoreszierenden Markierungen aus der Gruppe 5- und 6-Carboxyfluorescein, 5- oder 6-Carboxyfluorescein, 6-Fluorescein-5- und -6-carboxamidcapronsäure, Fluoresceinisothiocyanat, Rhodamin, Tetramethylrhodamin, Farbstoffen, Cy2, Cy3, Cy5, wahlweise substituiertes Kumarin, AMCA, PerCP, Phycobiliproteinen, R-Phycoerythrin, Allophycoerythrin, Texas Red, Princeston Red, grün fluoreszierendes Protein und Analoge davon, Konjugate von R-Phycoerythrin, Konjugate von Allophycoerythrin und Cy5 oder Texas Red, anorganische fluoreszierende Markierungen auf Basis von Halbleiternanokristallen, Quantum Dot und zeitlich aufgelöste fluoreszierende Markierungen auf Basis von Lanthaniden, zeitlich aufgelöste fluoreszierende Markierungen auf Basis von Eu3+ und zeitlich aufgelöste fluoreszierende Markierungen auf Basis von Sm3+;
(b) ausgewählt ist/sind aus Haptenen aus der Gruppe DNP, Biotin und Digoxiginin;
(c) ausgewählt ist/sind aus enzymatischen Markierungen aus der Gruppe Meerrettichperoxidase, alkalische Phosphatase, beta-Galactosidase, Glucose-6-phosphatdehydrogenase, beta-N-Acetylglucosaminidase, _-Glucuronidase, Invertase, Xanthin-Oxidase, Glühwürmchen-Luciferase und Glucoseoxidase;
(d) ausgewählt ist/sind aus Luminiszenzmarkierungen aus der Gruppe Luminol, Isoluminol, Acridiniumester, 1,2-Dioxetanen und Pyridopyridazinen; und
(e) ausgewählt ist/sind aus radioaktiven Markierungen aus der Gruppe aufgenommene Isotope von Jod, aufgenommene Isotope von Kobalt, aufgenommene Isotope von Selen, aufgenommene Isotope von Tritium und aufgenommene Isotope von Phosphor und
(f) ausgewählt ist/sind aus einer fluoreszierenden Markierung, einer Enzymmarkierung, einem Radioisotop, einer chemilumineszierenden Markierung, einer biolumineszierenden Markierung, einem Polymer, einem Metallpartikel, einem Hapten, einem Antikörper oder einem Farbstoff.

15. MHC-Molekülkonstrukt nach einem der Ansprüche 1 bis 14, das unmittelbar auf dem festen oder halbfesten Träger immobilisiert ist.

16. MHC-Molekülkonstrukt nach einem der Ansprüche 1 bis 15, das auf dem festen oder halbfesten Träger über einen Linker, einen Spacer oder einen Antikörper, ein Antikörperderivat oder ein Fragment davon immobilisiert ist.

17. MHC-Molekülkonstrukt nach einem der Ansprüche 15 bis 16, wobei der Träger ausgewählt ist aus der Gruppe bestehend aus Partikeln, Perlen, biologisch abbaubaren Partikeln, Folien, Gelen, Filtern, Membranen, Nylonmembranen, Fasern, Kapillaren, Nadeln, Mikrotiterstreifen, Röhren, Platten oder Mulden, Kämmen, Pipettenspitzen, Mikroarrays, Chips, Objektträgern, die Perlen und Partikeln sind Polymerperlen, Polymerpartikeln, magnetische Perlen, magnetische Partikeln, supermagnetische Perlen oder supermagnetische Partikeln.

18. MHC-Molekülkonstrukt nach einem der Ansprüche 1 bis 17 zur Verwendung in einem Durchflusszytometrieverfahren, einem histologischen Verfahren oder einem zytologischen Verfahren.

19. Zusammensetzung, umfassend ein MHC-Molekülkonstrukt nach einem der Ansprüche 1 bis 18.

20. Zusammensetzung nach Anspruch 19, wobei das MHC-Molekülkonstrukt peptidgefüllte MHC-Moleküle oder peptidfreie MHC-Moleküle umfasst; und wobei die Peptide zum Füllen der peptidfreien MHC-Moleküle und das MHC-Molekülkonstrukt, das peptidfreie MHC-Moleküle umfasst, separat bereitgestellt werden.

21. Zusammensetzung nach Anspruch 19, ferner umfassend ein oder mehr biologisch aktive Moleküle und/oder Vehikel.

22. Zusammensetzung nach einem der Ansprüche 19 bis 21 zur Verwendung beim Behandeln, Verhindern, Stabilisieren oder Lindern von Erkrankungen im Zusammenhang mit MHC erkennenden Zellen.

23. Zusammensetzung nach Anspruch 22, wobei die Erkrankung entzündlichen, autoimmunen, allergischen, viralen, Krebs-, infektiösen, allogenen, xenogenen, Transplantat-Wirt-Reaktion- oder Wirt-Transplantat-Reaktionursprungs, chronisch entzündliche Darmerkrankung, Morbus Crohn, Colitis ulcerosa, Sclerose, Diabetes Typ I, rheumatoide Arthritis, Psoriasis, atopische Dermatitis, Asthma, bösartiges Melanom, Nierenkarzinom, Brustkrebs, Lungenkrebs, Gebärmutterkrebs, Prostatakrebs, Gehirntumor, Kopf- und Nackenkrebs, Leukämie, kutanes Lymphom, Leberkarzinom, kolorektaler Krebs, Blasenkrebs, eine Erkrankung im Zusammenhang mit einer Abstoßung, eine Erkrankung im Zusammenhang mit einer Transplantat-Wirt-Reaktion , eine virale Erkrankung im Zusammenhang mit Hepatitis, AIDS, Masern, Pocken, Windpocken, Röteln oder Herpes ist.

24. Zusammensetzung nach einem der Ansprüche 19 bis 23, die zur parenteralen Verabreichung, einschließlich intravenöser, intramuskulärer, subkutaner, intradermaler, epikutanter/transdermaler und intraperitonealer Verabreichung, und zur Infusion formuliert ist.

25. Verwendung der Zusammensetzung nach einem der Ansprüche 19 bis 24 zur Herstellung eines Medikaments zum Steigern, Dämpfen oder Modulieren einer Immunantwort in Reaktion auf ein Tier, zum Induzieren von Anergie einer Zelle oder zur adoptiven zellulären Immuntherapie.

26. Verfahren zum Erlangen von MHC erkennenden Zellen umfassend In-Kontakt-Bringen eines MHC-Molekülkonstrukts nach einem der Ansprüche 1 bis 18 und einer Probe, von der vermutet wird, dass sie MHC erkennende Zellen umfasst, unter Bedingungen, wobei die MHC erkennenden Zellen an das MHC-Molekülkonstrukt anbinden, und Isolieren des MHC-Molekülkonstrukts und der MHC erkennenden Zellen, die aneinander gebunden sind.

27. Verfahren nach Anspruch 26, wobei die Isolation durch Anlegen eines Magnetfelds oder durch Durchflusszytometrie ausgeführt wird.

28. Verfahren zum Herstellen einer Zusammensetzung nach einem der Ansprüche 19 bis 24, umfassend Bereitstellen eines MHC-Molekülkonstrukts nach den Ansprüchen 1 bis 18, Lösen oder Dispergieren des MHC-Molekülkonstrukts in einem Medium, das für therapeutische Substanzen geeignet ist, und wahlweise Zusetzen weiterer Vehikel.

29. Verfahren zum Nachweisen oder Überwachen des Vorhandenseins von MHC erkennenden Zellen in einer Probe, folgende Schritte umfassend
(a) In-Kontakt-Bringen einer Probe, von der vermutet wird, dass sie MHC erkennende Zellen umfasst, mit einem MHC-Molekülkonstrukt nach einem der Ansprüche 1 bis 18, und
(b) Bestimmen einer Anbindung des MHC-Molekülkonstrukts, wobei die Anbindung das Vorhandensein MHC erkennender Zellen anzeigt.

30. Verfahren zum Erstellen einer Prognose einer Erkrankung, Bestimmen des Status einer Erkrankung oder Diagnostizieren einer Erkrankung im Zusammenhang mit MHC erkennenden Zellen, folgende Schritte umfassend
(a) In-Kontakt-Bringen einer Probe, von der vermutet wird, dass sie MHC erkennende Zellen umfasst, mit einem MHC-Molekülkonstrukt nach einem der Ansprüche 1 bis 18, und
(b) Bestimmen einer Anbindung des MHC-Molekülkonstrukts und dadurch Erstellen einer Prognose einer Erkrankung, Bestimmen des Status einer Erkrankung oder Diagnostizieren einer Erkrankung im Zusammenhang mit MHC erkennenden Zellen.

31. Verfahren zum Bestimmen der Wirksamkeit eines Medikaments gegen eine Erkrankung im Zusammenhang mit MHC erkennenden Zellen, folgende Schritte umfassend
(a) In-Kontakt-Bringen einer Probe von einem behandelten Patienten mit einem Medikament mit einem MHC-Molekülkonstrukt nach den Ansprüchen 1 bis 18, und
(b) Bestimmen einer Anbindung des MHC-Molekülkonstrukts und dadurch Bestimmen der Wirksamkeit des Medikaments.

32. Verfahren nach einem der Ansprüche 29 bis 31, wobei die MHC erkennenden Zellen an einer Erkrankung entzündlichen, autoimmunen, allergischen, viralen, Krebs-, infektiösen, allogenen, xenogenen, Wirt-Transplantat-Reaktion- oder Transplantat-Wirt-Reaktionursprungs, chronisch entzündliche Darmerkrankung, Morbus Crohn, Colitis ulcerosa, Sclerose, Diabetes Typ I, rheumatoide Arthritis, Psoriasis, atopische Dermatitis, Asthma, bösartiges Melanom, Nierenkarzinom, Brustkrebs, Lungenkrebs, Gebärmutterkrebs, Gebärmutterhalskrebs, Prostatakrebs, Gehirntumor, Kopf- und Nackenkrebs, Leukämie, kutanes Lymphom, Leberkarzinom, kolorektaler Krebs, Blasenkrebs, eine Erkrankung im Zusammenhang mit einer Abstoßung, eine Erkrankung im Zusammenhang mit einer Transplantat-Wirt-Reaktion, eine virale Erkrankung im Zusammenhang mit Hepatitis, AIDS, Masern, Pocken, Windpocken, Röteln oder Herpes beteiligt sind.

33. Verfahren nach Anspruch 29 bis 32, wobei die MHC erkennenden Zellen ausgewählt werden aus Subpopulationen von CD3+-T-Zellen, gamma-delta-T-Zellen, alpha-beta-T-Zellen, CD4+-T-Zellen, T-Helferzellen, CD8+-T-Zellen, regulatorischen T-Zellen, zytotoxischen CD8+-T-Zellen, CTLs, NK-Zellen, NKT-Zellen, LAK-Zellen und MAK.

34. Verfahren nach einem der Ansprüche 29 bis 33, wobei die Probe ausgewählt wird aus der Gruppe bestehend aus histologischem Material, zytologischem Material, Primärtumoren, sekundären Organmetastasen, Feinnadelaspiraten, Milzgewebe, Knochenmarkproben, Zellabstrichen, exfoliativen zytologischen Proben, Berührungspräparaten, Tupfern für orale Abstriche, Tupfern für Kehlkopfabstriche, Tupfern für Vaginalabstriche, Bronchialspülung, Darmspülung, einer Nabelschnurprobe, einer Körperfluidprobe, einer Blutprobe, einer Probe von einer peripheren mononuklearen Blutzellpopulation, die von Blut isoliert wurde, einer Probe von einer aus Blut gewonnenen Zubereitung, einer Probe von einem eine Leukophereseprodukt, Auswurfproben, Ausgehustetem, Bronchialaspiraten, Biopsien und Abschnitten von Biopsien.

35. Verfahren nach einem der Ansprüche 29 bis 34, wobei die Bestimmung der Bindung durch Untersuchung in einem Mikroskop, durch Licht, durch Fluoreszenz, durch Elektronentransmission oder durch Durchflusszytometrie ausgeführt wird.

36. Verfahren nach einem der Ansprüche 29 bis 35, wobei die Probe auf einen Träger, einen festen oder halbfesten Träger oder auf Glasobjektträger, Mikrotiterplatten mit einer oder mehreren Mulden, Perlen, Partikeln, Membranen, Filter, Filtermembranen, Polymerobjektträger, Polymermembranen, Kammerobjektträger, Schalen und Petrischalen aufgebracht wird.

37. Verfahren zum Herstellen der Zusammensetzung nach Anspruch 19, folgende Schritte umfassend
(i) Bereitstellen von wenigstens 5 MHC-Molekülen;
(ii) Bereitstellen eines dextranen Trägermoleküls;
(iii) Bereitstellen von mehr als einer Bindungseinheit;
(iv) Anbinden der wenigstens 5 MHC-Moleküle an den dextranen Träger über die mehr als eine Bindungseinheit; wobei das Anbinden bewirkt, dass jede Bindungseinheit derart angebunden wird, dass sie an 2 bis 4 MHC-Moleküle anbindet;
(v) wahlweise Anbinden von einem oder mehreren biologisch aktiven Molekülen an dem dextranen Träger entweder unmittelbar oder über die mehr als eine Bindungseinheit;
(vi) wahlweise Anbinden von einer oder mehreren Markierungsverbindungen an dem dextranen Trägermolekül, oder an einer oder mehreren der mehr als einen Bindungseinheit oder auf eins oder mehrere der wenigstens 5 MHC-Moleküle;
und dadurch Herstellen der Zusammensetzung nach Anspruch 19.

38. Verfahren nach Anspruch 26, wobei die MHC erkennenden Zellen erlangt werden, indem eine Probe von einem Patienten, die MHC erkennende Zellen umfasst, in Kontakt mit einem MHC-Molekülkonstrukt nach einem der Ansprüche 1 bis 18 gebracht wird, wodurch die MHC erkennenden Zellen an das MHC-Molekülkonstrukt gebunden werden, das MHC-Molekülkonstrukt und die MHC erkennenden Zellen, die aneinander gebunden sind, isoliert werden, und wahlweise die MHC erkennenden Zellen ex vivo auf eine klinisch relevante Anzahl vermehrt werden, und wobei die isolierten MHC erkennenden Zellen vor der Vermehrung von dem MHC-Molekülkonstrukt freigesetzt werden.

39. Verfahren nach einem der Ansprüche 26 oder 38, wobei das MHC-Molekülkonstrukt auf dem festen oder halbfesten Träger vor oder nach dem Kontakt mit der Probe immobilisiert wird.

40. Verfahren nach einem der Ansprüche 38 bis 39, wobei die Vermehrung in Anwesenheit von einem oder mehreren MHC-Molekülkonstrukten, wahlweise einem oder mehreren biologisch aktiven Molekülen und wahlweise Fütterzellen wie etwa dendritische Zellen oder Fütterzellen durchgeführt wird.

## Revendications

1. Construction de molécules du CMH sous forme soluble dans un milieu solubilisant ou immobilisée sur un support solide ou semi-solide, ladite construction de molécules du CMH comprenant une molécule de support de dextrane soluble fixée à au moins 5 molécules du CMH, où lesdites molécules du CMH sont fixées à la molécule de support de dextrane par l'intermédiaire de plus d'une entité de liaison, où chaque entité de liaison est fixée à 2 à 4 molécules du CMH.

2. Construction de molécules du CMH sous forme soluble dans un milieu solubilisant ou immobilisée sur un support solide ou semi-solide, ladite construction de molécules du CMH comprenant une molécule de support de dextrane soluble fixée à au moins 5 molécules du CMH, où lesdites molécules du CMH sont fixées directement à la molécule de support de dextrane et ladite construction de molécules du CMH comprend un ou plusieurs marqueurs.

3. Construction de molécules du CMH selon l'une quelconque des revendications 1 à 2, où la molécule du CMH est une molécule du CMH dépourvue de peptide ou une molécule du CMH remplie de peptides.

4. Construction de molécules du CMH selon l'une quelconque des revendications 1 à 3, où les molécules du CMH sont identiques ou au moins deux des molécules du CMH sont différentes.

5. Construction de molécules du CMH selon l'une quelconque des revendications 1 à 4, où les peptides abrités par les molécules de CMH sont identiques ou au moins deux des peptides abrités par les molécules du CMH sont différents.

6. Construction de molécules du CMH selon l'une quelconque des revendications 1 et 3 à 5, où les molécules du CMH sont fixées à la molécule de support par l'intermédiaire de deux entités de liaison identiques ou plus, ou des combinaisons d'une ou de plusieurs entités de liaison différentes.

7. Construction de molécules du CMH selon l'une quelconque des revendications 1 et 3 à 6, où l'entité de liaison est choisie parmi la streptavidine, et ses dérivés, l'avidine et ses dérivés, la biotine, des immunoglobulines, des anticorps monoclonaux, des anticorps polyclonaux, des anticorps recombinants, des fragments d'anticorps et leurs dérivés, le domaine leucine zipper de AP-1, jun, fos, hexa-his, hexa-hat, la glutathion-S-transférase, l'affinité pour le glutathion, le peptide de liaison de la calmoduline, le marqueur Strep, le domaine de liaison de la cellulose, la protéine de liaison du maltose, le marqueur S-Peptide, le marqueur de liaison de la chitine, les épitopes immunoréactifs, les marqueurs d'épitopes, le marqueur E2, le marqueur d'épitope HA, l'épitope Myc, l'épitope FLAG, les épitopes AU1 et AU5, l'épitope Glu-Glu, l'épitope KT3, l'épitope IRS, l'épitope Btag, l'épitope de protéine kinase C, l'épitope du VSV, les lectines qui médient la liaison à divers composés, y compris des glucides, des lipides et des protéines, de préférence Con A ou WGA et la tétranectine ou la protéine A et la protéine G.

8. Construction de molécules du CMH selon l'une quelconque des revendications 1 à 7, comprenant en outre une ou plusieurs molécules biologiquement actives.

9. Construction de molécules du CMH selon la revendication 8, où la molécule biologiquement active est fixée à la molécule de support soit directement soit par l'intermédiaire d'une ou de plusieurs des entités de liaison.

10. Construction de molécules du CMH selon l'une quelconque des revendications 8 à 9, où la molécule biologiquement active est choisie parmi
(a) des protéines du groupe des protéines de type CMH de classe I comme MIC A, MIC B, CD1d, HLA E, HLA F, HLA G, HLA H, ULBP-1, ULBP-2, et ULBP-3 ;
(b) des molécules costimulantes du groupe de CD2, CD3, CD4, CD5, CD8, CD9, CD27, CD28, CD30, CD69, CD134, CD137, CD147, CDw150, CD152, CD153, CD40L, NKG2D, ICOS, HVEM, HLA de classe II, PD-1, Fas, FasL exprimé sur des lymphocytes T et/ou des cellules NK, CD40, CD48, CD58, CD70, CD72, B7.1, B7.2, B7RP-1, B7-H3, PD-LI, PD-L2, CD134L, CD137L, ICOSL, LIGHT exprimé sur des cellules CPA et/ou tumorales ;
(c) des molécules modulant les cellules du groupe de CD16, NKp30, NKp44, NKp46, NKp80, 2B4, KIR, LIR, CD94/NKG2A, CD94/NKG2C exprimé sur des cellules NK, IFN-alpha, IFN-bêta, IFN-gamma, IL-1, IL-2, IL-3, IL-4, IL-6, IL-7, IL-8, IL-10, IL-11, IL-12, IL-15, facteurs de stimulation des colonies, vitamine D3, toxines d'IL-2, cyclosporine, FK-506, rapamycine, TGF-bêta, clotrimazole, nitrendipine, et charybdotoxine ;
(d) des molécules accessoires du groupe de LFA-1, CD11a/18, CD54, CD106, et CD49a,b,c,d,e,f/CD29 ;
(e) des molécules d'adhérence du groupe de ICAM-1, ICAM-2, GlyCAM-1, CD34, anti-LFA-1, anti-CD44, anti-bêta7, chimiokines, CXCR4, CCR5, anti-sélectine L, anti-sélectine E, et anti-sélectine P ;
(f) des molécules toxiques du groupe de cyclophosphamide, méthotrexate, azathioprine, mizoribine, 1,5-désoxyspergualine, néomycine, staurosporine, génistéine, herbimycine A, exotoxine A de Pseudomonas, saporine, Rituxan, ricine, gemtuzumab, ozogamicine, toxine Shiga, métaux lourds, mercuriels inorganiques, mercuriels organiques, FN18-CRM9, radioisotopes, isotopes incorporés d'iodure, isotopes incorporés de cobalt, isotopes incorporés de sélénium, isotopes incorporés de tritium, isotopes incorporés de phosphore, haptènes, DNP, et digoxigénine ; et des anticorps contre l'une quelconque des molécules en (a) à (f), des dérivés desdits anticorps et des fragments desdits anticorps, et leurs combinaisons et
(g) des protéines, des molécules costimulantes, des molécules modulant les cellules, des récepteurs, des molécules accessoires, des molécules d'adhérence, des ligands naturels, et des molécules toxiques, et des anticorps et des molécules de liaison recombinantes de ceux-ci, et leurs combinaisons.

11. Construction de molécules du CMH selon l'une quelconque des revendications 1 à 10, comprenant en outre un ou plusieurs composés de marquage.

12. Construction de molécules du CMH selon la revendication 11, où un ou plusieurs composés de marquage sont fixés à la molécule de support et/ou une ou plusieurs des entités de liaison et/ou une ou plusieurs molécules du CMH.

13. Construction de molécules du CMH selon la revendication 11 ou 12, où le composé de marquage est détectable directement ou indirectement.

14. Construction de molécules du CMH selon l'une quelconque des revendications 11 à 13, où le composé de marquage est
(a) choisi parmi des marqueurs fluorescents du groupe de 5- et 6-carboxyfluorescéine, 5- ou 6-carboxy-fluorescéine, acide 6-fluorescéine-5- et 6-carboxamido-hexanoïque, isothiocyanate de fluorescéine, rhodamine, tétraméthylrhodamine, colorants, Cy2, Cy3, Cy5, coumarine éventuellement substituée, AMCA, PerCP, phycobiliprotéines, phycoérythrine R, allophycoérythrine, rouge Texas, rouge Princeston, protéine fluorescente verte et ses analogues, conjugués de phycoérythrine R, conjugués d'allophycoérythrine et de Cy5 ou de rouge Texas, marqueurs fluorescents inorganiques à base de nanocristaux semi-conducteurs, point quantique, et marqueurs fluorescents résolus dans le temps à base de lanthanides, marqueurs fluorescents résolus dans le temps à base de Eu3+, et marqueurs fluorescents résolus dans le temps à base de Sm3+ ;
(b) choisi parmi des haptènes du groupe de DNP, biotine, et digoxigénine ;
(c) choisi parmi des marqueurs enzymatiques du groupe de la peroxydase de raifort, la phosphatase alcaline, la bêta-galactosidase, la glucose-6-phosphate déshydrogénase, la bêta-N-acétylglucosaminidase, la _-glucuronidase, l'invertase, la xanthine oxydase, la luciférase de luciole et la glucose oxydase ;
(d) choisi parmi des marqueurs luminescents du groupe du luminol, l'isoluminol, des esters d'acridinium, des 1,2-dioxétanes et des pyridopyridazines ; et
(e) choisi parmi des marqueurs radioactifs du groupe des isotopes incorporés d'iodure, des isotopes incorporés de cobalt, des isotopes incorporés de sélénium, des isotopes incorporés de tritium, et des isotopes incorporés de phosphore et
(f) choisi parmi un marqueur fluorescent, un marqueur enzymatique, un radio-isotope, un marqueur chimioluminescent, un marqueur bioluminescent, un polymère, une particule métallique, un haptène, un anticorps, ou un colorant.

15. Construction de molécules du CMH selon l'une quelconque des revendications 1 à 14, immobilisée directement sur le support solide ou semi-solide.

16. Construction de molécules du CMH selon l'une quelconque des revendications 1 à 15, immobilisée sur le support solide ou semi-solide par l'intermédiaire d'un lieur, d'un espaceur, ou d'un anticorps, d'un dérivé d'anticorps ou de l'un de ses fragments.

17. Construction de molécules du CMH selon l'une quelconque des revendications 15 à 16, où le support est choisi dans le groupe constitué de particules, billes, particules biodégradables, feuillets, gels, filtres, membranes, membranes de nylon, fibres, capillaires, aiguilles, bandelettes de microtitrage, tubes, plaques ou puits, peignes, embouts de pipette, micropuces, puces, lames, les billes et les particules sont des billes polymères, des particules polymères, des billes magnétiques, des particules magnétiques, des billes supermagnétiques, ou des particules supermagnétiques.

18. Construction de molécules du CMH selon l'une quelconque des revendications 1 à 17, pour une utilisation dans un procédé de cytométrie en flux, un procédé histologique ou un procédé cytologique.

19. Composition comprenant une construction de molécules du CMH selon l'une quelconque des revendications 1 à 18.

20. Composition selon la revendication 19, dans laquelle la construction de molécules du CMH comprend des molécules du CMH remplies de peptides ou des molécules du CMH dépourvues de peptide ; et dans laquelle lesdits peptides pour remplir les molécules du CMH dépourvues de peptide, et la construction de molécules du CMH comprenant des molécules du CMH dépourvues de peptide sont fournis séparément.

21. Composition selon la revendication 19, comprenant en outre une ou plusieurs molécules biologiquement actives et/ou un ou plusieurs excipients.

22. Composition selon l'une quelconque des revendications 19 à 21, pour une utilisation dans le traitement, la prévention, la stabilisation, ou le soulagement de maladies impliquant des cellules reconnaissant le CMH.

23. Composition selon la revendication 22, où la maladie est d'origine inflammatoire, auto-immune, allergique, virale, cancéreuse, infectieuse, allogénique, xénogénique, greffon contre l'hôte, ou hôte contre le greffon, une maladie intestinale inflammatoire chronique, la maladie de Crohn, la rectocolite hémorragique, la sclérose, le diabète de type I, la polyarthrite rhumatoïde, le psoriasis, la dermite atopique, l'asthme, le mélanome malin, le carcinome rénal, le cancer du sein, le cancer du poumon, le cancer de l'utérus, le cancer prostatique, le cancer du cerveau, le cancer de la tête et du cou, la leucémie, le lymphome cutané, le carcinome hépatique, le cancer colorectal, le cancer de la vessie, une maladie associée à un rejet, une maladie de type greffon contre l'hôte, une maladie virale associée à une hépatite, le SIDA, la rougeole, la variole, la varicelle, la rubéole ou l'herpès.

24. Composition selon l'une quelconque des revendications 19 à 23, formulée pour une administration parentérale, y compris une administration intraveineuse, intramusculaire, sous-cutanée, intradermique, épicutanée/transdermique, et intrapéritonéale, et pour une perfusion.

25. Utilisation de la composition selon l'une quelconque des revendications 19 à 24 pour la fabrication d'un médicament destiné à la régulation positive, la régulation négative ou la modulation d'une réponse immunitaire chez un animal, pour l'induction de l'anergie d'une cellule ou pour une immunothérapie cellulaire adoptive.

26. Procédé d'obtention de cellules reconnaissant le CMH comprenant la mise en contact d'une construction de molécules du CMH selon l'une quelconque des revendications 1 à 18 et d'un échantillon suspecté de comprendre des cellules reconnaissant le CMH dans des conditions grâce auxquelles les cellules reconnaissant le CMH se lient à la construction de molécules du CMH, et l'isolement de la construction de molécules du CMH et des cellules reconnaissant le CMH liées.

27. Procédé selon la revendication 26, dans lequel l'isolement est réalisé en appliquant un champ magnétique ou par cytométrie en flux.

28. Procédé de production d'une composition selon l'une quelconque des revendications 19 à 24, comprenant la fourniture d'une construction de molécules du CMH telle que définie dans les revendications 1 à 18, la solubilisation ou la dispersion de la construction de molécules du CMH dans un milieu approprié pour des substances thérapeutiques, et éventuellement l'addition d'autres excipients.

29. Procédé de détection ou de contrôle de la présence de cellules reconnaissant le CMH dans un échantillon comprenant les étapes suivantes
(a) la mise en contact d'un échantillon suspecté de comprendre des cellules reconnaissant le CMH avec une construction de molécules du CMH selon l'une quelconque des revendications 1 à 18, et
(b) la détermination de toute liaison de la construction de molécules du CMH, laquelle liaison indique la présence de cellules reconnaissant le CMH.

30. Procédé d'établissement d'un pronostic d'une maladie, de détermination de l'état d'une maladie ou de diagnostic d'une maladie impliquant des cellules reconnaissant le CMH comprenant les étapes suivantes
(a) la mise en contact d'un échantillon suspecté de comprendre des cellules reconnaissant le CMH avec une construction de molécules du CMH selon les revendications 1 à 18, et
(b) la détermination de toute liaison de la construction de molécules du CMH, établissant de cette façon un pronostic d'une maladie, la détermination de l'état d'une maladie ou le diagnostic d'une maladie impliquant des cellules reconnaissant le CMH.

31. Procédé de détermination de l'efficacité d'un médicament contre une maladie impliquant des cellules reconnaissant le CMH, comprenant les étapes suivantes
(a) la mise en contact d'un échantillon provenant d'un sujet recevant un traitement avec un médicament avec une construction de molécules du CMH selon les revendications 1 à 18, et
(b) la détermination de toute liaison de la construction de molécules du CMH, déterminant de cette façon l'efficacité dudit médicament.

32. Procédé selon l'une quelconque des revendications 29 à 31, dans lequel les cellules reconnaissant le CMH sont impliquées dans une maladie d'origine inflammatoire, auto-immune, allergique, virale, cancéreuse, infectieuse, allogénique, xénogénique, greffon contre l'hôte, ou hôte contre le greffon, une maladie intestinale inflammatoire chronique, la maladie de Crohn, la rectocolite hémorragique, la sclérose, le diabète de type I, la polyarthrite rhumatoïde, le psoriasis, la dermite atopique, l'asthme, le mélanome malin, le carcinome rénal, le cancer du sein, le cancer du poumon, le cancer de l'utérus, le cancer prostatique, le cancer du cerveau, le cancer de la tête et du cou, la leucémie, le lymphome cutané, le carcinome hépatique, le cancer colorectal, le cancer de la vessie, une maladie associée à un rejet, une maladie de type greffon contre l'hôte, une maladie virale associée à une hépatite, le SIDA, la rougeole, la variole, la varicelle, la rubéole ou l'herpès.

33. Procédé selon les revendications 29 à 32, dans lequel les cellules reconnaissant le CMH sont choisies parmi des sous-populations de lymphocytes T CD3+, lymphocytes T gamma, delta, lymphocytes T alpha, bêta, lymphocytes T CD4+, lymphocytes T auxiliaires, lymphocytes T CD8+, lymphocytes T suppresseurs, lymphocytes T cytotoxiques CD8+, LTC, cellules NK, cellules NKT, cellules LAL, et MAK.

34. Procédé selon l'une quelconque des revendications 29 à 33, dans lequel l'échantillon est choisi dans le groupe constitué de matériau histologique, matériau cytologique, tumeurs primaires, métastases d'organes secondaires, aspirats par aiguille fine, tissu splénique, prélèvements de moelle osseuse, frottis cellulaires, prélèvements cytologiques par exfoliation, préparations d'empreinte, écouvillons buccaux, écouvillons laryngés, écouvillons vaginaux, lavage bronchique, lavage gastrique, un échantillon du cordon ombilical, un échantillon de liquide corporel, un échantillon sanguin, un échantillon d'une population de cellules mononucléées du sang périphérique isolée du sang, un échantillon d'une préparation dérivée du sang, un échantillon d'un produit de leucophérèse, des échantillons de crachats, des expectorats, des aspirats bronchiques, des biopsies et des coupes de biopsies.

35. Procédé selon l'une quelconque des revendications 29 à 34, dans lequel la détermination de la liaison est réalisée par inspection au microscope, par transmission de lumière, de fluorescence, électronique, ou par cytométrie en flux.

36. Procédé selon l'une quelconque des revendications 29 à 35, dans lequel l'échantillon est monté sur un support, un support solide ou semi-solide ou des lames de verre, des plaques de microtitrage comportant un ou plusieurs puits, des billes, des particules, des membranes, des filtres, des membranes à filtre, des lames en polymère, des membranes en polymère, des lames à chambre, des boîtes, et des boîtes de Petri.

37. Procédé de fabrication de la composition selon la revendication 19, comprenant les étapes suivantes
(i) la fourniture d'au moins 5 molécules du CMH ;
(ii) la fourniture d'une molécule de support de dextrane ;
(iii) la fourniture de plus d'une entité de liaison ;
(iv) la fixation desdites au moins 5 molécules du CMH audit support de dextrane par l'intermédiaire de ladite plus d'une entité de liaison ; où la fixation entraîne la fixation de chaque entité de liaison à 2 à 4 molécules du CMH ;
(v) la fixation éventuelle d'une ou de plusieurs molécules biologiquement actives audit support de dextrane soit directement soit par l'intermédiaire de ladite plus d'une entité de liaison ;
(vi) la fixation éventuelle d'un ou de plusieurs composés de marquage à ladite molécule de support de dextrane, ou à une ou plusieurs de ladite plus d'une entité de liaison ou à une ou plusieurs desdites au moins 5 molécules du CMH ;
et la fabrication de cette façon de la composition selon la revendication 19.

38. Procédé selon la revendication 26, les cellules reconnaissant le CMH sont obtenues par la mise en contact d'un échantillon provenant d'un sujet comprenant des cellules reconnaissant le CMH avec une construction de molécules du CMH selon l'une quelconque des revendications 1 à 18, grâce à quoi les cellules reconnaissant le CMH se retrouvent liées à la construction de molécules du CMH, l'isolement de la construction de molécules du CMH et des cellules reconnaissant le CMH liées, et éventuellement l'expansion ex *vivo* de ces cellules reconnaissant le CMH jusqu'à un nombre cliniquement pertinent et où les cellules reconnaissant le CMH isolées sont libérées de la construction de molécules du CMH avant l'expansion.

39. Procédé selon l'une quelconque des revendications 26 à 38, dans lequel la construction de molécules du CMH est immobilisée sur le support solide ou semi-solide avant ou après la mise en contact avec l'échantillon.

40. Procédé selon l'une quelconque des revendications 38 à 39, dans lequel l'expansion est réalisée en présence d'une ou de plusieurs constructions de molécules du CMH, éventuellement d'une ou de plusieurs molécules biologiquement actives et éventuellement de cellules nourricières comme des cellules dendritiques ou des cellules nourricières.
